(19) Europäisches Patentamt
European Patent Office
Office européen des brevets

(11) Veröffentlichungsnummer: **0 431 270 A1**

# (12) EUROPÄISCHE PATENTANMELDUNG

(21) Anmeldenummer: **90118519.9**

(22) Anmeldetag: **27.09.90**

(51) Int. Cl.5: **C07D 403/12**, C07D 251/46, A01N 47/44, C07D 409/12, C07D 239/47, C07D 239/52, C07D 251/16, C07D 239/42, C07D 405/12, C07D 401/12

(30) Priorität: **10.10.89 DE 3933792**
**31.05.90 DE 4017460**

(43) Veröffentlichungstag der Anmeldung:
**12.06.91 Patentblatt 91/24**

(84) Benannte Vertragsstaaten:
**BE CH DE ES FR GB IT LI NL**

(71) Anmelder: **BAYER AG**

**W-5090 Leverkusen 1 Bayerwerk(DE)**

(72) Erfinder: **Fest, Christa, Dr.**
**Im Johannistal 20**
**W-5600 Wuppertal 1(DE)**
Erfinder: **Gesing, Ernst R.F., Dr.**
**Trillser Graben 4**
**W-4006 Erkrath-Hochdahl(DE)**
Erfinder: **Kirsten, Rolf, Dr.**
**Carl-Langhans-Strasse 27**
**W-4019 Monheim(DE)**
Erfinder: **Kluth, Joachim, Dr.**
**Tannenweg 9**
**W-4018 Langenfeld(DE)**
Erfinder: **Lantzsch, Reinhard, Dr.**
**Am Buschhäuschen 51**
**W-5600 Wuppertal 1(DE)**
Erfinder: **Müller, Klaus-Helmut, Dr.**
**Bockhackstrasse 55**
**W-4000 Düsseldorf 13(DE)**
Erfinder: **Pfister, Theodor, Dr.**
**Lichtenberger Strasse 30**
**W-4019 Monheim(DE)**
Erfinder: **Riebel, Hans-Jochem, Dr.**
**In der Beek 92**
**W-5600 Wuppertal 1(DE)**
Erfinder: **Rosenfeldt, Frank, Dr.**
**Zum Stadion 70**
**W-4018 Langenfeld(DE)**
Erfinder: **Santel, Hans-Joachim, Dr.**
**Gruenstrasse 9a**
**W-5090 Leverkusen 1(DE)**
Erfinder: **Lürssen, Klaus, Dr.**
**August-Kierspel-Strasse 145**
**W-5060 Bergisch-Gladbach 2(DE)**
Erfinder: **Schmidt, Robert R., Dr.**
**Im Waldwinkel 110**
**W-5060 Bergisch-Gladbach 2(DE)**

(54) **Substituierte Sulfonylamidinohydrazone.**

(57) Die Erfindung betrifft neue substituierte Sulfonylamidinohydrazone der allgemeinen Formel (I),

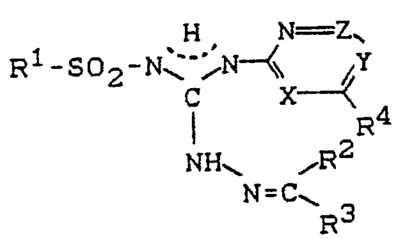

(I)

in welcher

EP 0 431 270 A1

$R^1$ für jeweils gegebenenfalls substituiertes Aryl, Aralkyl oder Heteroaryl steht,
X für Stickstoff oder eine -CH-Gruppierung steht,
Y für Stickstoff oder eine $-CR^5$-Gruppierung steht,
Z für Stickstoff oder eine $-CR^6$-Gruppierung steht,
(wobei die Substituenten $R^2$, $R^3$, $R^4$, $R^5$ und $R^6$ die in der Beschreibung angegebenen Bedeutungen haben), Verfahren zu ihrer Herstellung und ihre Verwendung als Herbizide.

## SUBSTITUIERTE SULFONYLAMIDINOHYDRAZONE

Die Erfindung betrifft neue substituierte Sulfonylamidinohydrazone, Verfahren zu ihrer Herstellung und ihre Verwendung als Herbizide.

Es ist bereits bekannt, das bestimmte substituierte Aminoguanidinoazine, wie z. B. N′-(4,6-Dimethyl-pyrimidin-2-yl)-N″-acetylamino-N‴-(2-chlor-phenylsulfonyl)-guanidin, herbizide Eigenschaften aufweisen (vgl. EP-A 121 082). Die herbizide Wirkung der bisher bekannten Aminoguanidinoazine ist jedoch nicht immer ganz zufriedenstellend.

Es wurden nun neue substituierte Sulfonylamidinohydrazone der allgemeinen Formel (I)

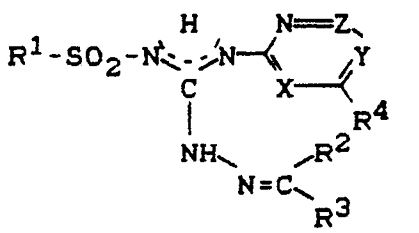

(I)

in welcher

$R^1$ für jeweils gegebenenfalls substituiertes Aryl, Aralkyl oder Heteroaryl steht,

$R^2$ für Wasserstoff oder für jeweils gegebenenfalls substituiertes Alkyl, Aryl oder Aralkyl steht,

$R^3$ für Wasserstoff oder für jeweils gegebenenfalls substituiertes Alkyl, Alkenyl, Alkadienyl, Alkinyl, (Hetero)-Aryl, Aralkyl, Aralkenyl, Alkoxy, Alkoxycarbonyl oder Dialkylamino steht, oder zusammen mit $R^2$ für gegebenenfalls substituiertes Alkandiyl steht,

$R^4$ für Wasserstoff, Halogen, Hydroxy, Alkyl, Halogenalkyl, Alkoxyalkyl, Alkoxy, Halogenalkoxy, Alkylthio, Halogenalkylthio, Amino, Alkylamino oder Dialkylamino steht,

X für Stickstoff oder eine -CH-Gruppierung steht,

Y für Stickstoff oder eine -$CR^5$-Gruppierung steht, worin

$R^5$ für Wasserstoff, Halogen, Cyano, Alkyl, Formyl, Alkyl-carbonyl oder Alkoxy-carbonyl steht, und

Z für Stickstoff oder eine -$CR^6$-Gruppierung steht, worin

$R^6$ für Wasserstoff, Halogen, Hydroxy, Alkyl, Halogenalkyl, Alkoxy, Halogenalkoxy, Alkylthio, Alkylamino oder Dialkylamino steht,

gefunden.

Die allgemeine Formel (I) steht für die einzelnen möglichen Tautomeren der Formeln (IA), (IB) und (IC)

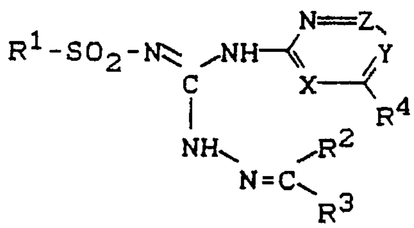

(IA)

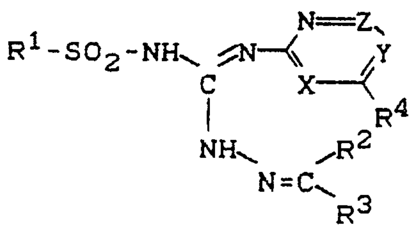

(IB)

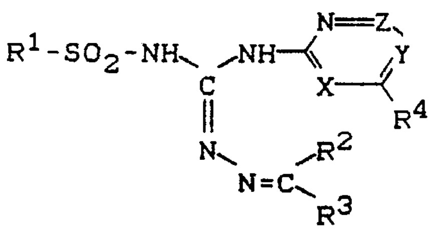

(IC)

und für die Gemische dieser Tautomeren sowie für die E-und Z-Isomeren, welche durch die jeweils vorhandenen C-N-Doppelbindungen bedingt sind, und deren Gemische.

Man erhält die neuen substituierten Sulfonylamidinohydrazone der allgemeinen Formel (I), wenn man

(a) Aminoguanidine der allgemeinen Formel (II)

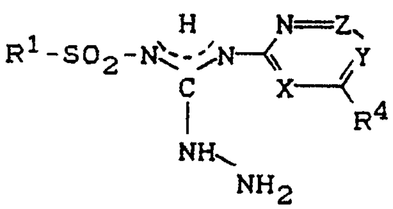

(II)

in welcher

$R^1$, $R^4$, X, Y und Z die oben angegebenen Bedeutungen haben,

mit Carbonylverbindungen der allgemeinen Formel (III)

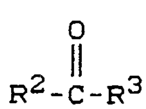

(III)

in welcher

$R^2$ und $R^3$ die oben angegebenen Bedeutungen haben, oder mit N,N-Dialkyl-carbonsäureamid-acetalen bzw. -ketalen

gegebenenfalls in Gegenwart eines Kondensations hilfsmittels und gegebenenfalls in Gegenwart eines Verdünnungsmittels umsetzt, oder wenn man

(b) Sulfonylverbindungen der allgemeinen Formel (IV)

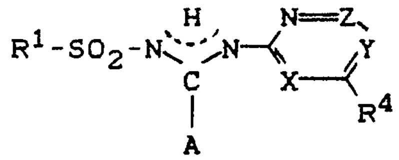

(IV)

in welcher
$R^1$, $R^4$, X, Y und Z die oben angegebenen Bedeutungen haben und
A für Halogen oder eine der nachstehend angegebenen Abgangsgruppen

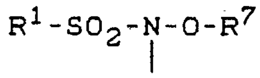 oder $-Q-R^8$ steht,

worin
$R^1$ die oben angegebene Bedeutung hat,
$R^7$ für Alkyl, Alkenyl oder Aralkyl steht,
$R^8$ für jeweils gegebenenfalls substituiertes Alkyl, Aralkyl oder Aryl steht und
Q für Sauerstoff oder Schwefel steht,
mit Hydrazonen der allgemeinen Formel (V)

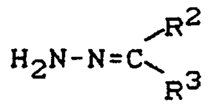

(V)

in welcher
$R^2$ und $R^3$ die oben angegebenen Bedeutungen haben,
gegebenenfalls in Gegenwart eines Verdünnungsmittels umsetzt.

Die neuen substituierten Sulfonylamidinohydrazone der allgemeinen Formel (I) zeichnen sich durch starke herbizide Wirksamkeit aus.

Überraschenderweise zeigen die neuen Verbindungen der allgemeinen Formel (I) erheblich stärkere herbizide Wirkung als vorbekannte Verbindungen ähnlicher Struktur und gleichartiger Wirkungsrichtung, wie z.B. die Verbindung N'-(4,6-Dimethylpyrimidin-2-yl)-N''-acetylamino-N'''-(2-chlor-phenylsulfonyl)-guanidin (vgl. EP-A 121082).

Gegenstand der vorliegenden Erfindung sind vorzugsweise Verbindungen der Formel (I), in welcher
$R^1$ für den Rest

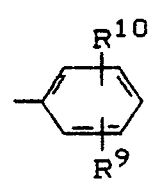

steht, worin
$R^9$ und $R^{10}$ gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di($C_1$-$C_4$-alkyl)-amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylaminocarbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsul-

finyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxy-carbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substitu iert ist), $C_3$-$C_6$-Alkinyloxy, $C_3$-$C_6$-Alkinylthio oder für den Rest $-S(O)_p-R^{11}$ stehen, wobei

p für die Zahlen 1 oder 2 steht und

$R^{11}$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, Phenyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^{12}$ steht, wobei

$R^{12}$ für $C_1$-$C_{12}$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxycarbonyl substituiert ist) steht,

$R^9$ und $R^{10}$ weiterhin für Phenyl oder Phenoxy, für Amino, $C_1$-$C_4$-Alkylcarbonylamino, $C_1$-$C_4$-Alkoxy-carbonylamino, $C_1$-$C_4$-Alkylamino-carbonyl-amino, Di-($C_1$-$C_4$-alkyl)-amino-carbonylamino, oder für den Rest $-CO-R^{13}$ stehen, wobei

$R^{13}$ für $C_1$-$C_6$-Alkyl, $C_1$-$C_6$-Alkoxy, (welches gegebenenfalls durch Fluor, Chlor, Methoxy oder Ethoxy substituiert ist), für $C_3$-$C_6$-Cycloalkoxy, $C_3$-$C_6$-Alkenyloxy, $C_1$-$C_4$Alkylthio, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^9$ und $R^{10}$ weiterhin für $C_1$-$C_4$-Alkylsulfonyloxy, Di-($C_1$-$C_4$-alkyl)-aminosulfonylamino, Thiazolyloxy oder für den Rest $-CH=N-R^{14}$ stehen, wobei

$R^{14}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsul finyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonyl-amino, $C_1$-$C_4$-Alkylsulfonylamino oder für gegebenenfalls durch Fluor, Chlor, Brom oder Methyl substituiertes Phenylsulfonylamino steht;

in welcher weiter

$R^1$ für den Rest

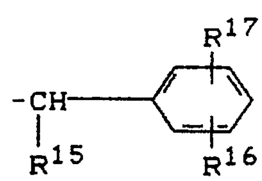

steht, worin

$R^{15}$ für Wasserstoff oder $C_1$-$C_4$-Alkyl steht,

$R^{16}$ und $R^{17}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Carboxy, $C_1$-$C_4$-Alkoxycarbonyl, $C_1$-$C_4$-Alkylsulfonyl oder Di-($C_1$-$C_4$-alkyl)-aminosulfonyl stehen;

in welcher weiter

$R^1$ für den Rest

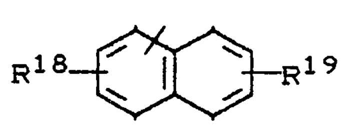

steht, worin
$R^{18}$ und $R^{19}$ gleich oder verschieden sind und für Wasserstoff, Fluor, Chlor, Brom, Nitro, Cyano, Dimethylamino, $C_1$-$C_4$-Alkyl (welches gegebenenfalls durch Fluor und/oder Chlor substituiert ist) oder $C_1$-$C_4$-Alkoxy (welches gegebenenfalls durch Fluor und) oder Chlor substituiert ist), stehen;
in welcher weiter
$R^1$ für den Rest

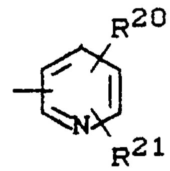

steht, worin
$R^{20}$ und $R^{21}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/ oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxycarbonyl, Dimethylaminocarbonyl oder Dioxolanyl stehen;
in welcher weiter
$R^1$ für den Rest

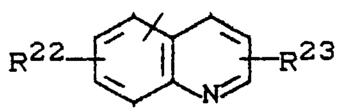

steht, worin
$R^{22}$ und $R^{23}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;
in welcher weiter
$R^1$ für den Rest

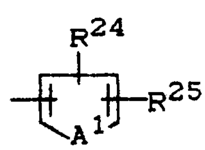

steht, worin
$R^{24}$ und $R^{25}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), Di-($C_1$-$C_4$-alkyl)-amino-sulfonyl, $C_1$-$C_4$-Alkoxy-carbonyl, Dioxolanyl oder 2-Thiazolyl stehen, und
$A^1$ für Sauerstoff, Schwefel oder die Gruppierung N-$Z^1$ steht, wobei
$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;
in welcher weiter
$R^1$ für den Rest

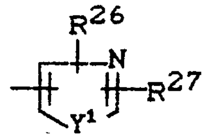

steht, worin
$R^{26}$ für Wasserstoff, $C_1$-$C_5$-Alkyl oder Halogen steht,
$R^{27}$ für Wasserstoff oder $C_1$-$C_5$-Alkyl steht und
$Y^1$ für Sauerstoff, Schwefel oder die Gruppierung N-$R^{28}$ steht, wobei
$R^{28}$ für Wasserstoff oder $C_1$-$C_5$-Alkyl steht;
in welcher weiter
$R^1$ für den Rest

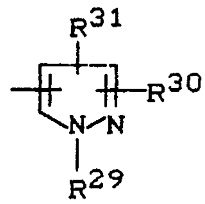

steht, worin
$R^{29}$ für Wasserstoff, $C_1$-$C_4$-Alkyl, Phenyl oder (Iso)Chinolinyl steht,
$R^{30}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und
$R^{31}$ für Wasserstoff, Halogen oder $C_1$-$C_4$-Alkyl steht;
in welcher weiter
$R^1$ für den Rest

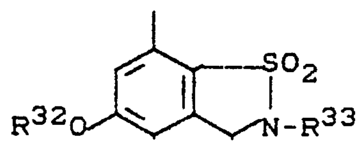

steht, worin
$R^{32}$ für $C_1$-$C_3$-Alkyl steht und
$R^{33}$ für $C_1$-$C_4$-Alkyl steht,
in welcher weiter
$R^1$ für den Rest

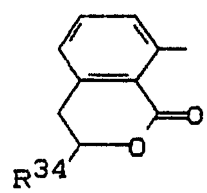

steht,
worin
$R^{34}$ für Wasserstoff oder Methyl steht;
in welcher weiter

$R^1$ für den Rest

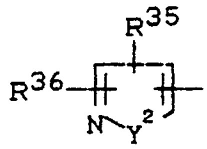

steht, worin
$R^{35}$ für Wasserstoff, Halogen oder $C_1$-$C_4$-Alkyl steht,
$R^{36}$ für Wasserstoff, Halogen, $C_1$-$C_4$-Alkyl, $C_1$-$C_4$-Halogenalkyl, $C_1$-$C_4$-Alkoxy oder $C_1$-$C_4$-Halogenalkoxy steht, und
$Y^2$ für Sauerstoff oder Schwefel steht,
in welcher weiter
$R^1$ für Pentamethylphenyl steht,
in welcher weiter
$R^2$ für Wasserstoff, für gegebenenfalls durch Fluor, Chlor und/oder Brom substituiertes $C_1$-$C_6$-Alkyl oder für jeweils gegebenenfalls durch Fluor, Chlor, Brom, $C_1$-$C_4$-Alkyl und/oder $C_1$-$C_4$-Alkoxy substituiertes Phenyl oder Benzyl steht,
$R^3$ für Wasserstoff, für jeweils gegebenenfalls durch Fluor, Chlor und/oder Brom substituiertes $C_1$-$C_{20}$-Alkyl, $C_2$-$C_{10}$-Alkenyl, $C_2$-$C_{10}$-Alkinyl oder $C_4$-$C_{10}$-Alkadienyl, für $C_1$-$C_4$-Alkoxy-$C_1$-$C_4$-alkyl, für $C_1$-$C_4$-Alkylthio-$C_1$-$C_4$-alkyl, für gegebenenfalls durch Fluor, Chlor, Brom, Carboxy, Cyano, Nitro, Amino, $C_1$-$C_4$-Alkyl (welches gegebenenfalls durch Fluor und/oder Chlor substituiert ist), $C_1$-$C_4$-Alkoxy (welches gegebenenfalls durch Fluor und/ oder Chlor substituiert ist), $C_1$-$C_4$-Alkylthio (welches gegebenenfalls durch Fluor und/oder Chlor substituiert ist), Di-($C_1$-$C_4$-alkyl)-amino, $C_1$-$C_4$-Alkoxy-carbonyl und/oder Phenoxy (welches gegebenenfalls durch Fluor, Chlor und/oder Trifluormethyl substituiert ist) substituiertes Phenyl, für Naphthyl, für jeweils gegebenenfalls durch Cyano, Nitro, Fluor, Chlor, Brom, $C_1$-$C_4$-Alkyl und/oder $C_1$-$C_4$-Alkoxy substituiertes Pyridyl, Pyrrolyl, Furyl, Thiazolyl oder Thienyl, für Dithienyl, für jeweils gegebenenfalls durch Fluor, Chlor, Brom, $C_1$-$C_4$-Alkyl und/oder $C_1$-$C_4$-Alkoxy substituiertes Phenyl-$C_1$-$C_2$-alkyl oder Phenylethenyl, für $C_1$-$C_6$-Alkoxy, $C_1$-$C_6$-Alkoxy-carbonyl oder Di-($C_1$-$C_4$-alkyl)-amino steht, oder zusammen mit $R^2$ für gegebenenfalls durch Fluor, Chlor, Brom, $C_1$-$C_4$-Alkyl und/oder $C_1$-$C_4$-Alkoxycarbonyl substituiertes $C_2$-$C_6$-Alkandiyl steht, $R^4$ für Wasserstoff, Fluor, Chlor, Brom, $C_1$-$C_4$-Alkyl, $C_1$-$C_4$-Halogenalkyl, $C_1$-$C_2$-Alkoxy-$C_1$-$C_2$-alkyl, Bis-($C_1$-$C_2$-Alkoxy)-$C_1$-$C_2$-alkyl, $C_1$- $C_4$-Alkoxy, $C_1$-$C_4$-Halogenalkoxy, $C_1$-$C_4$-Alkylthio, $C_1$-$C_4$-Halogenalkylthio, Amino, $C_1$-$C_4$-Alkylamino, Dimethylamino oder Diethylamino steht,
X für Stickstoff oder eine -CH-Gruppierung steht,
Y für Stickstoff oder eine -$CR^5$-Gruppierung steht, worin
$R^5$ für Wasserstoff, Fluor, Chlor, Brom, Cyano, Methyl, Formyl, Acetyl, Methoxycarbonyl oder Ethoxycarbonyl steht, und
Z für Stickstoff oder eine -$CR^6$-Gruppierung steht, worin
$R^6$ für Wasserstoff, Fluor, Chlor, Brom, $C_1$-$C_4$-Alkyl, Trifluormethyl, $C_1$-$C_4$-Alkoxy, Difluormethoxy, $C_1$-$C_4$-Alkylthio, $C_1$-$C_4$-Alkylamino, Dimethylamino oder Diethylamino steht.

Die Erfindung betrifft insbesondere Verbindungen der Formel (I), in welcher
$R^1$ für den Rest

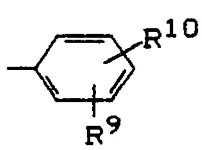

steht, worin
$R^9$ für Fluor, Chlor, Brom, Cyano, Methyl, Trifluormethyl, Methoxy, Ethoxy, Difluormethoxy, Trifluormethoxy, $C_1$-$C_3$-Alkylthio, $C_1$-$C_3$-Alkylsulfinyl, $C_1$-$C_3$-Alkylsulfonyl, Phenylsulfonyl, Dimethylaminosulfonyl, Diethylaminosulfonyl, N-Methoxy-N-methylaminosulfonyl, Phenyl, Phenoxy oder $C_1$-$C_3$-Alkoxy-carbonyl steht und
$R^{10}$ für Wasserstoff, Fluor, Chlor, Brom, Methyl oder Methoxy steht;
in welcher weiter
$R^1$ für den Rest

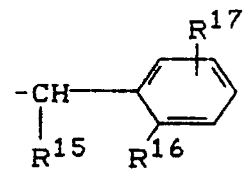

steht, worin
$R^{15}$ für Wasserstoff steht
$R^{16}$ für Fluor, Chlor, Brom, Cyano, Methyl, Methoxy, Ethoxy, Difluormethoxy, Trifluormethoxy, Methoxycarbonyl, Ethoxycarbonyl, Methylsulfonyl oder Dimethylaminosulfonyl steht und
$R^{17}$ für Wasserstoff oder Chlor steht;
in welcher weiter
$R^1$ für den Rest

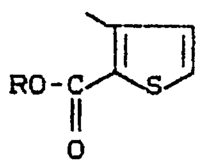

steht, worin
R für $C_1$-$C_2$-Alkyl steht, oder
$R^1$ für den Rest

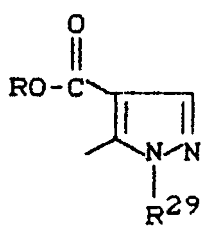

steht, worin
$R^{29}$ für Methyl oder Phenyl steht und
R für $C_1$-$C_2$-Alkyl steht, oder
$R^1$ für Naphthyl steht;
in welcher weiter
$R^2$ für Wasserstoff, $C_1$-$C_4$-Alkyl oder Phenyl steht,
$R^3$ für $C_1$-$C_{10}$-Alkyl, $C_2$-$C_{10}$-Alkenyl oder $C_4$-$C_{10}$-Alkadienyl, für gegebenenfalls durch Fluor, Chlor, Brom, Carboxy, Cyano, Nitro, Methyl, Ethyl, Trifluormethyl, Methoxy, Ethoxy, Difluormethoxy, Trifluormethoxy, Methylthio, Ethylthio, Difluormethylthio, Trifluormethylthio, Dimethylamino und/oder Phenoxy (welches gegebenenfalls durch Fluor, Chlor und/oder Trifluormethyl substituiert ist) substituiertes Phenyl, für jeweils gegebenenfalls durch Cyano, Nitro, Fluor, Chlor, Brom, Methyl, Ethyl, Methoxy oder Ethoxy substituiertes Pyridyl, Furyl, Thiazolyl oder Thienyl, für Dithienyl, für Benzyl oder Phenylethenyl, für $C_1$-$C_4$-Alkoxy, $C_1$-$C_4$-Alkoxycarbonyl oder Dimethylamino, oder zusammen mit $R^2$ für Butan-1,4-diyl (Tetramethylen) oder Pentan-1,5-diyl (Pentamethylen) steht,
$R^4$ für Wasserstoff, Fluor, Chlor, Brom, Methyl, Methoxymethyl, Trifluormethyl, Methoxy, Ethoxy, Difluormethoxy, Methylthio, Ethylthio, Amino, Methylamino, Ethylamino, Dimethylamino oder Diethylamino steht,
X für Stickstoff oder eine -CH-Gruppierung steht,
Y für Stickstoff oder eine $-CR^5$-Gruppierung steht, worin
$R^5$ für Wasserstoff, Fluor, Chlor oder Methyl steht, und
Z für Stickstoff oder eine $-CR^6$-Gruppierung steht, worin
$R^6$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino, Dimethylamino oder Diethylamino steht.

Verwendet man beispielsweise N′-(4,6-Dimethoxypyrimidin-2-yl)-N″-amino-N‴-(2,6-difluorphenylsulfonyl)-guanidin und Aceton als Ausgangsstoffe, so kann der Reaktionsablauf beim erfindungsgemäßen Verfahren (a) durch das folgende Formelschema skizziert werden:

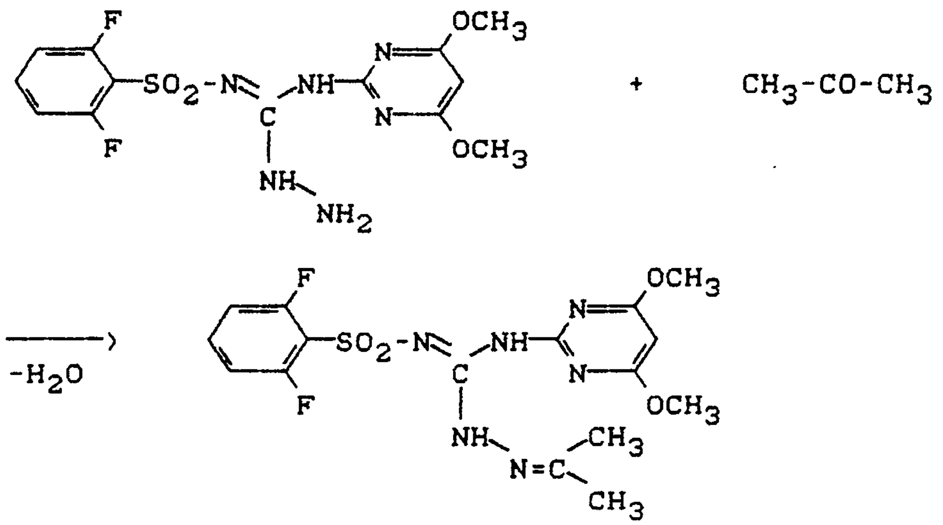


Verwendet man beispielsweise N′-(4-Methoxy-6-methyl-s-triazin-2-yl)-N″-(2-trifluormethyl-phenylsulfonyl)-O-methyl-isoharnstoff und Acetophenon-hydrazon als Ausgangsstoffe, so kann der Reaktionsablauf beim erfindungsgemäßen Verfahren (b) durch das folgende Formelschema skizziert werden:

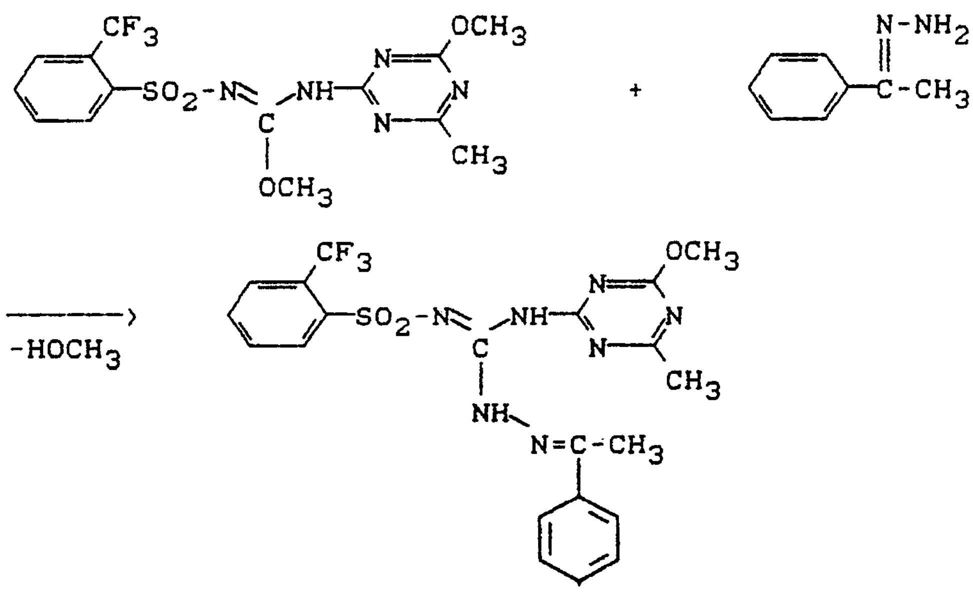


Die beim erfindungsgemäßen Verfahren (a) zur Herstellung von Verbindungen der Formel (I) als Ausgangsstoffe zu verwendenden Aminoguanidine sind durch die Formel (II) allgemein definiert.

In Formel (II) haben $R^1$, $R^4$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^4$, X, Y und Z angegeben wurden.

Beispiele für die Ausgangsstoffe der Formel (II) sind in der nachstehenden Tabelle 1 aufgeführt.

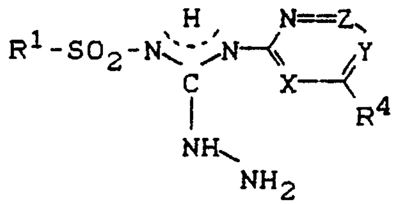

(II)

Tabelle 1: Beispiele für die Verbindungen der Formel (II)

| $R^1$ | $R^4$ | X | Y | Z |
|---|---|---|---|---|
| 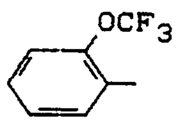 | $OCH_3$ | N | CH | $C-OCH_3$ |
| 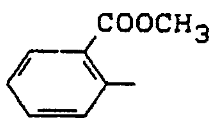 | $OCH_3$ | N | CH | $C-OCH_3$ |
| 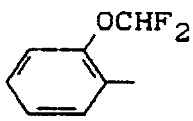 | $CH_3$ | N | CH | $C-CH_3$ |
| 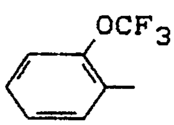 | $CH_3$ | N | N | $C-OCH_3$ |
| 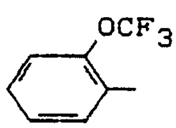 | $OCH_3$ | N | N | $C-OCH_3$ |
| 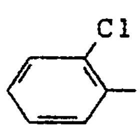 | $CH_3$ | N | CH | $C-OCH_3$ |
| 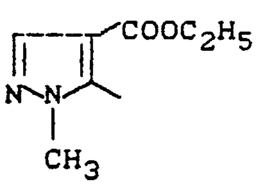 | $CH_3$ | N | CH | $C-CH_3$ |

Tabelle 1 - Fortsetzung

| $R^1$ | $R^4$ | X | Y | Z |
|---|---|---|---|---|
| 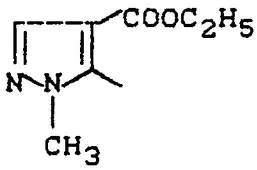 | $CH_3$ | N | N | $C-CH_3$ |
| 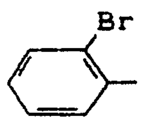 | $CH_3$ | N | CH | $C-CH_3$ |
| 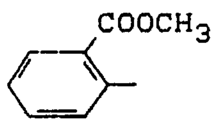 | $CH_3$ | N | N | $C-OCH_3$ |
| 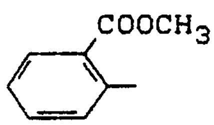 | $OCH_3$ | N | CH | $C-OCH_3$ |
| 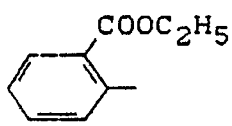 | $CH_3$ | N | CH | $C-OCH_3$ |
| 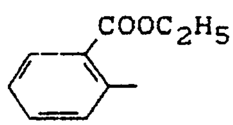 | $OCH_3$ | N | CH | $C-OCH_3$ |
| 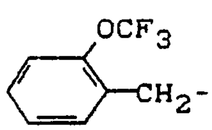 | $OCH_3$ | N | CH | $C-OCH_3$ |
| 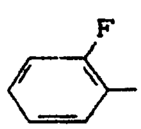 | $OCH_3$ | N | N | $C-OCH_3$ |

Tabelle 1 - Fortsetzung

| $R^1$ | $R^4$ | X | Y | Z |
|---|---|---|---|---|
| 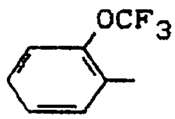 | $C_2H_5$ | N | CH | $C-OCH_3$ |
| 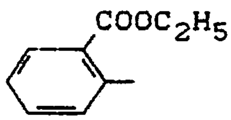 | $CH_3$ | N | N | $C-OCH_3$ |
| 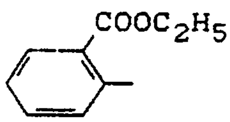 | $OCH_3$ | N | N | $C-OCH_3$ |
| 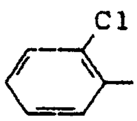 | $CH_3$ | N | N | $C-OCH_3$ |
| 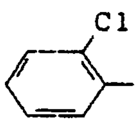 | $OCH_3$ | N | N | $C-OCH_3$ |
| 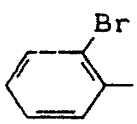 | $OCH_3$ | N | N | $C-OCH_3$ |
| 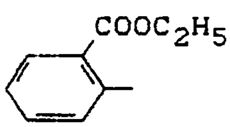 | $CH_3$ | N | CH | $C-CH_3$ |
| 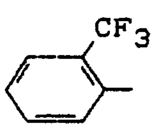 | $CH_3$ | N | CH | $C-CH_3$ |

Tabelle 1 - Fortsetzung

| $R^1$ | $R^4$ | X | Y | Z |
|---|---|---|---|---|
| 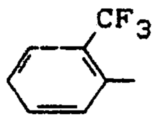 | $OCH_3$ | N | CH | $C-OCH_3$ |
| 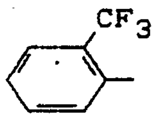 | $CH_3$ | N | N | $C-OCH_3$ |
| 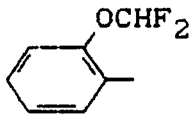 | $OCH_3$ | N | CH | $C-OCH_3$ |
| 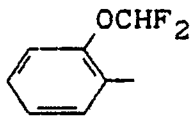 | $OCH_3$ | N | N | $C-OCH_3$ |
| 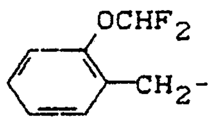 | $OCH_3$ | N | CH | $C-OCH_3$ |
| 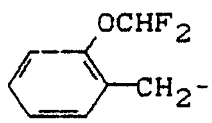 | $OCH_3$ | N | N | $C-OCH_3$ |
| 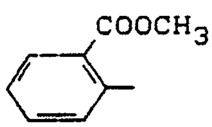 | $C_2H_5$ | N | N | $C-OCH_3$ |
| 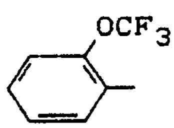 | $CH_3$ | N | CH | $C-OC_2H_5$ |

<u>Tabelle 1</u> - Fortsetzung

| $R^1$ | $R^4$ | X | Y | Z |
|---|---|---|---|---|
| 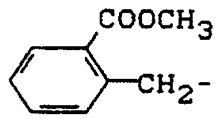 | $OCH_3$ | N | N | $C-OCH_3$ |
| 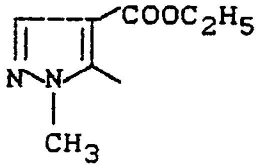 | $OCH_3$ | N | CH | $C-OCH_3$ |
| 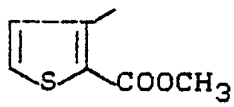 | $CH_3$ | N | N | $C-OCH_3$ |
| 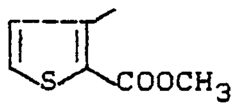 | $OCH_3$ | N | N | $C-OCH_3$ |
| 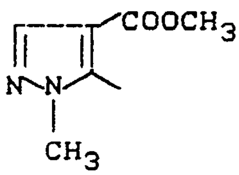 | $OCH_3$ | N | N | $C-OCH_3$ |
| 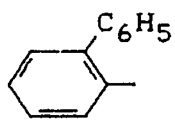 | $OCH_3$ | N | CH | $C-OCH_3$ |
| 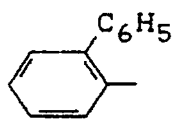 | $OCH_3$ | N | N | $C-OCH_3$ |
| 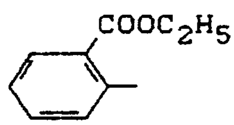 | $OCH_3$ | N | CH | C-Cl |

Tabelle 1 - Fortsetzung

| $R^1$ | $R^4$ | X | Y | Z |
|---|---|---|---|---|
| 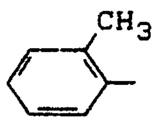 | $CH_3$ | N | N | $C-OCH_3$ |
| 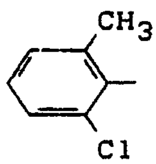 | $CH_3$ | N | CH | $C-CH_3$ |
| 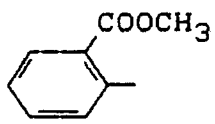 | $CF_3$ | N | CH | $C-OCH_3$ |
| 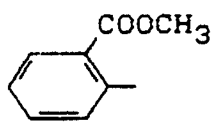 | $CH_3$ | N | CH | $C-OCHF_2$ |
| 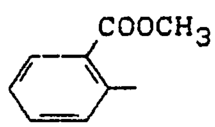 | $OCH_3$ | N | CH | $C-OCHF_2$ |
| 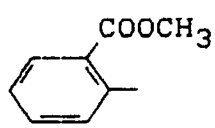 | $OCHF_2$ | N | CH | $C-OCHF_2$ |
| 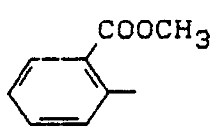 | $NHCH_3$ | N | N | $C-OC_2H_5$ |
| 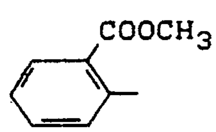 | $NHC_2H_5$ | N | N | $C-OCH_3$ |

Tabelle 1 - Fortsetzung

| $R^1$ | $R^4$ | X | Y | Z |
|---|---|---|---|---|
| 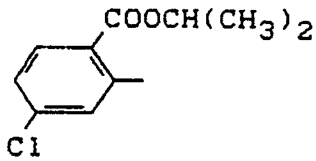 | $CH_3$ | N | N | $C-OCH_3$ |
| 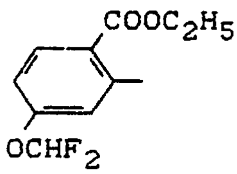 | $OCH_3$ | N | CH | $C-OCH_3$ |
| 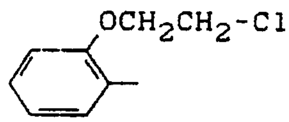 | $CH_3$ | N | N | $C-OCH_3$ |
| 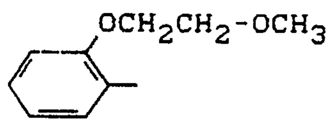 | $OCH_3$ | N | N | $C-OCH_3$ |
| 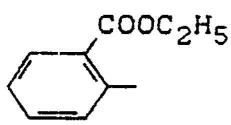 | Cl | N | CH | $C-OCH_3$ |
| 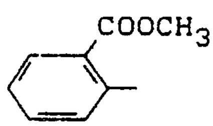 | $CH(OCH_3)_2$ | N | CH | $C-OCH_3$ |
| 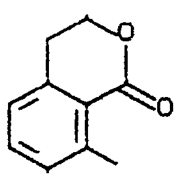 | $NHCH_3$ | N | N | $C-OC_2H_5$ |
| 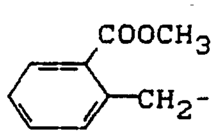 | $OCH_3$ | N | CH | $C-OCH_3$ |

Tabelle 1 - Fortsetzung

| $R^1$ | $R^4$ | X | Y | Z |
|---|---|---|---|---|
| 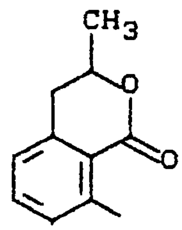 | $NHCH_3$ | N | N | $C-OC_2H_5$ |
| 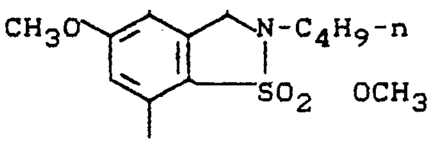 | $OCH_3$ | N | CH | $C-OCH_3$ |
| 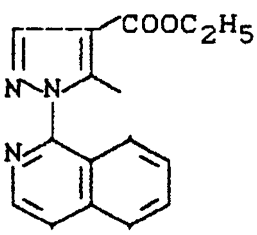 | $OCH_3$ | N | CH | $C-OCH_3$ |
| 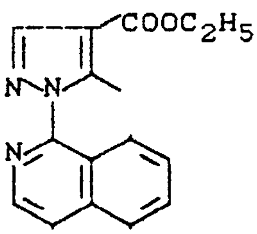 | $OCH_3$ | N | N | $C-OCH_3$ |
| 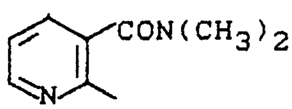 | $OCH_3$ | N | CH | $C-OCH_3$ |
| 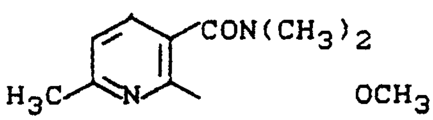 | $OCH_3$ | N | CH | $C-OCH_3$ |
| 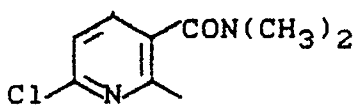 | $OCH_3$ | N | CH | $C-OCH_3$ |

Tabelle 1 - Fortsetzung

| $R^1$ | $R^4$ | X | Y | Z |
|---|---|---|---|---|
| 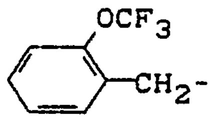 | $OCH_3$ | N | N | $C-OCH_3$ |
| 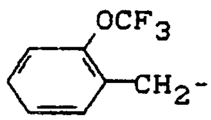 | $CH_3$ | N | N | $C-OCH_3$ |
| 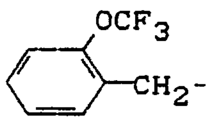 | $CH_3$ | N | N | $C-CH_3$ |
| 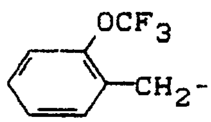 | $NHCH_3$ | N | N | $C-OC_2H_5$ |
| 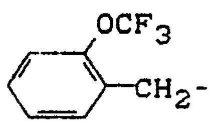 | $C_2H_5$ | N | N | $C-OCH_3$ |
| 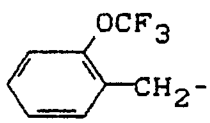 | $CH_3$ | N | N | $C-OC_2H_5$ |
| 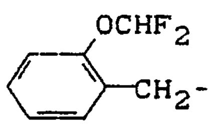 | $CH_3$ | N | N | $C-OCH_3$ |
| 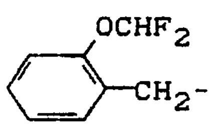 | $CH_3$ | N | N | $C-CH_3$ |

Tabelle 1 - Fortsetzung

| $R^1$ | $R^4$ | X | Y | Z |
| --- | --- | --- | --- | --- |
| 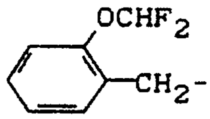 | $NHCH_3$ | N | N | $C-OC_2H_5$ |
| 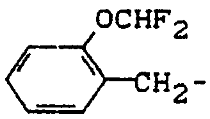 | $C_2H_5$ | N | N | $C-OCH_3$ |
| 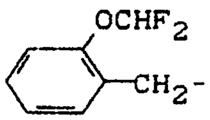 | $CH_3$ | N | N | $C-OC_2H_5$ |
| 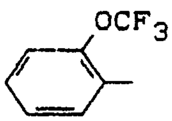 | $CF_3$ | N | CH | $C-OCH_3$ |
| 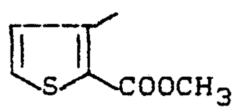 | $OCHF_2$ | N | CH | $C-OCHF_2$ |
| 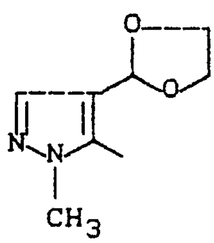 | $OCH_3$ | N | CH | $C-OCH_3$ |
| 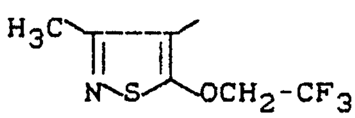 | $OCH_3$ | N | CH | $C-OCH_3$ |
| 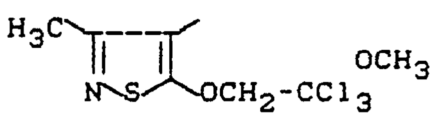 | $OCH_3$ | N | N | $C-OCH_3$ |

Tabelle 1 - Fortsetzung

| $R^1$ | $R^4$ | X | Y | Z |
|---|---|---|---|---|
| 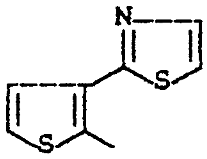 | $OCH_3$ | N | CH | $C-OCH_3$ |
| 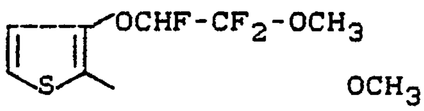 | $OCH_3$ | N | N | $C-OCH_3$ |
| 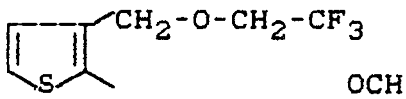 | $OCH_3$ | N | CH | $C-OCH_3$ |
| 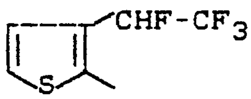 | $OCH_3$ | N | CH | $C-OCH_3$ |
| 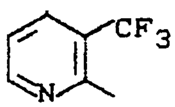 | $OCH_3$ | N | CH | $C-OCH_3$ |
| 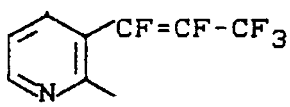 | $OCH_3$ | N | CH | $C-OCH_3$ |
| 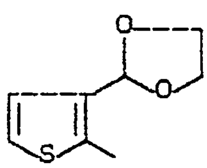 | $OCH_3$ | N | N | $C-OCH_3$ |
| 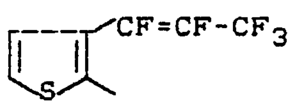 | $OCH_3$ | N | CH | $C-OCH_3$ |
| 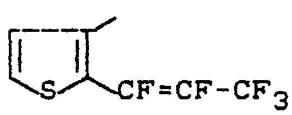 | $OCH_3$ | N | N | $C-OCH_3$ |

Tabelle 1 - Fortsetzung

| $R^1$ | $R^4$ | X | Y | Z |
|---|---|---|---|---|
| 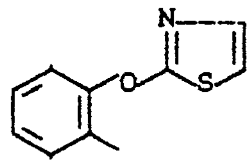 | $OCH_3$ | N | N | $C-OCH_3$ |
| 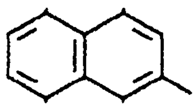 | $CH_3$ | N | N | $C-OCH_3$ |
| 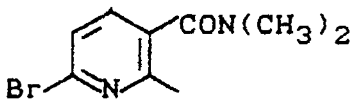 | $OCH_3$ | N | CH | $C-OCH_3$ |
| 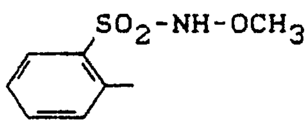 | $OCH_3$ | N | CH | $C-OCH_3$ |
| 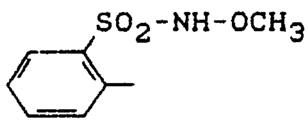 | $OCH_3$ | N | CH | $C-CH_3$ |
| 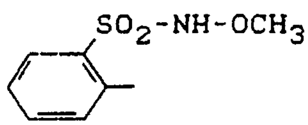 | $OCH_3$ | N | N | $C-OCH_3$ |
| 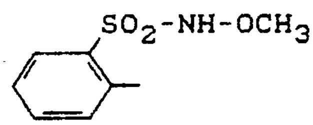 | $CF_3$ | N | CH | $C-OCH_3$ |
| 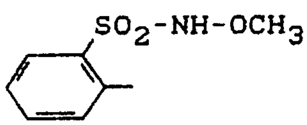 | $CH_3$ | N | CH | $C-CH_3$ |
| 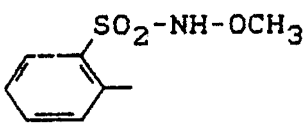 | $OCH_3$ | N | N | $C-CH_3$ |

Die Ausgangsstoffe der Formel (II) sind teilweise bekannt (vgl. EP-A 224 078, US-P 4 725 303) und teilweise als Herbizide Gegenstand einer eigenen älteren, nicht vorveröffentlichten DE-Patentanmeldung (P 38 18 040.5 vom 27.05.88).

Man erhält die Verbindungen der allgemeinen Formel (II), wenn man Sulfonylverbindungen der allgemeinen Formel (IV)

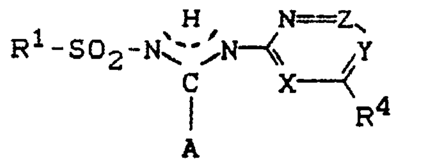

(IV)

in welcher
$R^1$, $R^4$, X, Y und Z die oben angegebenen Bedeutungen haben und
A für Halogen oder eine der nachstehend angegebenen Abgangsgruppen

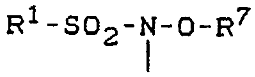 oder $-Q-R^8$ steht,

worin
$R^1$ die oben angegebene Bedeutung hat,
$R^7$ für Alkyl, Alkenyl oder Aralkyl steht,
$R^8$ für jeweils gegebenenfalls substituiertes Alkyl, Aralkyl oder Aryl steht und
Q für Sauerstoff oder Schwefel steht,
mit Hydrazin oder einem Hydrazin-Wasser- oder Hydrazin-Säure-Addukt,
gegebenenfalls in Gegenwart eines Säureakzeptors, wie z.B. Kaliumcarbonat, und gegebenenfalls in Gegenwart eines Verdünnungsmittels, wie z.B. Methanol, Ethanol und/oder Wasser, bei Temperaturen zwischen -20 °C und +100 °C umsetzt.

Ein Teil der Verbindungen der Formel (II) kann auch wie nachstehend skizziert erhalten werden (R wie oben für $R^{12}$ angegeben)

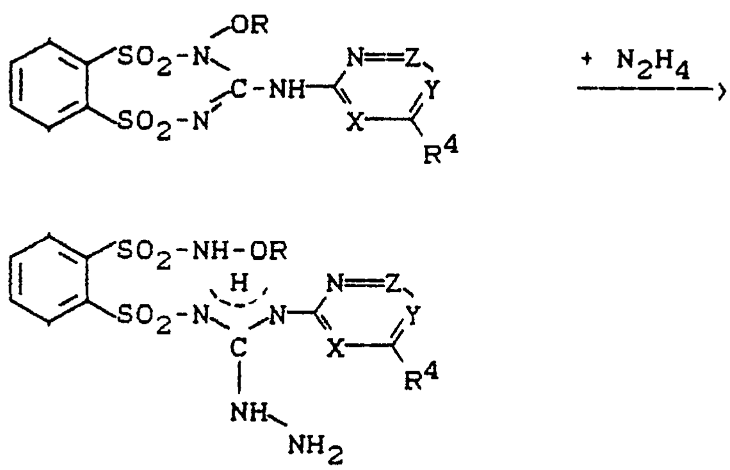


(zum Reaktionsprinzip vgl. US-P 4 659 364, EP-A 173319).

Die als Zwischenprodukte benötigten Sulfonylverbindungen sind durch die Formel (IV) allgemein definiert. In Formel (IV) haben $R^1$, $R^4$, X, Y und Z vorzugsweise bzw. insbesondere diejenigen Bedeutungen, die bereits oben im Rahmen der Beschreibung der erfindungsgemäßen Verbindungen der Formel (I) als bevorzugt bzw. als insbesondere bevorzugt für $R^1$, $R^4$, X, Y und Z angegeben wurden und
A steht vorzugsweise für Chlor oder eine der nachstehend angegebenen Abgangsgruppen

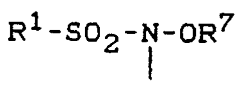 oder $-Q-R^8$,

worin
$R^1$ die oben vorzugsweise angegebene Bedeutung hat,

$R^7$ für $C_1$-$C_4$-Alkyl, $C_3$-$C_4$-Alkenyl oder Benzyl steht,
$R^8$ für gegebenenfalls durch Carboxy, $C_1$-$C_4$-Alkoxy-carbonyl oder $C_1$-$C_4$-Alkoxy substituiertes $C_1$-$C_4$-Alkyl oder für jeweils gegebenenfalls durch Fluor, Chlor, Brom, $C_1$-$C_4$-Alkyl oder $C_1$-$C_4$-Alkoxy substituiertes Benzyl oder Phenyl steht, und
Q für Sauerstoff oder Schwefel steht.

Beispiele für die Verbindungen der Formel (IV) sind in der nachstehenden Tabelle 2 aufgeführt.

<u>Tabelle 2:</u> Beispiele für die Verbindungen der Formel (IV)

| A | $R^1$ | $R^4$ | X | Y | Z |
|---|---|---|---|---|---|
| 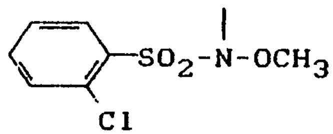 | 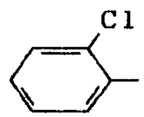 | $CH_3$ | N | CH | $C-OCH_3$ |
| 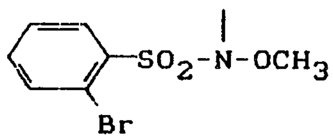 | 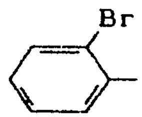 | $OCH_3$ | N | CH | $C-OCH_3$ |
| 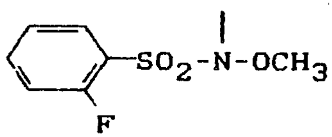 | 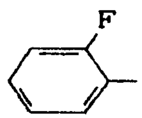 | $OCH_3$ | N | CH | $C-OCH_3$ |
| 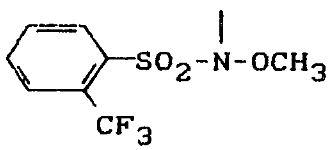 | 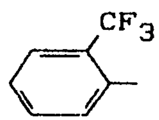 | $OCH_3$ | N | CH | $C-OCH_3$ |
| 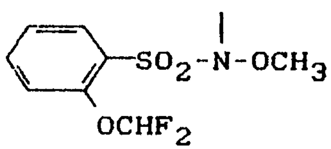 | 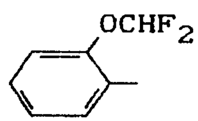 | $OCH_3$ | N | CH | $C-OCH_3$ |

Tabelle 2 - Fortsetzung

| A | $R^1$ | $R^4$ | X | Y | Z |
| --- | --- | --- | --- | --- | --- |
| 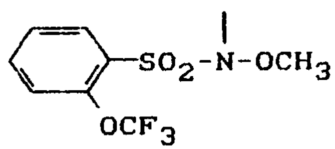 | 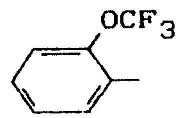 | $OCH_3$ | N | CH | $C-OCH_3$ |
| 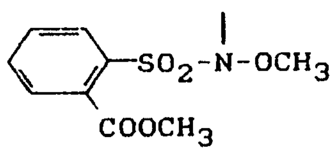 | 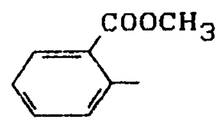 | $CH_3$ | N | N | $C-OCH_3$ |
| 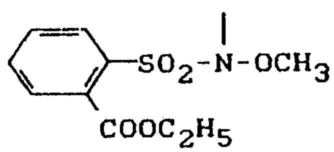 | 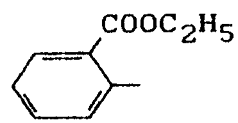 | $CH_3$ | N | CH | $C-CH_3$ |
| 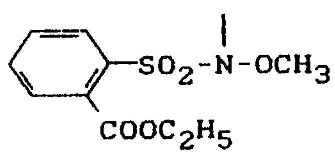 | 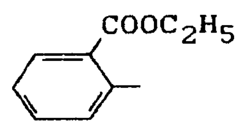 | $OCH_3$ | N | CH | C-Cl |
| 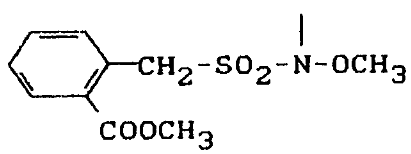 | 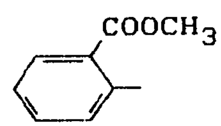 | $OCH_3$ | N | CH | $C-OCH_3$ |

Tabelle 2 - Fortsetzung

| A | $R^1$ | $R^4$ | X | Y | Z |
|---|---|---|---|---|---|
| 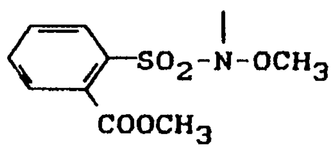 | 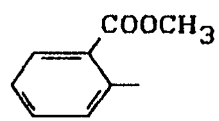 | $OCHF_2$ | N | CH | C-$OCHF_2$ |
| 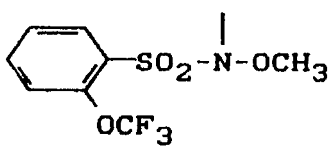 | 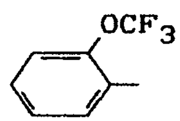 | $OCH_3$ | N | N | C-$OCH_3$ |
| -$OCH_3$ | 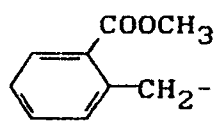 | $OCH_3$ | N | CH | C-$OCH_3$ |
| -$SCH_3$ | 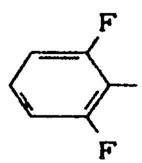 | $CH_3$ | N | N | C-$OCH_3$ |
| -$OC_6H_5$ | 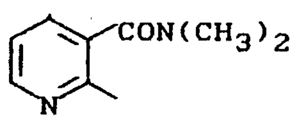 | $OCH_3$ | N | CH | C-$OCH_3$ |

**Tabelle 2** - Fortsetzung

| A | $R^1$ | $R^4$ | X | Y | Z |
|---|---|---|---|---|---|
| $-SC_6H_5$ | 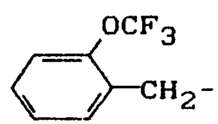 | $CH_3$ | N | CH | $C-OCH_3$ |
| $-SCH_3$ | 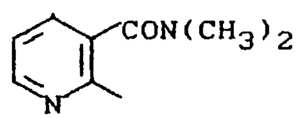 | $OCH_3$ | N | N | $C-OCH_3$ |
| $-OC_6H_5$ | 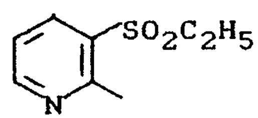 | $OCH_3$ | N | CH | $C-OCH_3$ |

Die Verbindungen der Formel (IV) sind bekannt und/oder können nach an sich bekannten Verfahren hergestellt werden (vgl. EP-A 5 986, EP-A 24 215, EP-A 121 082, EP-A 172 957, EP-A 173 321, EP-A 173 956, EP-A 224 078, DE-OS 36 34 928, DE-OS 36 34 929).

Die beim erfindungsgemäßen Verfahren (a) zur Herstellung von Verbindungen der Formel (I) weiter als Ausgangsstoffe zu verwendenden Carbonylverbindungen sind durch die Formel (III) allgemein definiert.

In Formel (III) haben $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ und $R^3$ angegeben wurden.

Als Beispiele für die Ausgangsstoffe der Formel (III) seien genannt:
Formaldehyd, Acetaldehyd, Propionaldehyd, Butyraldehyd, Isobutyraldehyd, Valeraldehyd, Isovaleraldehyd, Benzaldehyd, Pyridin-4-, -2- und -3-carbaldehyd, Furan-2- und -3-carbaldehyd sowie Thiophen-2- und -3-carbaldehyd, ferner auch Aceton, Methylethylketon, Methylpropylketon, Methylisopropylketon, Methylbutylketon, Methylisobutylketon, Diethylketon, Acetophenon, Benzophenon, Cyclopentanon, Cyclohexanon, Phenylaceton, Chloraceton, Chloral, Glyoxylsäuremethylester und -ethylester, Brenztraubensäuremethylester und -ethylester, Phthalaldehydsäure und 6-Chlor-pyridin-3-aldehyd.

N,N-Dialkyl-carbonsäureamid-acetale bzw. -ketale, welche weiter als Ausgangsstoffe beim erfindungsgemäßen Verfahren (a) eingesetzt werden können, sind vorzugsweise N,N-Dialkyl-formamid- oder -acetamidacetale bzw. -ketale mit an N- bzw. O gebundenen geradkettigen oder verzweigten Alkylresten mit 1 bis 4 Kohlenstoffatomen, wie z.B. N,N-Dimethyl- und N,N-Diethyl-formamid- und -acetamid-dimethylacetal, -diethylacetal, -dipropylacetal, -diisopropylacetal, -dibutylacetal, -diisobutylacetal, -di-sec-butyl-acetal und -ditert-butyl-acetal.

Die Ausgangsstoffe der Formel (III) sind bekannte organische Synthesechemikalien.

Das erfindungsgemäße Verfahren (a) zur Herstellung der neuen Verbindungen der Formel (I) wird gegebenenfalls unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol, Propanol, Isopropanol, Butanol und Isobutanol, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (a) wird gegebenenfalls in Gegenwart eines Kondensationshilfsmittels durchgeführt. Als solche kommen vorzugsweise die in der organischen Chemie üblichen Trockenmittel in Betracht. Hierzu gehören vorzugsweise wasserfreie Salze, wie z.B. Natriumsulfat, Magnesiumsulfat und Kaliumcarbonat.

Die Reaktionstemperaturen können bei dem erfindungsgemäßen Verfahren (a) in einem größeren Bereich variiert werden. Im allgemeinen arbeitet man bei Temperaturen zwischen 0 °C und 100 °C, vorzugsweise bei Temperaturen zwischen 10 °C und 80 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man auf 1 Mol Aminoguanidin der Formel (II) im allgemeinen zwischen 1 und 1000 Mol, vorzugsweise zwischen 1 und 500 Mol Carbonylverbindung der Formel (III) ein.

Die Reaktionskomponenten werden im allgemeinen bei Raumtemperatur oder unter leichtem Kühlen vermischt und gegebenenfalls bei erhöhter Temperatur bis zum Ende der Umsetzung gerührt. Die Aufarbeitung erfolgt nach üblichen Methoden (vgl. die Herstellungsbeispiele).

Die beim erfindungsgemäßen Verfahren (b) zur Herstellung von Verbindungen der Formel (I) als Ausgangsstoffe zu verwendenden Sulfonylverbindungen sind durch die Formel (IV) allgemein definiert. Bevorzugte und besonders bevorzugte Untergruppen der Sulfonylverbindungen (IV) sind bereits oben (vor Tabelle 2) definiert worden. Beispiele für die Ausgangsstoffe der Formel (IV) sind in Tabelle 2 (oben) aufgeführt.

Die beim erfindungsgemäßen Verfahren (b) zur Herstellung von Verbindungen der Formel (I) weiter als Ausgangsstoffe zu verwendenden Hydrazone sind durch die Formel (V) allgemein definiert.

In Formel (V) haben $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$ und $R^3$ angegeben wurden.

Als Beispiele für die Ausgangsstoffe der Formel (V) seien genannt:
Acetaldehyd-, Propionaldehyd-, Butyraldehyd-, Isobutyraldehyd-, Valeraldehyd-, Isovaleraldehyd-, Benzaldehyd-, Pyridin-2-, -3- und -4-aldehyd-, Furan-2-und -3-carbaldehyd-, Thiophen-2- und -3-carbaldehyd-, Aceton-, Methylethylketon-, Methylpropylketon-, Methylisopropylketon-, Methylbutylketon-, Methylisobutylketon-, Diethylketon-, Acetophenon-, Benzophenon-, Cyclopentanon- und Cyclohexanon-hydrazon.

Die Ausgangsstoffe der Formel (V) sind bekannte organische Chemikalien.

Das erfindungsgemäße Verfahren (b) zur Herstellung der neuen Verbindungen der Formel (I) wird vorzugsweise unter Verwendung von Verdünnungsmitteln durchgeführt. Es kommen hierbei die gleichen organischen Lösungsmittel in Betracht, die oben für das erfindungsgemäße Verfahren (a) als Verdünnungsmittel angegeben wurden.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man auf 1 Mol Sulfonylverbindung der Formel (IV) im allgemeinen zwischen 1 und 10 Mol, vorzugsweise zwischen 1 und 5 Mol, Hydrazon der Formel (V) ein.

Die Reaktionskomponenten werden im allgemeinen bei Raumtemperatur zusammengegeben und gegebenenfalls bei erhöhter Temperatur bis zum Ende der Umsetzung gerührt. Die Aufarbeitung erfolgt nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
<u>Dikotyle Unkräuter der Gattungen</u>: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
<u>Dikotyle Kulturen der Gattungen</u>: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
<u>Monokotyle Unkräuter der Gattungen</u>: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
<u>Monokotyle Kulturen der Gattungen</u>: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergeholz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdolfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Link verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 2-{4-[(6-Chlor-2-benzoxazolyl)-oxy]-phenoxy}-propansäure, deren Methyl-oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); O-(6-Chlor-3-phenyl-pyridazin-4-yl)-S-octyl-thiolcarbamat (PYRIDATE); 4-Ethylamino-2-t-butylamino-6-methylthio-s-triazin (TERBUTRYNE); 3-[[[[(4-Methoxy-6-methyl-1,3,5-triazin-2-yl)amino]-carbonyl]-amino]-sulfonyl)-thiophen-2-carbonsäu re-methylester (THIAMETURON); N,N-Diisopropyl-S-(2,3,3-trichlorallyl)-thiolcarbamat (TRIALLATE). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen,

Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

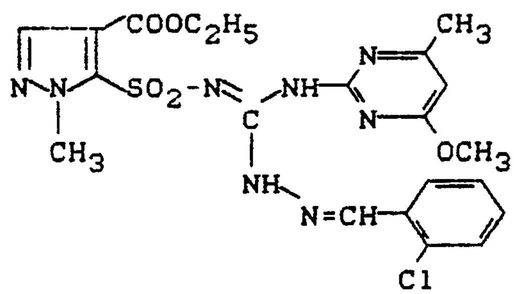

(Verfahren (a))

Eine Mischung aus 8,2 g (0,02 Mol) N′-(4-Methoxy-6-methyl-pyrimidin-2-yl)-N″-amino-N‴-(1-methyl-4-ethoxycarbonyl-pyrazol-5-yl-sulfonyl)-guanidin, 5,6 g (0,04 Mol) 2-Chlor-benzaldehyd und 50 ml Acetonitril wird 20 Stunden bei 20 °C gerührt. Das kristallin angefallene Produkt wird durch Absaugen isoliert.

Man erhält 7,5 g (70% der Theorie) N′-(4-Methoxy-6-methyl-pyrimidin-2-yl)-N″-(2-chlor-benzylidenamino)-N‴-(1-methyl-4-ethoxycarbonyl-pyrazol-5-yl-sulfonyl)-guanidin vom Schmelzpunkt 185 °C.

Beispiel 2

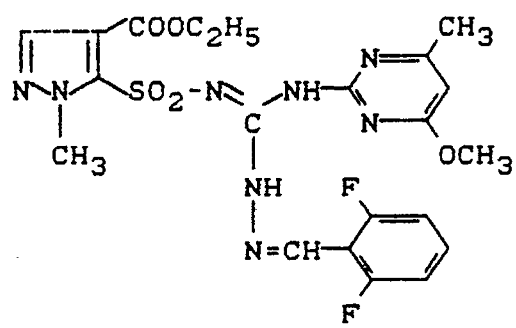

(Verfahren (a))

Eine Mischung aus 8,2 g (0,02 Mol) N′-(4-Methoxy-6-methyl-pyrimidin-2-yl)-N″-amino-N‴-(1-methyl-4-ethoxycarbonyl-pyrazol-5-yl-sulfonyl)-guanidin, 5,7 g (0,04 Mol) 2,6-Difluor-benzaldehyd und 50 ml Ethanol wird 20 Stunden bei 60 °C gerührt. Nach Abkühlen auf 20 °C wird das kristallin angefallene Produkt durch Absaugen isoliert.

Man erhält 9,2 g (86% der Theorie) N′-(4-Methoxy-6-methyl-pyrimidin-2-yl)-N″-(2,6-difluor-benzylidenamino)N‴-(1-methyl-4-ethoxycarbonyl-pyrazol-5-yl-sulfonyl)guanidin vom Schmelzpunkt 177 °C.

Beispiel 3

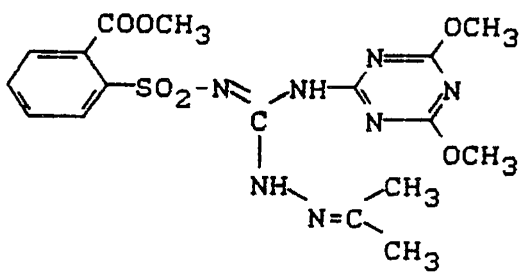

(Verfahren (a))

Eine Mischung aus 0,5 g (1,2 mMol) N′-(4,6-Dimethoxy-s-triazin-2-yl)-N″-amino-N‴-(2-methoxycarbonyl-phenylsulfonyl)-guanidin, 20 ml Aceton, 2 g Natriumsulfat und 10 ml Methylenchlorid wird 20 Stunden bei 20 °C gerührt und anschließend filtriert. Der Filterrückstand wird mit 50 ml Methylenchlorid gewaschen, die Mutterlauge eingedampft, der Rückstand durch Verreiben mit Diethylether zur Kristallisation gebracht und das kristalline Produkt durch Absaugen isoliert.

Man erhält 0,34 g (62% der Theorie) N′-(4,6-Dimethoxy-s-triazin-2-yl)-N″-(1-methyl-ethylidenamino)-N‴-(2-methoxycarbonyl-phenylsulfonyl)-guanidin vom Schmelzpunkt 175 °C.

Beispiel 4

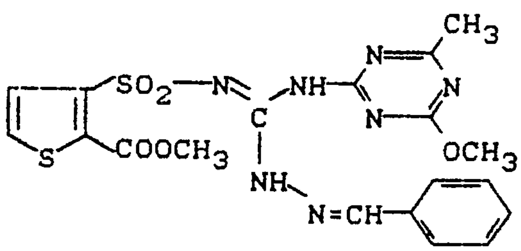

(Verfahren (a))

Eine Mischung aus 1,8 g (4,5 mMol) N′-(4-Methoxy-6-methyl-s-triazin-2-yl)-N″-amino-N‴-(2-methoxycarbonylthiophen-3-yl-sulfonyl)-guanidin, 0,71 g (6,7 mMol) Benzaldehyd, 2 g Natriumsulfat und 30 ml Chloroform wird 12 Stunden unter Rückfluß erhitzt und anschließend filtriert. Der Filterrückstand wird mit 50 ml Chloroform gewaschen, die filtrierte Chloroformlösung eingedampft, der Rückstand mit Diethylether verrührt und das kristallin angefallene Produkt durch Absaugen isoliert.

Man erhält 2,05 g (94% der Theorie) N′-(4-Methoxy-6-methyl-s-triazin-2-yl)-N″-benzylidenamino-N‴-(2-methoxycarbonyl-thiophen-3-yl-sulfonyl)-guanidin vom Schmelzpunkt 215 °C.

Beispiel 5

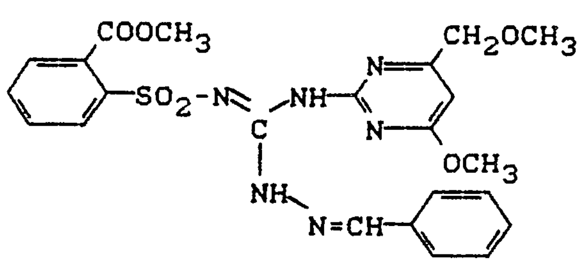

(Verfahren (a))

Eine Mischung aus 4,2 g (0,01 Mol) N'-(4-Methoxy-6-methoxymethyl-pyrimidin-2-yl)-N''-amino-N'''-(2-methoxycarbonyl-phenylsulfonyl)-guanidin und 50 ml Benzaldehyd wird 7 Tage bei 20° C gerührt. Dann wird das kristallin angefallene Produkt durch Absaugen isoliert.

Man erhält 2,0 g (39% der Theorie) N'-(4-Methoxy-6-methoxymethyl-pyrimidin-2-yl)-N''-benzylidenamino-N'''-(2-methoxycarbonyl-phenylsulfonyl)-guanidin vom Schmelzpunkt 209° C.

Beispiel 6

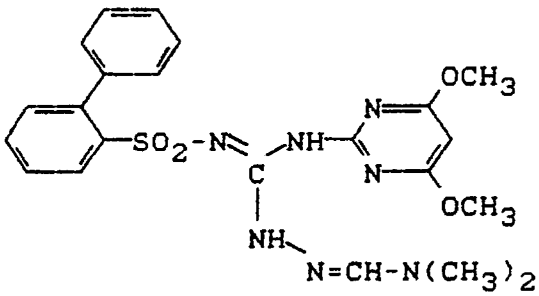

(Verfahren (a))

Eine Mischung aus 4,3 g (0,01 Mol) N'-(4,6-Dimethoxypyrimidin-2-yl)-N''-amino-N'''-(2-phenyl-phenylsulfonyl)-guanidin und 2 ml Dimethylformamid-dimethylacetal wird 60 Minuten bei 20° C gerührt; dann wird das kristallin angefallene Produkt durch Absaugen isoliert.

Man erhält 4,6 g (95% der Theorie) N'-(4,6-Dimethoxypyrimidin-2-yl)-N''-dimethylaminomethylenamino-N'''-(2-phenyl-phenylsulfonyl)-guanidin vom Schmelzpunkt 139° C.

Beispiel 7

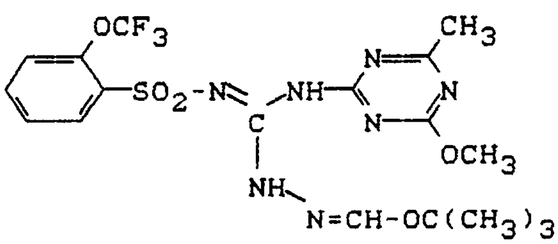

(Verfahren (a))

Eine Mischung aus 8,42 g (0,02 Mol) N'-(4-Methoxy-6-methyl-s-triazin-2-yl)-N''-amino-N'''-(2-trifluormethoxyphenylsulfonyl)-guanidin, 5 ml Dimethylformamid-di-tertbutyl-acetal und 100 ml Tetrahydrofuran wird 15 Stunden bei 20° C gerührt und dann mit Wasser langsam auf etwa das doppelte Volumen verdünnt. Das hierbei kristallin angefallene Produkt wird durch Absaugen isoliert.

Man erhält 2,2 g (22% der Theorie) N'-(4-Methoxy-6-methyl-s-triazin-2-yl)-N''-(tert-butoxy-methylenamino)-N'''-(2-trifluormethoxy-phenylsulfonyl)-guanidin vom Schmelzpunkt 182° C.

Beispiel 8

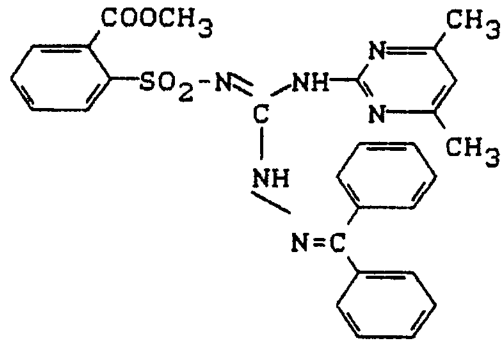

(Verfahren (b))

Eine Mischung aus 11,8 g (0,02 Mol) N′-(4,6-Dimethyl-pyrimidin-2-yl)-N″-methoxy-N″,N‴-bis-(2-methoxycarbonyl-phenylsulfonyl)-guanidin, 5,9 g (0,03 Mol) Benzophenon-hydrazon und 100 ml Dioxan wird 2 Tage unter Rückfluß erhitzt. Nach Abkühlen wird das nicht umgesetzte Benzophenon-hydrazon durch Filtration abgetrennt, das Filtrat eingeengt und der Rückstand aus Ethanol umkristallisiert.

Man erhält 4,7 g (43% der Theorie) N′-(4,6-Dimethylpyrimidin-2-yl)-N″-diphenylmethylenamino-N‴-(2-methoxycarbonyl-phenylsulfonyl)-guanidin vom Schmelzpunkt 153 °C.

Analog zu den Beispielen 1 bis 8 und entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

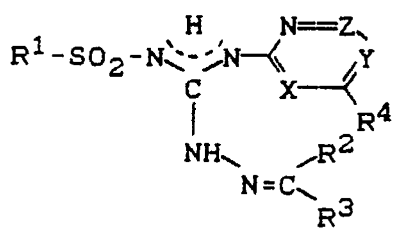

(I)

(Die in Tabelle 3 verwendete Abkürzung "Zers." bedeutet "Zersetzung").

<u>Tabelle 3:</u> Beispiele für die Verbindungen der Formel (I)

| Bsp.-Nr. | $R^1$ | $R^2$ | $R^3$ | $R^4$ | X | Y | Z | Schmelzpunkt (°C) |
|---|---|---|---|---|---|---|---|---|
| 9 | 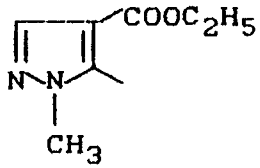 | H | $C_6H_5$ | $CH_3$ | N | CH | $C-OCH_3$ | 182 |
| 10 | 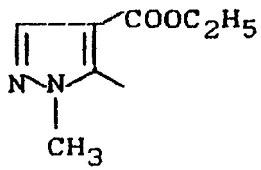 | $CH_3$ | $CH_3$ | $CH_3$ | N | CH | $C-OCH_3$ | 168 |
| 11 | 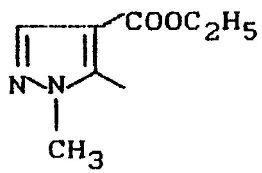 | $-(CH_2)_5-$ | | $CH_3$ | N | CH | $C-OCH_3$ | 188 |
| 12 | 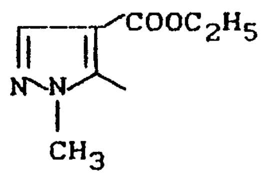 | $CH_3$ | $C_2H_5$ | $CH_3$ | N | CH | $C-OCH_3$ | 134 |
| 13 | 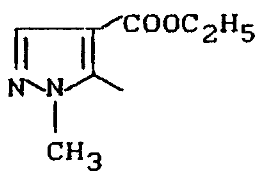 | H | 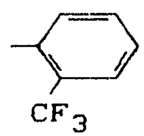 | $CH_3$ | N | CH | $C-OCH_3$ | 183 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 14 | 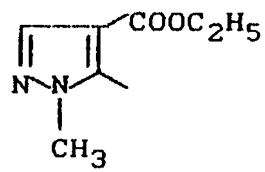 | H | 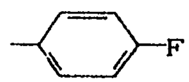 | $CH_3$ | N | CH | $C-OCH_3$ | 191 |
| 15 | 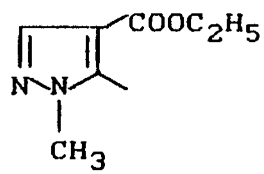 | H | 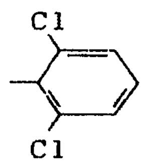 | $CH_3$ | N | CH | $C-OCH_3$ | 181 |
| 16 | 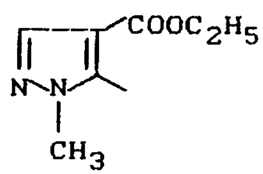 | H | 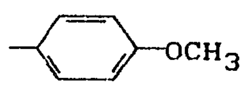 | $CH_3$ | N | CH | $C-OCH_3$ | 193 |
| 17 | 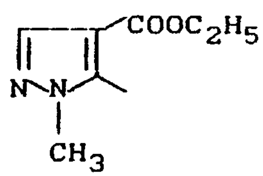 | H | 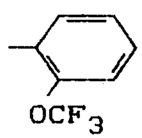 | $CH_3$ | N | CH | $C-OCH_3$ | 161 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 18 | 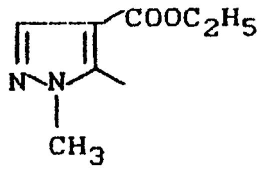 | H | 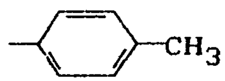 | $CH_3$ | N | CH | $C-OCH_3$ | 186 |
| 19 | 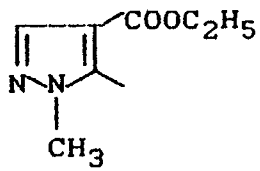 | H | 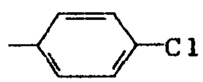 | $CH_3$ | N | CH | $C-OCH_3$ | 194 |
| 20 | 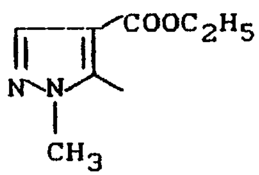 | H | 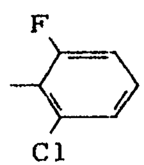 | $CH_3$ | N | CH | $C-OCH_3$ | 175 |
| 21 | 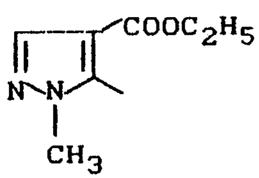 | H | 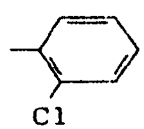 | $OCH_3$ | N | CH | $C-OCH_3$ | 216 |
| 22 | 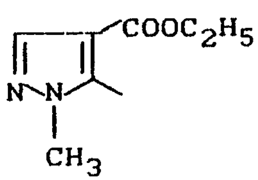 | H | 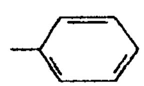 | $OCH_3$ | N | CH | $C-OCH_3$ | 189 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 23 | 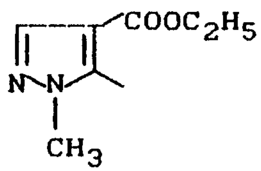 | $CH_3$ | $C_2H_5$ | $OCH_3$ | N | CH | $C-OCH_3$ | 149 |
| 24 | 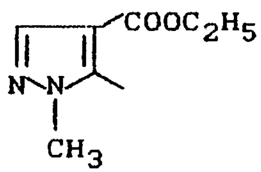 | $CH_3$ | $CH_3$ | $OCH_3$ | N | CH | $C-OCH_3$ | 182 |
| 25 | 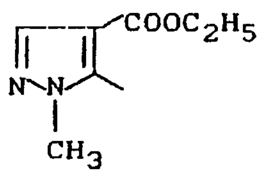 | H | 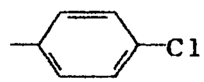 | $OCH_3$ | N | CH | $C-OCH_3$ | 224 |
| 26 | 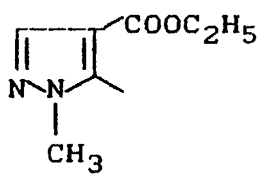 | $-(CH_2)_5-$ | | $OCH_3$ | N | CH | $C-OCH_3$ | 148 |
| 27 | 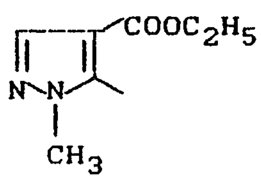 | H | 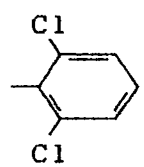 | $OCH_3$ | N | CH | $C-OCH_3$ | 202 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 28 | 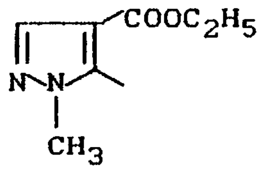 | H | 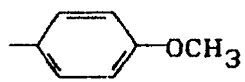 | $OCH_3$ | N | CH | $C-OCH_3$ | 211 |
| 29 | 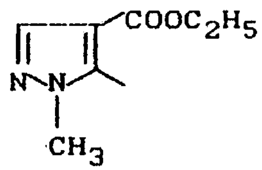 | H | 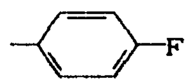 | $OCH_3$ | N | CH | $C-OCH_3$ | 213 |
| 30 | 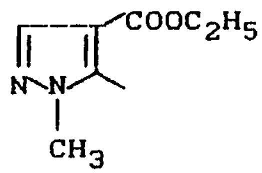 | H | 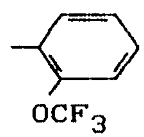 | $OCH_3$ | N | CH | $C-OCH_3$ | 152 |
| 31 | 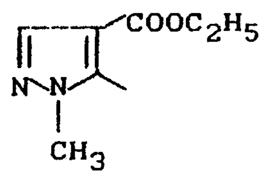 | H | 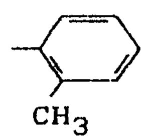 | $OCH_3$ | N | CH | $C-OCH_3$ | 200 |
| 32 | 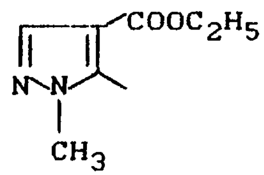 | H | 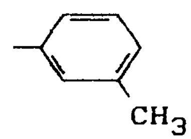 | $OCH_3$ | N | CH | $C-OCH_3$ | 180 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 33 | 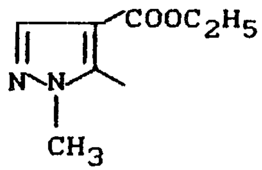 | H | 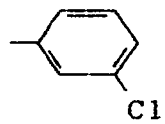 | $OCH_3$ | N | CH | $C-CH_3$ | 185 |
| 34 | 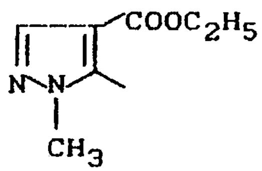 | H | 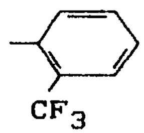 | $OCH_3$ | N | CH | $C-OCH_3$ | 165 |
| 35 | 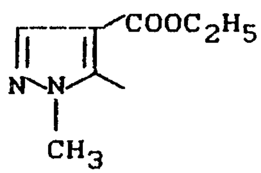 | H | 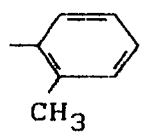 | $CH_3$ | N | CH | $C-OCH_3$ | 186 |
| 36 | 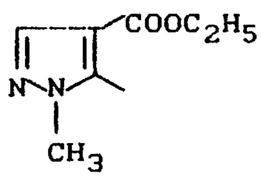 | $CH_3$ | $CH_3$ | $CH_3$ | N | CH | $C-CH_3$ | 164 |
| 37 | 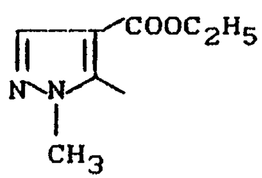 | $CH_3$ | $C_2H_5$ | $CH_3$ | N | CH | $C-CH_3$ | 140 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 33 | 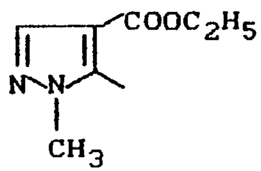 | H | 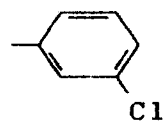 | $OCH_3$ | N | CH | $C-CH_3$ | 185 |
| 34 | 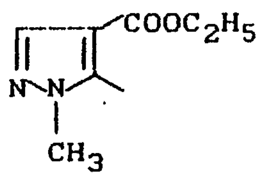 | H | 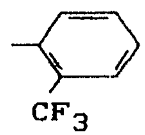 | $OCH_3$ | N | CH | $C-OCH_3$ | 165 |
| 35 | 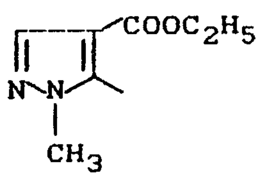 | H | 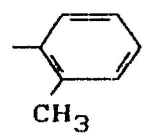 | $CH_3$ | N | CH | $C-OCH_3$ | 186 |
| 36 | 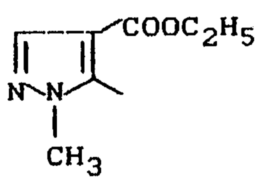 | $CH_3$ | $CH_3$ | $CH_3$ | N | CH | $C-CH_3$ | 164 |
| 37 | 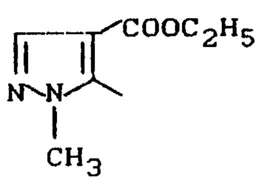 | $CH_3$ | $C_2H_5$ | $CH_3$ | N | CH | $C-CH_3$ | 140 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 38 | 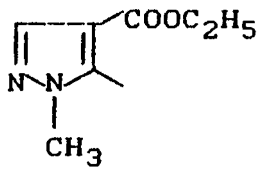 | H | 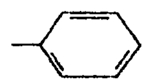 | $CH_3$ | N | CH | $C-CH_3$ | 187 |
| 39 | 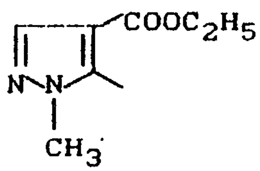 | $-(CH_2)_5-$ | | $CH_3$ | N | CH | $C-CH_3$ | 176 |
| 40 | 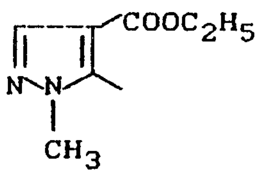 | H | 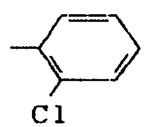 | $CH_3$ | N | CH | $C-CH_3$ | 189 |
| 41 | 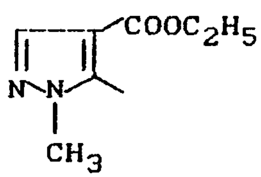 | H | 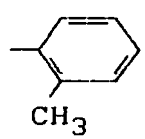 | $CH_3$ | N | CH | $C-CH_3$ | 190 |
| 42 | 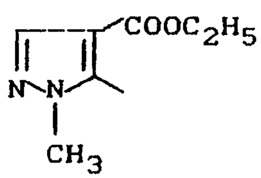 | H | 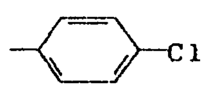 | $CH_3$ | N | CH | $C-CH_3$ | 186 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 48 | 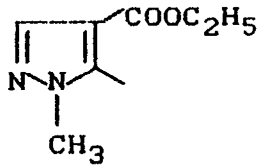 | H | 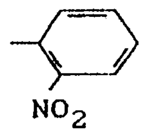 | $OCH_3$ | N | CH | $C-OCH_3$ | 192 |
| 49 | 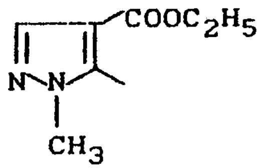 | H | 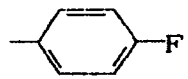 | $CH_3$ | N | CH | $C-CH_3$ | 198 |
| 50 | 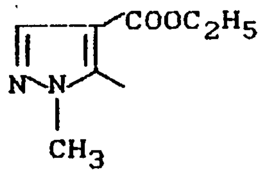 | H | 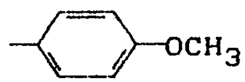 | $CH_3$ | N | CH | $C-CH_3$ | 185 |
| 51 | 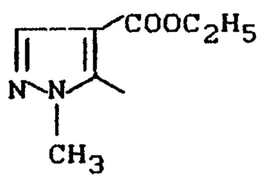 | H | 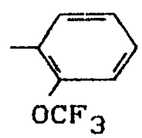 | $CH_3$ | N | CH | $C-CH_3$ | 181 |
| 52 | 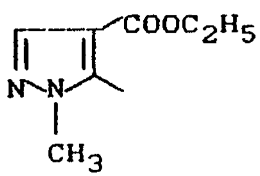 | H | 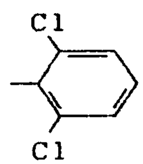 | $CH_3$ | N | CH | $C-CH_3$ | 179 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 53 | 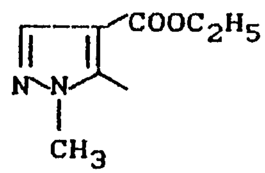 | H | 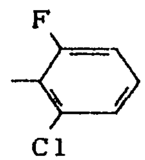 | $CH_3$ | N | CH | $C-CH_3$ | 187 |
| 54 | 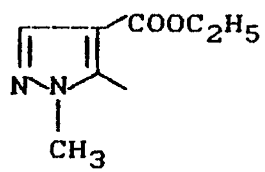 | H | 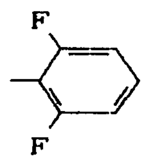 | $CH_3$ | N | CH | $C-CH_3$ | 177 |
| 55 | 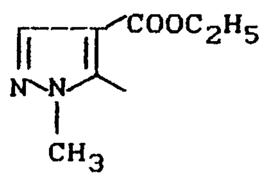 | H | 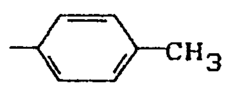 | $CH_3$ | N | CH | $C-CH_3$ | 202 |
| 56 | 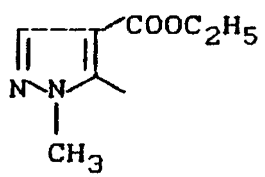 | H | 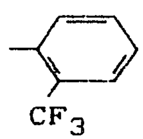 | $CH_3$ | N | CH | $C-CH_3$ | 185 |
| 57 | 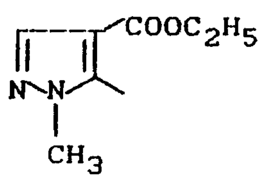 | H | 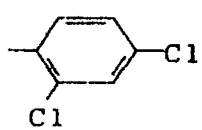 | $CH_3$ | N | CH | $C-CH_3$ | 186 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 58 | 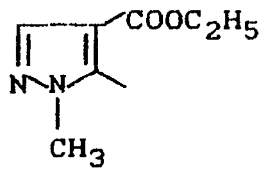 | H | 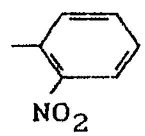 | $CH_3$ | N | CH | $C-CH_3$ | 187 |
| 59 | 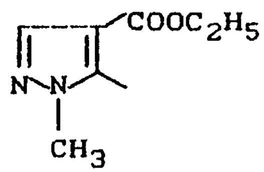 | H | 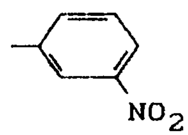 | $CH_3$ | N | CH | $C-CH_3$ | 197 |
| 60 | 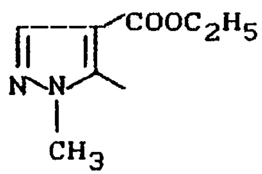 | H | 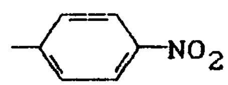 | $CH_3$ | N | CH | $C-CH_3$ | 192 |
| 61 | 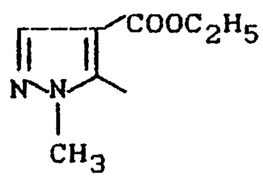 | H | 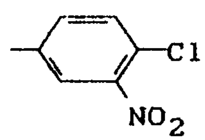 | $CH_3$ | N | CH | $C-CH_3$ | 215 |
| 62 | 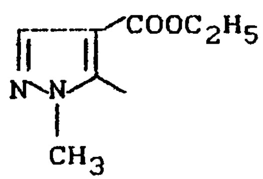 | H | 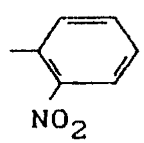 | $CH_3$ | N | CH | $C-OCH_3$ | 176 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 63 | 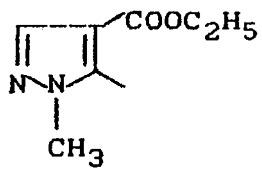 | H | 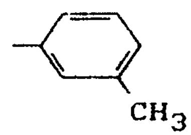 | $CH_3$ | N | CH | $C-OCH_3$ | 174 |
| 64 | 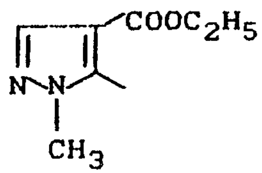 | H | 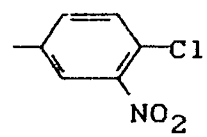 | $CH_3$ | N | CH | $C-OCH_3$ | 193 |
| 65 | 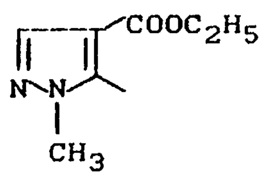 | H | 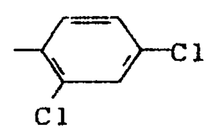 | $CH_3$ | N | CH | $C-OCH_3$ | 192 |
| 66 | 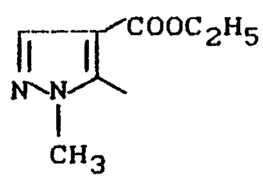 | H | 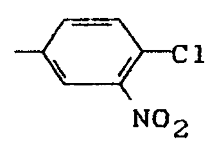 | $OCH_3$ | N | CH | $C-OCH_3$ | 222 |
| 67 | 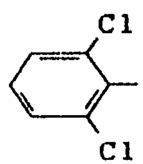 | $CH_3$ | $CH_3$ | $OCH_3$ | N | N | $C-OCH_3$ | 213 (Zers.) |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 68 | 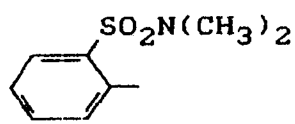 | $CH_3$ | $CH_3$ | $OCH_3$ | N | N | $C-OCH_3$ | 222 |
| 69 | 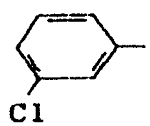 | $CH_3$ | $CH_3$ | $OCH_3$ | N | N | $C-OCH_3$ | 146 |
| 70 | 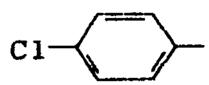 | $CH_3$ | $CH_3$ | $OCH_3$ | N | N | $C-OCH_3$ | 160 |
| 71 | 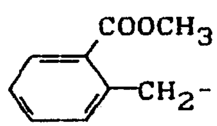 | $-(CH_2)_5-$ | | $OCH_3$ | N | N | $C-OCH_3$ | 173 |
| 72 | 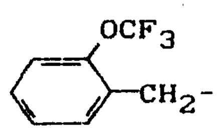 | $-(CH_2)_5-$ | | $OCH_3$ | N | N | $C-OCH_3$ | 167 |
| 73 | 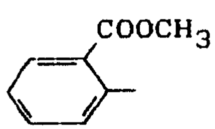 | H | 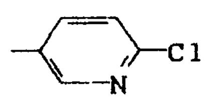 | $OCH_3$ | N | N | $C-OCH_3$ | 230 (Zers.) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 74 | 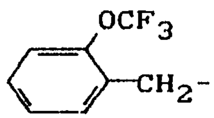 | H | $C_6H_5$ | $CH_3$ | N | N | $C-OCH_3$ | 198 |
| 75 | 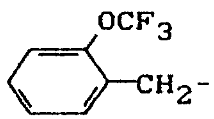 | H | $C_6H_5$ | $CH_3$ | N | N | $C-CH_3$ | 180 |
| 76 | 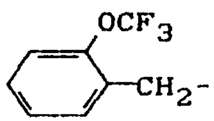 | H | $C_6H_5$ | $C_2H_5$ | N | N | $C-OCH_3$ | 174 |
| 77 | 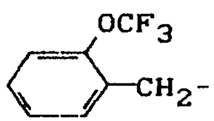 | H | $C_6H_5$ | $CH_3$ | N | N | $C-OC_2H_5$ | 186 |
| 78 | 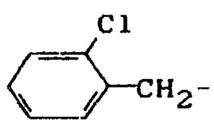 | H | $C_6H_5$ | $CH_3$ | N | N | $C-OC_2H_5$ | 184 |
| 79 | 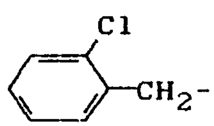 | H | $C_6H_5$ | $CH_3$ | N | N | $C-SCH_3$ | 218 |

Tabelle 3 - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 80 | 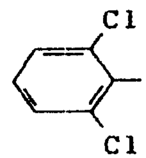 | $CH_3$ | $CH_3$ | $CH_3$ | N | N | $C-OC_2H_5$ | 176 |
| 81 | 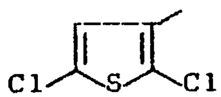 | $CH_3$ | $CH_3$ | $CH_3$ | N | N | $C-OCH_3$ | 152 |
| 82 | 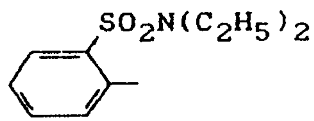 | $CH_3$ | $CH_3$ | $CH_3$ | N | N | $C-OCH_3$ | 180 |
| 83 | 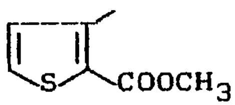 | $CH_3$ | $CH_3$ | $CH_3$ | N | N | $C-OCH_3$ | 183 |
| 84 | 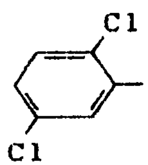 | $CH_3$ | $CH_3$ | $CH_3$ | N | N | $C-OCH_3$ | 187 |
| 85 | 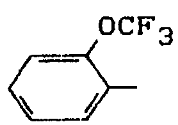 | $CH_3$ | $CH_3$ | $CH_3$ | N | N | $C-OC_2H_5$ | 146 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 86 | 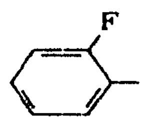 | $CH_3$ | $CH_3$ | $OCH_3$ | N | N | $C-OCH_3$ | 194 |
| 87 | 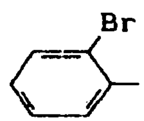 | $CH_3$ | $CH_3$ | $OCH_3$ | N | N | $C-OCH_3$ | 211 |
| 88 | 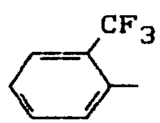 | $CH_3$ | $CH_3$ | $OCH_3$ | N | N | $C-OCH_3$ | 152 |
| 89 | 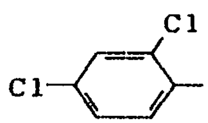 | $CH_3$ | $CH_3$ | $OCH_3$ | N | N | $C-OCH_3$ | 191 |
| 90 | 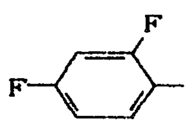 | $CH_3$ | $CH_3$ | $OCH_3$ | N | N | $C-OCH_3$ | 166 |
| 91 | 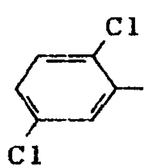 | $CH_3$ | $CH_3$ | $OCH_3$ | N | N | $C-OCH_3$ | 214 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 92 | 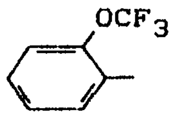 | $CH_3$ | $CH_3$ | $OCH_3$ | N | N | $C-OCH_3$ | 164 |
| 93 | 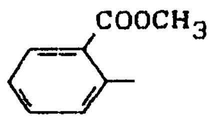 | $CH_3$ | $CH_3$ | $CH_3$ | N | N | $C-OCH_3$ | 165 |
| 94 | 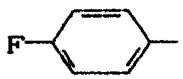 | $CH_3$ | $CH_3$ | $CH_3$ | N | N | $C-OCH_3$ | 112 |
| 95 | 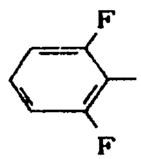 | $CH_3$ | $CH_3$ | $OCH_3$ | N | N | $C-OCH_3$ | 171 |
| 96 | 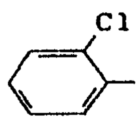 | $CH_3$ | $CH_3$ | $CH_3$ | N | N | $C-CH_3$ | 110 |
| 97 | 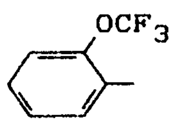 | H | 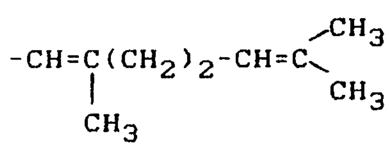 | $OCH_3$ | N | N | $C-OCH_3$ | 152 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 98 | 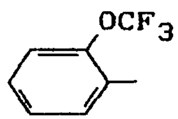 | H | 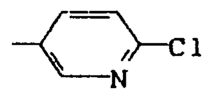 | $OCH_3$ | N | N | $C-OCH_3$ | 109 |
| 99 | 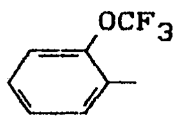 | H | $C_6H_5$ | $OCH_3$ | N | N | $C-OCH_3$ | 174 |
| 100 | 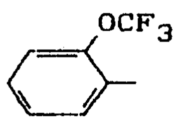 | H | 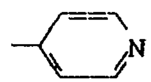 | $OCH_3$ | N | N | $C-OCH_3$ | 137 |
| 101 | 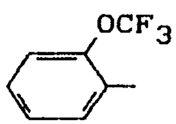 | H | 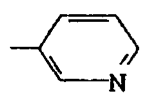 | $OCH_3$ | N | N | $C-OCH_3$ | 198 |
| 102 | 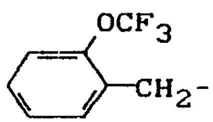 | $CH_3$ | $CH_3$ | $OCH_3$ | N | N | $C-OCH_3$ | 192 |
| 103 | 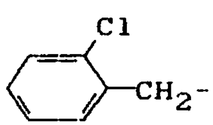 | $CH_3$ | $CH_3$ | $OCH_3$ | N | N | $C-OCH_3$ | 175 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 104 | 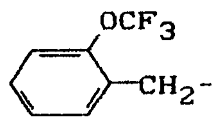 | H | 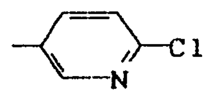 | $OCH_3$ | N | N | $C-OCH_3$ | 242 (Zers.) |
| 105 | 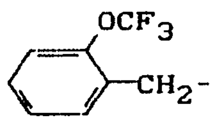 | H | 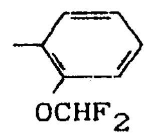 | $OCH_3$ | N | N | $C-OCH_3$ | 170 |
| 106 | 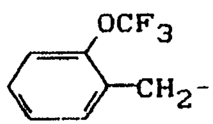 | H | 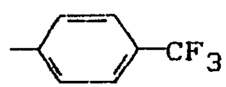 | $OCH_3$ | N | N | $C-OCH_3$ | 210 |
| 107 | 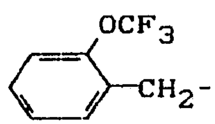 | H | $C_6H_5$ | $OCH_3$ | N | N | $C-OCH_3$ | 199 |
| 108 | 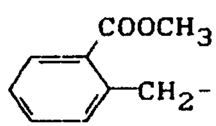 | $CH_3$ | $CH_3$ | $CH_3$ | N | N | $C-OCH_3$ | 201 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 109 | 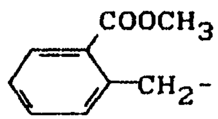 | $CH_3$ | $CH_3$ | $OCH_3$ | N | N | $C-OCH_3$ | 210 |
| 110 | 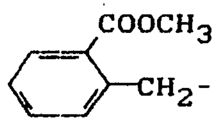 | $CH_3$ | $CH_3$ | $CH_3$ | N | N | $C-CH_3$ | 200 (Zers.) |
| 111 | 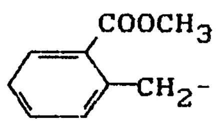 | $CH_3$ | $CH_3$ | $C_2H_5$ | N | N | $C-OCH_3$ | 185 |
| 112 | 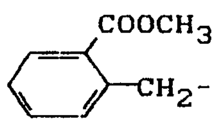 | $CH_3$ | $CH_3$ | $CH_3$ | N | N | $C-SCH_3$ | 184 |
| 113 | 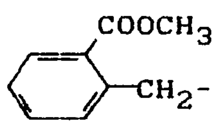 | $CH_3$ | $CH_3$ | $CH_3$ | N | N | $C-OC_2H_5$ | 183 |
| 114 | 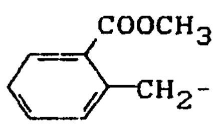 | $CH_3$ | $CH_3$ | $CH_3$ | N | N | $C-N(CH_3)_2$ | 227 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 115 | 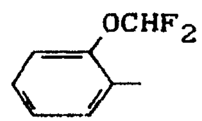 | $CH_3$ | $CH_3$ | $C_2H_5$ | N | N | $C-OCH_3$ | 128 |
| 116 | 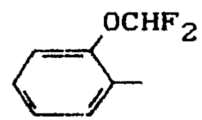 | $CH_3$ | $CH_3$ | $CH_3$ | N | N | $C-OCH_3$ | 177 |
| 117 | 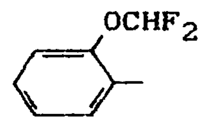 | $CH_3$ | $CH_3$ | $OCH_3$ | N | N | $C-OCH_3$ | 169 |
| 118 | 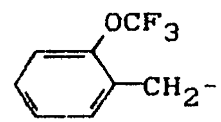 | $CH_3$ | $CH_3$ | $CH_3$ | N | N | $C-OCH_3$ | 185 |
| 119 | 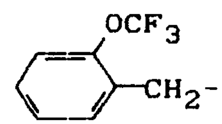 | $CH_3$ | $CH_3$ | $CH_3$ | N | N | $C-CH_3$ | 184 |
| 120 | 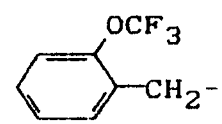 | $CH_3$ | $CH_3$ | $C_2H_5$ | N | N | $C-OCH_3$ | 175 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 121 | 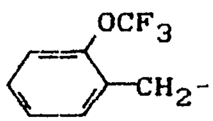 | $CH_3$ | $CH_3$ | $CH_3$ | N | N | $C-OC_2H_5$ | 210 |
| 122 | 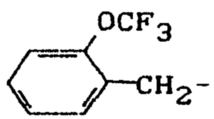 | $CH_3$ | $CH_3$ | $CH_3$ | N | N | $C-SCH_3$ | 188 |
| 123 | 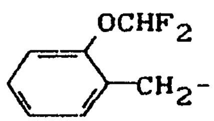 | $CH_3$ | $CH_3$ | $OCH_3$ | N | N | $C-OCH_3$ | 163 |
| 124 | 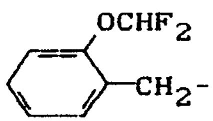 | $CH_3$ | $CH_3$ | $CH_3$ | N | N | $C-CH_3$ | 165 |
| 125 | 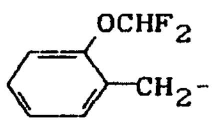 | $CH_3$ | $CH_3$ | $CH_3$ | N | N | $C-OCH_3$ | 154 |
| 126 | 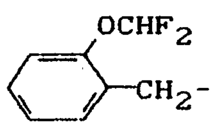 | $CH_3$ | $CH_3$ | $C_2H_5$ | N | N | $C-OCH_3$ | 115 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 127 | 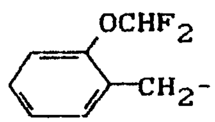 | $CH_3$ | $CH_3$ | $CH_3$ | N | N | $C-OC_2H_5$ | 169 |
| 128 | 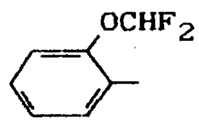 | $CH_3$ | $CH_3$ | $CH_3$ | N | N | $C-CH_3$ | 205 |
| 129 | 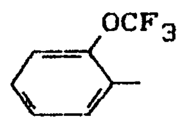 | $CH_3$ | $CH_3$ | $CH_3$ | N | N | $C-CH_3$ | 179 |
| 130 | 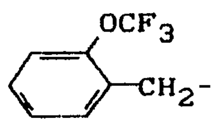 | H | $C_6H_5$ | $CH_3$ | N | N | $C-SCH_3$ | 208 |
| 131 | 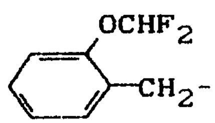 | H | $C_6H_5$ | $OCH_3$ | N | N | $C-OCH_3$ | 227 (Zers.) |
| 132 | 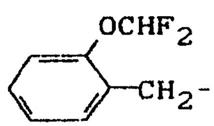 | H | $C_6H_5$ | $CH_3$ | N | N | $C-OCH_3$ | 207 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 133 | 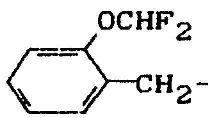 | H | $C_6H_5$ | $CH_3$ | N | N | $C-CH_3$ | 195 |
| 134 | 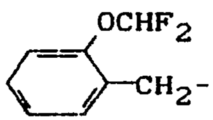 | H | $C_6H_5$ | $C_2H_5$ | N | N | $C-OCH_3$ | 190 |
| 135 | 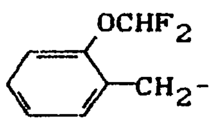 | H | $C_6H_5$ | $CH_3$ | N | N | $C-OC_2H_5$ | 212 |
| 136 | 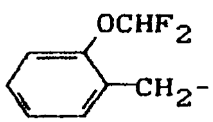 | $CH_3$ | $CH_3$ | $CH_3$ | N | N | $C-SCH_3$ | 143 |
| 137 | 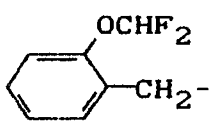 | H | $C_6H_5$ | $CH_3$ | N | N | $C-SCH_3$ | 210 |
| 138 | 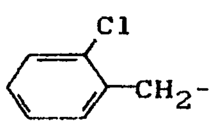 | $CH_3$ | $CH_3$ | $CH_3$ | N | N | $C-OC_2H_5$ | 168 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 139 | 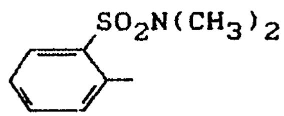 | H | 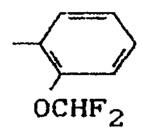 | $CH_3$ | N | N | $C-OCH_3$ | 210 |
| 140 | 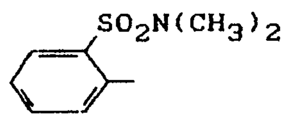 | H | 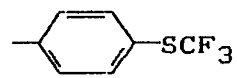 | $CH_3$ | N | N | $C-OCH_3$ | 200 |
| 141 | 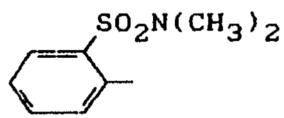 | H | 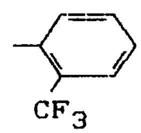 | $OCH_3$ | N | N | $C-OCH_3$ | 231 (Zers.) |
| 142 | 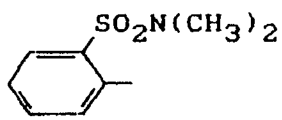 | H | 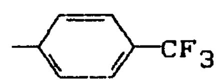 | $CH_3$ | N | N | $C-OCH_3$ | 210 |
| 143 | 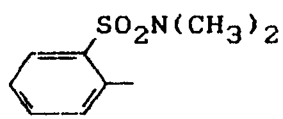 | H | 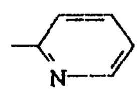 | $CH_3$ | N | N | $C-OCH_3$ | 207 (Zers.) |
| 144 | 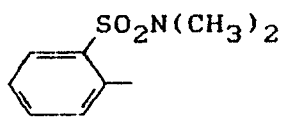 | H | 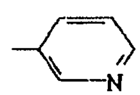 | $CH_3$ | N | N | $C-OCH_3$ | 208 (Zers.) |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 145 | 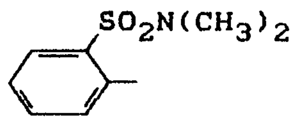 | H | 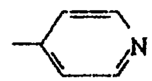 | $CH_3$ | N | N | $C-OCH_3$ | 209 (Zers.) |
| 146 | 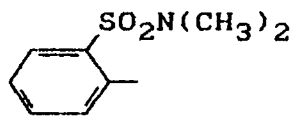 | H | 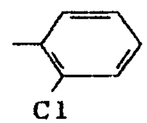 | $CH_3$ | N | N | $C-OCH_3$ | 215 |
| 147 | 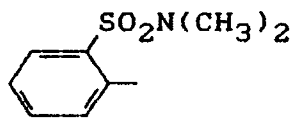 | H | 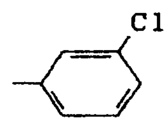 | $CH_3$ | N | N | $C-OCH_3$ | 221 (Zers.) |
| 148 | 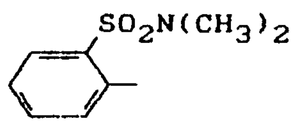 | H | 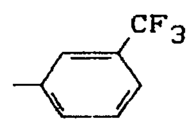 | $CH_3$ | N | N | $C-OCH_3$ | 213 |
| 149 | 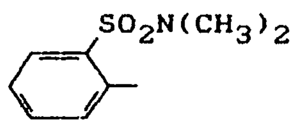 | H | 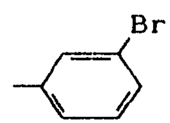 | $CH_3$ | N | N | $C-OCH_3$ | 222 (Zers.) |
| 150 | 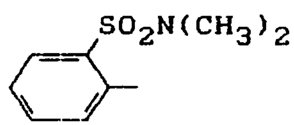 | H | 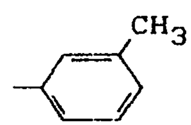 | $CH_3$ | N | N | $C-OCH_3$ | 238 (Zers.) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 151 | 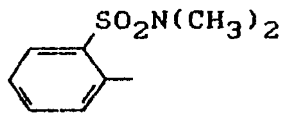 | H | 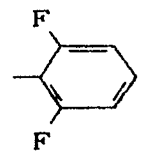 | $CH_3$ | N | N | $C-OCH_3$ | 225 (Zers.) |
| 152 | 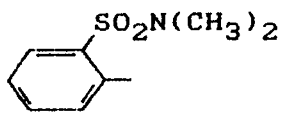 | H | 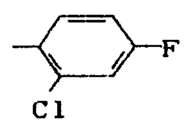 | $CH_3$ | N | N | $C-OCH_3$ | 196 (Zers.) |
| 153 | 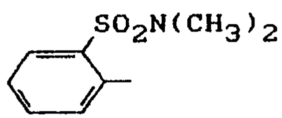 | H | 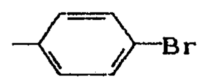 | $CH_3$ | N | N | $C-OCH_3$ | 236 (Zers.) |
| 154 | 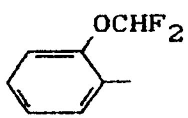 | H | 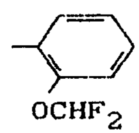 | $CH_3$ | N | N | $C-OCH_3$ | 184 |
| 155 | 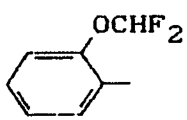 | H | 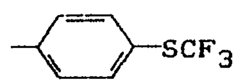 | $CH_3$ | N | N | $C-OCH_3$ | 149 |
| 156 | 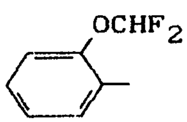 | H | 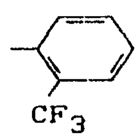 | $CH_3$ | N | N | $C-OCH_3$ | 166 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 157 | 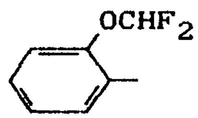 | H | 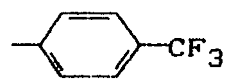 | $CH_3$ | N | N | $C-OCH_3$ | 165 (Zers.) |
| 158 | 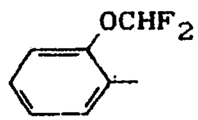 | H | 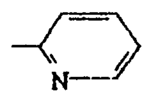 | $CH_3$ | N | N | $C-OCH_3$ | 176 |
| 159 | 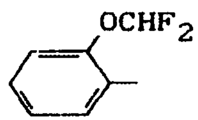 | H | 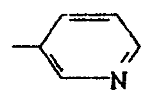 | $CH_3$ | N | N | $C-OCH_3$ | 197 |
| 160 | 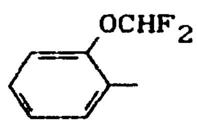 | H | 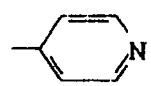 | $CH_3$ | N | N | $C-OCH_3$ | 177 |
| 161 | 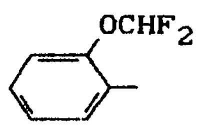 | H | 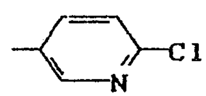 | $CH_3$ | N | N | $C-OCH_3$ | 200 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 162 | 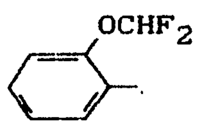 | H | 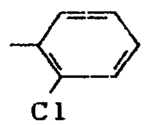 | $CH_3$ | N | N | $C-OCH_3$ | 134 |
| 163 | 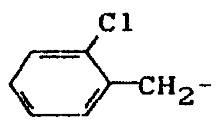 | $CH_3$ | $CH_3$ | $CH_3$ | N | N | $C-SCH_3$ | 180 |
| 164 | 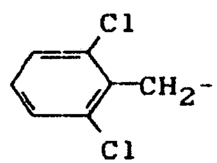 | $CH_3$ | $CH_3$ | $CH_3$ | N | N | $C-OC_2H_5$ | 199 |
| 165 | 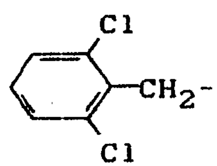 | H | $C_6H_5$ | $CH_3$ | N | N | $C-OC_2H_5$ | 179 |
| 166 | 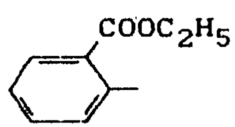 | $CH_3$ | $CH_3$ | $OCH_3$ | N | N | $C-OCH_3$ | 191 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 167 | 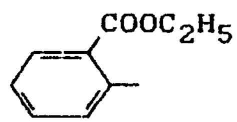 | H | $C_6H_5$ | $OCH_3$ | N | N | $C-OCH_3$ | 192 |
| 168 | 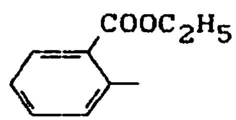 | $CH_3$ | $CH_3$ | $CH_3$ | N | N | $C-OCH_3$ | 169 |
| 169 | 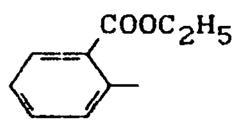 | H | $C_6H_5$ | $CH_3$ | N | N | $C-OCH_3$ | 187 |
| 170 | 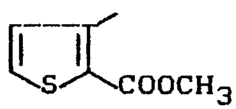 | $CH_3$ | $CH_3$ | $CH_3$ | N | N | $C-SCH_3$ | 198 |
| 171 | 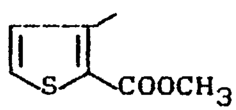 | H | $C_6H_5$ | $CH_3$ | N | N | $C-SCH_3$ | 211 |
| 172 | 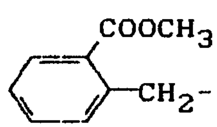 | H | $C_6H_5$ | $CH_3$ | N | N | $C-OCH_3$ | 156 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 173 | 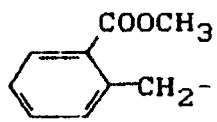 | H | $C_6H_5$ | $CH_3$ | N | N | $C-OC_2H_5$ | 153 |
| 174 | 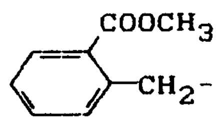 | H | $C_6H_5$ | $CH_3$ | N | N | $C-SCH_3$ | 165 |
| 175 | 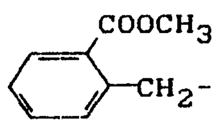 | H | $C_6H_5$ | $CH_3$ | N | N | $C-N(CH_3)_2$ | 188 |
| 176 | 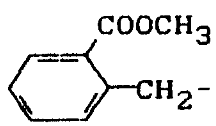 | H | $C_6H_5$ | $C_2H_5$ | N | N | $C-OCH_3$ | 121 (Zers.) |
| 177 | 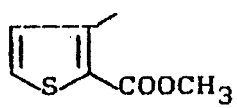 | $CH_3$ | $CH_3$ | $CH_3$ | N | N | $C-OC_2H_5$ | 180 |
| 178 | 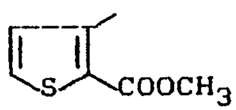 | H | $C_6H_5$ | $CH_3$ | N | N | $C-OC_2H_5$ | 216 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 179 | 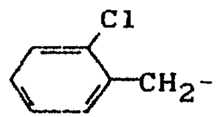 | $CH_3$ | $CH_3$ | $CH_3$ | N | N | $C-OCH_3$ | 164 |
| 180 | 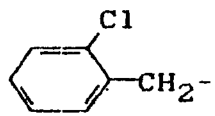 | H | $C_6H_5$ | $CH_3$ | N | N | $C-OCH_3$ | 206 |
| 181 | 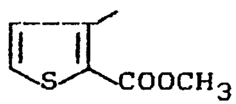 | H | 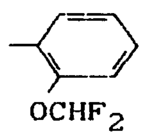 | $CH_3$ | N | N | $C-OC_2H_5$ | 182 |
| 182 | 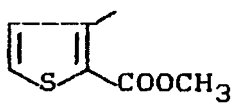 | H | 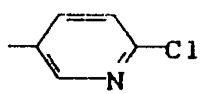 | $CH_3$ | N | N | $C-OC_2H_5$ | 167 (Zers.) |
| 183 | 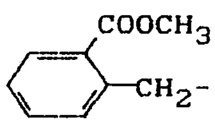 | H | $C_6H_5$ | $CH_3$ | N | N | $C-CH_3$ | 132 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 184 | 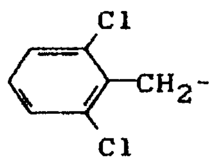 | $CH_3$ | $CH_3$ | $CH_3$ | N | N | $C-OCH_3$ | 190 |
| 185 | 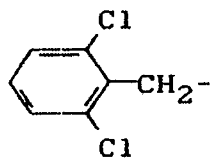 | H | $C_6H_5$ | $CH_3$ | N | N | $C-OCH_3$ | 202 |
| 186 | 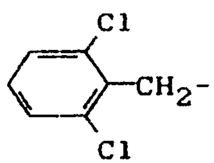 | $CH_3$ | $CH_3$ | $OCH_3$ | N | N | $C-OCH_3$ | 224 |
| 187 | 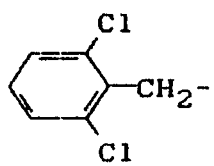 | H | $C_6H_5$ | $OCH_3$ | N | N | $C-OCH_3$ | 185 |
| 188 | 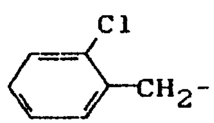 | $CH_3$ | $CH_3$ | $CH_3$ | N | N | $C-CH_3$ | 181 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 189 | 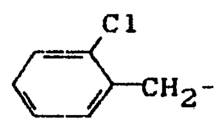 | H | $C_6H_5$ | $CH_3$ | N | N | $C-CH_3$ | 157 |
| 190 | 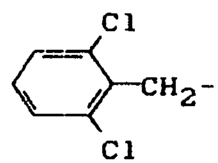 | $CH_3$ | $CH_3$ | $CH_3$ | N | N | $C-SCH_3$ | 200 |
| 191 | 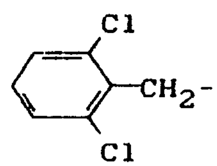 | H | $C_6H_5$ | $CH_3$ | N | N | $C-SCH_3$ | 230 |
| 192 | 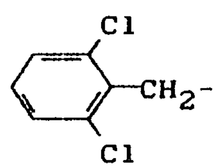 | $CH_3$ | $CH_3$ | $C_2H_5$ | N | N | $C-OCH_3$ | 170 |
| 193 | 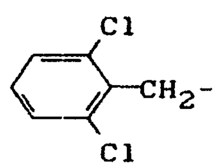 | H | $C_6H_5$ | $C_2H_5$ | N | N | $C-OCH_3$ | 190 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 194 | 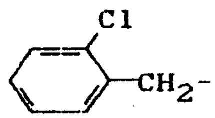 | $CH_3$ | $CH_3$ | $C_2H_5$ | N | N | $C-OCH_3$ | 174 |
| 195 | 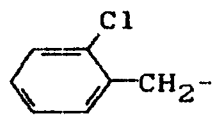 | H | $C_6H_5$ | $C_2H_5$ | N | N | $C-OCH_3$ | 189 |
| 196 | 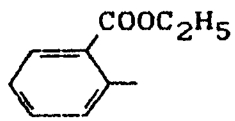 | $CH_3$ | $CH_3$ | $CH_3$ | N | N | $C-SCH_3$ | 187 |
| 197 | 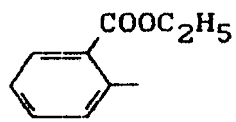 | H | $C_6H_5$ | $CH_3$ | N | N | $C-SCH_3$ | 207 |
| 198 | 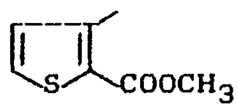 | H | 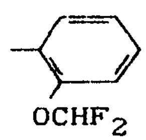 | $CH_3$ | N | N | $C-OCH_3$ | 195 (Zers.) |
| 199 | 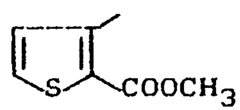 | H | 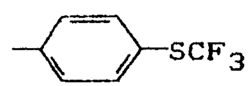 | $CH_3$ | N | N | $C-OCH_3$ | 194 (Zers.) |

Tabelle 3 - Fortsetzung

| Bsp.-Nr. | $R^1$ | $R^2$ | $R^3$ | $R^4$ | X | Y | Z | Schmelzpunkt ($^0$C) |
|---|---|---|---|---|---|---|---|---|
| 200 | 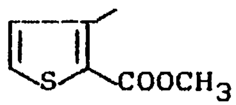 | H | 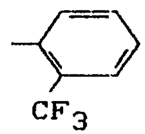 | $CH_3$ | N | N | $C-OCH_3$ | 173 |
| 201 | 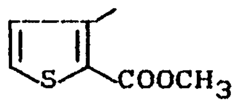 | H | 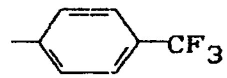 | $CH_3$ | N | N | $C-OCH_3$ | 208 |
| 202 | 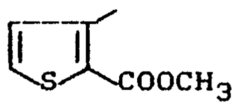 | H | 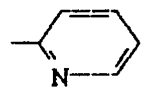 | $CH_3$ | N | N | $C-OCH_3$ | 177 (Zers.) |
| 203 | 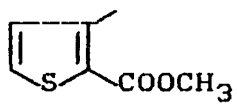 | H | 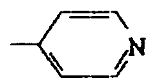 | $CH_3$ | N | N | $C-OCH_3$ | 174 (Zers.) |
| 204 | 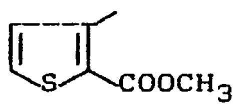 | H | 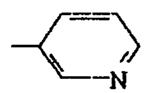 | $CH_3$ | N | N | $C-OCH_3$ | 186 (Zers.) |
| 205 | 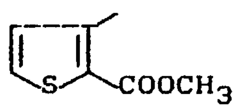 | H | 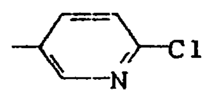 | $CH_3$ | N | N | $C-OCH_3$ | 210 (Zers.) |
| 206 | 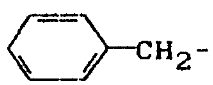 | H | $C_6H_5$ | $CH_3$ | N | N | $C-OCH_3$ | 179 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 207 | 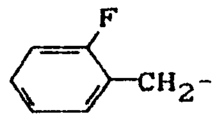 | H | $C_6H_5$ | $CH_3$ | N | N | $C-OCH_3$ | 192 |
| 208 | 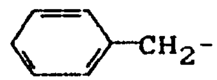 | $CH_3$ | $CH_3$ | $CH_3$ | N | N | $C-OCH_3$ | 182 |
| 209 | 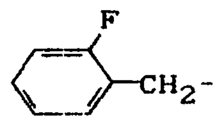 | $CH_3$ | $CH_3$ | $CH_3$ | N | N | $C-OCH_3$ | 160 |
| 210 | 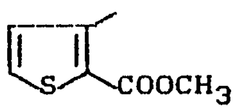 | H | 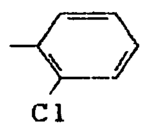 | $CH_3$ | N | N | $C-OCH_3$ | 200 (Zers.) |
| 211 | 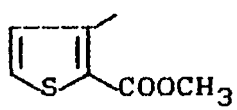 | H | 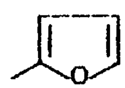 | $CH_3$ | N | N | $C-OCH_3$ | 208 (Zers.) |
| 212 | 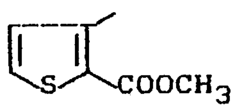 | H | 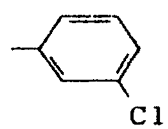 | $CH_3$ | N | N | $C-OCH_3$ | 210 (Zers.) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 213 | 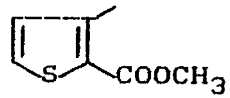 | H | 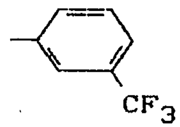 | $CH_3$ | N | N | $C-OCH_3$ | 210 (Zers.) |
| 214 | 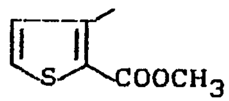 | H | 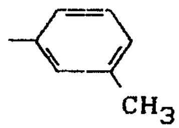 | $CH_3$ | N | N | $C-OCH_3$ | 209 (Zers.) |
| 215 | 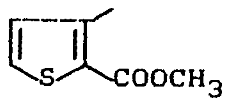 | H | 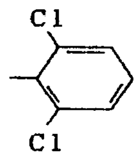 | $CH_3$ | N | N | $C-OCH_3$ | 140 (Zers.) |
| 216 | 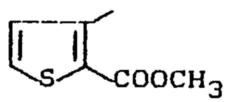 | H | 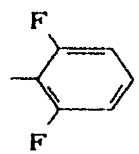 | $CH_3$ | N | N | $C-OCH_3$ | 213 (Zers.) |
| 217 | 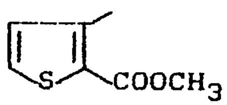 | H | 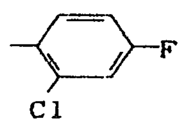 | $CH_3$ | N | N | $C-OCH_3$ | 195 (Zers.) |
| 218 | 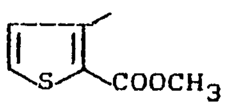 | H | 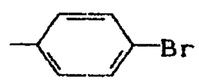 | $CH_3$ | N | N | $C-OCH_3$ | 220 (Zers.) |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 219 | 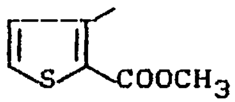 | H | 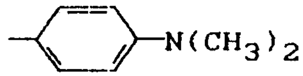 | $CH_3$ | N | N | $C-OCH_3$ | 219 (Zers.) |
| 220 | 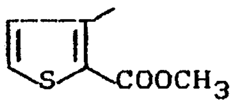 | H | 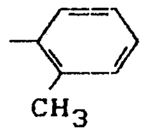 | $CH_3$ | N | N | $C-OCH_3$ | 185 (Zers.) |
| 221 | 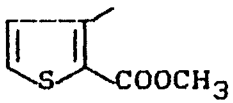 | H | 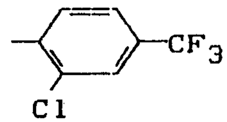 | $CH_3$ | N | N | $C-OCH_3$ | 202 (Zers.) |
| 222 | 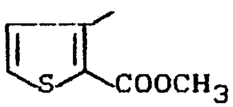 | H | 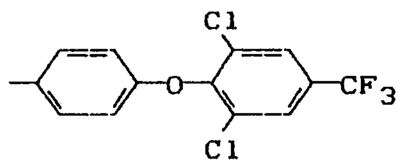 | $CH_3$ | N | N | $C-OCH_3$ | 159 (Zers.) |
| 223 | 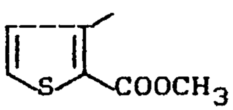 | H | 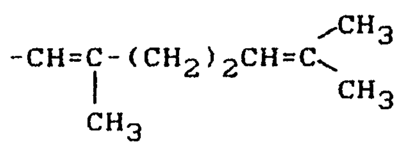 | $CH_3$ | N | N | $C-OCH_3$ | 176 |
| 224 | 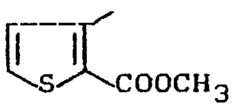 | H | 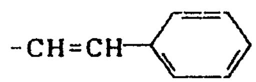 | $CH_3$ | N | N | $C-OCH_3$ | 222 (Zers.) |

Tabelle 3 - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 225 | 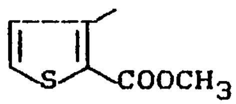 | H | 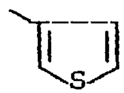 | $CH_3$ | N | N | $C-OCH_3$ | 206 |
| 226 | 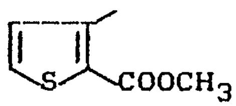 | H | 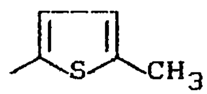 | $CH_3$ | N | N | $C-OCH_3$ | 207 (Zers.) |
| 227 | 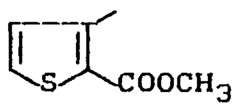 | $CH_3$ | 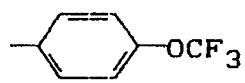 | $CH_3$ | N | N | $C-OCH_3$ | 140 |
| 228 | 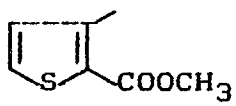 | H | 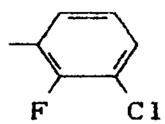 | $CH_3$ | N | N | $C-OCH_3$ | 196 (Zers.) |
| 229 | 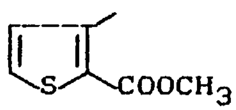 | H | 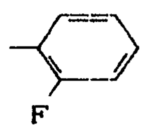 | $CH_3$ | N | N | $C-OCH_3$ | 198 (Zers.) |
| 230 | 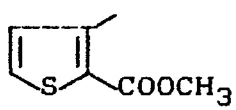 | H | 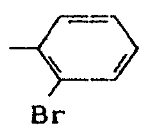 | $CH_3$ | N | N | $C-OCH_3$ | 191 (Zers.) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 231 | 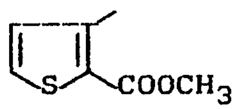 | H | $CH_3$ | $CH_3$ | N | N | $C-OCH_3$ | 179 (Zers.) |
| 232 | 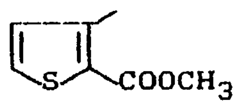 | H | $CH(CH_3)_2$ | $CH_3$ | N | N | $C-OCH_3$ | 189 |
| 233 | 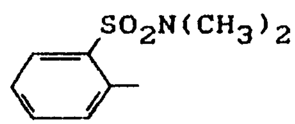 | $CH_3$ | $CH_3$ | $CH_3$ | N | N | $C-OCH_3$ | 193 (Zers.) |
| 234 | 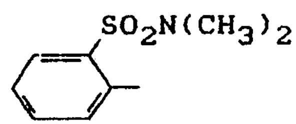 | H | $C_6H_5$ | $CH_3$ | N | N | $C-OCH_3$ | 241 (Zers.) |
| 235 | 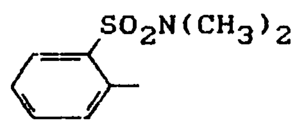 | H | $CH_3$ | $CH_3$ | N | N | $C-OCH_3$ | 173 (Zers.) |
| 236 | 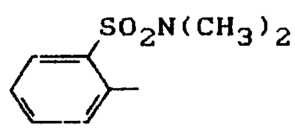 | H | $CH(CH_3)_2$ | $CH_3$ | N | N | $C-OCH_3$ | 174 (Zers.) |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 237 | 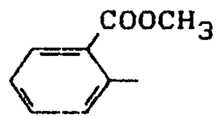 | H | $C_6H_5$ | $CH_3$ | N | N | $C-OCH_3$ | 198 |
| 238 | 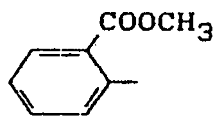 | H | 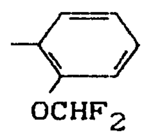 | $CH_3$ | N | N | $C-OCH_3$ | 201 |
| 239 | 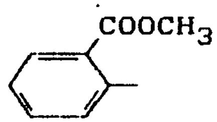 | H | 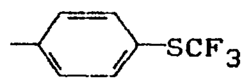 | $CH_3$ | N | N | $C-OCH_3$ | 208 |
| 240 | 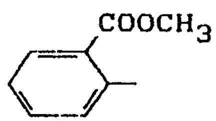 | H | 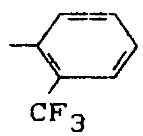 | $CH_3$ | N | N | $C-OCH_3$ | 165 (Zers.) |
| 241 | 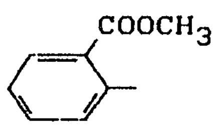 | H | 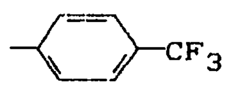 | $CH_3$ | N | N | $C-OCH_3$ | 215 |
| 242 | 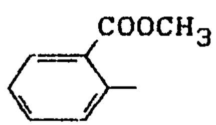 | H | 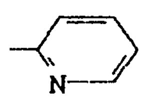 | $CH_3$ | N | N | $C-OCH_3$ | 201 (Zers.) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 243 | 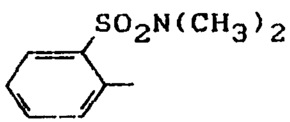 | H | 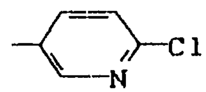 | $CH_3$ | N | N | $C-OCH_3$ | 242 (Zers.) |
| 244 | 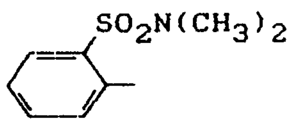 | H | 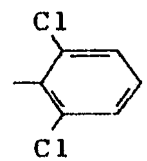 | $CH_3$ | N | N | $C-OCH_3$ | 202 (Zers.) |
| 245 | 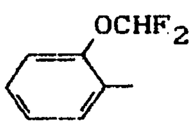 | H | $CH_3$ | $CH_3$ | N | N | $C-OCH_3$ | 139 |
| 246 | 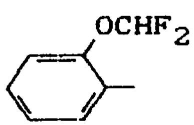 | H | $CH(CH_3)_2$ | $CH_3$ | N | N | $C-OCH_3$ | 136 |
| 247 | 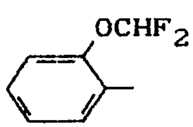 | H | 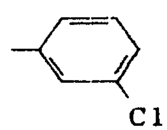 | $CH_3$ | N | N | $C-OCH_3$ | 182 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 248 | 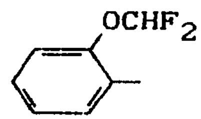 | H | 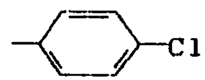 | $CH_3$ | N | N | $C-OCH_3$ | 202 |
| 249 | 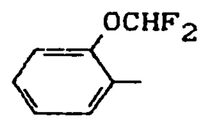 | H | 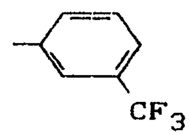 | $CH_3$ | N | N | $C-OCH_3$ | 183 |
| 250 | 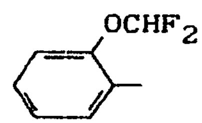 | H | 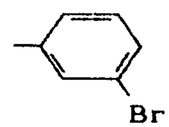 | $CH_3$ | N | N | $C-OCH_3$ | 182 |
| 251 | 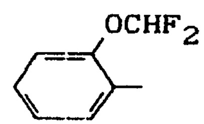 | H | 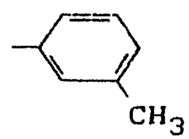 | $CH_3$ | N | N | $C-OCH_3$ | 188 |
| 252 | 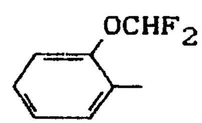 | H | 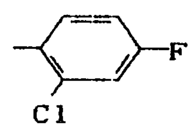 | $CH_3$ | N | N | $C-OCH_3$ | 154 |
| 253 | 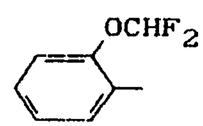 | H | 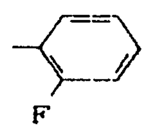 | $CH_3$ | N | N | $C-OCH_3$ | 179 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 254 | 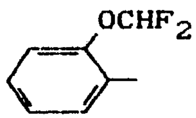 | H | 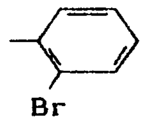 | $CH_3$ | N | N | $C-OCH_3$ | 174 |
| 255 | 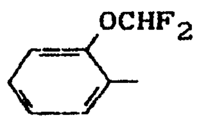 | H | 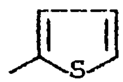 | $CH_3$ | N | N | $C-OCH_3$ | 216 |
| 256 | 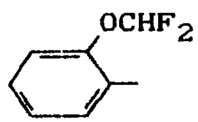 | H | 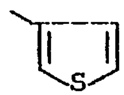 | $CH_3$ | N | N | $C-OCH_3$ | 193 |
| 257 | 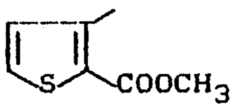 | $CH_3$ | $CH_3$ | $CH_3$ | N | CH | $C-CH_3$ | 229 |
| 258 | 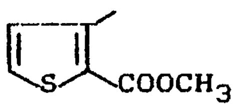 | $CH_3$ | $C_2H_5$ | $CH_3$ | N | CH | $C-CH_3$ | 199 |
| 259 | 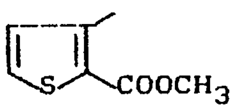 | $CH_3$ | $COOC_2H_5$ | $CH_3$ | N | CH | $C-CH_3$ | 222 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 260 | 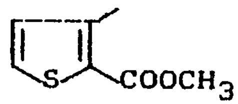 | H | $C_6H_5$ | $CH_3$ | N | CH | $C-CH_3$ | 222 |
| 261 | 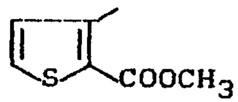 | H | $C_6H_5$ | $OCH_3$ | N | CH | $C-OCH_3$ | 230 |
| 262 | 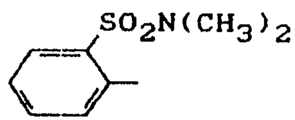 | H | $C_6H_5$ | $OCH_3$ | N | CH | $C-OCH_3$ | 239 |
| 263 | 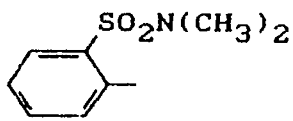 | H | $C_6H_5$ | H | N | CH | $C-CH_3$ | 212 |
| 264 | 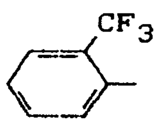 | H | $C_6H_5$ | $OCH_3$ | N | CH | $C-OCH_3$ | 229 |
| 265 | 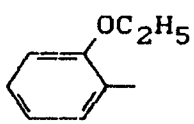 | H | $C_6H_5$ | $CH_3$ | N | CH | $C-CH_3$ | 210 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 266 | 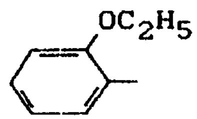 | H | $C_6H_5$ | $OCH_3$ | N | CH | $C-OCH_3$ | 203 |
| 267 | 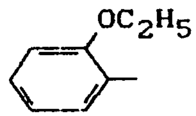 | H | $C_6H_5$ | $CH_3$ | N | CH | CH | 186 |
| 268 | 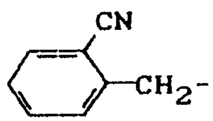 | H | $C_6H_5$ | $CH_3$ | N | CH | $C-OCH_3$ | 213 |
| 269 | 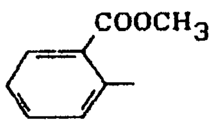 | H | $N(CH_3)_2$ | $CH_3$ | N | CH | $C-CH_3$ | 136 |
| 270 | 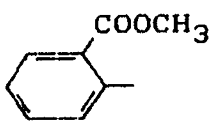 | $CH_3$ | $CH_3$ | $CH_3$ | N | CH | CH | 185 |
| 271 | 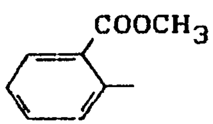 | H | $C_6H_5$ | $CH_3$ | N | CH | $C-CH_3$ | 244 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 272 | 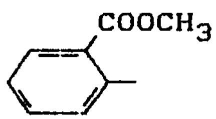 | $-(CH_2)_5-$ | | $CH_3$ | N | CH | $C-CH_3$ | 182 |
| 273 | 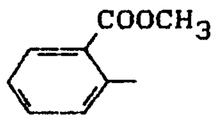 | H | $CH(CH_3)_2$ | $CH_3$ | N | CH | $C-CH_3$ | 171 |
| 274 | 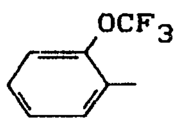 | H | $C_6H_5$ | $CH_3$ | N | CH | $C-CH_3$ | $^1H$-NMR: $\delta$ = 13,5 ppm (N<u>H</u>; in $CDCl_3$) |
| 275 | 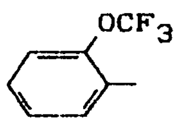 | $-(CH_2)_5-$ | | $CH_3$ | N | CH | $C-CH_3$ | $^1H$-NMR: $\delta$ = 13,05 ppm (N<u>H</u>; in $CDCl_3$) |
| 276 | 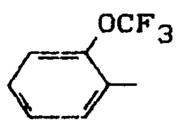 | H | $CH(CH_3)_2$ | $CH_3$ | N | CH | $C-CH_3$ | 205 |
| 277 | 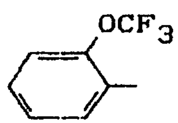 | H | $C_6H_5$ | $CH_3$ | N | CH | CH | 200 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 278 | 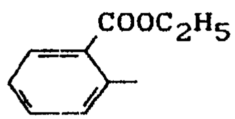 | H | $C_6H_5$ | $CH_3$ | N | CH | CH | 198 |
| 279 | 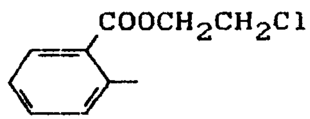 | H | $C_6H_5$ | $CH_3$ | N | CH | CH | 180 |
| 280 | 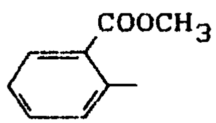 | $CH_3$ | $CH_3$ | $OCH_3$ | N | CH | $C-OCH_3$ | 200 |
| 281 | 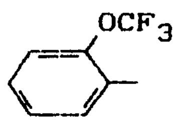 | H | $C_6H_5$ | $OCH_3$ | N | CH | $C-OCH_3$ | 218 |
| 282 | 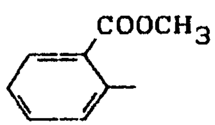 | H | 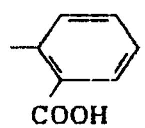 | $CH_3$ | N | CH | $C-CH_3$ | 240 |
| 283 | 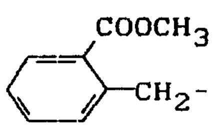 | $CH_3$ | $CH_3$ | $OCH_3$ | N | CH | $C-OCH_3$ | 176 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 284 | 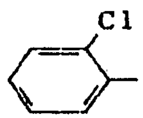 | $CH_3$ | $CH_3$ | $CH_3$ | N | CH | $C-CH_3$ | 223 |
| 285 | 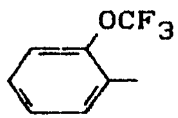 | $CH_3$ | $CH_3$ | $CH_3$ | N | CH | $C-CH_3$ | 225 |
| 286 | 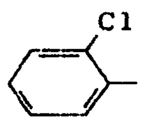 | $CH_3$ | $CH_3$ | $OCH_3$ | N | N | $C-OCH_3$ | 135 |
| 287 | 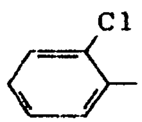 | H | 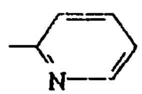 | $OCH_3$ | N | N | $C-OCH_3$ | 183 |
| 288 | 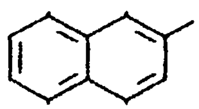 | $CH_3$ | $CH_3$ | $OCH_3$ | N | N | $C-OCH_3$ | 182 |
| 289 | 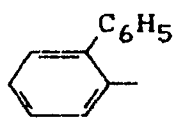 | H | $C_6H_5$ | $OCH_3$ | N | N | $C-OCH_3$ | 204 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 290 | 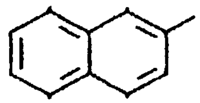 | H | $C_6H_5$ | $OCH_3$ | N | N | $C-OCH_3$ | 191 |
| 291 | 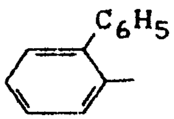 | $CH_3$ | $CH_3$ | $OCH_3$ | N | N | $C-OCH_3$ | 147 |
| 292 | 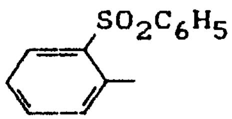 | H | $C_6H_5$ | $OCH_3$ | N | N | $C-OCH_3$ | 229 (Zers.) |
| 293 | 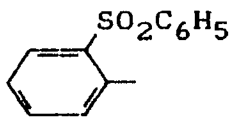 | $CH_3$ | $CH_3$ | $OCH_3$ | N | N | $C-OCH_3$ | 210 |
| 294 | 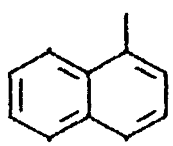 | H | $C_6H_5$ | $OCH_3$ | N | N | $C-OCH_3$ | 209 |
| 295 | 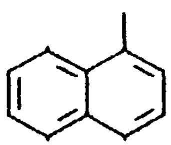 | $CH_3$ | $CH_3$ | $OCH_3$ | N | N | $C-OCH_3$ | 185 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 296 | 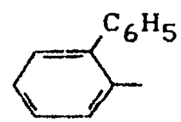 | H | $N(CH_3)_2$ | $OCH_3$ | N | N | $C-OCH_3$ | 130 |
| 297 | 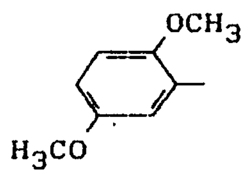 | H | $C_6H_5$ | $OCH_3$ | N | N | $C-OCH_3$ | 219 |
| 298 | 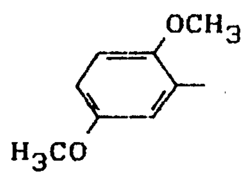 | $CH_3$ | $CH_3$ | $OCH_3$ | N | N | $C-OCH_3$ | 185 |
| 299 | 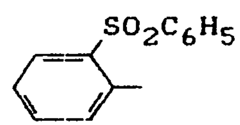 | H | $N(CH_3)_2$ | $OCH_3$ | N | N | $C-OCH_3$ | 157 |
| 300 | 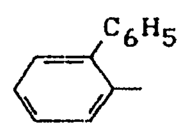 | $CH_3$ | $CH_3$ | $CH_3$ | N | CH | $C-CH_3$ | 242 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 301 | 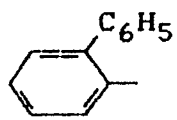 | H | $C_6H_5$ | H | N | CH | CH | 214 |
| 302 | 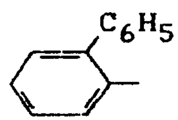 | H | $N(CH_3)_2$ | $CH_3$ | N | CH | $C-CH_3$ | 144 |
| 303 | 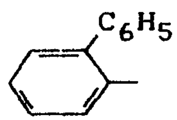 | H | $C_6H_5$ | $OCH_3$ | N | CH | $C-OCH_3$ | 206 |
| 304 | 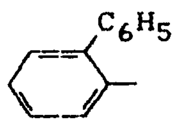 | $CH_3$ | $CH_3$ | $OCH_3$ | N | CH | $C-OCH_3$ | 181 |
| 305 | 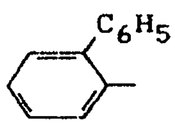 | $CH_3$ | $CH_3$ | $CH_3$ | N | CH | $C-OCH_3$ | 192 |
| 306 | 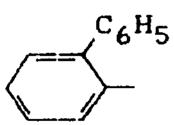 | H | $C_6H_5$ | $CH_3$ | N | CH | $C-OCH_3$ | 174 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 307 | 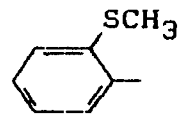 | H | $C_6H_5$ | $OCH_3$ | N | CH | $C-OCH_3$ | 214 |
| 308 | 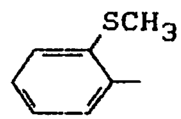 | $CH_3$ | $CH_3$ | $OCH_3$ | N | CH | $C-OCH_3$ | 216 |
| 309 | 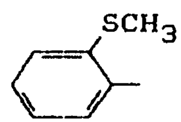 | H | $C_6H_5$ | $CH_3$ | N | CH | $C-CH_3$ | 228 |
| 310 | 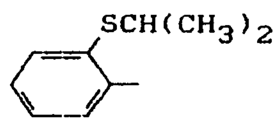 | $CH_3$ | $CH_3$ | $OCH_3$ | N | CH | $C-OCH_3$ | 153 |
| 311 | 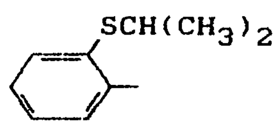 | H | $C_6H_5$ | $OCH_3$ | N | CH | $C-OCH_3$ | 228 |
| 312 | 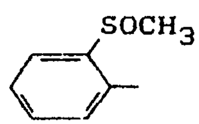 | $CH_3$ | $CH_3$ | $OCH_3$ | N | CH | $C-OCH_3$ | 178 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 313 | 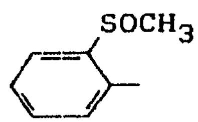 | H | $C_6H_5$ | $OCH_3$ | N | CH | $C-OCH_3$ | 204 |
| 314 | 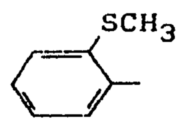 | $CH_3$ | $CH_3$ | $OCH_3$ | N | N | $C-OCH_3$ | 197 |
| 315 | 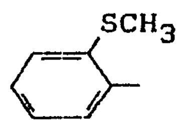 | H | $C_6H_5$ | $OCH_3$ | N | N | $C-OCH_3$ | 199 |
| 316 | 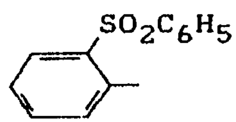 | $CH_3$ | $CH_3$ | $CH_3$ | N | N | $C-OCH_3$ | 144 |
| 317 | 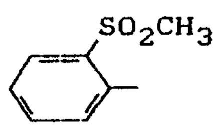 | $CH_3$ | $CH_3$ | $OCH_3$ | N | N | $C-OCH_3$ | 175 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 318 | 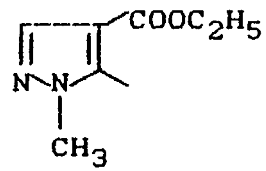 | H | 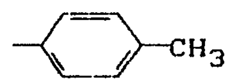 | $OCH_3$ | N | CH | $C-OCH_3$ | 208 |
| 319 | 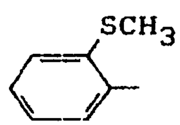 | $CH_3$ | $CH_3$ | $CH_3$ | N | CH | $C-CH_3$ | 203 |
| 320 | 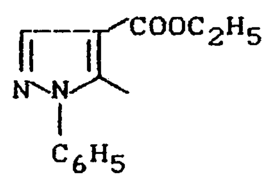 | H | $C_6H_5$ | $CH_3$ | N | CH | $C-CH_3$ | 197 |
| 321 | 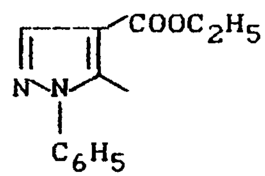 | $CH_3$ | $CH_3$ | $CH_3$ | N | CH | $C-OCH_3$ | 186 |
| 322 | 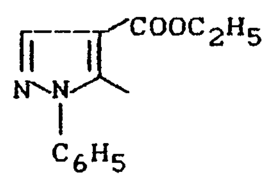 | H | $C_6H_5$ | $CH_3$ | N | CH | $C-OCH_3$ | 173 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 323 | 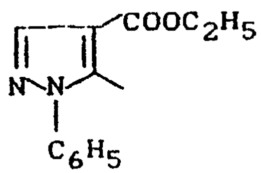 | $CH_3$ | $CH_3$ | $CH_3$ | N | CH | $C-CH_3$ | 170 |
| 324 | 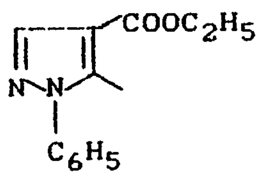 | $CH_3$ | $C_2H_5$ | $CH_3$ | N | CH | $C-CH_3$ | 152 |
| 325 | 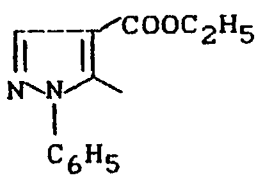 | $-(CH_2)_5-$ | | $CH_3$ | N | CH | $C-CH_3$ | 169 |
| 326 | 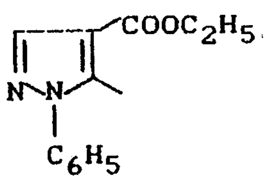 | H | 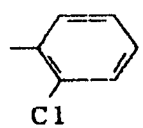 | $CH_3$ | N | CH | $C-CH_3$ | 161 |
| 327 | 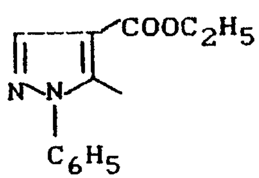 | H | 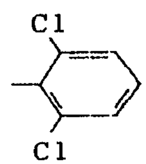 | $CH_3$ | N | CH | $C-CH_3$ | 140 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 328 | 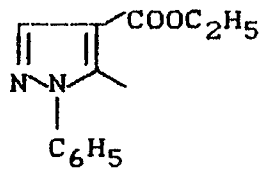 | H | 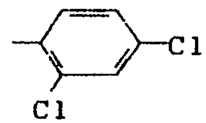 | $CH_3$ | N | CH | $C-CH_3$ | 218 |
| 329 | 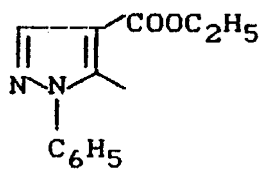 | H | 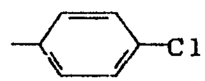 | $CH_3$ | N | CH | $C-CH_3$ | 178 |
| 330 | 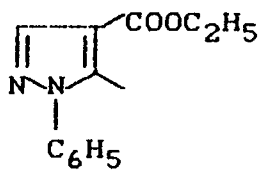 | H | 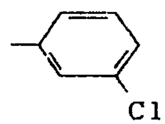 | $CH_3$ | N | CH | $C-CH_3$ | 168 |
| 331 | 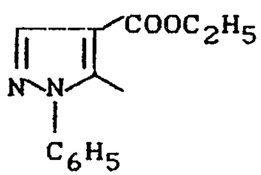 | H | 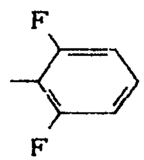 | $CH_3$ | N | CH | $C-CH_3$ | 159 |
| 332 | 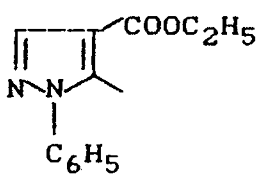 | H | 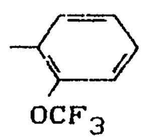 | $CH_3$ | N | CH | $C-CH_3$ | 137 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 333 | 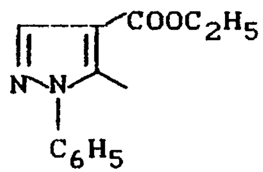 | H | 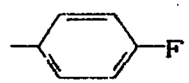 | $CH_3$ | N | CH | $C-CH_3$ | 198 |
| 334 | 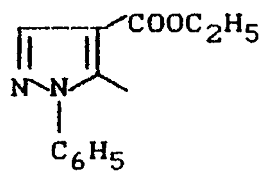 | H | 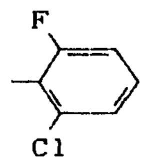 | $CH_3$ | N | CH | $C-CH_3$ | 145 |
| 335 | 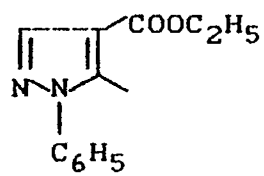 | H | 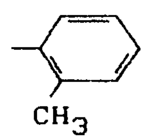 | $CH_3$ | N | CH | $C-CH_3$ | 164 |
| 336 | 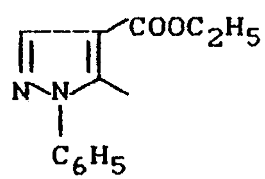 | $CH_3$ | $C_2H_5$ | $CH_3$ | N | CH | $C-OCH_3$ | 189 |
| 337 | 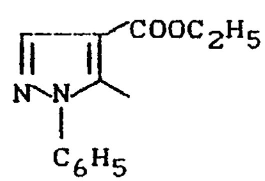 | $-(CH_2)_5-$ | | $CH_3$ | N | CH | $C-OCH_3$ | 161 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 338 | 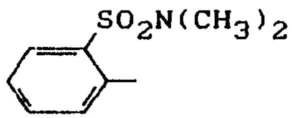 | H | 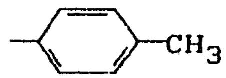 | $CH_3$ | N | N | $C-OCH_3$ | 246 (Zers.) |
| 339 | 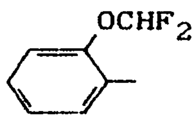 | H | 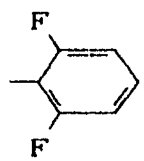 | $CH_3$ | N | N | $C-OCH_3$ | 189 |
| 340 | 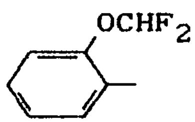 | H | 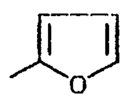 | $CH_3$ | N | N | $C-OCH_3$ | 205 |
| 341 | 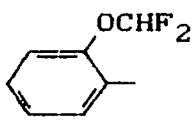 | H | 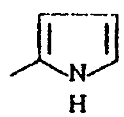 | $CH_3$ | N | N | $C-OCH_3$ | 195 |
| 342 | 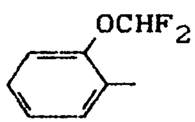 | H | 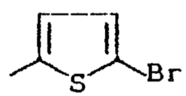 | $CH_3$ | N | N | $C-OCH_3$ | 195 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 343 | 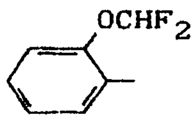 | H | 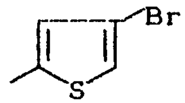 | $CH_3$ | N | N | $C-OCH_3$ | 229 |
| 344 | 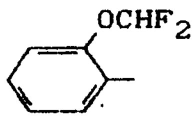 | H | 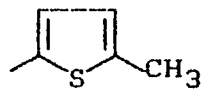 | $CH_3$ | N | N | $C-OCH_3$ | 187 |
| 345 | 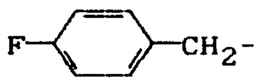 | H | $C_6H_5$ | $CH_3$ | N | N | $C-OCH_3$ | 188 |
| 346 | 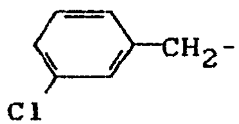 | H | $C_6H_5$ | $CH_3$ | N | N | $C-OCH_3$ | 207 |
| 347 | 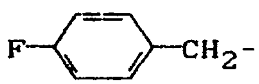 | $CH_3$ | $CH_3$ | $CH_3$ | N | N | $C-OCH_3$ | 189 |
| 348 | 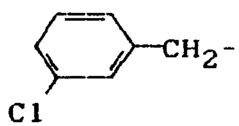 | $CH_3$ | $CH_3$ | $CH_3$ | N | N | $C-OCH_3$ | 204 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 349 | 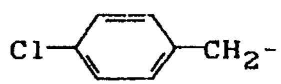 | H | $C_6H_5$ | $CH_3$ | N | N | $C-OCH_3$ | 215 |
| 350 | 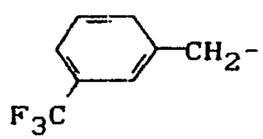 | H | $C_6H_5$ | $CH_3$ | N | N | $C-OCH_3$ | 225 |
| 351 | 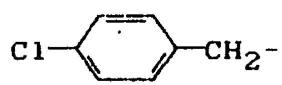 | $CH_3$ | $CH_3$ | $CH_3$ | N | N | $C-OCH_3$ | 156 |
| 352 | 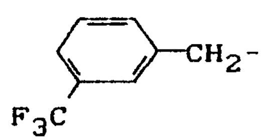 | $CH_3$ | $CH_3$ | $CH_3$ | N | N | $C-OCH_3$ | 174 |
| 353 | 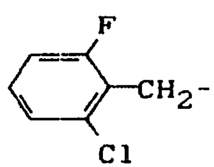 | H | $C_6H_5$ | $CH_3$ | N | N | $C-OCH_3$ | 191 |
| 354 | 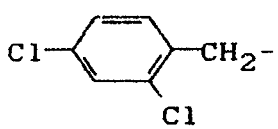 | H | $C_6H_5$ | $CH_3$ | N | N | $C-OCH_3$ | 190 |

Tabelle 3 - Fortsetzung

| Bsp.-Nr. | $R^1$ | $R^2$ | $R^3$ | $R^4$ | X | Y | Z | Schmelzpunkt ($^0C$) |
|---|---|---|---|---|---|---|---|---|
| 355 | 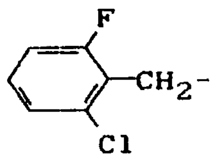 | $CH_3$ | $CH_3$ | $CH_3$ | N | N | $C-OCH_3$ | 198 |
| 356 | 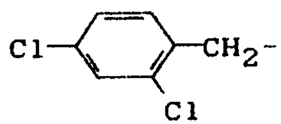 | $CH_3$ | $CH_3$ | $CH_3$ | N | N | $C-OCH_3$ | 176 |
| 357 | 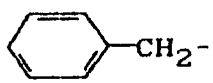 | H | $C_6H_5$ | $OCH_3$ | N | N | $C-OCH_3$ | 175 |
| 358 | 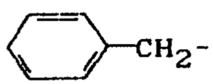 | $CH_3$ | $CH_3$ | $OCH_3$ | N | N | $C-OCH_3$ | 189 |
| 359 | 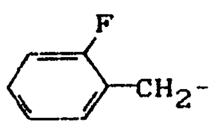 | H | $C_6H_5$ | $OCH_3$ | N | N | $C-OCH_3$ | 174 |
| 360 | 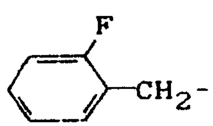 | $CH_3$ | $CH_3$ | $OCH_3$ | N | N | $C-OCH_3$ | 185 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 361 | 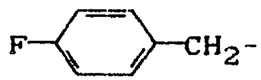 | H | $C_6H_5$ | $OCH_3$ | N | N | $C-OCH_3$ | 190 |
| 362 | 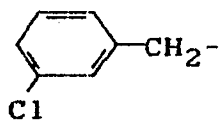 | H | $C_6H_5$ | $OCH_3$ | N | N | $C-OCH_3$ | 164 |
| 363 | 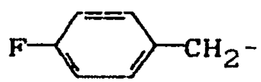 | $CH_3$ | $CH_3$ | $OCH_3$ | N | N | $C-OCH_3$ | 189 |
| 364 | 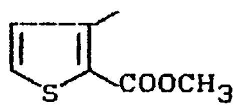 | H | 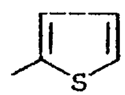 | $CH_3$ | N | N | $C-OCH_3$ | 193 (Zers.) |
| 365 | 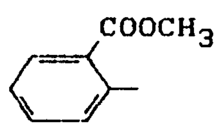 | H | 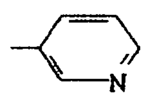 | $CH_3$ | N | N | $C-OCH_3$ | 202 (Zers.) |
| 366 | 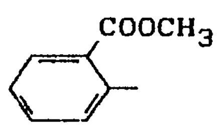 | H | 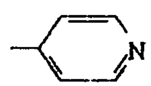 | $CH_3$ | N | N | $C-OCH_3$ | 179 (Zers.) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 367 | 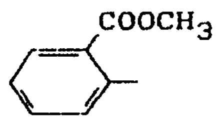 | H | 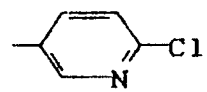 | $CH_3$ | N | N | $C-OCH_3$ | 199 (Zers.) |
| 368 | 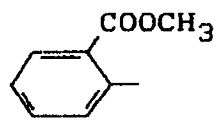 | H | 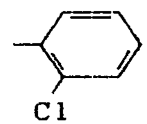 | $CH_3$ | N | N | $C-OCH_3$ | 203 |
| 369 | 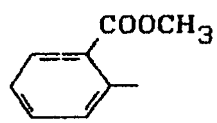 | H | 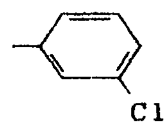 | $CH_3$ | N | N | $C-OCH_3$ | 206 |
| 370 | 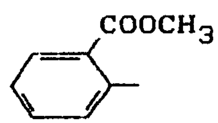 | H | 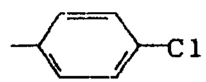 | $CH_3$ | N | N | $C-OCH_3$ | 203 |
| 371 | 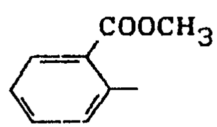 | H | 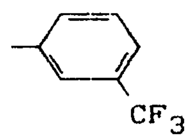 | $CH_3$ | N | N | $C-OCH_3$ | 191 |
| 372 | 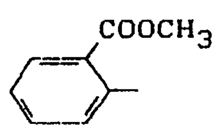 | H | 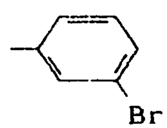 | $CH_3$ | N | N | $C-OCH_3$ | 194 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 373 | 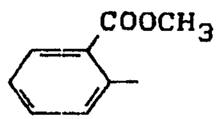 | H | 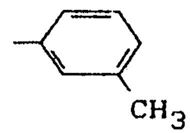 | $CH_3$ | N | N | $C-OCH_3$ | 182 |
| 374 | 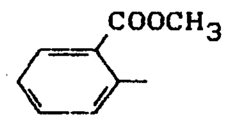 | H | 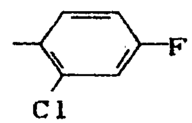 | $CH_3$ | N | N | $C-OCH_3$ | 192 |
| 375 | 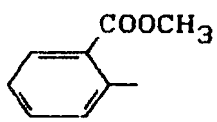 | H | 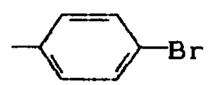 | $CH_3$ | N | N | $C-OCH_3$ | 231 (Zers.) |
| 376 | 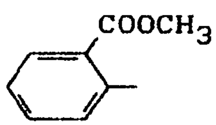 | H | 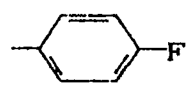 | $CH_3$ | N | N | $C-OCH_3$ | 219 |
| 377 | 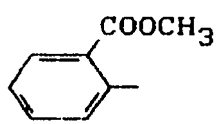 | H | 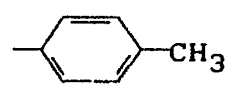 | $CH_3$ | N | N | $C-OCH_3$ | 227 (Zers.) |
| 378 | 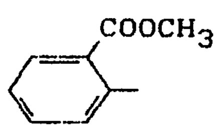 | H | 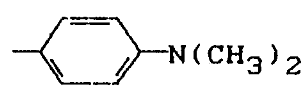 | $CH_3$ | N | N | $C-OCH_3$ | 213 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 379 | 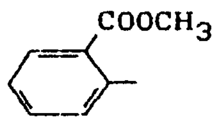 | H | 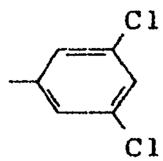 | $CH_3$ | N | N | $C-OCH_3$ | 205 |
| 380 | 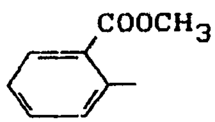 | H | 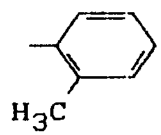 | $CH_3$ | N | N | $C-OCH_3$ | 210 |
| 381 | 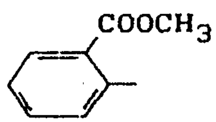 | H | 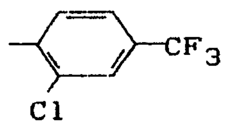 | $CH_3$ | N | N | $C-OCH_3$ | 221 |
| 382 | 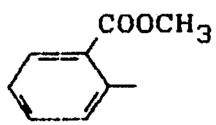 | H | 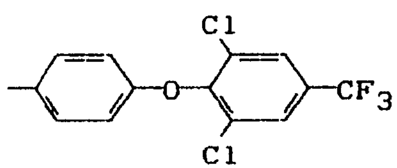 | $CH_3$ | N | N | $C-OCH_3$ | 198 |
| 383 | 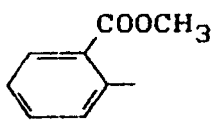 | H | 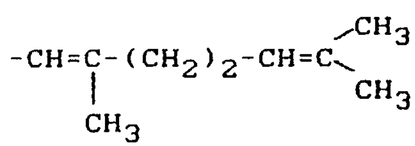 | $CH_3$ | N | N | $C-OCH_3$ | 109 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 384 | 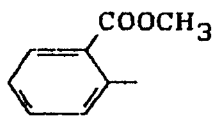 | H | -CH=CH- 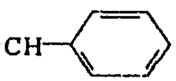 | $CH_3$ | N | N | $C-OCH_3$ | 209 |
| 385 | 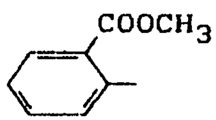 | $CH_3$ | 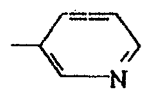 | $CH_3$ | N | N | $C-OCH_3$ | 207 |
| 386 | 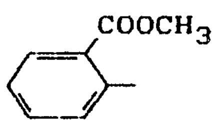 | H | 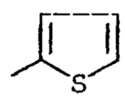 | $CH_3$ | N | N | $C-OCH_3$ | 206 (Zers.) |
| 387 | 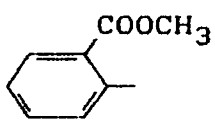 | $CH_3$ | 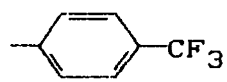 | $CH_3$ | N | N | $C-OCH_3$ | 196 (Zers.) |
| 388 | 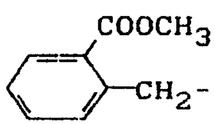 | H | $C_6H_5$ | $CH_3$ | N | CH | $C-OCH_3$ | 180 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 389 | 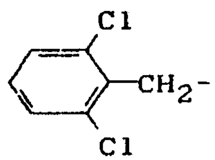 | H | $C_6H_5$ | $CH_3$ | N | CH | $C-OCH_3$ | 146 |
| 390 | 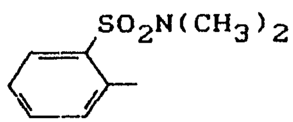 | H | $C_6H_5$ | $CH_3$ | N | CH | $C-OCH_3$ | 206 |
| 391 | 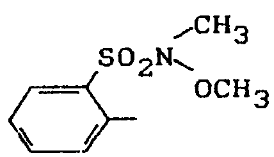 | H | $C_6H_5$ | $CH_3$ | N | CH | $C-OCH_3$ | 217 |
| 392 | 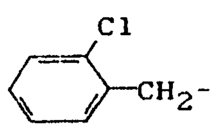 | H | $C_6H_5$ | $CH_3$ | N | CH | CH | 188 |

**Tabelle 3 - Fortsetzung**

| Bsp.-Nr. | $R^1$ | $R^2$ | $R^3$ | $R^4$ | X | Y | Z | Schmelzpunkt (°C) |
|---|---|---|---|---|---|---|---|---|
| 393 | 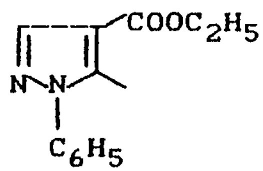 | H | $C_6H_5$ | $OCH_3$ | N | CH | $C-OCH_3$ | 198 |
| 394 | 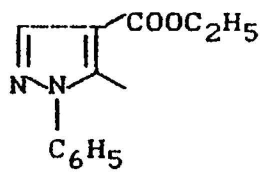 | $CH_3$ | $CH_3$ | $OCH_3$ | N | CH | $C-OCH_3$ | 173 |
| 395 | 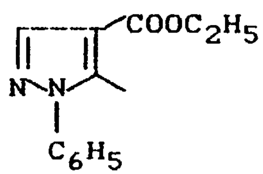 | $CH_3$ | $C_2H_5$ | $OCH_3$ | N | CH | $C-OCH_3$ | 149 |
| 396 | 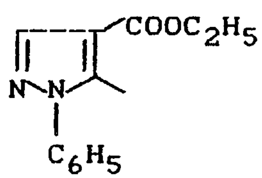 | H | 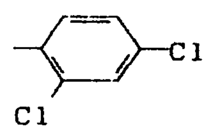 | $OCH_3$ | N | CH | $C-OCH_3$ | 238 |
| 397 | 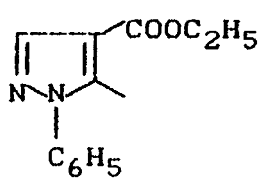 | H | 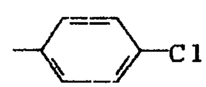 | $OCH_3$ | N | CH | $C-OCH_3$ | 208 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 398 | 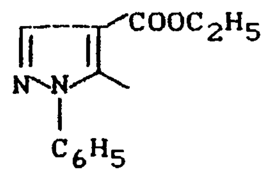 | H | 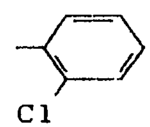 | $OCH_3$ | N | CH | $C-OCH_3$ | 173 |
| 399 | 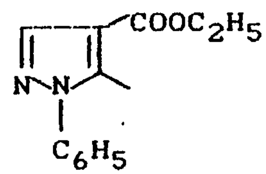 | H | 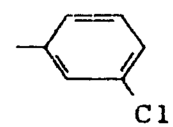 | $OCH_3$ | N | CH | $C-OCH_3$ | 181 |
| 400 | 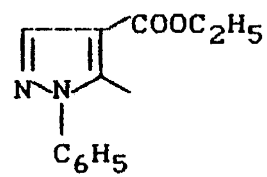 | H | 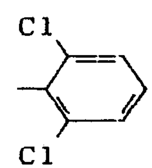 | $OCH_3$ | N | CH | $C-OCH_3$ | 145 |
| 401 | 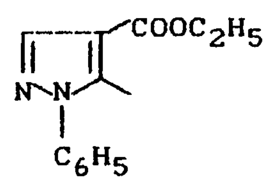 | H | 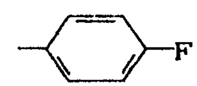 | $OCH_3$ | N | CH | $C-OCH_3$ | 206 |
| 402 | 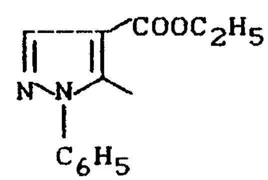 | H | 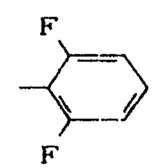 | $OCH_3$ | N | CH | $C-OCH_3$ | 176 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 403 | 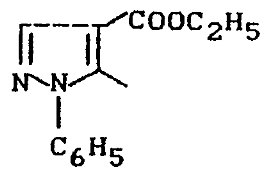 | H | 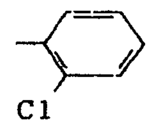 | $OCH_3$ | N | CH | $C-CH_3$ | 152 |
| 404 | 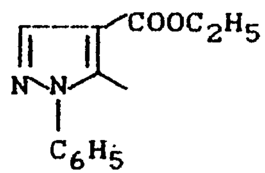 | H | 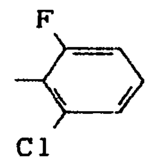 | $OCH_3$ | N | CH | $C-OCH_3$ | 175 |
| 405 | 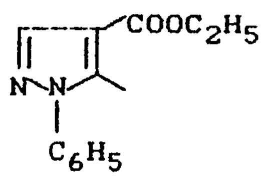 | H | 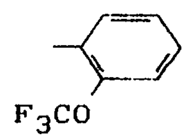 | $OCH_3$ | N | CH | $C-OCH_3$ | 154 |
| 406 | 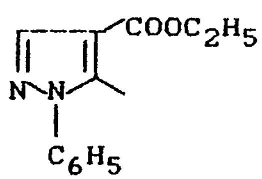 | H | 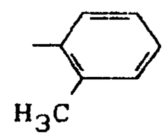 | $OCH_3$ | N | CH | $C-OCH_3$ | 161 |
| 407 | 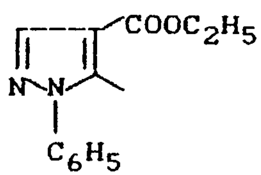 | H | 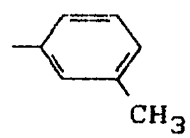 | $OCH_3$ | N | CH | $C-OCH_3$ | 159 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 408 | 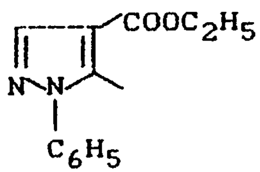 | H | 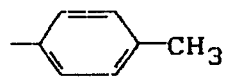 | $OCH_3$ | N | CH | $C-OCH_3$ | 195 |
| 409 | 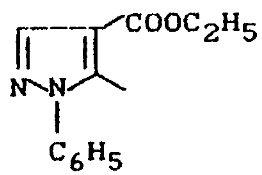 | H | 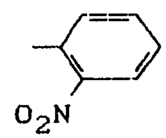 | $OCH_3$ | N | CH | $C-OCH_3$ | 123 |
| 410 | 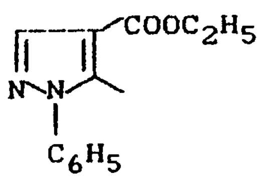 | H | 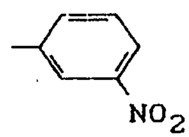 | $OCH_3$ | N | CH | $C-OCH_3$ | 196 |
| 411 | 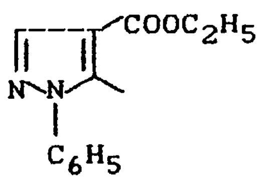 | H | 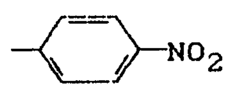 | $OCH_3$ | N | CH | $C-OCH_3$ | 225 |
| 412 | 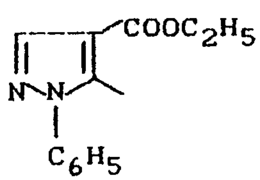 | H | 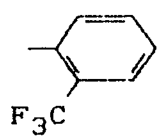 | $OCH_3$ | N | CH | $C-OCH_3$ | 177 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 413 | 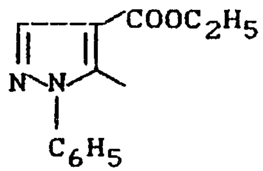 | H | 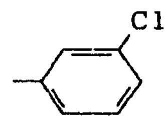 | $OCH_3$ | N | CH | $C-CH_3$ | 166 |
| 414 | 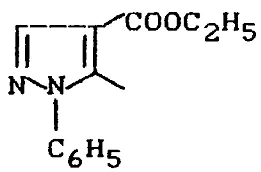 | H | 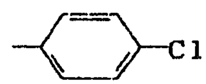 | $OCH_3$ | N | CH | $C-CH_3$ | 165 |
| 415 | 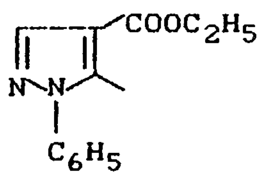 | H | 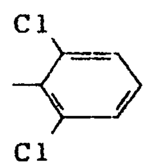 | $OCH_3$ | N | CH | $C-CH_3$ | 118 |
| 416 | 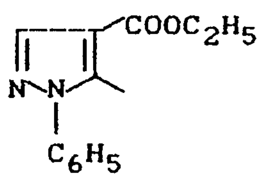 | H | 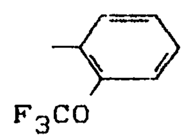 | $OCH_3$ | N | CH | $C-CH_3$ | 139 |
| 417 | 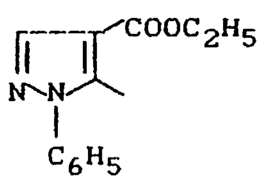 | H | 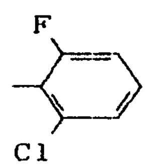 | $OCH_3$ | N | CH | $C-CH_3$ | 147 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 418 | 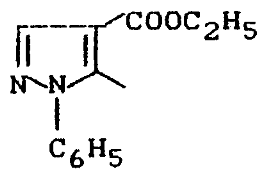 | H | 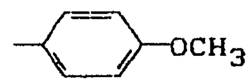 | $OCH_3$ | N | CH | $C-OCH_3$ | 173 |
| 419 | 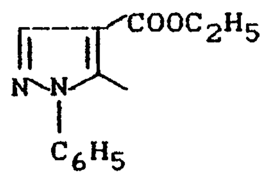 | H | 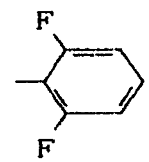 | $OCH_3$ | N | CH | $C-CH_3$ | 189 |
| 420 | 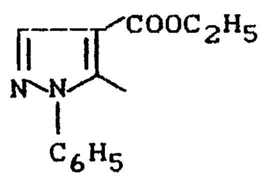 | H | 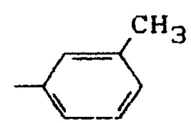 | $OCH_3$ | N | CH | $C-CH_3$ | 143 |
| 421 | 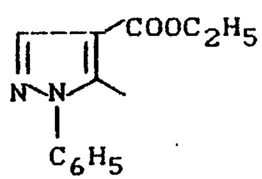 | H | 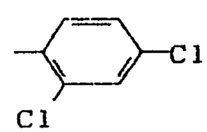 | $OCH_3$ | N | CH | $C-CH_3$ | 232 |
| 422 | 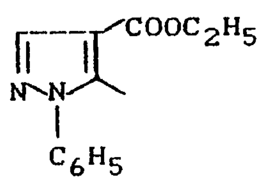 | $-(CH_2)_5-$ | | $OCH_3$ | N | CH | $C-OCH_3$ | 135 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 423 | 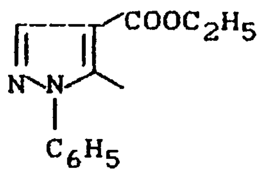 | H | 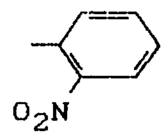 | $OCH_3$ | N | CH | $C-CH_3$ | 152 |
| 424 | 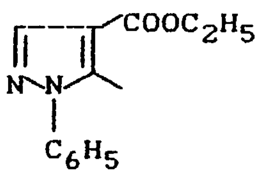 | H | 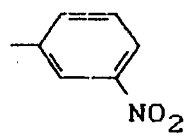 | $OCH_3$ | N | CH | $C-CH_3$ | 235 |
| 425 | 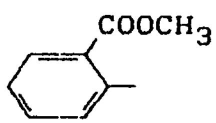 | H | $CH(CH_3)_2$ | $OCH_3$ | N | N | $C-OCH_3$ | 121 |
| 426 | 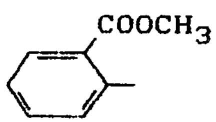 | H | $C_6H_5$ | $OCH_3$ | N | N | $C-OCH_3$ | 209 |
| 427 | 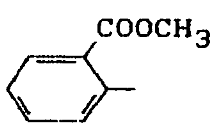 | H | 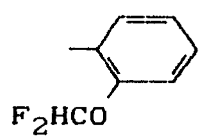 | $OCH_3$ | N | N | $C-OCH_3$ | 173 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 428 | 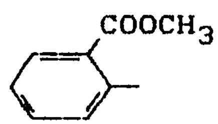 | H | 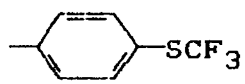 | $OCH_3$ | N | N | $C-OCH_3$ | 213 |
| 429 | 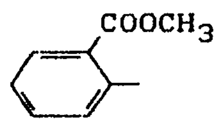 | H | 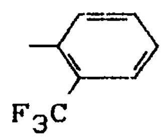 | $OCH_3$ | N | N | $C-OCH_3$ | 187 |
| 430 | 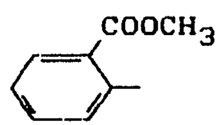 | H | 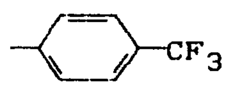 | $OCH_3$ | N | N | $C-OCH_3$ | 139 |
| 431 | 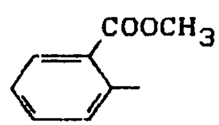 | H | 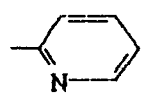 | $OCH_3$ | N | N | $C-OCH_3$ | 168 |
| 432 | 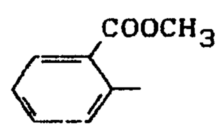 | H | 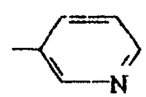 | $OCH_3$ | N | N | $C-OCH_3$ | 188 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 433 | 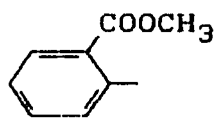 | H | 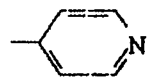 | $OCH_3$ | N | N | C-$OCH_3$ | 189 |
| 434 | 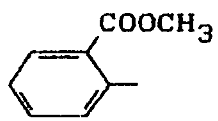 | H | 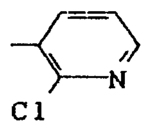 | $OCH_3$ | N | N | C-$OCH_3$ | 207 |
| 435 | 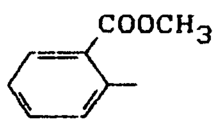 | H | 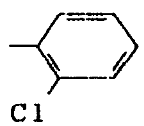 | $OCH_3$ | N | N | C-$OCH_3$ | 219 |
| 436 | 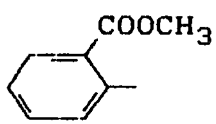 | H | 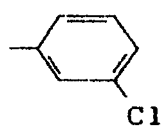 | $OCH_3$ | N | N | C-$OCH_3$ | 217 |
| 437 | 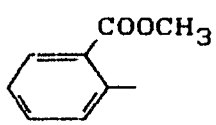 | H | 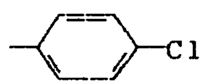 | $OCH_3$ | N | N | C-$OCH_3$ | 225 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 438 | 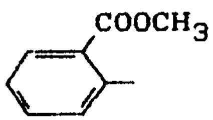 | H | 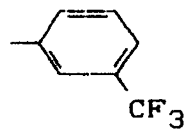 | $OCH_3$ | N | N | C-$OCH_3$ | 141 |
| 439 | 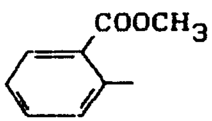 | H | 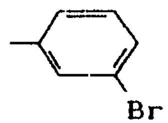 | $OCH_3$ | N | N | C-$OCH_3$ | 224 |
| 440 | 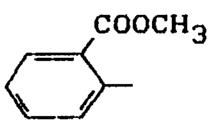 | H | 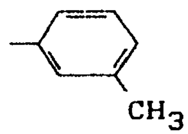 | $OCH_3$ | N | N | C-$OCH_3$ | 224 |
| 441 | 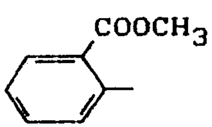 | H | 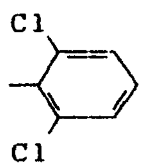 | $OCH_3$ | N | N | C-$OCH_3$ | 182 |
| 442 | 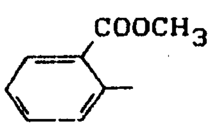 | H | 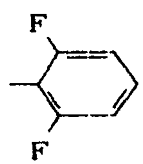 | $OCH_3$ | N | N | C-$OCH_3$ | 188 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 443 | 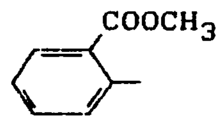 | H | 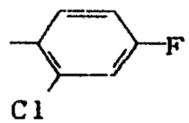 | $OCH_3$ | N | N | $C-OCH_3$ | 216 |
| 444 | 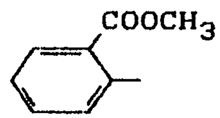 | H | 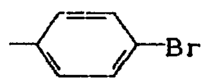 | $OCH_3$ | N | N | $C-OCH_3$ | 224 |
| 445 | 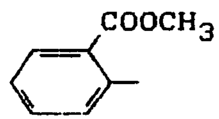 | H | 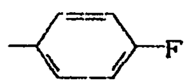 | $OCH_3$ | N | N | $C-OCH_3$ | 221 |
| 446 | 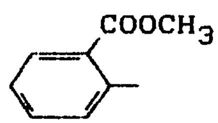 | H | 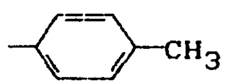 | $OCH_3$ | N | N | $C-OCH_3$ | 212 |
| 447 | 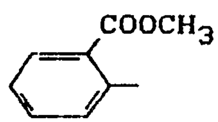 | H | 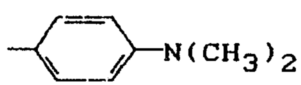 | $OCH_3$ | N | N | $C-OCH_3$ | 212 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 448 | 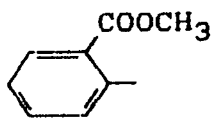 | H | 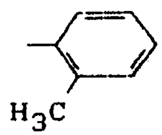 | $OCH_3$ | N | N | $C-OCH_3$ | 216 |
| 449 | 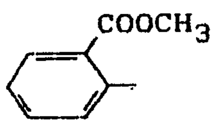 | H | 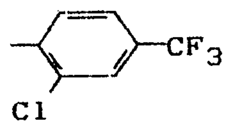 | $OCH_3$ | N | N | $C-OCH_3$ | 213 |
| 450 | 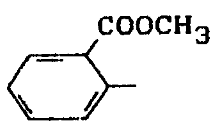 | H | 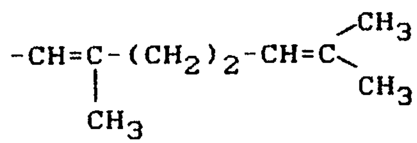 | $OCH_3$ | N | N | $C-OCH_3$ | 137 |
| 451 | 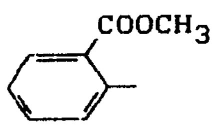 | H | 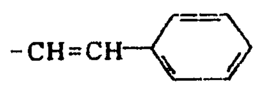 | $OCH_3$ | N | N | $C-OCH_3$ | 218 |
| 452 | 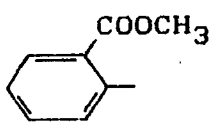 | H | 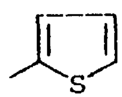 | $OCH_3$ | N | N | $C-OCH_3$ | 226 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 453 | 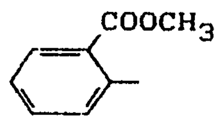 | H | 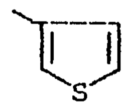 | $OCH_3$ | N | N | $C-OCH_3$ | 196 |
| 454 | 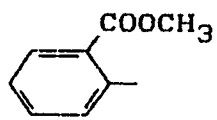 | H | 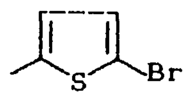 | $OCH_3$ | N | N | $C-OCH_3$ | 229 |
| 455 | 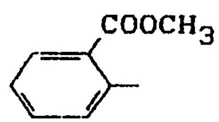 | H | 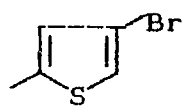 | $OCH_3$ | N | N | $C-OCH_3$ | 222 |
| 456 | 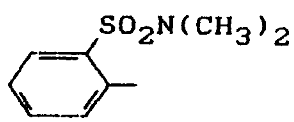 | H | 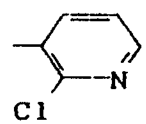 | $OCH_3$ | N | N | $C-CH_3$ | 188 |
| 457 | 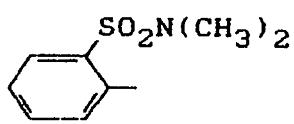 | H | 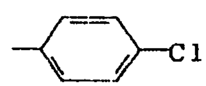 | $OCH_3$ | N | N | $C-CH_3$ | 224 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 458 | 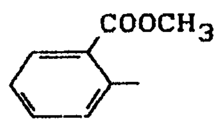 | H | 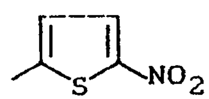 | $OCH_3$ | N | N | $C-OCH_3$ | 215 |
| 459 | 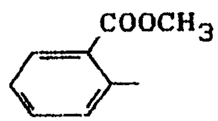 | H | 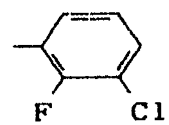 | $OCH_3$ | N | N | $C-OCH_3$ | 200 |
| 460 | 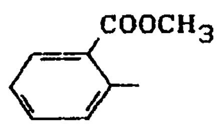 | H | 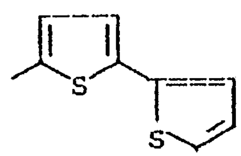 | $OCH_3$ | N | N | $C-OCH_3$ | 214 |
| 461 | 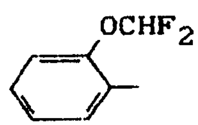 | H | 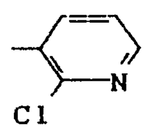 | $OCH_3$ | N | N | $C-CH_3$ | 114 |
| 462 | 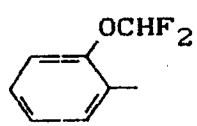 | H | 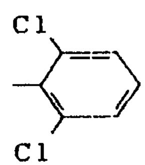 | $OCH_3$ | N | N | $C-CH_3$ | 86 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 463 | 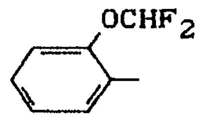 | | $-(CH_2)_5-$ | $OCH_3$ | N | N | $C-CH_3$ | 161 |
| 464 | 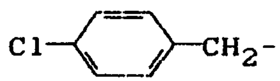 | H | $C_6H_5$ | $OCH_3$ | N | N | $C-OCH_3$ | 211 |
| 465 | 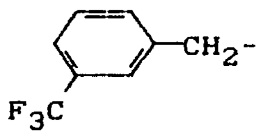 | H | $C_6H_5$ | $OCH_3$ | N | N | $C-OCH_3$ | 194 |
| 466 | 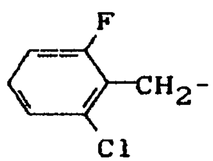 | H | $C_6H_5$ | $OCH_3$ | N | N | $C-OCH_3$ | 166 |
| 467 | 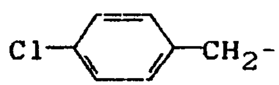 | $CH_3$ | $CH_3$ | $OCH_3$ | N | N | $C-OCH_3$ | 178 |
| 468 | 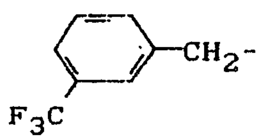 | $CH_3$ | $CH_3$ | $OCH_3$ | N | N | $C-OCH_3$ | 166 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 469 | 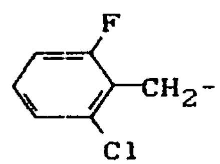 | $CH_3$ | $CH_3$ | $OCH_3$ | N | N | $C-OCH_3$ | 195 |
| 470 | 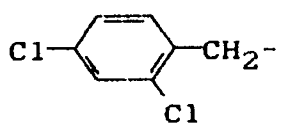 | H | $C_6H_5$ | $OCH_3$ | N | N | $C-OCH_3$ | 201 |
| 471 | 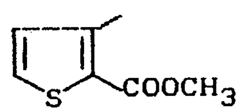 | H | 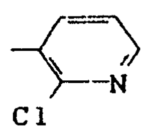 | $OCH_3$ | N | N | $C-CH_3$ | 165 |
| 472 | 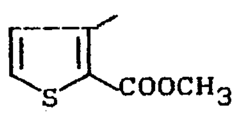 | H | 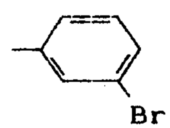 | $OCH_3$ | N | N | $C-CH_3$ | 212 |
| 473 | 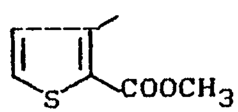 | H | 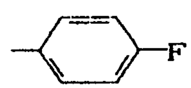 | $OCH_3$ | N | N | $C-CH_3$ | 225 |
| 474 | 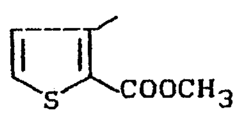 | H | 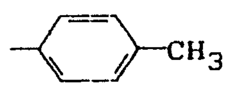 | $OCH_3$ | N | N | $C-CH_3$ | 224 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 475 | 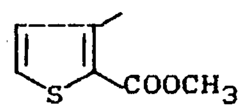 | H | 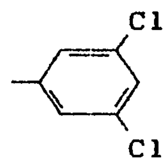 | $OCH_3$ | N | N | $C-CH_3$ | 246 |
| 476 | 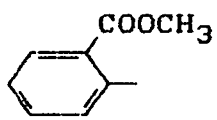 | H | 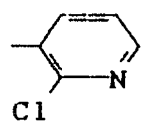 | $OCH_3$ | N | N | $C-CH_3$ | 150 |
| 477 | 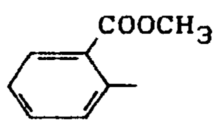 | H | 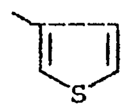 | $OCH_3$ | N | N | $C-CH_3$ | 195 (Zers.) |
| 478 | 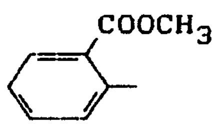 | H | 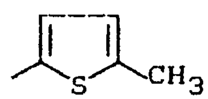 | $OCH_3$ | N | N | $C-CH_3$ | 173 (Zers.) |
| 479 | 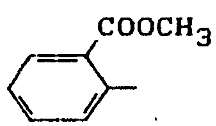 | H | 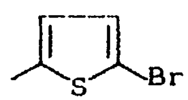 | $OCH_3$ | N | N | $C-CH_3$ | 229 (Zers.) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 480 | 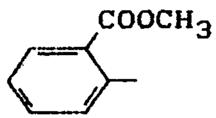 | H | 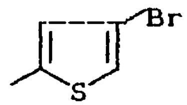 | $OCH_3$ | N | N | $C-CH_3$ | 234 |
| 481 | 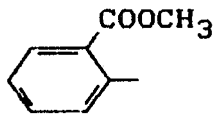 | H | 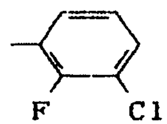 | $OCH_3$ | N | N | $C-CH_3$ | 167 |
| 482 | 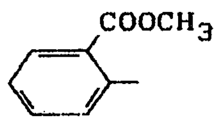 | H | 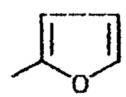 | $OCH_3$ | N | N | $C-CH_3$ | 196 |
| 483 | 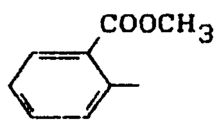 | H | 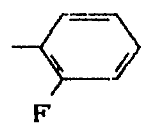 | $OCH_3$ | N | N | $C-CH_3$ | 201 |
| 484 | 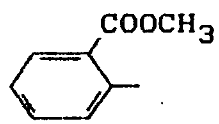 | H | 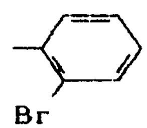 | $OCH_3$ | N | N | $C-CH_3$ | 210 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 485 | 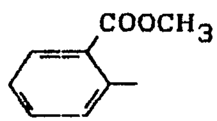 | H | 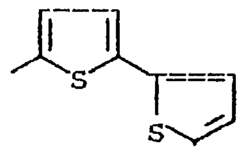 | $OCH_3$ | N | N | $C-CH_3$ | 250 |
| 486 | 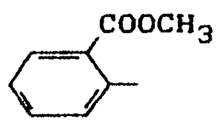 | H | 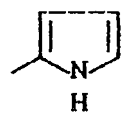 | $OCH_3$ | N | N | $C-CH_3$ | 225 |
| 487 | 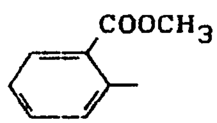 | $-(CH_2)_5-$ | | $OCH_3$ | N | N | $C-CH_3$ | 178 |
| 488 | 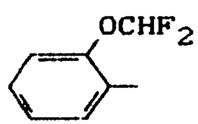 | H | 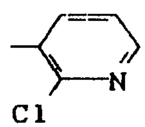 | $OCH_3$ | N | N | $C-OCH_3$ | 168 |
| 489 | 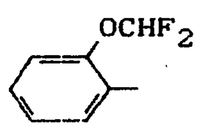 | H | $CH_3$ | $OCH_3$ | N | N | $C-OCH_3$ | 118 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 490 | 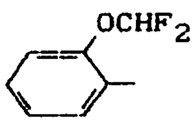 | H | $CH(CH_3)_2$ | $OCH_3$ | N | N | $C-OCH_3$ | 135 |
| 491 | 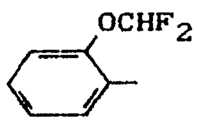 | H | $C_6H_5$ | $OCH_3$ | N | N | $C-OCH_3$ | 243 |
| 492 | 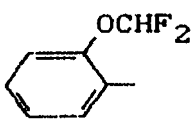 | H | 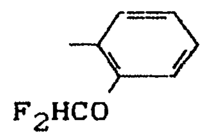 | $OCH_3$ | N | N | $C-OCH_3$ | 190 |
| 493 | 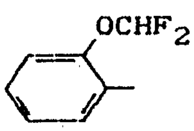 | H | 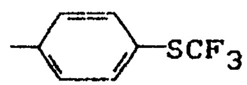 | $OCH_3$ | N | N | $C-OCH_3$ | 168 |
| 494 | 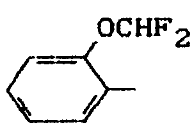 | H | 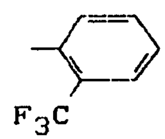 | $OCH_3$ | N | N | $C-OCH_3$ | 169 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 495 | 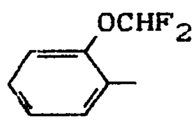 | H | 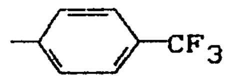 | $OCH_3$ | N | N | $C-OCH_3$ | 208 |
| 496 | 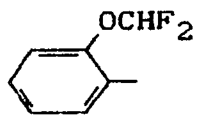 | H | 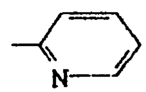 | $OCH_3$ | N | N | $C-OCH_3$ | 199 |
| 497 | 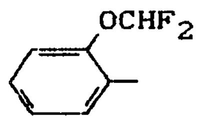 | H | 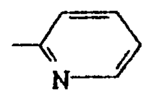 | $OCH_3$ | N | N | $C-OCH_3$ | 189 |
| 498 | 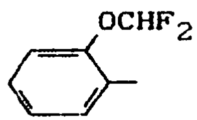 | H | 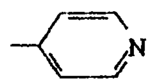 | $OCH_3$ | N | N | $C-OCH_3$ | 175 |
| 499 | 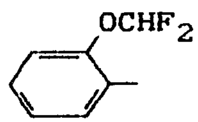 | H | 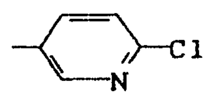 | $OCH_3$ | N | N | $C-OCH_3$ | 124 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 500 | 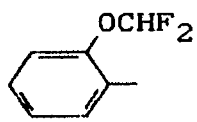 | H | 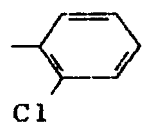 | $OCH_3$ | N | N | $C-OCH_3$ | 139 |
| 501 | 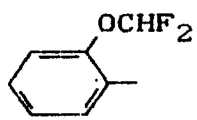 | H | 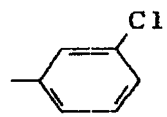 | $OCH_3$ | N | N | $C-OCH_3$ | 192 |
| 502 | 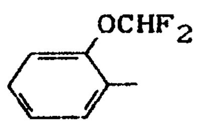 | H | 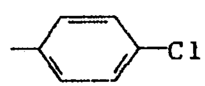 | $OCH_3$ | N | N | $C-OCH_3$ | 171 |
| 503 | 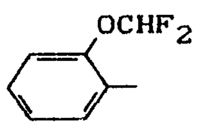 | H | 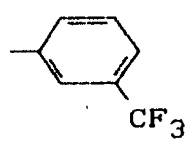 | $OCH_3$ | N | N | $C-OCH_3$ | 200 |
| 504 | 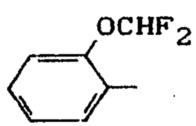 | H | 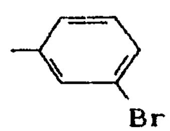 | $OCH_3$ | N | N | $C-OCH_3$ | 210 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 505 | 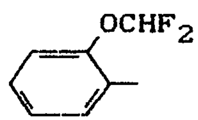 | H | 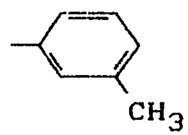 | $OCH_3$ | N | N | $C-OCH_3$ | 207 |
| 506 | 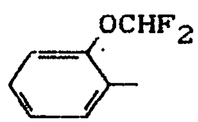 | H | 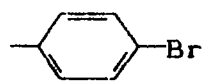 | $OCH_3$ | N | N | $C-OCH_3$ | 170 |
| 507 | 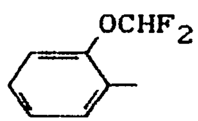 | H | 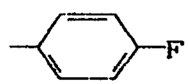 | $OCH_3$ | N | N | $C-OCH_3$ | 223 |
| 508 | 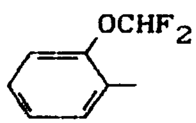 | H | 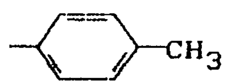 | $OCH_3$ | N | N | $C-OCH_3$ | 222 |
| 509 | 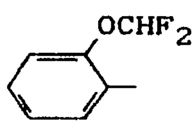 | H | 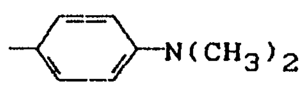 | $OCH_3$ | N | N | $C-OCH_3$ | 227 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 510 | 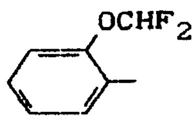 | H | 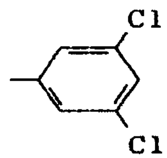 | $OCH_3$ | N | N | $C-OCH_3$ | 204 |
| 511 | 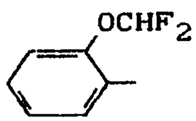 | H | 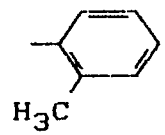 | $OCH_3$ | N | N | $C-OCH_3$ | 216 |
| 512 | 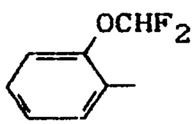 | H | 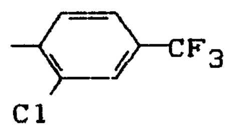 | $OCH_3$ | N | N | $C-OCH_3$ | 133 |
| 513 | 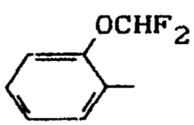 | H | 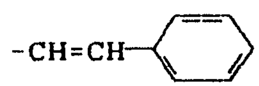 | $OCH_3$ | N | N | $C-OCH_3$ | 213 |
| 514 | 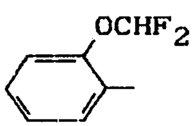 | H | 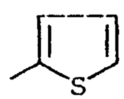 | $OCH_3$ | N | N | $C-OCH_3$ | 240 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 515 | 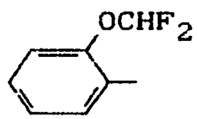 | H | 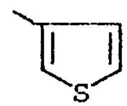 | $OCH_3$ | N | N | $C-OCH_3$ | 206 |
| 516 | 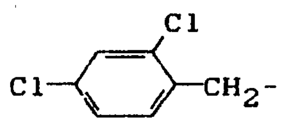 | $CH_3$ | $CH_3$ | $OCH_3$ | N | N | $C-OCH_3$ | 176 |
| 517 | 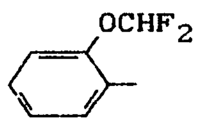 | H | 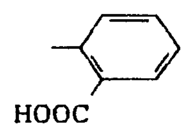 | $OCH_3$ | N | N | $C-OCH_3$ | 196 |
| 518 | 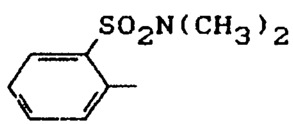 | H | 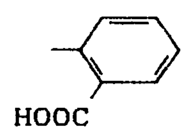 | $OCH_3$ | N | N | $C-CH_3$ | 180 |
| 519 | 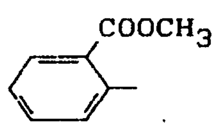 | H | 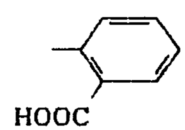 | $OCH_3$ | N | N | $C-CH_3$ | 184 |

<u>Tabelle 3</u> - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 520 | 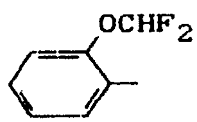 | H | 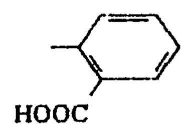 | $OCH_3$ | N | N | $C-CH_3$ | 176 |
| 521 | 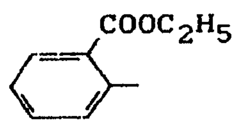 | H | 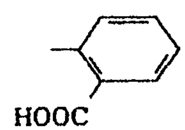 | $OCH_3$ | N | N | $C-CH_3$ | 212 |
| 522 | 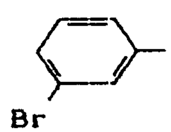 | $CH_3$ | $CH_3$ | $OCH_3$ | N | N | $C-OCH_3$ | 185 |
| 523 | 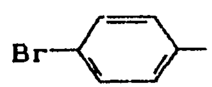 | $CH_3$ | $CH_3$ | $OCH_3$ | N | N | $C-OCH_3$ | 159 |
| 524 | 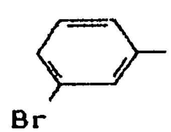 | H | $C_6H_5$ | $OCH_3$ | N | N | $C-OCH_3$ | 172 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 525 | 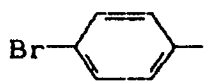 | H | $C_6H_5$ | $OCH_3$ | N | N | $C-OCH_3$ | 185 |
| 526 | 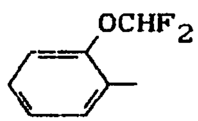 | H | 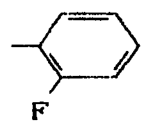 | $OCH_3$ | N | N | $C-OCH_3$ | 194 |
| 527 | 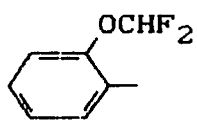 | $-(CH_2)_5-$ | | $OCH_3$ | N | N | $C-OCH_3$ | 187 |
| 528 | 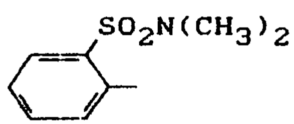 | H | $CH(CH_3)_2$ | $OCH_3$ | N | N | $C-OCH_3$ | 103 |
| 529 | 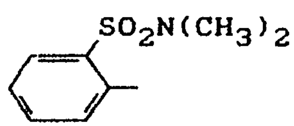 | H | 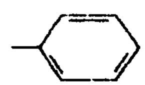 | $OCH_3$ | N | N | $C-OCH_3$ | 230 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 530 | 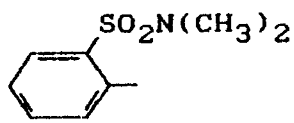 | H | 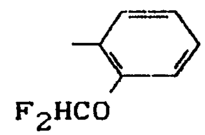 | $OCH_3$ | N | N | $C-OCH_3$ | 217 |
| 531 | 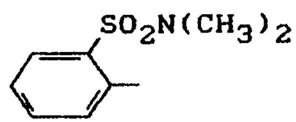 | H | 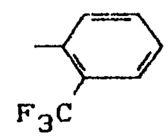 | $OCH_3$ | N | N | $C-OCH_3$ | 231 |
| 532 | 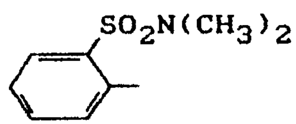 | H | 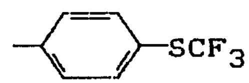 | $OCH_3$ | N | N | $C-OCH_3$ | 187 |
| 533 | 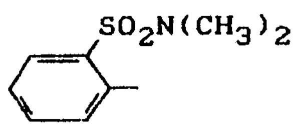 | H | 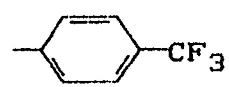 | $OCH_3$ | N | N | $C-OCH_3$ | 201 |
| 534 | 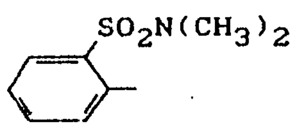 | H | 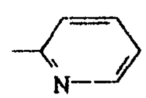 | $OCH_3$ | N | N | $C-OCH_3$ | 195 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 535 | 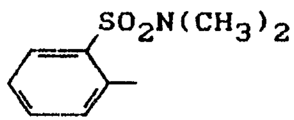 | H | 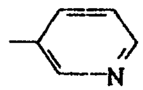 | $OCH_3$ | N | N | $C-OCH_3$ | 198 (Zers.) |
| 536 | 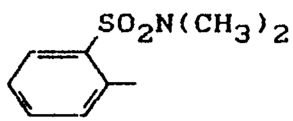 | H | 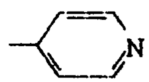 | $OCH_3$ | N | N | $C-OCH_3$ | 194 |
| 537 | 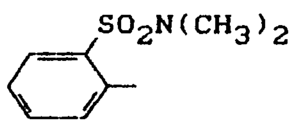 | H | 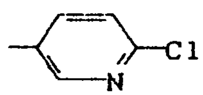 | $OCH_3$ | N | N | $C-OCH_3$ | 212 |
| 538 | 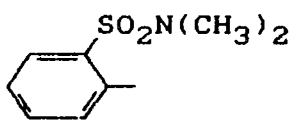 | H | 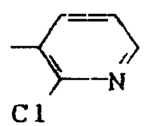 | $OCH_3$ | N | N | $C-OCH_3$ | 199 |
| 539 | 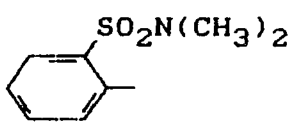 | H | 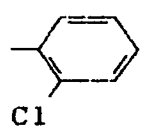 | $OCH_3$ | N | N | $C-OCH_3$ | 230 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 540 | 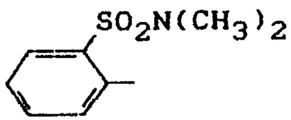 | H | 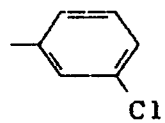 | $OCH_3$ | N | N | $C-OCH_3$ | 219 |
| 541 | 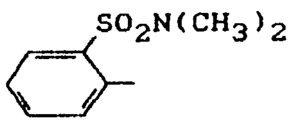 | H | 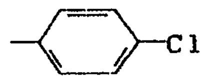 | $OCH_3$ | N | N | $C-OCH_3$ | 240 |
| 542 | 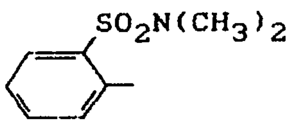 | H | 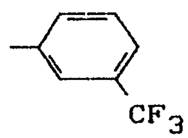 | $OCH_3$ | N | N | $C-OCH_3$ | 198 |
| 543 | 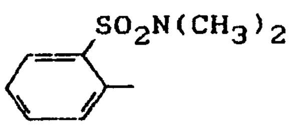 | H | 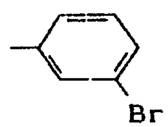 | $OCH_3$ | N | N | $C-OCH_3$ | 231 |
| 544 | 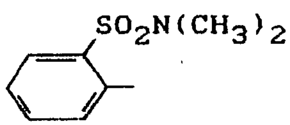 | H | 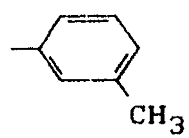 | $OCH_3$ | N | N | $C-OCH_3$ | 218 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 545 | 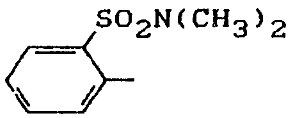 | H | 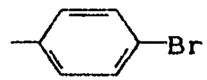 | $OCH_3$ | N | N | $C-OCH_3$ | 248 (Zers.) |
| 546 | 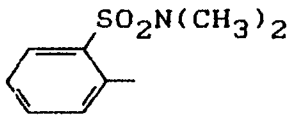 | H | 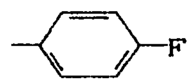 | $OCH_3$ | N | N | $C-OCH_3$ | 239 |
| 547 | 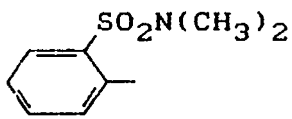 | H | 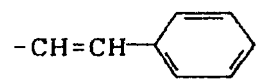 | $OCH_3$ | N | N | $C-OCH_3$ | 230 |
| 548 | 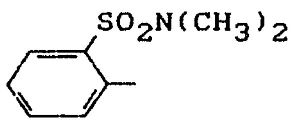 | H | 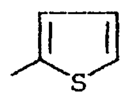 | $OCH_3$ | N | N | $C-OCH_3$ | 248 |
| 549 | 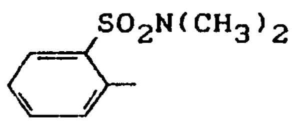 | H | 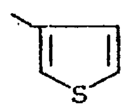 | $OCH_3$ | N | N | $C-OCH_3$ | 236 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 550 | 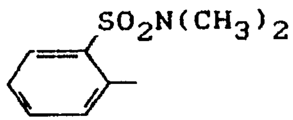 | H | 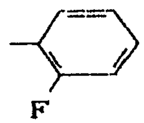 | $OCH_3$ | N | N | $C-OCH_3$ | 221 |
| 551 | 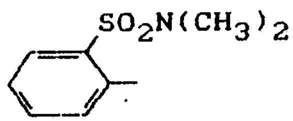 | H | 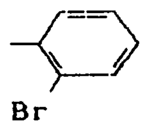 | $OCH_3$ | N | N | $C-OCH_3$ | 226 |
| 552 | 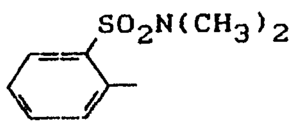 | $-(CH_2)_5-$ | | $OCH_3$ | N | N | $C-OCH_3$ | 179 |
| 553 | 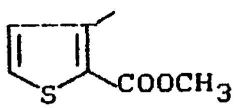 | $CH_3$ | $CH_3$ | $OCH_3$ | N | N | $C-OCH_3$ | 215 |
| 554 | 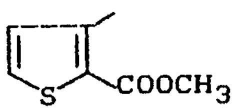 | H | $CH_3$ | $OCH_3$ | N | N | $C-OCH_3$ | 145 |
| 555 | 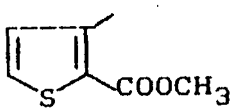 | H | $CH(CH_3)_2$ | $OCH_3$ | N | N | $C-OCH_3$ | 164 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 556 | 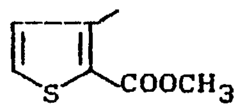 | H | $C_6H_5$ | $OCH_3$ | N | N | $C-OCH_3$ | 209 |
| 557 | 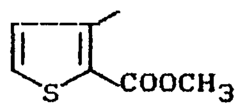 | H | 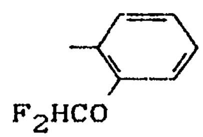 | $OCH_3$ | N | N | $C-OCH_3$ | 165 |
| 558 | 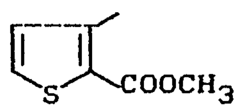 | H | 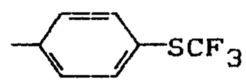 | $OCH_3$ | N | N | $C-OCH_3$ | 185 |
| 559 | 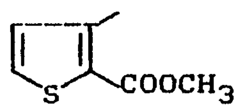 | H | 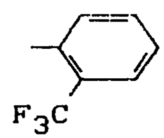 | $OCH_3$ | N | N | $C-OCH_3$ | 119 (Zers.) |
| 560 | 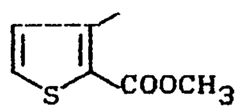 | H | 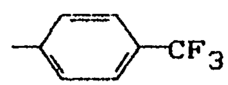 | $OCH_3$ | N | N | $C-OCH_3$ | 196 |
| 561 | 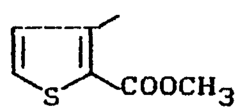 | H | 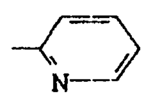 | $OCH_3$ | N | N | $C-OCH_3$ | 193 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 562 | 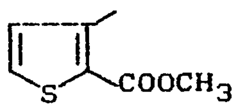 | H | 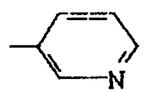 | $OCH_3$ | N | N | C-$OCH_3$ | 191 (Zers.) |
| 563 | 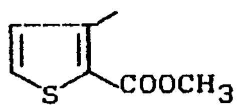 | H | 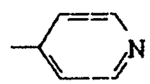 | $OCH_3$ | N | N | C-$OCH_3$ | 159 |
| 564 | 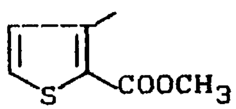 | H | 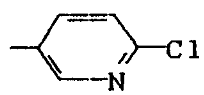 | $OCH_3$ | N | N | C-$OCH_3$ | 130 |
| 565 | 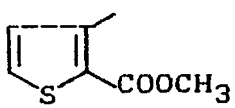 | H | 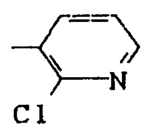 | $OCH_3$ | N | N | C-$OCH_3$ | 183 |
| 566 | 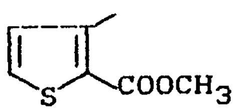 | H | 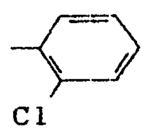 | $OCH_3$ | N | N | C-$OCH_3$ | 215 |
| 567 | 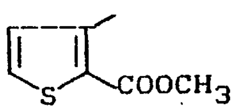 | H | 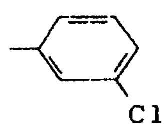 | $OCH_3$ | N | N | C-$OCH_3$ | 233 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 568 | 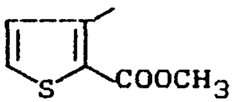 | H | 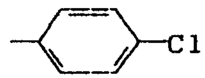 | $OCH_3$ | N | N | $C-OCH_3$ | 218 |
| 569 | 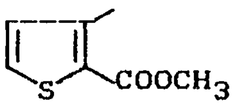 | H | 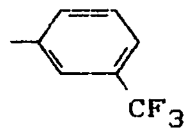 | $OCH_3$ | N | N | $C-OCH_3$ | 235 |
| 570 | 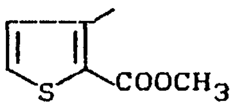 | H | 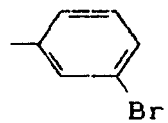 | $OCH_3$ | N | N | $C-OCH_3$ | 225 |
| 571 | 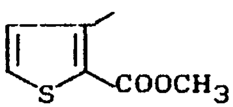 | H | 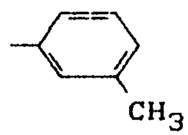 | $OCH_3$ | N | N | $C-OCH_3$ | 205 |
| 572 | 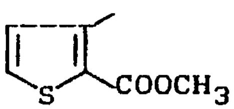 | H | 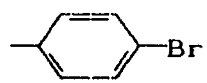 | $OCH_3$ | N | N | $C-OCH_3$ | 221 |
| 573 | 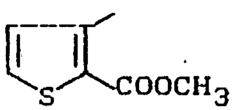 | H | 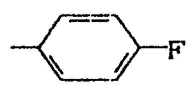 | $OCH_3$ | N | N | $C-OCH_3$ | 205 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 574 | 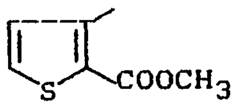 | H | -CH=CH- 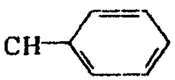 | $OCH_3$ | N | N | $C-OCH_3$ | 212 |
| 575 | 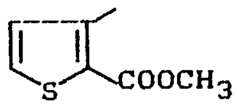 | H | 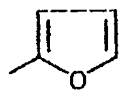 | $OCH_3$ | N | N | $C-OCH_3$ | 201 |
| 576 | 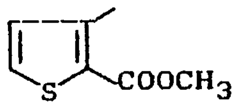 | H | 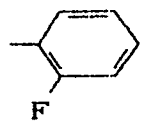 | $OCH_3$ | N | N | $C-OCH_3$ | 226 |
| 577 | 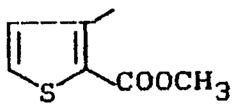 | H | 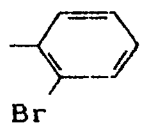 | $OCH_3$ | N | N | $C-OCH_3$ | 219 |
| 578 | 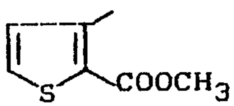 | $-(CH_2)_5-$ | | $OCH_3$ | N | N | $C-OCH_3$ | 203 |
| 579 | 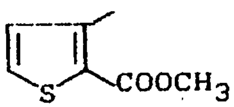 | H | 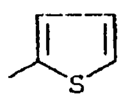 | $OCH_3$ | N | N | $C-OCH_3$ | 239 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 580 | 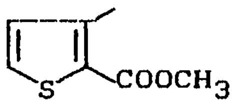 | H | 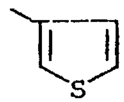 | $OCH_3$ | N | N | $C-OCH_3$ | 209 |
| 581 | 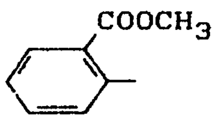 | | $-(CH_2)_5-$ | $OCH_3$ | N | N | $C-OCH_3$ | 167 |
| 582 | 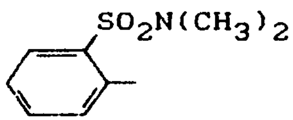 | H | 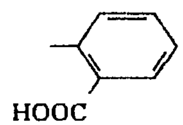 | $OCH_3$ | N | N | $C-OCH_3$ | 238 |
| 583 | 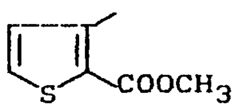 | H | 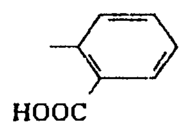 | $OCH_3$ | N | N | $C-OCH_3$ | 225 (Zers.) |
| 584 | 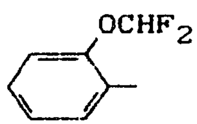 | H | $C_6H_5$ | $OCH_3$ | N | N | $C-CH_3$ | 192 |
| 585 | 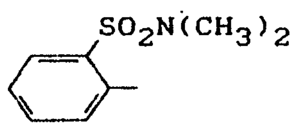 | H | -CH=CH- 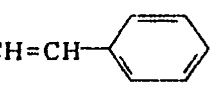 | $OCH_3$ | N | N | $C-CH_3$ | 249 |

Tabelle 3 - Fortsetzung

| Bsp.- Nr. | $R^1$ | $R^2$ | $R^3$ | $R^4$ | X | Y | Z | Schmelzpunkt (°C) |
|---|---|---|---|---|---|---|---|---|
| 586 | 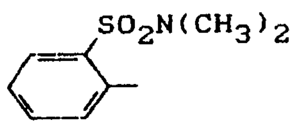 | H | 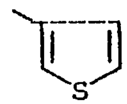 | $OCH_3$ | N | N | $C-CH_3$ | 236 |
| 587 | 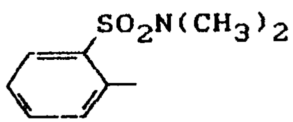 | H | 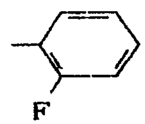 | $OCH_3$ | N | N | $C-CH_3$ | 241 |
| 588 | 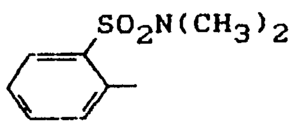 | H | 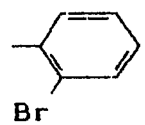 | $OCH_3$ | N | N | $C-CH_3$ | 208 |
| 589 | 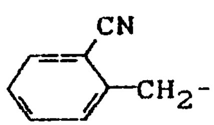 | $CH_3$ | $CH_3$ | $OCH_3$ | N | N | $C-OCH_3$ | 214 |
| 590 | 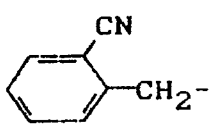 | H | $C_6H_5$ | $OCH_3$ | N | N | $C-OCH_3$ | 227 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 591 | 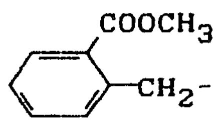 | H | $C_6H_5$ | $OCH_3$ | N | N | $C-OCH_3$ | 203 |
| 592 | 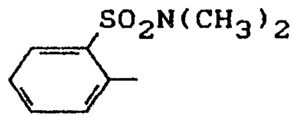 | | $-(CH_2)_5-$ | $OCH_3$ | N | N | $C-CH_3$ | 174 |
| 593 | 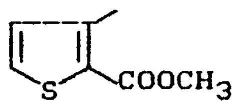 | | $-(CH_2)_5-$ | $OCH_3$ | N | N | $C-CH_3$ | 177 |
| 594 | 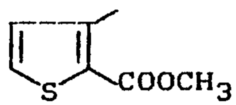 | H | 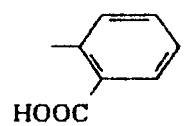 | $OCH_3$ | N | N | $C-CH_3$ | 115-123 |
| 595 | 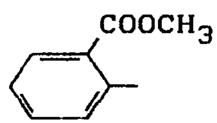 | H | $C_6H_5$ | $OC_2H_5$ | N | N | $C-CH_3$ | 85-93 |
| 596 | 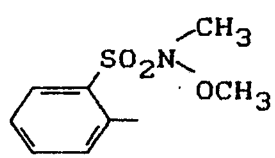 | H | 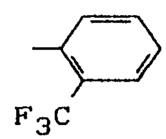 | $OCH_3$ | N | N | $C-OCH_3$ | 205-210 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 597 | 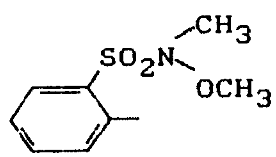 | H | 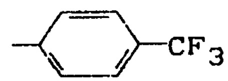 | $OCH_3$ | N | N | $C-OCH_3$ | 205-207 |
| 598 | 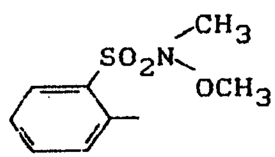 | H | 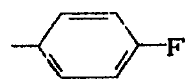 | $OCH_3$ | N | N | $C-OCH_3$ | 225 |
| 599 | 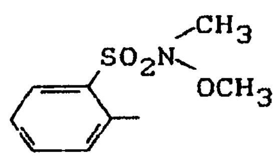 | H | 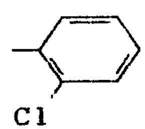 | $OCH_3$ | N | N | $C-OCH_3$ | 203-206 |
| 600 | 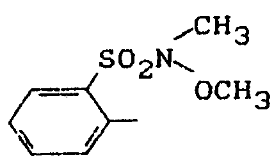 | H | 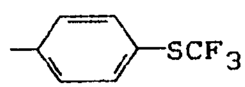 | $OCH_3$ | N | N | $C-OCH_3$ | 210 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 601 | 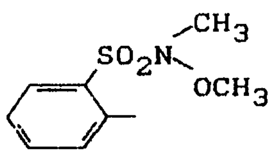 | H | -CH=CH- 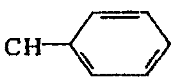 | $OCH_3$ | N | N | $C\text{-}OCH_3$ | 233-235 |
| 602 | 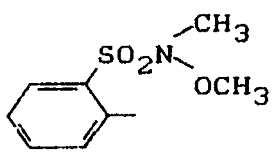 | $-(CH_2)_5-$ | | $OCH_3$ | N | N | $C\text{-}OCH_3$ | 194 |
| 603 | 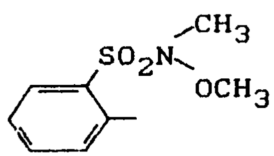 | H | $C(CH_3)_3$ | $OCH_3$ | N | N | $C\text{-}OCH_3$ | 175-177 |
| 604 | 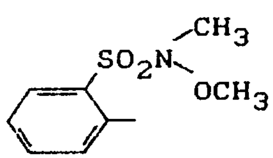 | H | $CH(CH_3)_2$ | $OCH_3$ | N | N | $C\text{-}OCH_3$ | 156-157 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 605 | 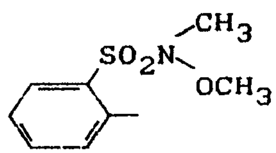 | $CH_3$ | $CH_2CH(CH_3)_2$ | $OCH_3$ | N | N | $C-OCH_3$ | 169 |
| 606 | 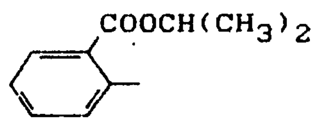 | $CH_3$ | $CH_3$ | $OCH_3$ | N | N | $C-CH_3$ | 158 |
| 607 | 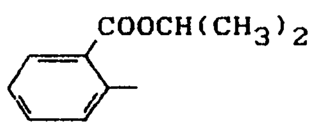 | H | $C_6H_5$ | $OCH_3$ | N | N | $C-CH_3$ | 190 |
| 608 | 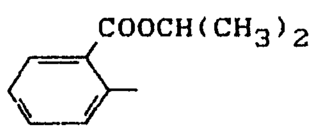 | $CH_3$ | $CH_3$ | $OCH_3$ | N | N | $C-C_2H_5$ | 148-150 |
| 609 | 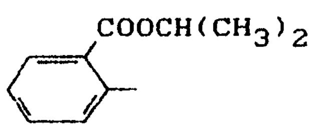 | H | $C_6H_5$ | $OCH_3$ | N | N | $C-C_2H_5$ | 170 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 610 | 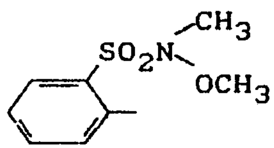 | H | $CH_3$ | $OCH_3$ | N | N | $C-OCH_3$ | 145 |
| 611 | 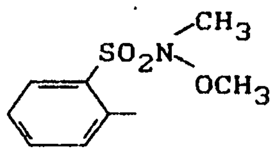 | H | 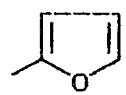 | $OCH_3$ | N | N | $C-OCH_3$ | 174 |
| 612 | 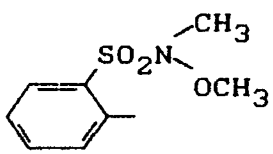 | H | 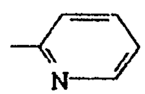 | $OCH_3$ | N | N | $C-OCH_3$ | 166-170 |
| 613 | 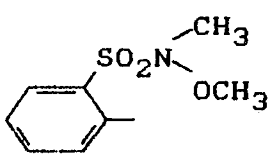 | H | 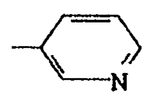 | $OCH_3$ | N | N | $C-OCH_3$ | 166-168 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 614 | 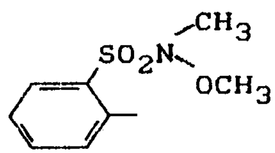 | H | 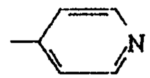 | $OCH_3$ | N | N | $C-OCH_3$ | 173-174 |
| 615 | 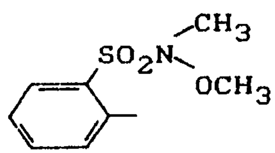 | H | 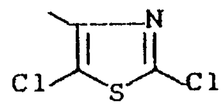 | $OCH_3$ | N | N | $C-OCH_3$ | 87-100 |
| 616 | 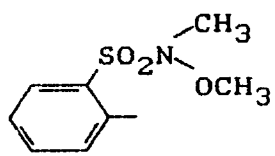 | H | 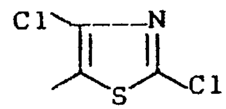 | $OCH_3$ | N | N | $C-OCH_3$ | 216 |
| 617 | 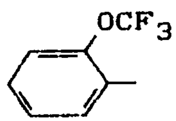 | H | $C_6H_5$ | $OC_2H_5$ | N | N | $C-CH_3$ | 175 |
| 618 | 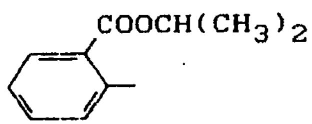 | $CH_3$ | $CH_3$ | $OC_2H_5$ | N | N | $C-CH_3$ | 152 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 619 | 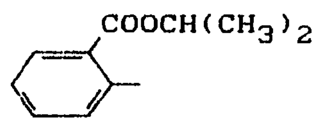 | H | $C_6H_5$ | $OC_2H_5$ | N | N | $C-CH_3$ | 162 |
| 620 | 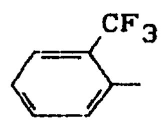 | H | 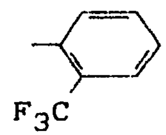 | $OCH_3$ | N | N | $C-OCH_3$ | 209 |
| 621 | 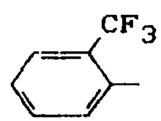 | H | 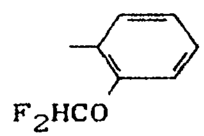 | $OCH_3$ | N | N | $C-OCH_3$ | 195-198 |
| 622 | 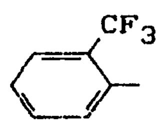 | H | $C_6H_5$ | $OCH_3$ | N | N | $C-OCH_3$ | 200 |
| 623 | 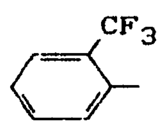 | H | 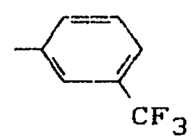 | $OCH_3$ | N | N | $C-OCH_3$ | 209-211 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 624 | 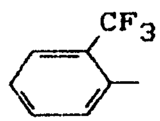 | H | 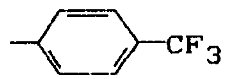 | $OCH_3$ | N | N | $C-OCH_3$ | 175-177 |
| 625 | 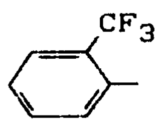 | H | 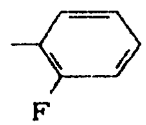 | $OCH_3$ | N | N | $C-OCH_3$ | 217 |
| 626 | 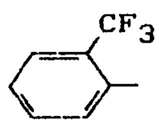 | H | 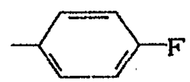 | $OCH_3$ | N | N | $C-OCH_3$ | 231 |
| 627 | 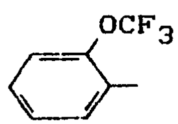 | $CH_3$ | $CH_3$ | $OCH_3$ | N | N | $C-C_2H_5$ | 151 |
| 628 | 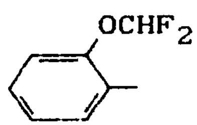 | $CH_3$ | $CH_3$ | $SCH_3$ | N | N | $C-CH_3$ | 141 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 629 | 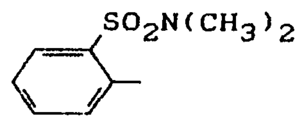 | H | $C_6H_5$ | $SCH_3$ | N | N | C-$CH_3$ | 226 |
| 630 | 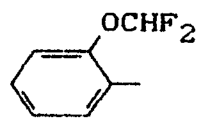 | H | $C_6H_5$ | $SCH_3$ | N | N | C-$CH_3$ | 169 |
| 631 | 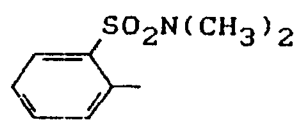 | $CH_3$ | $CH_3$ | $SCH_3$ | N | N | C-$CH_3$ | 228 |
| 632 | 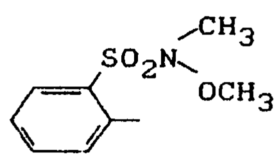 | H | 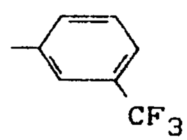 | $OCH_3$ | N | N | C-$OCH_3$ | 202-204 |
| 633 | 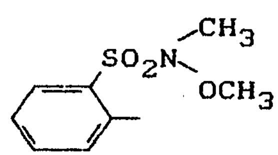 | H | 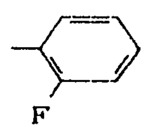 | $OCH_3$ | N | N | C-$OCH_3$ | 194-197 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 634 | 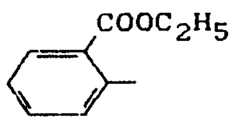 | $CH_3$ | $CH_3$ | $OC_2H_5$ | N | N | $C-CH_3$ | 184-185 |
| 635 | 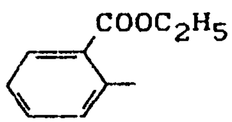 | H | $C_6H_5$ | $OC_2H_5$ | N | N | $C-CH_3$ | 147 |
| 636 | 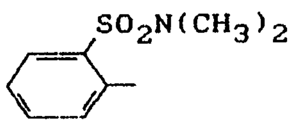 | $CH_3$ | $CH_3$ | $OC_2H_5$ | N | N | $C-CH_3$ | 214-217 |
| 637 | 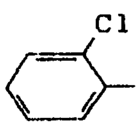 | H | $C_6H_5$ | $OCH_3$ | N | N | $C-OCH_3$ | 192 |
| 638 | 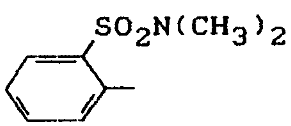 | H | $C_6H_5$ | $OC_2H_5$ | N | N | $C-CH_3$ | 204 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 639 | 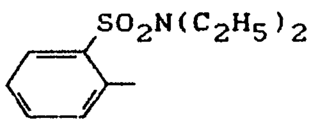 | H | 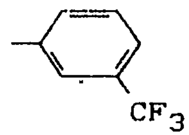 | $OCH_3$ | N | N | $C-CH_3$ | 151 |
| 640 | 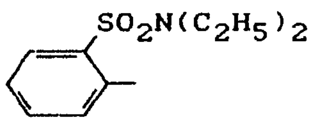 | H | 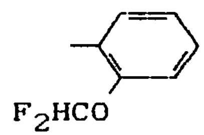 | $OCH_3$ | N | N | $C-CH_3$ | 179 |
| 641 | 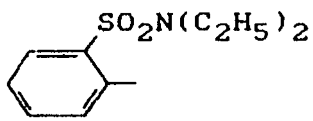 | H | 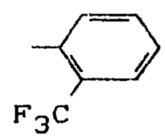 | $OCH_3$ | N | N | $C-CH_3$ | 204-207 |
| 642 | 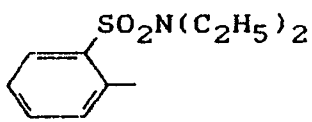 | H | 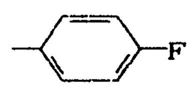 | $OCH_3$ | N | N | $C-CH_3$ | 236 |
| 643 | 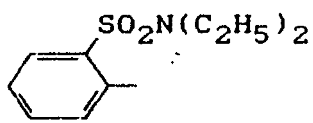 | H | 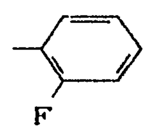 | $OCH_3$ | N | N | $C-CH_3$ | 230 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 644 | 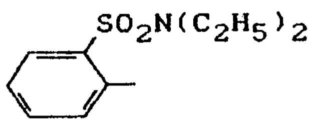 | H | 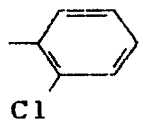 | $OCH_3$ | N | N | $C-CH_3$ | 198-203 |
| 645 | 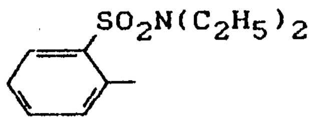 | H | 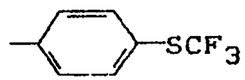 | $OCH_3$ | N | N | $C-CH_3$ | 213-215 |
| 646 | 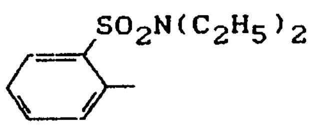 | H | $-CH=CH-$ 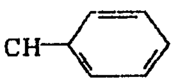 | $OCH_3$ | N | N | $C-CH_3$ | 223-226 |
| 647 | 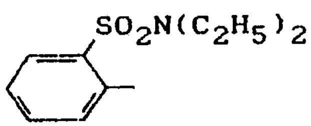 | $-(CH_2)_5-$ | | $OCH_3$ | N | N | $C-CH_3$ | 200 |
| 648 | 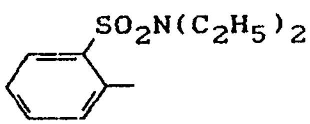 | H | $CH(CH_3)_2$ | $OCH_3$ | N | N | $C-CH_3$ | 166-172 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 649 | 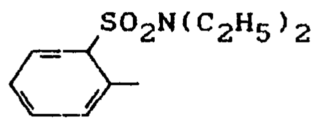 | H | $C(CH_3)_3$ | $OCH_3$ | N | N | $C-CH_3$ | 173-175 |
| 650 | 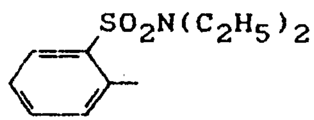 | H | 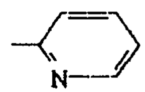 | $OCH_3$ | N | N | $C-CH_3$ | 217 |
| 651 | 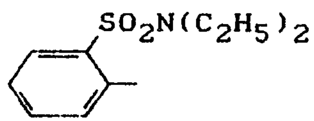 | H | 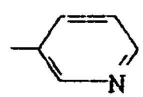 | $OCH_3$ | N | N | $C-CH_3$ | 180-184 |
| 652 | 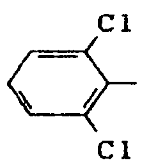 | H | 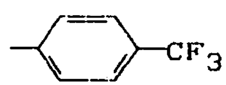 | $OCH_3$ | N | N | $C-CH_3$ | 167-173 |
| 653 | 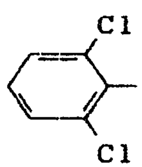 | H | $CH_3$ | $OCH_3$ | N | N | $C-CH_3$ | 144-149 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 654 | 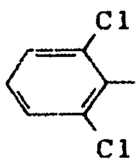 | H | 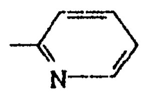 | $OCH_3$ | N | N | $C-CH_3$ | 171 |
| 655 | 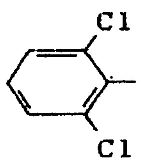 | H | 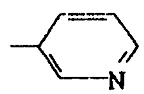 | $OCH_3$ | N | N | $C-CH_3$ | 122 |
| 656 | 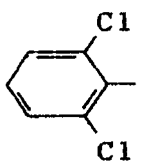 | H | 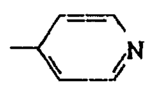 | $OCH_3$ | N | N | $C-CH_3$ | 195 |
| 657 | 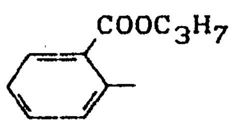 | $CH_3$ | $CH_3$ | $OCH_3$ | N | N | $C-CH_3$ | 160 |
| 658 | 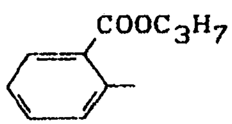 | H | $C_6H_5$ | $OCH_3$ | N | N | $C-CH_3$ | 173-182 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 659 | 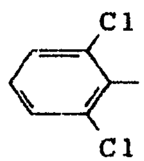 | H | 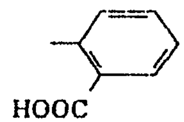 | $OCH_3$ | N | N | $C-CH_3$ | 192 |
| 660 | 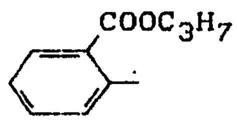 | H | $C_6H_5$ | $OCH_3$ | N | N | $C-C_2H_5$ | 141 |
| 661 | 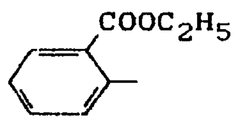 | H | 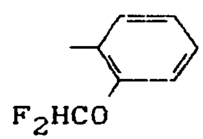 | $OCH_3$ | N | N | $C-CH_3$ | 159 |
| 662 | 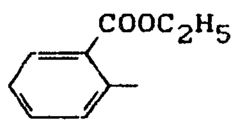 | H | 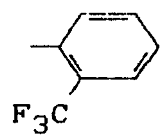 | $OCH_3$ | N | N | $C-CH_3$ | 125 |
| 663 | 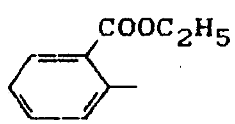 | H | 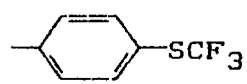 | $OCH_3$ | N | N | $C-CH_3$ | 163 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 664 | 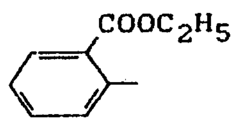 | H | 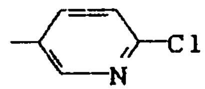 | $OCH_3$ | N | N | $C-CH_3$ | 191 |
| 665 | 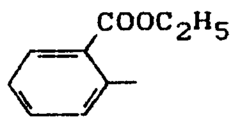 | H | 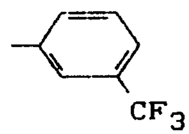 | $OCH_3$ | N | N | $C-CH_3$ | 160-162 |
| 666 | 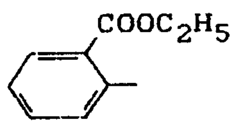 | H | 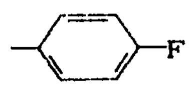 | $OCH_3$ | N | N | $C-CH_3$ | 186 |
| 667 | 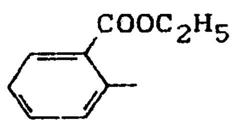 | H | 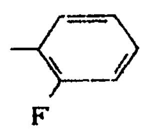 | $OCH_3$ | N | N | $C-CH_3$ | 192 |
| 668 | 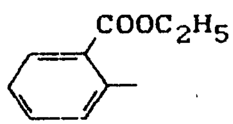 | $-(CH_2)_5-$ | | $OCH_3$ | N | N | $C-CH_3$ | 166 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 669 | 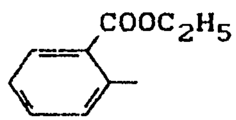 | H | 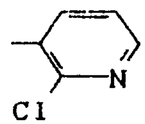 | $OCH_3$ | N | N | $C-CH_3$ | 254 |
| 670 | 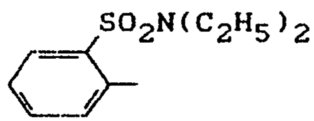 | H | 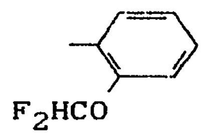 | $OCH_3$ | N | N | $C-OCH_3$ | 182 |
| 671 | 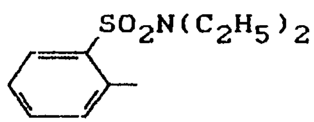 | H | 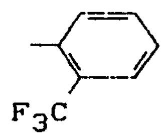 | $OCH_3$ | N | N | $C-OCH_3$ | 205-210 |
| 672 | 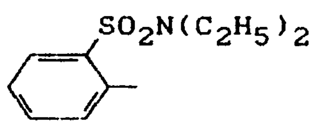 | H | 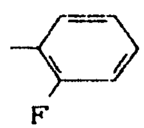 | $OCH_3$ | N | N | $C-OCH_3$ | 200 |
| 673 | 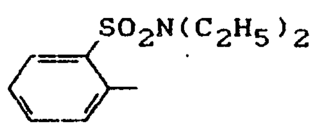 | H | 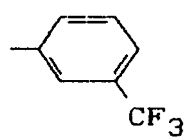 | $OCH_3$ | N | N | $C-OCH_3$ | 182 |

Tabelle 3 - Fortsetzung

| Bsp.-Nr. | $R^1$ | $R^2$ | $R^3$ | $R^4$ | X | Y | Z | Schmelzpunkt ($^oC$) |
|---|---|---|---|---|---|---|---|---|
| 674 | 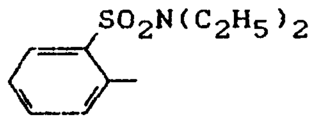 | H | 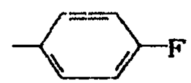 | $OCH_3$ | N | N | $C-OCH_3$ | 235 |
| 675 | 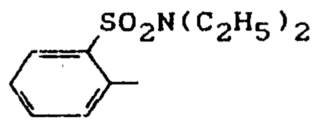 | H | 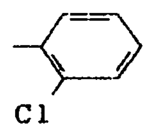 | $OCH_3$ | N | N | $C-OCH_3$ | 194 |
| 676 | 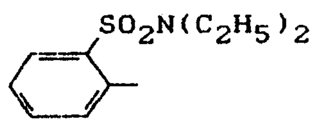 | H | 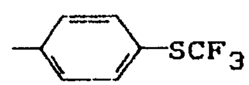 | $OCH_3$ | N | N | $C-OCH_3$ | 203 |
| 677 | 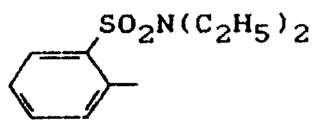 | H | $C_6H_5$ | $OCH_3$ | N | N | $C-OCH_3$ | 216-218 |
| 678 | 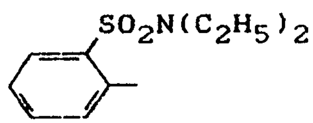 | $-(CH_2)_5-$ | | $OCH_3$ | N | N | $C-OCH_3$ | 171 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 679 | 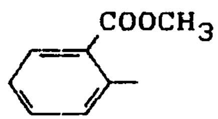 | H | $C_6H_5$ | $OCH_3$ | N | N | $C-C_2H_5$ | 170 |
| 680 | 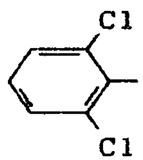 | H | 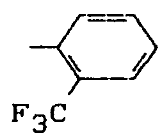 | $OC_2H_5$ | N | N | $C-CH_3$ | 202-204 |
| 681 | 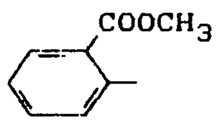 | $CH_3$ | $CH_3$ | $OC_2H_5$ | N | N | $C-CH_3$ | 160-163 |
| 682 | 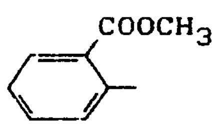 | H | 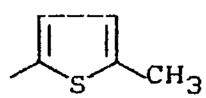 | $OCH_3$ | N | N | $C-OCH_3$ | 211-212 |
| 683 | 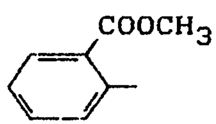 | H | 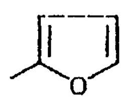 | $OCH_3$ | N | N | $C-OCH_3$ | 219-220 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 684 | 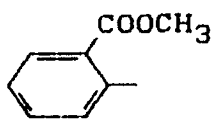 | H | 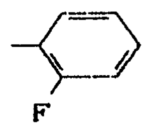 | $OCH_3$ | N | N | $C-OCH_3$ | 208-209 |
| 685 | 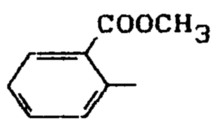 | H | 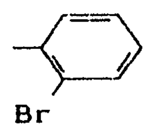 | $OCH_3$ | N | N | $C-OCH_3$ | 223-224 |
| 686 | 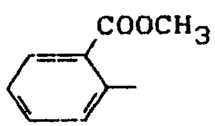 | H | 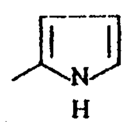 | $OCH_3$ | N | N | $C-OCH_3$ | 205 |
| 687 | 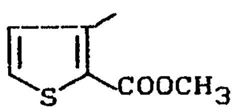 | H | 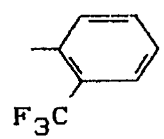 | $OC_2H_5$ | N | N | $C-CH_3$ | 160-165 |
| 688 | 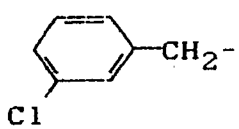 | $CH_3$ | $CH_3$ | $OCH_3$ | N | N | $C-OCH_3$ | 217 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 689 | 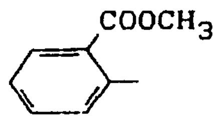 | $CH_3$ | $CH_3$ | $OCH_3$ | N | N | $C-C_2H_5$ | 138-140 |
| 690 | 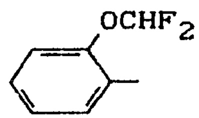 | H | 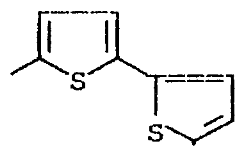 | $OCH_3$ | N | N | $C-CH_3$ | 249-251 |
| 691 | 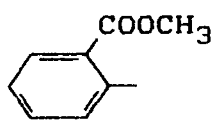 | H | 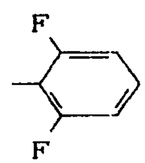 | $OCH_3$ | N | N | $C-CH_3$ | 180-183 |
| 692 | 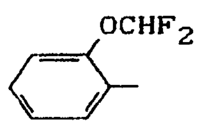 | H | 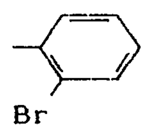 | $OCH_3$ | N | N | $C-OCH_3$ | 134-139 |
| 693 | 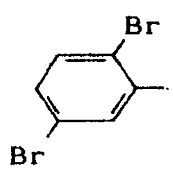 | $CH_3$ | $CH_3$ | $OCH_3$ | N | N | $C-OCH_3$ | 220 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 694 | 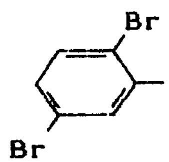 | H | $C_6H_5$ | $OCH_3$ | N | N | $C-OCH_3$ | 143-145 |
| 695 | 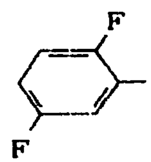 | $CH_3$ | $CH_3$ | $OCH_3$ | N | N | $C-OCH_3$ | 198-200 |
| 696 | 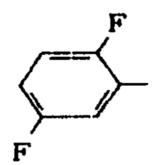 | H | $C_6H_5$ | $OCH_3$ | N | N | $C-OCH_3$ | 176-178 |
| 697 | 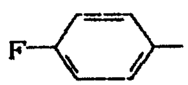 | $CH_3$ | $CH_3$ | $OCH_3$ | N | N | $C-OCH_3$ | 163-167 |
| 698 | 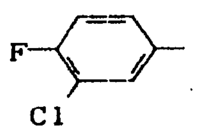 | $CH_3$ | $CH_3$ | $OCH_3$ | N | N | $C-OCH_3$ | 153-155 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 699 | 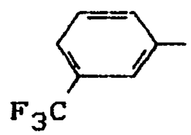 | $CH_3$ | $CH_3$ | $OCH_3$ | N | N | $C-OCH_3$ | 129-134 |
| 700 | 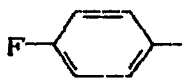 | H | $C_6H_5$ | $OCH_3$ | N | N | $C-OCH_3$ | 192-200 |
| 701 | 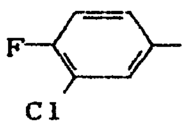 | H | $C_6H_5$ | $OCH_3$ | N | N | $C-OCH_3$ | 117-122 |
| 702 | 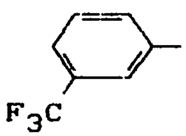 | H | $C_6H_5$ | $OCH_3$ | N | N | $C-OCH_3$ | 177 |
| 703 | 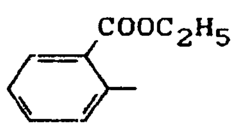 | H | 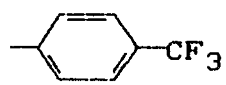 | $OCH_3$ | N | N | $C-CH_3$ | 199 |
| 704 | 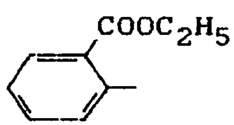 | H | 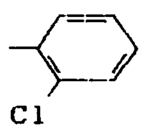 | $OCH_3$ | N | N | $C-CH_3$ | 140-144 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 705 | 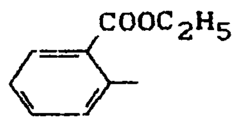 | H | -CH=CH 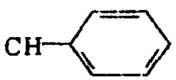 | $OCH_3$ | N | N | $C-CH_3$ | 175-179 |
| 706 | 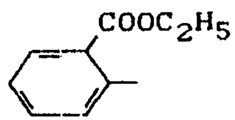 | H | $CH(CH_3)_2$ | $OCH_3$ | N | N | $C-CH_3$ | 137-140 |
| 707 | 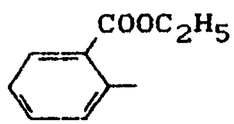 | H | $C(CH_3)_3$ | $OCH_3$ | N | N | $C-CH_3$ | 176-180 |
| 708 | 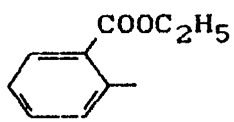 | $CH_3$ | $CH_2CH(CH_3)_2$ | $OCH_3$ | N | N | $C-CH_3$ | 136-139 |
| 709 | 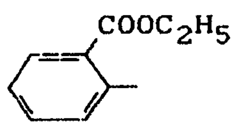 | H | $CH_3$ | $OCH_3$ | N | N | $C-CH_3$ | 130-132 |
| 710 | 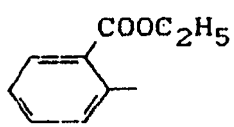 | H | $C_6H_5$ | $OCH_3$ | N | N | $C-C_2H_5$ | 172 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 711 | 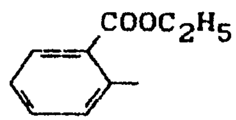 | $CH_3$ | $CH_3$ | $OCH_3$ | N | N | $C-C_2H_5$ | 137-142 |
| 712 | 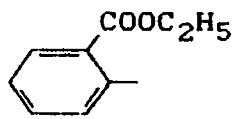 | H | 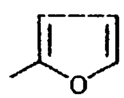 | $OCH_3$ | N | N | $C-CH_3$ | 128-136 |
| 713 | 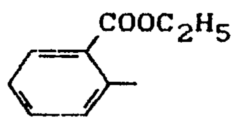 | H | 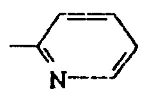 | $OCH_3$ | N | N | $C-CH_3$ | 182-188 |
| 714 | 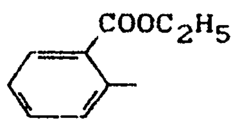 | H | 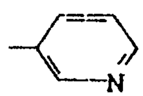 | $OCH_3$ | N | N | $C-CH_3$ | 156-166 |
| 715 | 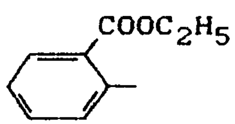 | H | 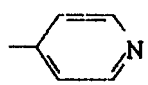 | $OCH_3$ | N | N | $C-CH_3$ | 157-163 |
| 716 | 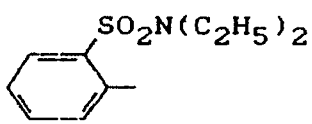 | H | $C_6H_5$ | $OCH_3$ | N | N | $C-OCH_3$ | 227-228 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 717 | 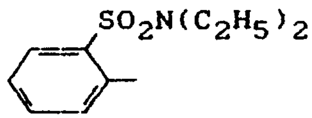 | H | $C_6H_5$ | $OCH_3$ | N | N | $C-CH_3$ | 228-231 |
| 718 | 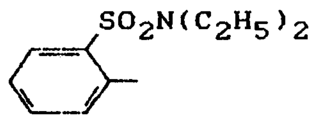 | $CH_3$ | $CH_3$ | $OCH_3$ | N | N | $C-OCH_3$ | 158-172 |
| 719 | 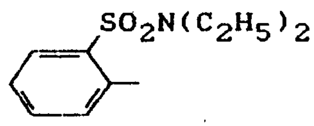 | H | 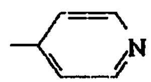 | $OCH_3$ | N | N | $C-OCH_3$ | 182 |
| 720 | 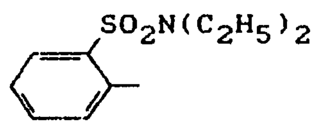 | H | 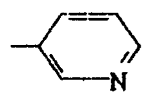 | $OCH_3$ | N | N | $C-OCH_3$ | 189-193 |
| 721 | 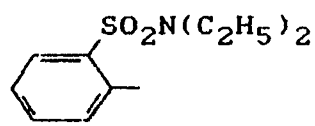 | H | 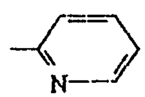 | $OCH_3$ | N | N | $C-OCH_3$ | 188-191 |
| 722 | 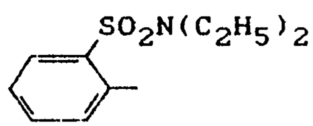 | H | 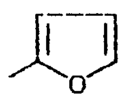 | $OCH_3$ | N | N | $C-OCH_3$ | 219-221 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 723 | 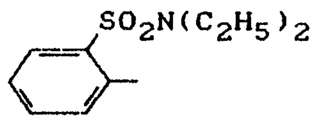 | H | $CH_3$ | $OCH_3$ | N | N | $C-OCH_3$ | 125-132 |
| 724 | 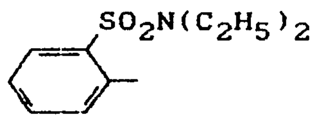 | $CH_3$ | $CH_2CH(CH_3)_2$ | $OCH_3$ | N | N | $C-OCH_3$ | 168 |
| 725 | 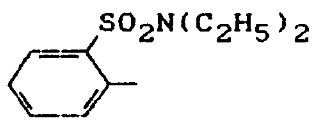 | H | $C(CH_3)_3$ | $OCH_3$ | N | N | $C-OCH_3$ | 180-182 |
| 726 | 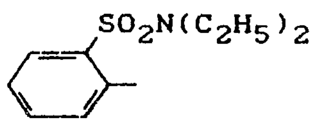 | H | $CH(CH_3)_2$ | $OCH_3$ | N | N | $C-OCH_3$ | 187-189 |
| 727 | 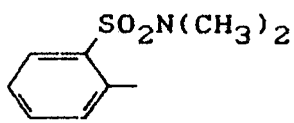 | $CH_3$ | $CH_3$ | $CH_3$ | N | N | $C-CH_3$ | 246-251 (Z.) |
| 728 | 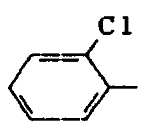 | $CH_3$ | $CH_3$ | $OCH_3$ | N | N | $C-CH_3$ | 170-176 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 729 | 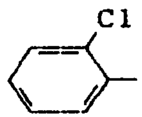 | H | $C_6H_5$ | $OCH_3$ | N | N | $C-CH_3$ | 212-218 |
| 730 | 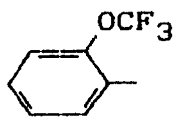 | $CH_3$ | $CH_3$ | $OCH_3$ | N | N | $C-CH_3$ | 146 |
| 731 | 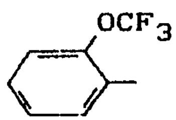 | H | $C_6H_5$ | $OCH_3$ | N | N | $C-CH_3$ | 170 |
| 732 | 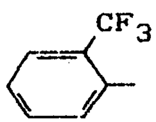 | $CH_3$ | $CH_3$ | $OCH_3$ | N | N | $C-CH_3$ | 180-181 |
| 733 | 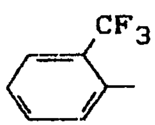 | H | $CH_3$ | $OCH_3$ | N | N | $C-CH_3$ | 197 |
| 734 | 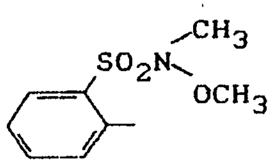 | $CH_3$ | $CH_3$ | $OCH_3$ | N | N | $C-CH_3$ | 191 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 735 | 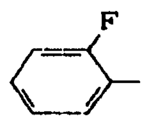 | $CH_3$ | $CH_3$ | $OCH_3$ | N | N | $C-CH_3$ | 185 |
| 736 | 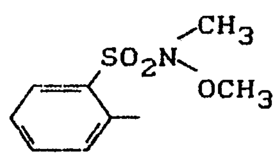 | H | $C_6H_5$ | $OCH_3$ | N | N | $C-CH_3$ | 223 |
| 737 | 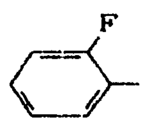 | H | $C_6H_5$ | $OCH_3$ | N | N | $C-CH_3$ | 230 |
| 738 | 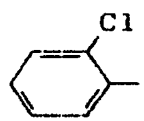 | $CH_3$ | $CH_3$ | $OC_2H_5$ | N | N | $C-CH_3$ | 163-167 |
| 739 | 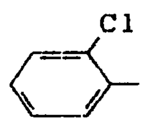 | H | $C_6H_5$ | $OC_2H_5$ | N | N | $C-CH_3$ | 178-180 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 740 | 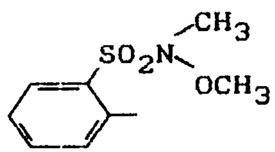 | $CH_3$ | $CH_3$ | $SCH_3$ | N | N | $C-CH_3$ | 187-191 |
| 741 | 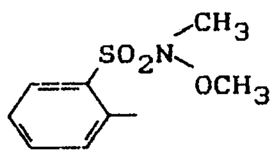 | H | $C_6H_5$ | $SCH_3$ | N | N | $C-CH_3$ | 219-223 |
| 742 | 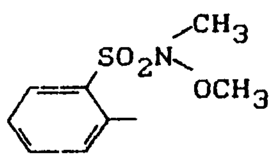 | $CH_3$ | $CH_3$ | $OCH_3$ | N | N | $C-C_2H_5$ | 171-175 |
| 743 | 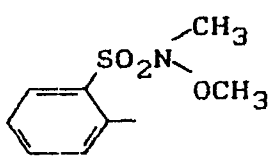 | H | $C_6H_5$ | $OCH_3$ | N | N | $C-C_2H_5$ | 204-207 |
| 744 | 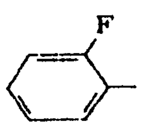 | $CH_3$ | $CH_3$ | $OCH_3$ | N | N | $C-C_2H_5$ | 146 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 745 | 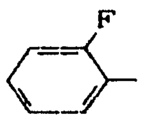 | H | $C_6H_5$ | $OCH_3$ | N | N | $C-C_2H_5$ | 215 |
| 746 | 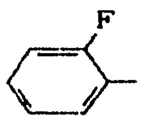 | H | $C_6H_5$ | $SCH_3$ | N | N | $C-CH_3$ | 220-223 |
| 747 | 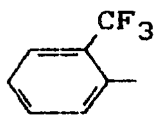 | $CH_3$ | $CH_3$ | $OCH_3$ | N | N | $C-C_2H_5$ | 148 |
| 748 | 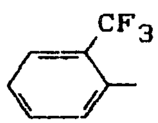 | H | $C_6H_5$ | $OCH_3$ | N | N | $C-C_2H_5$ | 185-188 |
| 749 | 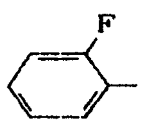 | H | $C_6H_5$ | $OC_2H_5$ | N | N | $C-CH_3$ | 195 |
| 750 | 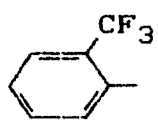 | $CH_3$ | $CH_3$ | $OC_2H_5$ | N | N | $C-CH_3$ | 152 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 751 | 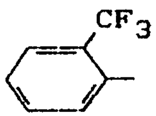 | H | $C_6H_5$ | $OC_2H_5$ | N | N | $C-CH_3$ | 209 |
| 752 | 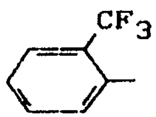 | H | $CH(CH_3)_2$ | $OCH_3$ | N | N | $C-CH_3$ | 143-147 |
| 753 | 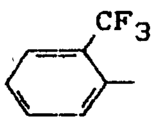 | H | $C(CH_3)_3$ | $OCH_3$ | N | N | $C-CH_3$ | 136-138 |
| 754 | 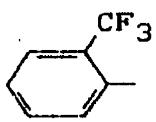 | $CH_3$ | $CH_2CH(CH_3)_2$ | $OCH_3$ | N | N | $C-CH_3$ | 126 |
| 755 | 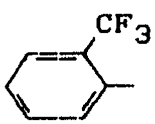 | H | $CH_3$ | $OCH_3$ | N | N | $C-CH_3$ | 142-144 |
| 756 | 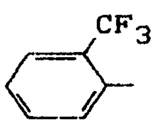 | H | 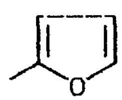 | $OCH_3$ | N | N | $C-CH_3$ | 147-158 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 757 | 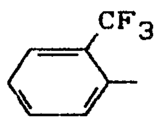 | H | 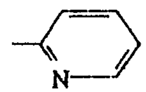 | $OCH_3$ | N | N | $C-CH_3$ | 174-179 |
| 758 | 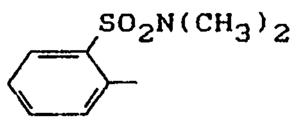 | $CH_3$ | $CH_2CH(CH_3)_2$ | $OCH_3$ | N | N | $C-CH_3$ | $^1H$ NMR*) $\delta=4,03$ppm ($OCH_3$) |
| 759 | 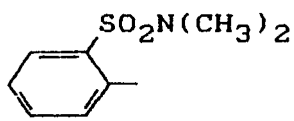 | $CH_3$ | $CH_2CH(CH_3)_2$ | $OCH_3$ | N | N | $C-OCH_3$ | 180-184 |
| 760 | 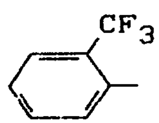 | H | 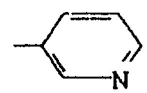 | $OCH_3$ | N | N | $C-CH_3$ | 180-185 |
| 761 | 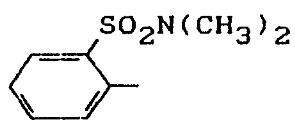 | H | $CH(C_2H_5)_2$ | $OCH_3$ | N | N | $C-OCH_3$ | 135 |
| 762 | 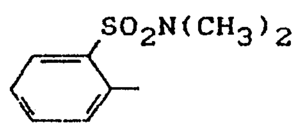 | H | $CH(C_2H_5)_2$ | $OCH_3$ | N | N | $C-CH_3$ | 124 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 763 | 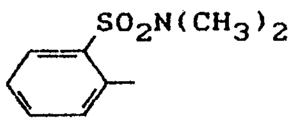 | H | $-CH=CH_2$ | $OCH_3$ | N | N | $C-OCH_3$ | 189-191 |
| 764 | 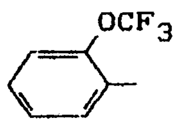 | H | 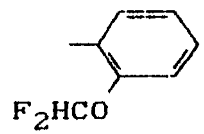 | $OCH_3$ | N | N | $C-CH_3$ | 154 |
| 765 | 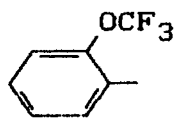 | H | 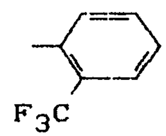 | $OCH_3$ | N | N | $C-CH_3$ | 160-162 |
| 766 | 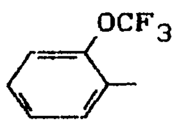 | H | 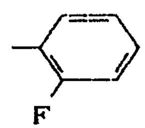 | $OCH_3$ | N | N | $C-CH_3$ | 149-158 |
| 767 | 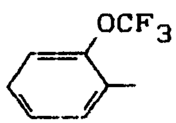 | H | 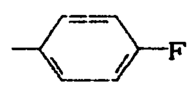 | $OCH_3$ | N | N | $C-CH_3$ | 187-189 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 768 | 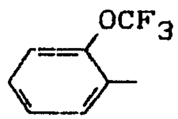 | H | 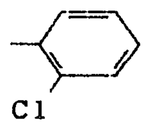 | $OCH_3$ | N | N | $C-CH_3$ | 171-174 |
| 769 | 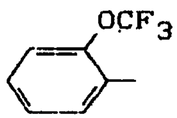 | H | -CH=CH- 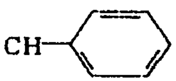 | $OCH_3$ | N | N | $C-CH_3$ | 182 |
| 770 | 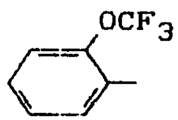 | H | $CH(CH_3)_2$ | $OCH_3$ | N | N | $C-CH_3$ | 128 |
| 771 | 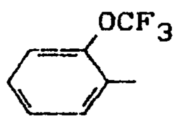 | H | $C(CH_3)_3$ | $OCH_3$ | N | N | $C-CH_3$ | 139-141 |
| 772 | 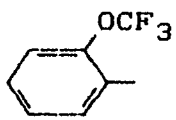 | H | $CH_3$ | $OCH_3$ | N | N | $C-CH_3$ | 137 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 773 | 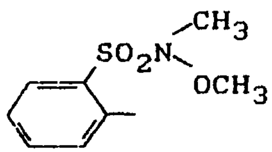 | H | 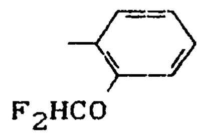 | $OCH_3$ | N | N | $C-OCH_3$ | 182 |
| 774 | 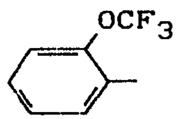 | H | 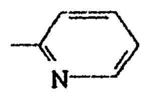 | $OCH_3$ | N | N | $C-CH_3$ | 166 |
| 775 | 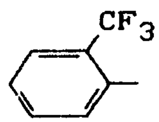 | H | 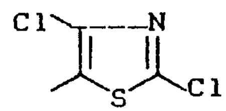 | $OCH_3$ | N | N | $C-CH_3$ | 196-199 |
| 776 | 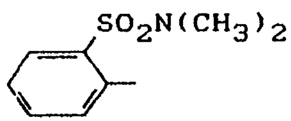 | $CH_3$ | $C_2H_5$ | $OCH_3$ | N | N | $C-OCH_3$ | 196-204 (Zers.) |
| 777 | 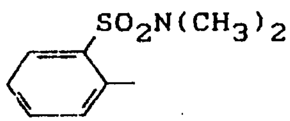 | $CH_3$ | $C_2H_5$ | $OCH_3$ | N | N | $C-CH_3$ | 190-196 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 778 | 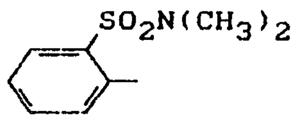 | $CH_3$ | $CH_2OCH_3$ | $OCH_3$ | N | N | $C-CH_3$ | 165-170 |
| 779 | 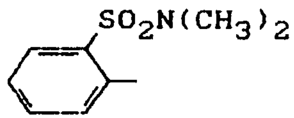 | H | 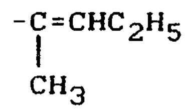 | $OCH_3$ | N | N | $C-CH_3$ | 178 |
| 780 | 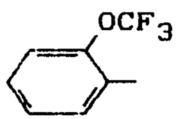 | H | 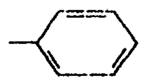 | $OCH_3$ | N | N | $C-C_2H_5$ | 157 |
| 781 | 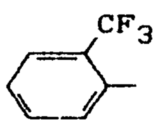 | H | 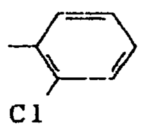 | $OCH_3$ | N | N | $C-OCH_3$ | 202 |
| 782 | 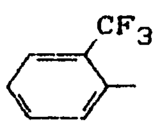 | H | 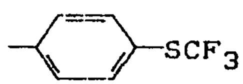 | $OCH_3$ | N | N | $C-OCH_3$ | 152 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 783 | 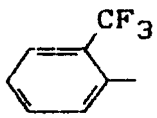 | H | -CH=CH- 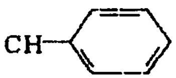 | $OCH_3$ | N | N | $C-OCH_3$ | 200-203 |
| 784 | 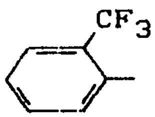 | H | $CH(CH_3)_2$ | $OCH_3$ | N | N | $C-OCH_3$ | 150-153 |
| 785 | 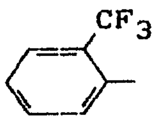 | $-(CH_2)_5-$ | | $OCH_3$ | N | N | $C-OCH_3$ | 145 |
| 786 | 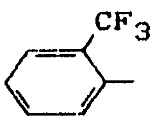 | H | $C(CH_3)_3$ | $OCH_3$ | N | N | $C-OCH_3$ | 70-78 |
| 787 | 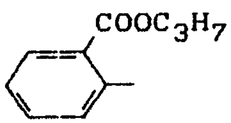 | $CH_3$ | $CH_3$ | $OCH_3$ | N | N | $C-OCH_3$ | 185-189 |
| 788 | 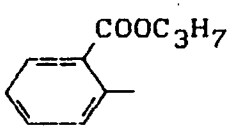 | H | $C_6H_5$ | $OCH_3$ | N | N | $C-OCH_3$ | 180-183 |

Tabelle 3 - Fortsetzung

| Bsp.- Nr. | $R^1$ | $R^2$ | $R^3$ | $R^4$ | X | Y | Z | Schmelzpunkt (°C) |
|---|---|---|---|---|---|---|---|---|
| 789 | 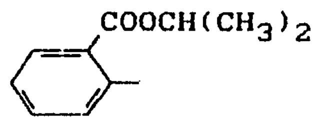 | $CH_3$ | $CH_3$ | $OCH_3$ | N | N | $C-OCH_3$ | 191 |
| 790 | 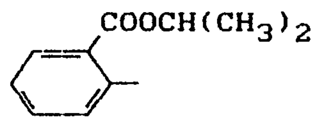 | H | $C_6H_5$ | $OCH_3$ | N | N | $C-OCH_3$ | 201-202 |
| 791 | 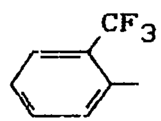 | H | 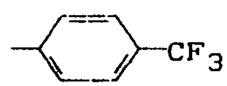 | $OCH_3$ | N | N | $C-CH_3$ | 188-190 |
| 792 | 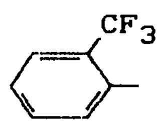 | H | 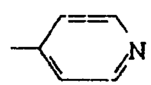 | $OCH_3$ | N | N | $C-CH_3$ | 144 |
| 793 | 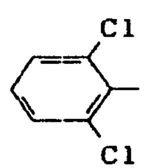 | H | 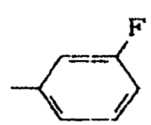 | $OCH_3$ | N | N | $C-CH_3$ | 205-207 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 794 | 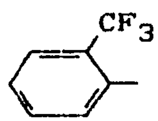 | H | 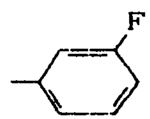 | $OCH_3$ | N | N | $C-CH_3$ | 175-177 |
| 795 | 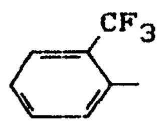 | H | 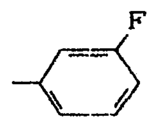 | $OCH_3$ | N | N | $C-OCH_3$ | 177 |
| 796 | 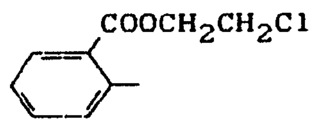 | $CH_3$ | $CH_3$ | $OCH_3$ | N | N | $C-OCH_3$ | 201-203 |
| 797 | 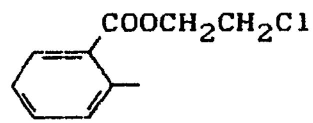 | H | $C_6H_5$ | $OCH_3$ | N | N | $C-OCH_3$ | 151 |
| 798 | 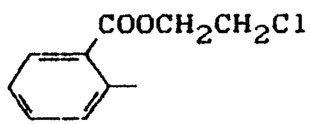 | $CH_3$ | $CH_3$ | $OCH_3$ | N | N | $C-CH_3$ | 180 |
| 799 | 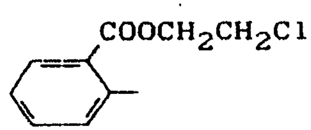 | H | $C_6H_5$ | $OCH_3$ | N | N | $C-CH_3$ | 172 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 800 | 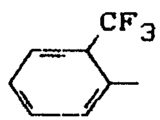 | $CH_3$ | $CH_2CH(CH_3)_2$ | $OCH_3$ | N | N | $C-OCH_3$ | 122-123 |
| 801 | 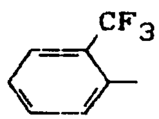 | H | $CH_3$ | $OCH_3$ | N | N | $C-OCH_3$ | 153-159 |
| 802 | 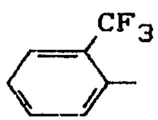 | H | 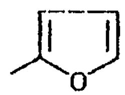 | $OCH_3$ | N | N | $C-OCH_3$ | 204-205 |
| 803 | 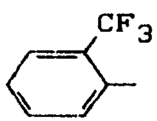 | H | 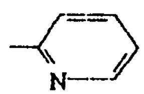 | $OCH_3$ | N | N | $C-OCH_3$ | 160-166 |
| 804 | 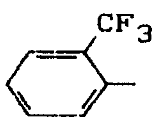 | H | 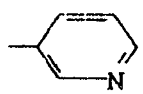 | $OCH_3$ | N | N | $C-OCH_3$ | 139 (Zers.) |
| 805 | 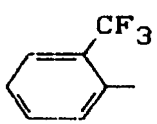 | H | 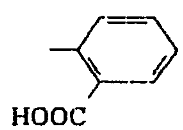 | $OCH_3$ | N | N | $C-OCH_3$ | 211 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 806 | 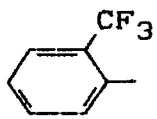 | H | 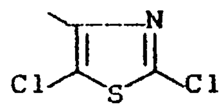 | $OCH_3$ | N | N | $C-OCH_3$ | 152-158 |
| 807 | 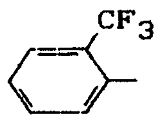 | H | 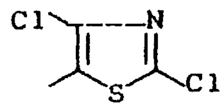 | $OCH_3$ | N | N | $C-OCH_3$ | 153 |
| 808 | 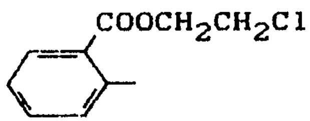 | $CH_3$ | $CH_3$ | $OCH_3$ | N | N | $C-C_2H_5$ | 165 |
| 809 | 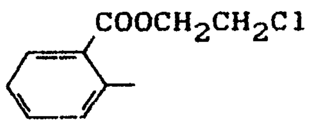 | H | $C_6H_5$ | $OCH_3$ | N | N | $C-C_2H_5$ | 154 |
| 810 | 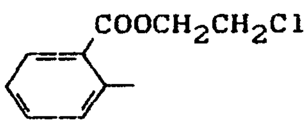 | $CH_3$ | $CH_3$ | $OC_2H_5$ | N | N | $C-CH_3$ | 165-166 |
| 811 | 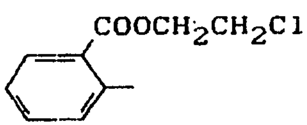 | H | $C_6H_5$ | $OC_2H_5$ | N | N | $C-CH_3$ | 148 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 812 | 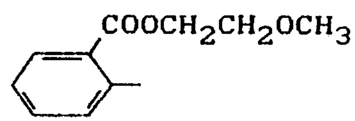 | $CH_3$ | $CH_3$ | $OCH_3$ | N | N | $C-OCH_3$ | 158 |
| 813 | 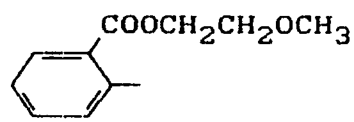 | H | $C_6H_5$ | $OCH_3$ | N | N | $C-OCH_3$ | 157 |
| 814 | 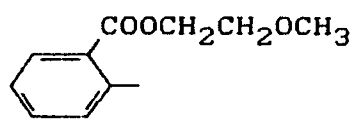 | $CH_3$ | $CH_3$ | $OCH_3$ | N | N | $C-CH_3$ | 160 |
| 815 | 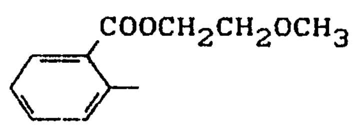 | H | $C_6H_5$ | $OCH_3$ | N | N | $C-CH_3$ | 153 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 816 | 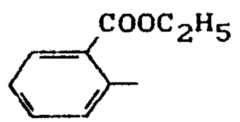 | H | 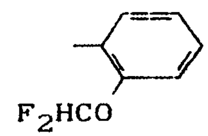 | $OCH_3$ | N | N | $C-OCH_3$ | 155 |
| 817 | 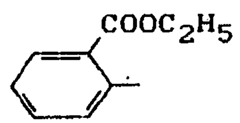 | H | 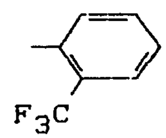 | $OCH_3$ | N | N | $C-OCH_3$ | 171-172 |
| 818 | 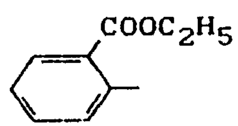 | H | 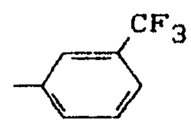 | $OCH_3$ | N | N | $C-OCH_3$ | 171 |
| 819 | 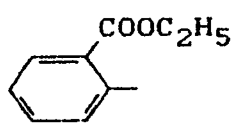 | H | 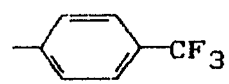 | $OCH_3$ | N | N | $C-OCH_3$ | 205 |
| 820 | 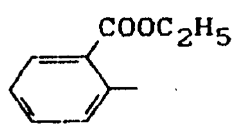 | H | 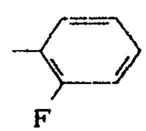 | $OCH_3$ | N | N | $C-OCH_3$ | 189 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 821 | 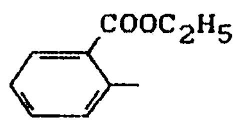 | H | 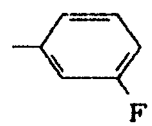 | $OCH_3$ | N | N | $C-OCH_3$ | 187 |
| 822 | 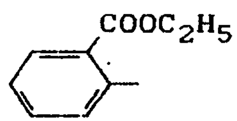 | H | 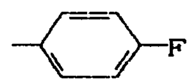 | $OCH_3$ | N | N | $C-OCH_3$ | 200-203 |
| 823 | 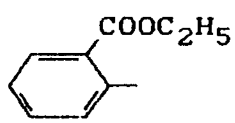 | H | 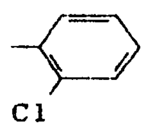 | $OCH_3$ | N | N | $C-OCH_3$ | 188-190 |
| 824 | 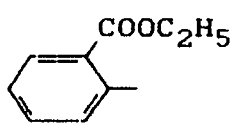 | H | 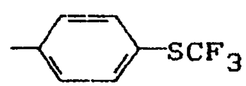 | $OCH_3$ | N | N | $C-OCH_3$ | 195-196 |
| 825 | 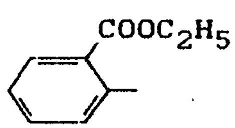 | H | 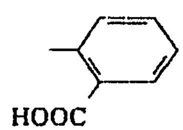 | $OCH_3$ | N | N | $C-OCH_3$ | 201-202 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 826 | 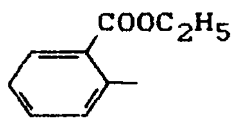 | H | $-CH=CH-$ 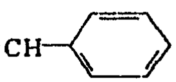 | $OCH_3$ | N | N | $C-OCH_3$ | 201-202 |
| 827 | 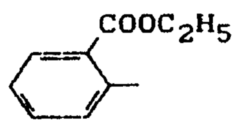 | | $-(CH_2)_5-$ | $OCH_3$ | N | N | $C-OCH_3$ | 164-165 |
| 828 | 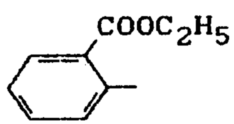 | H | $CH(CH_3)_2$ | $OCH_3$ | N | N | $C-OCH_3$ | 135-138 |
| 829 | 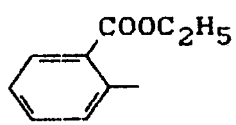 | H | $C(CH_3)_3$ | $OCH_3$ | N | N | $C-OCH_3$ | 150-151 |
| 830 | 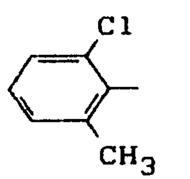 | H | 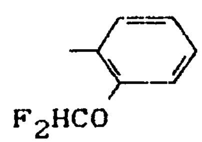 | $OCH_3$ | N | N | $C-OCH_3$ | 187 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 831 | 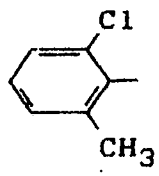 | H | 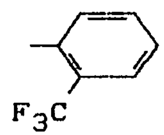 | $OCH_3$ | N | N | $C-OCH_3$ | 113-118 |
| 832 | 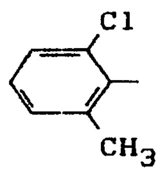 | H | 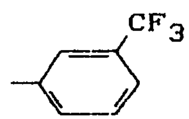 | $OCH_3$ | N | N | $C-OCH_3$ | 187 |
| 833 | 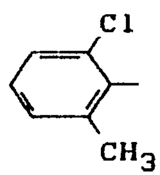 | H | 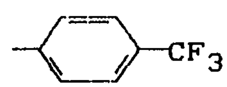 | $OCH_3$ | N | N | $C-OCH_3$ | 200-205 |
| 834 | 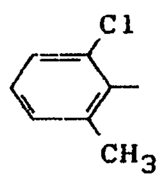 | H | 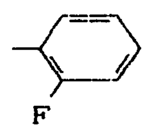 | $OCH_3$ | N | N | $C-OCH_3$ | 189 |
| 835 | 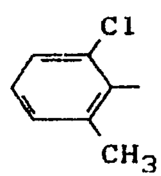 | H | 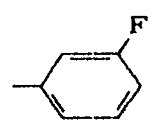 | $OCH_3$ | N | N | $C-OCH_3$ | 193-194 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 836 | 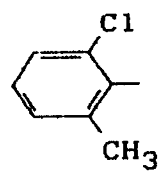 | H | 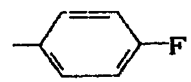 | $OCH_3$ | N | N | $C-OCH_3$ | 170 |
| 837 | 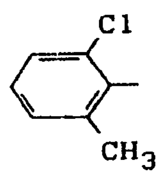 | H | 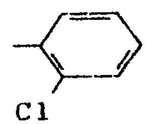 | $OCH_3$ | N | N | $C-OCH_3$ | 220-222 |
| 838 | 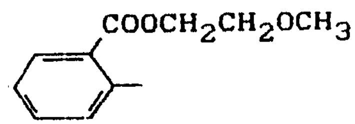 | $CH_3$ | $CH_3$ | $OCH_3$ | N | N | $C-C_2H_5$ | 142 |
| 839 | 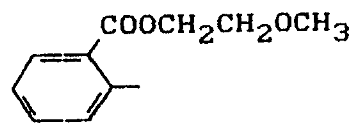 | H | $C_6H_5$ | $OCH_3$ | N | N | $C-C_2H_5$ | 140-141 |
| 840 | 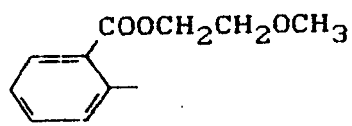 | H | $C_6H_5$ | $OC_2H_5$ | N | N | $C-CH_3$ | 150-152 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 841 | 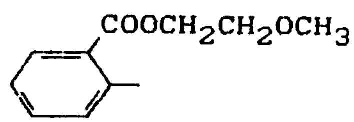 | $CH_3$ | $CH_3$ | $OC_2H_5$ | N | N | $C-CH_3$ | 149-152 |
| 842 | 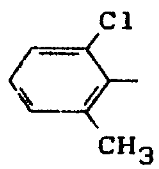 | H | 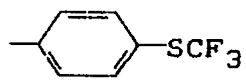 | $OCH_3$ | N | N | $C-OCH_3$ | 177 |
| 843 | 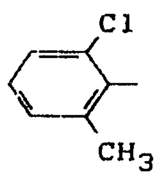 | H | 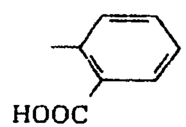 | $OCH_3$ | N | N | $C-OCH_3$ | 180 |
| 844 | 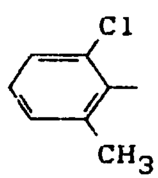 | H | $-CH=CH-$ 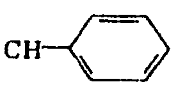 | $OCH_3$ | N | N | $C-OCH_3$ | 165-168 |
| 845 | 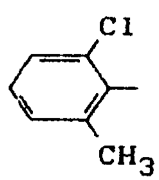 | $-(CH_2)_5-$ | | $OCH_3$ | N | N | $C-OCH_3$ | 193-195 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 846 | 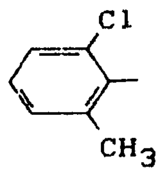 | H | $CH(CH_3)_2$ | $OCH_3$ | N | N | $C-OCH_3$ | 165-168 |
| 847 | 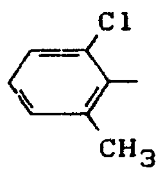 | H | $C(CH_3)_3$ | $OCH_3$ | N | N | $C-OCH_3$ | 84 |
| 848 | 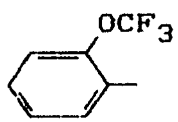 | H | 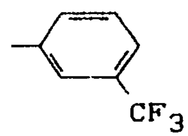 | $OCH_3$ | N | N | $C-CH_3$ | 175-176 |
| 849 | 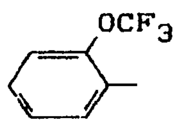 | H | 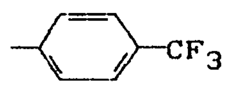 | $OCH_3$ | N | N | $C-CH_3$ | 158 |
| 850 | 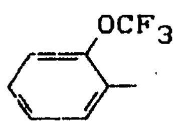 | H | 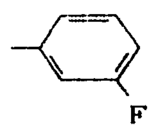 | $OCH_3$ | N | N | $C-CH_3$ | 153-160 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 851 | 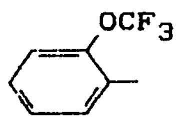 | H | 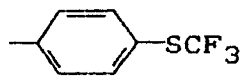 | $OCH_3$ | N | N | $C-CH_3$ | 139 |
| 852 | 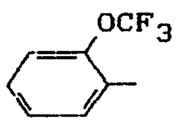 | H | 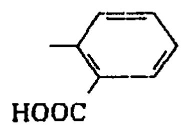 | $OCH_3$ | N | N | $C-CH_3$ | 183-186 |
| 853 | 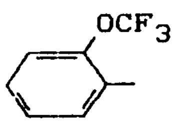 | $-(CH_2)_5-$ | | $OCH_3$ | N | N | $C-CH_3$ | 140 |
| 854 | 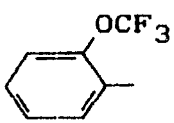 | $CH_3$ | $CH_2CH(CH_3)_2$ | $OCH_3$ | N | N | $C-CH_3$ | 102 |
| 855 | 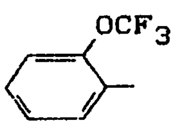 | H | 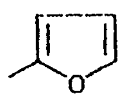 | $OCH_3$ | N | N | $C-CH_3$ | 168 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 856 | 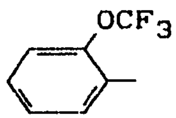 | H | 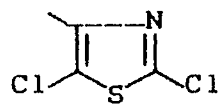 | $OCH_3$ | N | N | $C-CH_3$ | 176-178 |
| 857 | 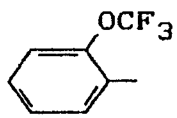 | H | 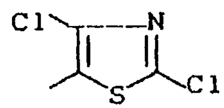 | $OCH_3$ | N | N | $C-CH_3$ | 198 |
| 858 | 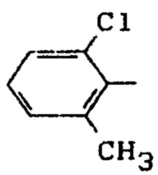 | H | 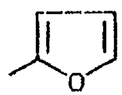 | $OCH_3$ | N | N | $C-OCH_3$ | 139 |
| 859 | 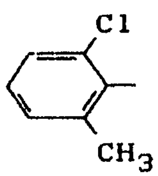 | H | 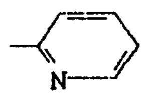 | $OCH_3$ | N | N | $C-OCH_3$ | 145-161 |
| 860 | 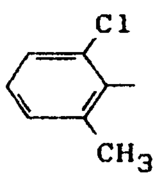 | H | 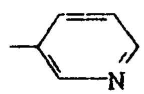 | $OCH_3$ | N | N | $C-OCH_3$ | 172 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 861 | 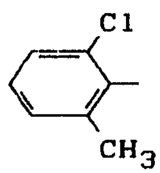 | H | 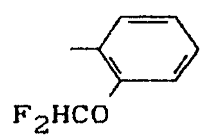 | $OCH_3$ | N | N | $C-OCH_3$ | 195-197 |
| 862 | 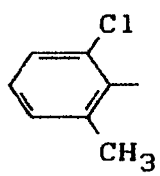 | H | 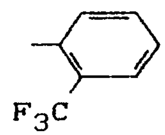 | $OCH_3$ | N | N | $C-OCH_3$ | 126-131 |
| 863 | 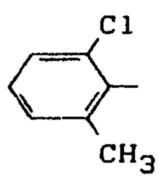 | H | 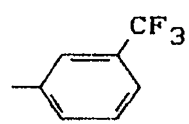 | $OCH_3$ | N | N | $C-OCH_3$ | 198-202 |
| 864 | 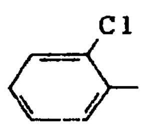 | $CH_3$ | $CH_3$ | $OCH_3$ | N | N | $C-C_2H_5$ | 175-177 |
| 865 | 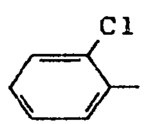 | H | $C_6H_5$ | $OCH_3$ | N | N | $C-C_2H_5$ | 189 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 866 | 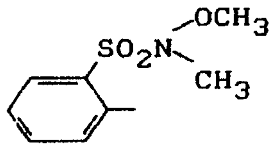 | H | $C_6H_5$ | $OCH_3$ | N | N | $C-OCH_3$ | 198-200 |
| 867 | 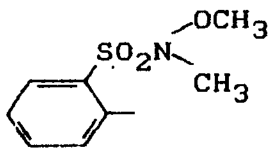 | $CH_3$ | $CH_3$ | $OCH_3$ | N | N | $C-OCH_3$ | 203-206 |
| 868 | 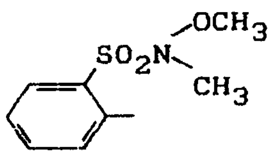 | $CH_3$ | $CH_3$ | $CH_3$ | N | N | $C-CH_3$ | 239 |
| 869 | 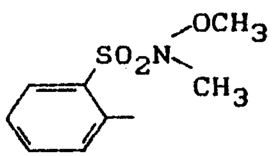 | H | $C_6H_5$ | $CH_3$ | N | N | $C-CH_3$ | 238-243 |
| 870 | 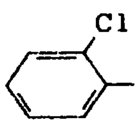 | $CH_3$ | $CH_3$ | $SCH_3$ | N | N | $C-CH_3$ | 188 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 871 | 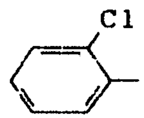 | H | $C_6H_5$ | $SCH_3$ | N | N | $C-CH_3$ | 216 |
| 872 | 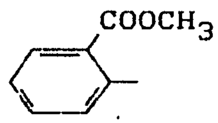 | $CH_3$ | $CH_3$ | $SCH_3$ | N | N | $C-CH_3$ | 182-187 |
| 873 | 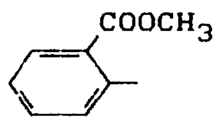 | H | $C_6H_5$ | $SCH_3$ | N | N | $C-CH_3$ | 210 |
| 874 | 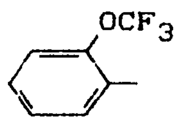 | $CH_3$ | $CH_3$ | $SCH_3$ | N | N | $C-CH_3$ | 162-165 |
| 875 | 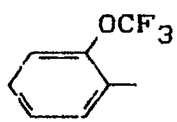 | H | $C_6H_5$ | $SCH_3$ | N | N | $C-CH_3$ | 149 |
| 876 | 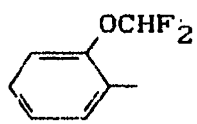 | H | $C_6H_5$ | $OC_2H_5$ | N | N | $C-CH_3$ | 199 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 877 | 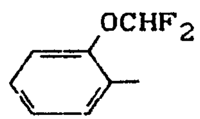 | $CH_3$ | $CH_3$ | $OC_2H_5$ | N | N | $C-CH_3$ | 150-155 |
| 878 | 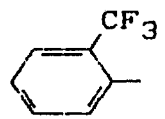 | $CH_3$ | $CH_3$ | $SCH_3$ | N | N | $C-CH_3$ | 158 |
| 879 | 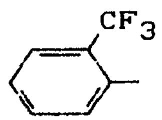 | H | $C_6H_5$ | $SCH_3$ | N | N | $C-CH_3$ | 206 |
| 880 | 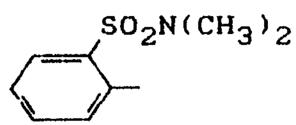 | $CH_3$ | $CH_3$ | $OCH_3$ | N | N | $C-C_2H_5$ | 196 |
| 881 | 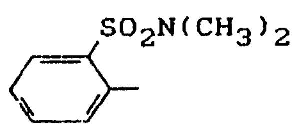 | H | $C_6H_5$ | $OCH_3$ | N | N | $C-C_2H_5$ | 188-191 |
| 882 | 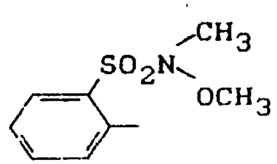 | $CH_3$ | $CH_3$ | $OC_2H_5$ | N | N | $C-CH_3$ | 189-193 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 883 | 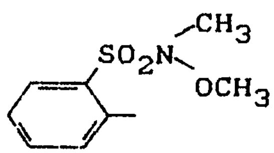 | H | $C_6H_5$ | $OC_2H_5$ | N | N | $C-CH_3$ | 180-187 |
| 884 | 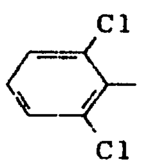 | $CH_3$ | $CH_3$ | $OCH_3$ | N | N | $C-CH_3$ | 174-181 |
| 885 | 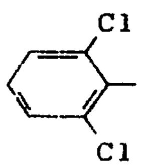 | H | $C_6H_5$ | $OCH_3$ | N | N | $C-CH_3$ | 171-185 |
| 886 | 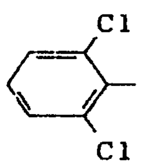 | H | 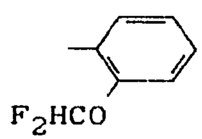 | $OCH_3$ | N | N | $C-CH_3$ | 190 |
| 887 | 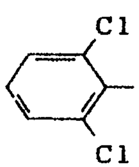 | H | 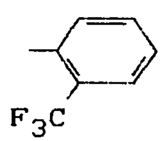 | $OCH_3$ | N | N | $C-CH_3$ | 180 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 888 | 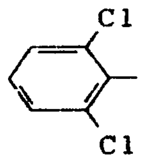 | H | 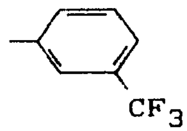 | $OCH_3$ | N | N | $C-CH_3$ | 180 |
| 889 | 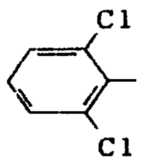 | H | 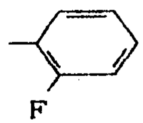 | $OCH_3$ | N | N | $C-CH_3$ | 224-225 |
| 890 | 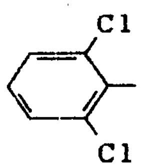 | H | 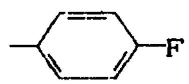 | $OCH_3$ | N | N | $C-CH_3$ | 220-221 |
| 891 | 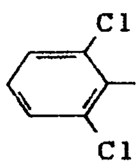 | H | 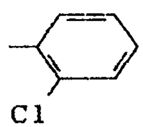 | $OCH_3$ | N | N | $C-CH_3$ | 212 |
| 892 | 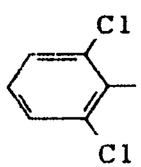 | H | 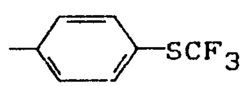 | $OCH_3$ | N | N | $C-CH_3$ | 147-149 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 893 | 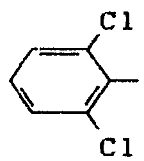 | H | -CH=CH- 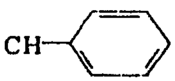 | $OCH_3$ | N | N | $C-CH_3$ | 211-215 |
| 894 | 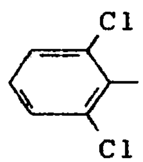 | $-(CH_2)_5-$ | | $OCH_3$ | N | N | $C-CH_3$ | 208 |
| 895 | 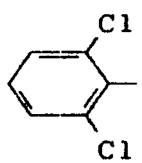 | H | $CH(CH_3)_2$ | $OCH_3$ | N | N | $C-CH_3$ | 198-202 |
| 896 | 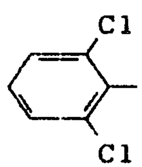 | $CH_3$ | $CH_2CH(CH_3)_2$ | $OCH_3$ | N | N | $C-CH_3$ | 167-169 |
| 897 | 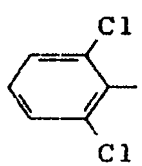 | H | $C(CH_3)_3$ | $OCH_3$ | N | N | $C-CH_3$ | 194 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 898 | 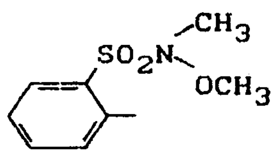 | H | 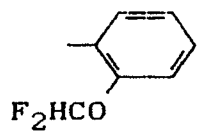 | $OCH_3$ | N | N | $C-CH_3$ | 165-169 |
| 899 | 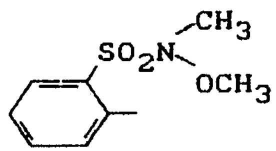 | H | 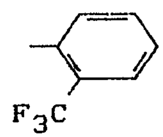 | $OCH_3$ | N | N | $C-CH_3$ | 163-188 |
| 900 | 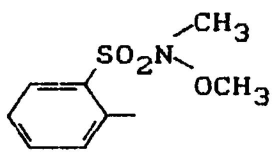 | H | 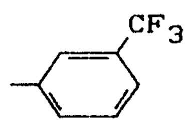 | $OCH_3$ | N | N | $C-CH_3$ | 204-206 |
| 901 | 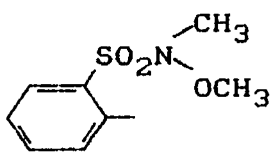 | H | 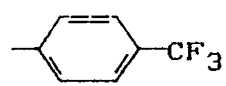 | $OCH_3$ | N | N | $C-CH_3$ | 207-209 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 902 | 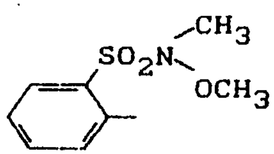 | H | 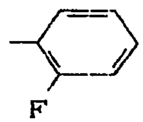 | $OCH_3$ | N | N | $C-CH_3$ | 220-223 |
| 903 | 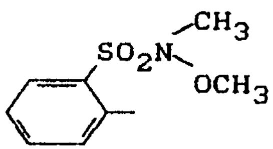 | H | 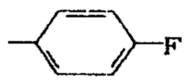 | $OCH_3$ | N | N | $C-CH_3$ | 208-209 |
| 904 | 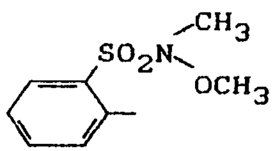 | H | 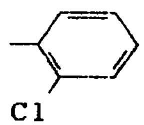 | $OCH_3$ | N | N | $C-CH_3$ | 205-210 |
| 905 | 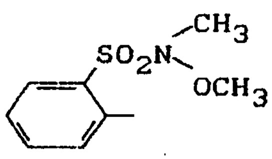 | H | 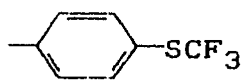 | $OCH_3$ | N | N | $C-CH_3$ | 206 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 906 | 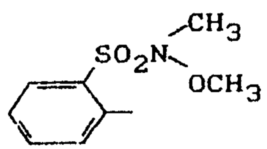 | H | 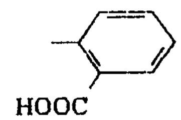 | $OCH_3$ | N | N | $C-CH_3$ | 216 |
| 907 | 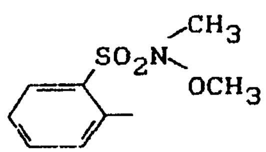 | H | 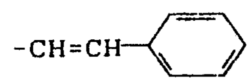 | $OCH_3$ | N | N | $C-CH_3$ | 226-228 |
| 908 | 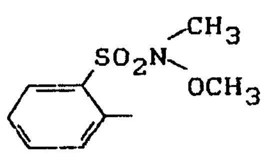 | | $-(CH_2)_5-$ | $OCH_3$ | N | N | $C-CH_3$ | 190-198 |
| 909 | 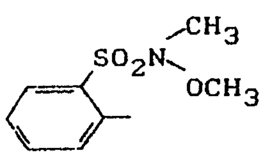 | H | $CH(CH_3)_2$ | $OCH_3$ | N | N | $C-CH_3$ | 155-159 |
| 910 | 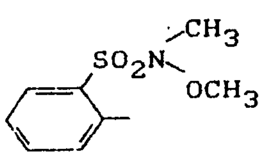 | H | $C(CH_3)_3$ | $OCH_3$ | N | N | $C-CH_3$ | $^1H$ NMR*) $\delta$ = 4,04 ppm ($OCH_3$) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 911 | 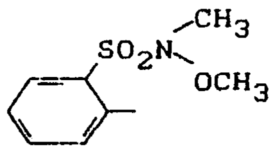 | $CH_3$ | $CH_2CH(CH_3)_2$ | $OCH_3$ | N | N | $C-CH_3$ | 161-163 |
| 912 | 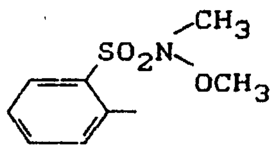 | H | $CH_3$ | $OCH_3$ | N | N | $C-CH_3$ | 149 |
| 913 | 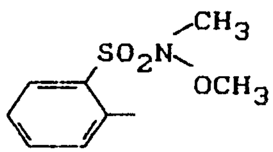 | H | 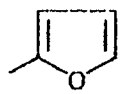 | $OCH_3$ | N | N | $C-CH_3$ | 208 |
| 914 | 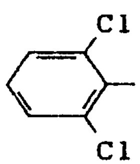 | H | 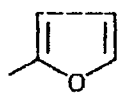 | $OCH_3$ | N | N | $C-CH_3$ | 210-215 |
| 915 | 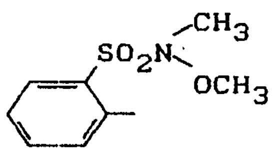 | H | 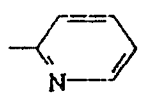 | $OCH_3$ | N | N | $C-CH_3$ | 186 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 916 | 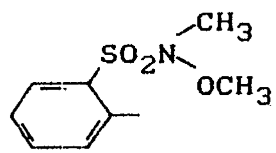 | H | 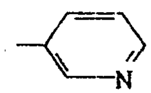 | $OCH_3$ | N | N | $C-CH_3$ | 180-185 |
| 917 | 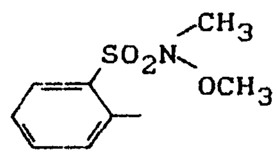 | H | 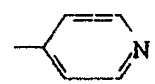 | $OCH_3$ | N | N | $C-CH_3$ | 175-178 |
| 918 | 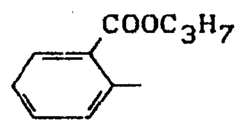 | $CH_3$ | $CH_3$ | $OCH_3$ | N | N | $C-C_2H_5$ | 145-148 |
| 919 | 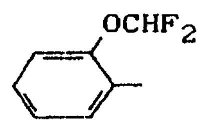 | H | $C(CH_3)_3$ | $OCH_3$ | N | N | $C-OCH_3$ | 153-156 |
| 920 | 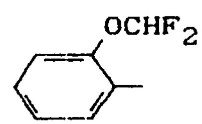 | $CH_3$ | $CH_2CH(CH_3)_2$ | $OCH_3$ | N | N | $C-OCH_3$ | 163 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 921 | 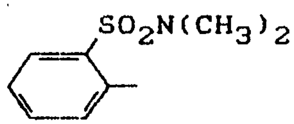 | H | $C(CH_3)_3$ | $OCH_3$ | N | N | $C-OCH_3$ | 136 |
| 922 | 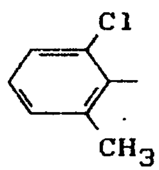 | H | $C_6H_5$ | $OCH_3$ | N | N | $C-CH_3$ | 181 |
| 923 | 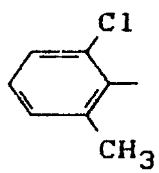 | $CH_3$ | $CH_3$ | $OCH_3$ | N | N | $C-CH_3$ | 175-176 |
| 924 | 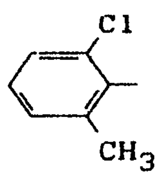 | H | 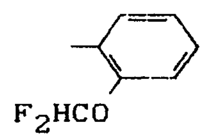 | $OCH_3$ | N | N | $C-CH_3$ | 167 |
| 925 | 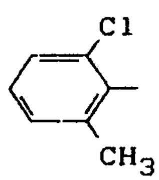 | H | 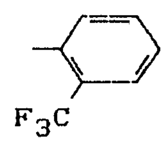 | $OCH_3$ | N | N | $C-CH_3$ | 151 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 926 | 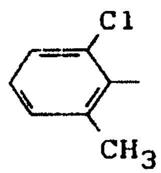 | H | 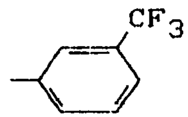 | $OCH_3$ | N | N | $C-CH_3$ | 172 |
| 927 | 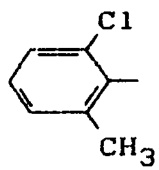 | H | 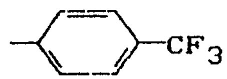 | $OCH_3$ | N | N | $C-CH_3$ | 129-132 |
| 928 | 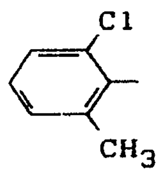 | H | 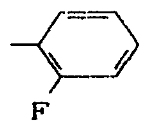 | $OCH_3$ | N | N | $C-CH_3$ | 182 |
| 929 | 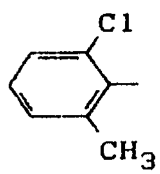 | H | 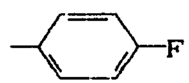 | $OCH_3$ | N | N | $C-CH_3$ | 202 |
| 930 | 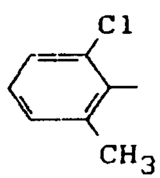 | H | 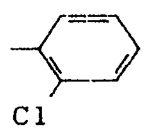 | $OCH_3$ | N | N | $C-CH_3$ | 208 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 931 | 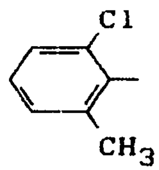 | H | 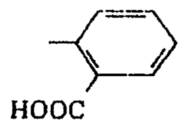 | $OCH_3$ | N | N | $C-CH_3$ | 172-173 |
| 932 | 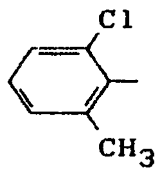 | H | $-CH=CH-$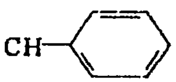 | $OCH_3$ | N | N | $C-CH_3$ | 180-183 |
| 933 | 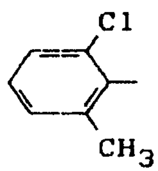 | $-(CH_2)_5-$ | | $OCH_3$ | N | N | $C-CH_3$ | 195 |
| 934 | 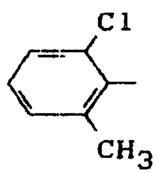 | H | $CH(CH_3)_2$ | $OCH_3$ | N | N | $C-CH_3$ | 148 |
| 935 | 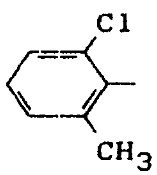 | H | $C(CH_3)_3$ | $OCH_3$ | N | N | $C-CH_3$ | 157 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 936 | 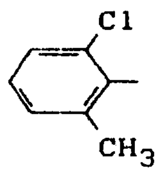 | $CH_3$ | $CH_2CH(CH_3)_2$ | $OCH_3$ | N | N | $C-CH_3$ | 130 |
| 937 | 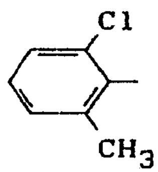 | H | 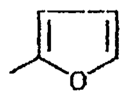 | $OCH_3$ | N | N | $C-CH_3$ | 160 |
| 938 | 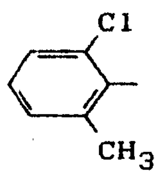 | H | 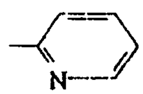 | $OCH_3$ | N | N | $C-CH_3$ | 161 |
| 939 | 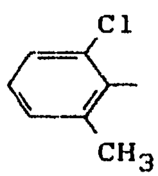 | H | 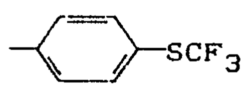 | $OCH_3$ | N | N | $C-CH_3$ | 140 |
| 940 | 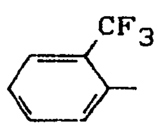 | H | 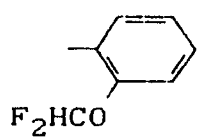 | $OCH_3$ | N | N | $C-CH_3$ | 177 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 941 | 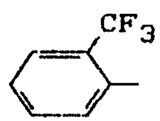 | H | 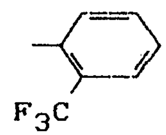 | $OCH_3$ | N | N | $C-CH_3$ | 193 |
| 942 | 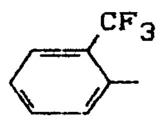 | H | 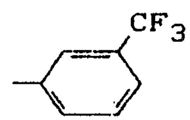 | $OCH_3$ | N | N | $C-CH_3$ | 183 |
| 943 | 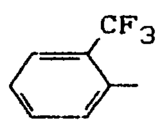 | H | 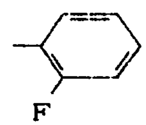 | $OCH_3$ | N | N | $C-CH_3$ | 201-203 |
| 944 | 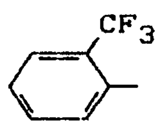 | H | 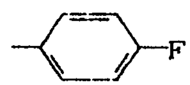 | $OCH_3$ | N | N | $C-CH_3$ | 209 |
| 945 | 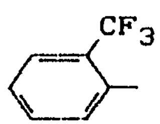 | H | 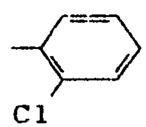 | $OCH_3$ | N | N | $C-CH_3$ | 184 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 946 | 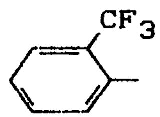 | H | 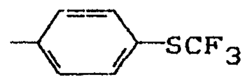 | $OCH_3$ | N | N | $C-CH_3$ | 149 |
| 947 | 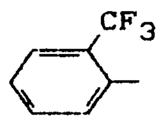 | H | 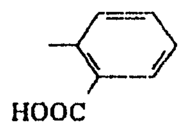 | $OCH_3$ | N | N | $C-CH_3$ | 150 |
| 948 | 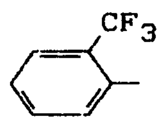 | H | -CH=CH- 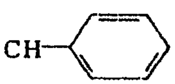 | $OCH_3$ | N | N | $C-CH_3$ | 184 |
| 949 | 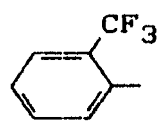 | $-(CH_2)_5-$ | | $OCH_3$ | N | N | $C-CH_3$ | 153 |
| 950 | 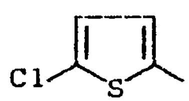 | H | $C_6H_5$ | $OCH_3$ | N | N | $C-OCH_3$ | 185 |
| 951 | 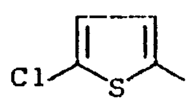 | $CH_3$ | $CH_3$ | $OCH_3$ | N | N | $C-CH_3$ | 148 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 952 | 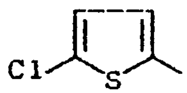 | H | $C_6H_5$ | $OCH_3$ | N | N | $C-CH_3$ | 206 |
| 953 | 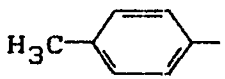 | $CH_3$ | $CH_3$ | $OCH_3$ | N | N | $C-CH_3$ | 149 |
| 954 | 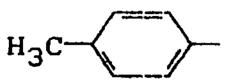 | H | $C_6H_5$ | $OCH_3$ | N | N | $C-CH_3$ | 153 |
| 955 | 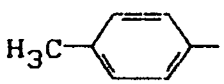 | $CH_3$ | $CH_3$ | $OCH_3$ | N | N | $C-OCH_3$ | 185 |
| 956 | 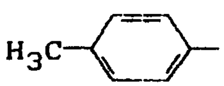 | H | $C_6H_5$ | $OCH_3$ | N | N | $C-OCH_3$ | 164 |
| 957 | 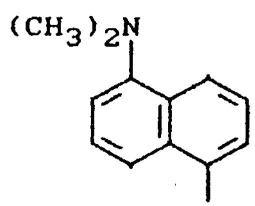 | $CH_3$ | $CH_3$ | $OCH_3$ | N | N | $C-CH_3$ | 210 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 958 | 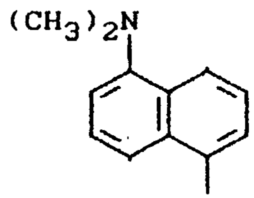 | H | $C_6H_5$ | $OCH_3$ | N | N | $C-CH_3$ | 241 |
| 959 | 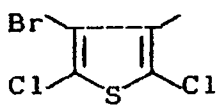 | H | $C_6H_5$ | $OCH_3$ | N | N | $C-OCH_3$ | 176 |
| 960 | 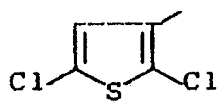 | $CH_3$ | $CH_3$ | $OCH_3$ | N | N | $C-OCH_3$ | 136 |
| 961 | 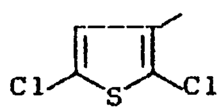 | H | $C_6H_5$ | $OCH_3$ | N | N | $C-OCH_3$ | 145 |
| 962 | 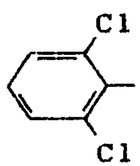 | H | 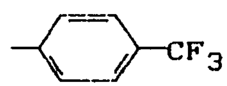 | $OCH_3$ | N | N | $C-OCH_3$ | 183 |
| 963 | 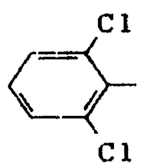 | H | 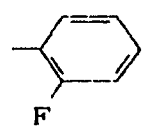 | $OCH_3$ | N | N | $C-OCH_3$ | 180-184 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 964 | 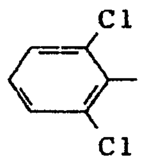 | H | 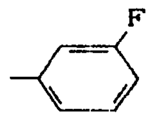 | $OCH_3$ | N | N | $C-OCH_3$ | 174-180 |
| 965 | 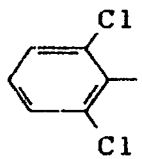 | H | 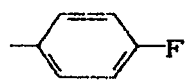 | $OCH_3$ | N | N | $C-OCH_3$ | 186-188 |
| 966 | 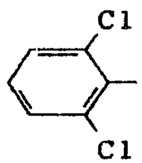 | H | 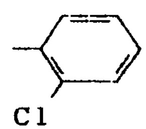 | $OCH_3$ | N | N | $C-OCH_3$ | 221 |
| 967 | 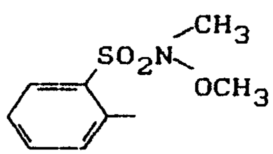 | H | 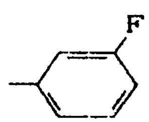 | $OCH_3$ | N | N | $C-CH_3$ | 216 |
| 968 | 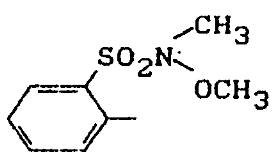 | H | 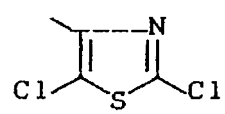 | $OCH_3$ | N | N | $C-CH_3$ | 177 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 969 | 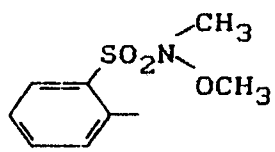 | H | 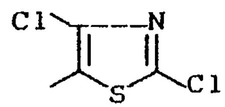 | $OCH_3$ | N | N | $C-CH_3$ | 199-204 |
| 970 | 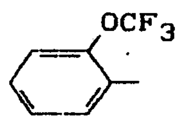 | H | 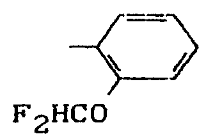 | $OCH_3$ | N | N | $C-OCH_3$ | 188-190 |
| 971 | 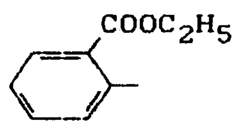 | $CH_3$ | $CH_2CH(CH_3)_2$ | $OCH_3$ | N | N | $C-OCH_3$ | 150-153 |
| 972 | 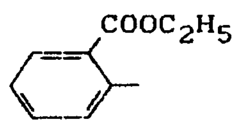 | H | $CH_3$ | $OCH_3$ | N | N | $C-OCH_3$ | 140-145 |
| 973 | 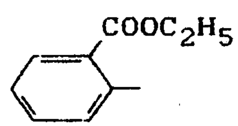 | H | 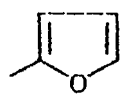 | $OCH_3$ | N | N | $C-OCH_3$ | 184 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 974 | 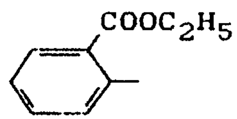 | H | 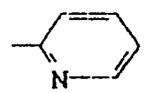 | $OCH_3$ | N | N | $C-OCH_3$ | 148 |
| 975 | 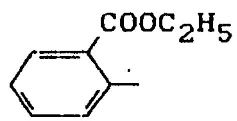 | H | 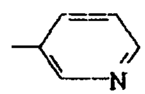 | $OCH_3$ | N | N | $C-OCH_3$ | 146 |
| 976 | 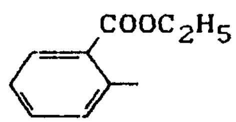 | H | 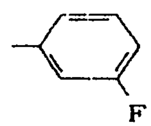 | $OCH_3$ | N | N | $C-CH_3$ | 168 |
| 977 | 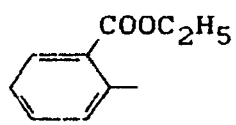 | H | 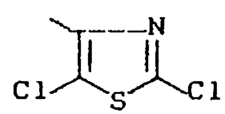 | $OCH_3$ | N | N | $C-CH_3$ | 156 |
| 978 | 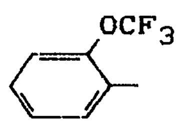 | H | 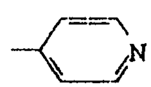 | $OCH_3$ | N | N | $C-CH_3$ | 152 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 979 | 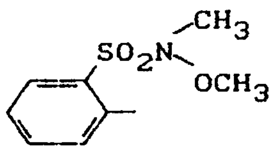 | H | 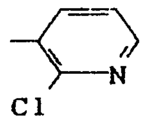 | $OCH_3$ | N | N | $C-CH_3$ | 175 |
| 980 | 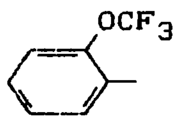 | H | 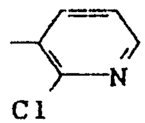 | $OCH_3$ | N | N | $C-CH_3$ | 149 |
| 981 | 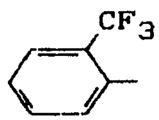 | H | 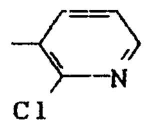 | $OCH_3$ | N | N | $C-CH_3$ | 173-175 |
| 982 | 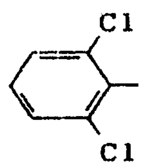 | H | 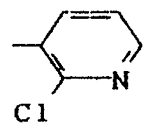 | $OCH_3$ | N | N | $C-CH_3$ | 215 |
| 983 | 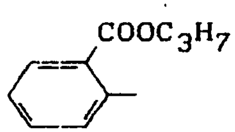 | $CH_3$ | $CH_3$ | $OC_2H_5$ | N | N | $C-CH_3$ | 146-148 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 984 | 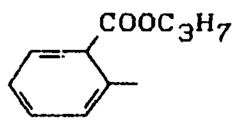 | H | $C_6H_5$ | $OC_2H_5$ | N | N | $C-CH_3$ | 155-158 |
| 985 | 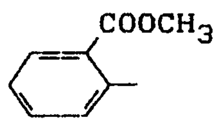 | H | $C_6H_5$ | $CH_3$ | N | N | $C-CH_3$ | 212-214 |
| 986 | 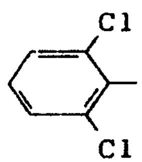 | H | 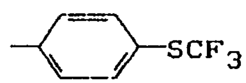 | $OCH_3$ | N | N | $C-OCH_3$ | 173-174 |
| 987 | 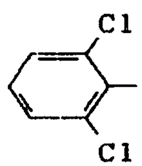 | H | 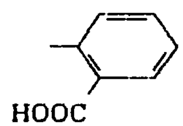 | $OCH_3$ | N | N | $C-OCH_3$ | 190-194 |
| 988 | 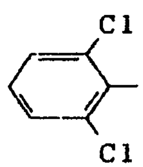 | H | -CH=CH- 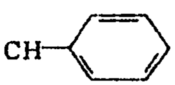 | $OCH_3$ | N | N | $C-OCH_3$ | 190-196 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 989 | 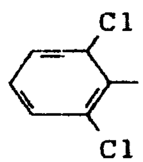 | | $-(CH_2)_5-$ | $OCH_3$ | N | N | $C-OCH_3$ | 223 |
| 990 | 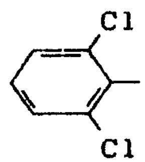 | H | $CH(CH_3)_2$ | $OCH_3$ | N | N | $C-OCH_3$ | 175-179 |
| 991 | 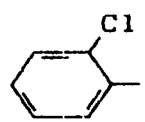 | H | $C_6H_5$ | $CH_3$ | N | N | $C-CH_3$ | 190 |
| 992 | 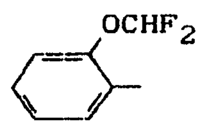 | H | $C_6H_5$ | $CH_3$ | N | N | $C-CH_3$ | 175 |
| 993 | 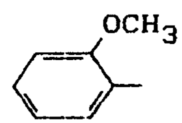 | H | $C_6H_5$ | $OC_2H_5$ | N | N | $C-CH_3$ | 197-199 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 994 | 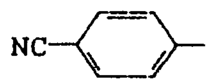 | H | $C_6H_5$ | $OCH_3$ | N | N | $C-OCH_3$ | 226-230 |
| 995 | 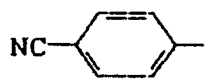 | $CH_3$ | $CH_3$ | $OCH_3$ | N | N | $C-OCH_3$ | 190 |
| 996 | 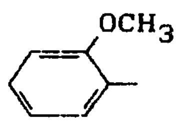 | H | $C_6H_5$ | $OCH_3$ | N | N | $C-C_2H_5$ | 195-199 |
| 997 | 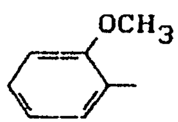 | H | $C_6H_5$ | $OCH_3$ | N | N | $C-CH_3$ | 166-168 |
| 998 | 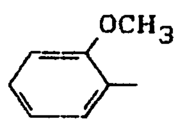 | $CH_3$ | $CH_3$ | $OCH_3$ | N | N | $C-CH_3$ | 167-168 |
| 999 | 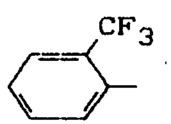 | H | $C_6H_5$ | $CH_3$ | N | N | $C-CH_3$ | 201-203 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1000 | 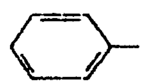 | $CH_3$ | $CH_3$ | $OCH_3$ | N | N | C-$OCH_3$ | 185-187 |
| 1001 | 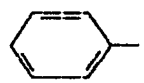 | H | $C_6H_5$ | $OCH_3$ | N | N | C-$OCH_3$ | 203-205 |
| 1002 | 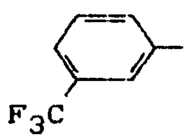 | H | $C_6H_5$ | $OCH_3$ | N | N | C-$CH_3$ | 194-198 |
| 1003 | 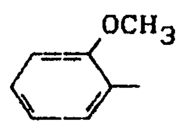 | $CH_3$ | $CH_3$ | $OC_2H_5$ | N | N | C-$CH_3$ | 176-178 |
| 1004 | 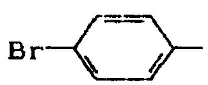 | H | $C_6H_5$ | $OCH_3$ | N | N | C-$CH_3$ | 224-226 |
| 1005 | 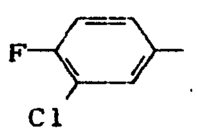 | H | $C_6H_5$ | $OCH_3$ | N | N | C-$CH_3$ | 181-183 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1006 | 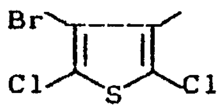 | H | $C_6H_5$ | $OCH_3$ | N | N | $C-CH_3$ | 174-175 |
| 1007 | 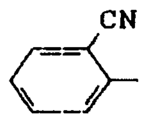 | $CH_3$ | $CH_3$ | $OCH_3$ | N | N | $C-CH_3$ | 180-182 |
| 1008 | 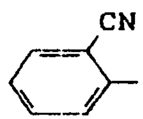 | H | $C_6H_5$ | $OCH_3$ | N | N | $C-CH_3$ | 214-216 |
| 1009 | 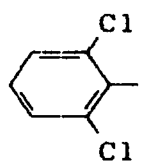 | H | $C(CH_3)_3$ | $OCH_3$ | N | N | $C-OCH_3$ | 171-172 |
| 1010 | 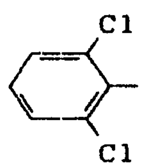 | $CH_3$ | $CH_2CH(CH_3)_2$ | $OCH_3$ | N | N | $C-OCH_3$ | 150-156 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1011 | 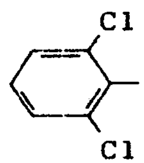 | H | $CH_3$ | $OCH_3$ | N | N | C-$OCH_3$ | 145-150 |
| 1012 | 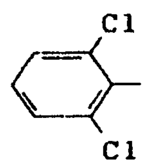 | H | 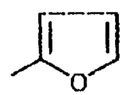 | $OCH_3$ | N | N | C-$OCH_3$ | 140 |
| 1013 | 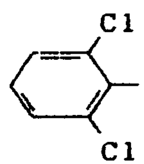 | H | 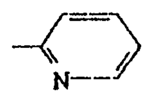 | $OCH_3$ | N | N | C-$OCH_3$ | 169-174 |
| 1014 | 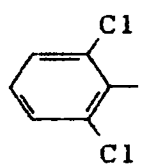 | H | 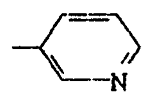 | $OCH_3$ | N | N | C-$OCH_3$ | 177 |
| 1015 | 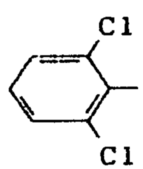 | H | 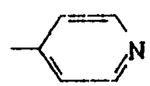 | $OCH_3$ | N | N | C-$OCH_3$ | 114-120 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1016 | 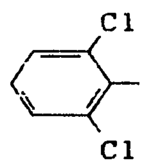 | H | 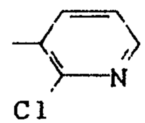 | $OCH_3$ | N | N | $C-OCH_3$ | 196-199 |
| 1017 | 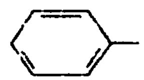 | $CH_3$ | $CH_3$ | $OCH_3$ | N | N | $C-CH_3$ | 173 |
| 1018 | 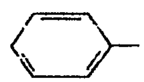 | H | $C_6H_5$ | $OCH_3$ | N | N | $C-CH_3$ | 186 |
| 1019 | 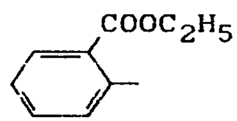 | H | 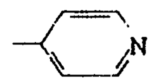 | $OCH_3$ | N | N | $C-OCH_3$ | 193 |
| 1020 | 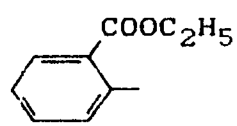 | H | 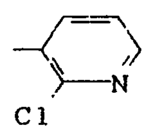 | $OCH_3$ | N | N | $C-OCH_3$ | 171 |
| 1021 | 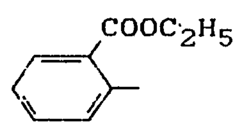 | H | 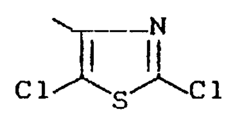 | $OCH_3$ | N | N | $C-OCH_3$ | 153-156 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1022 | 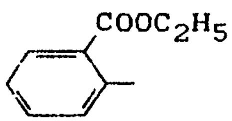 | H | 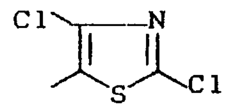 | $OCH_3$ | N | N | $C-OCH_3$ | 205 |
| 1023 | 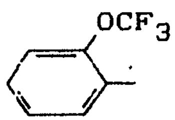 | H | 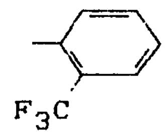 | $OCH_3$ | N | N | $C-OCH_3$ | 162-169 |
| 1024 | 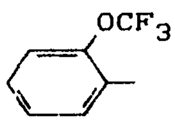 | H | 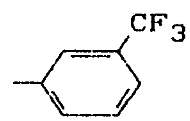 | $OCH_3$ | N | N | $C-OCH_3$ | 145 |
| 1025 | 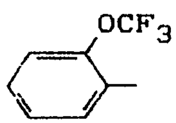 | H | 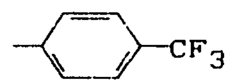 | $OCH_3$ | N | N | $C-OCH_3$ | 158 |
| 1026 | 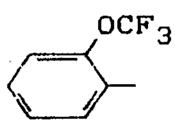 | H | 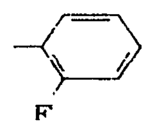 | $OCH_3$ | N | N | $C-OCH_3$ | 204 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1027 | 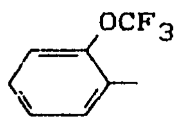 | H | 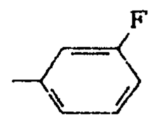 | $OCH_3$ | N | N | C-$OCH_3$ | 179 |
| 1028 | 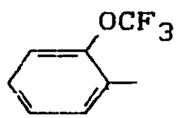 | H | 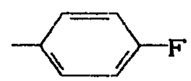 | $OCH_3$ | N | N | C-$OCH_3$ | 178-182 |
| 1029 | 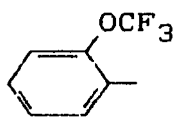 | H | 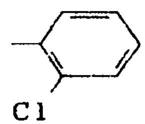 | $OCH_3$ | N | N | C-$OCH_3$ | 190-192 |
| 1030 | 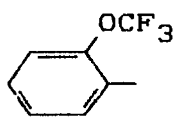 | H | 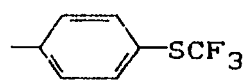 | $OCH_3$ | N | N | C-$OCH_3$ | 142 |
| 1031 | 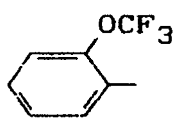 | H | 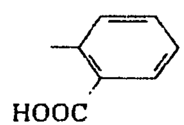 | $OCH_3$ | N | N | C-$OCH_3$ | 223 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1032 | 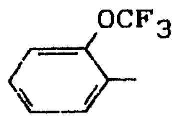 | H | -CH=CH- 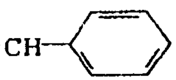 | $OCH_3$ | N | N | $C-OCH_3$ | 206 |
| 1033 | 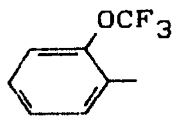 | | $-(CH_2)_5-$ | $OCH_3$ | N | N | $C-OCH_3$ | 134 |
| 1034 | 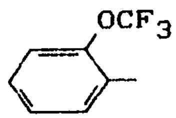 | H | $CH(CH_3)_2$ | $OCH_3$ | N | N | $C-OCH_3$ | 119 |
| 1035 | 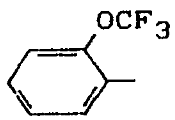 | H | $C(CH_3)_3$ | $OCH_3$ | N | N | $C-OCH_3$ | 152-155 |
| 1036 | 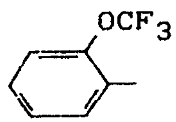 | $CH_3$ | $CH_2CH(CH_3)_2$ | $OCH_3$ | N | N | $C-OCH_3$ | 141 |
| 1037 | 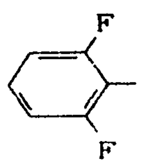 | $CH_3$ | $CH_3$ | $OCH_3$ | N | N | $C-CH_3$ | 171 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1038 | 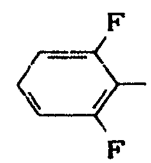 | H | $C_6H_5$ | $OCH_3$ | N | N | $C-CH_3$ | 236 |
| 1039 | 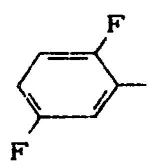 | $CH_3$ | $CH_3$ | $OCH_3$ | N | N | $C-CH_3$ | 174-175 |
| 1040 | 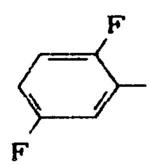 | H | $C_6H_5$ | $OCH_3$ | N | N | $C-CH_3$ | 193 |
| 1041 | 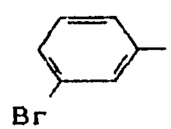 | $CH_3$ | $CH_3$ | $OCH_3$ | N | N | $C-CH_3$ | 134 |
| 1042 | 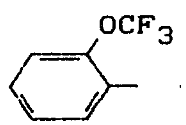 | H | $CH_3$ | $OCH_3$ | N | N | $C-OCH_3$ | 127-135 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1043 | 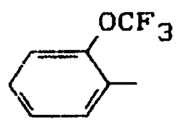 | H | 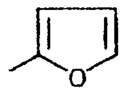 | $OCH_3$ | N | N | $C-OCH_3$ | 176-179 |
| 1044 | 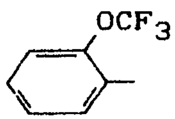 | H | 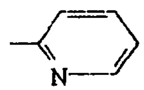 | $OCH_3$ | N | N | $C-OCH_3$ | 151 |
| 1045 | 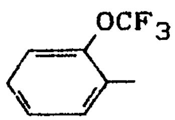 | H | 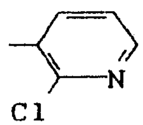 | $OCH_3$ | N | N | $C-OCH_3$ | 186-193 |
| 1046 | 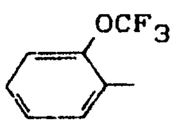 | H | $C_6H_5$ | $CH_3$ | N | N | $C-CH_3$ | 193-195 |
| 1047 | 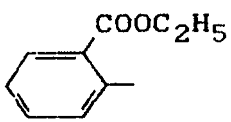 | $CH_3$ | $CH_3$ | $CH_3$ | N | N | $C-CH_3$ | 103-105 |
| 1048 | 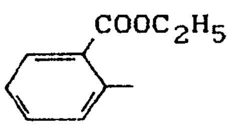 | H | $C_6H_5$ | $CH_3$ | N | N | $C-CH_3$ | 74 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1049 | 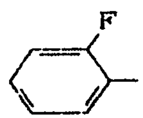 | H | $C_6H_5$ | $CH_3$ | N | N | $C-CH_3$ | 187 |
| 1050 | 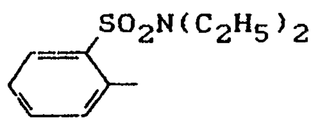 | H | 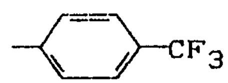 | $OCH_3$ | N | N | $C-OCH_3$ | 205-206 |
| 1051 | 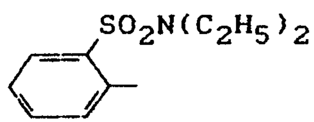 | H | 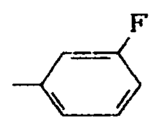 | $OCH_3$ | N | N | $C-OCH_3$ | 180-183 |
| 1052 | 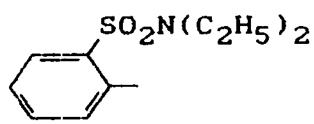 | H | 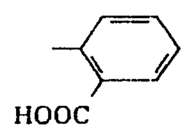 | $OCH_3$ | N | N | $C-OCH_3$ | $^1H$ NMR*) $\delta$ = 3,98 ppm ($OCH_3$) |
| 1053 | 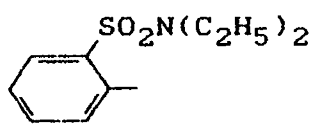 | H | 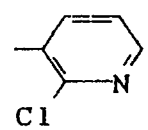 | $OCH_3$ | N | N | $C-OCH_3$ | 132-134 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1054 | 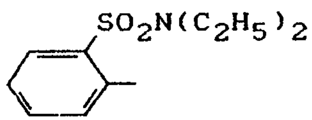 | H | 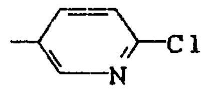 | $OCH_3$ | N | N | $C-OCH_3$ | 222-224 |
| 1055 | 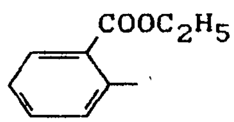 | H | 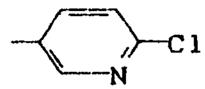 | $OCH_3$ | N | N | $C-OCH_3$ | 204-205 |
| 1056 | 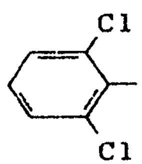 | H | 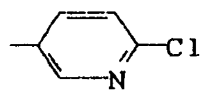 | $OCH_3$ | N | N | $C-OCH_3$ | 198-205 |
| 1057 | 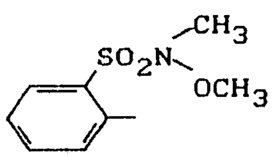 | H | 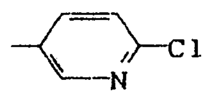 | $OCH_3$ | N | N | $C-CH_3$ | 202-204 |
| 1058 | 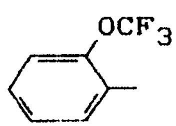 | H | 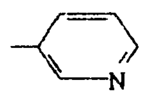 | $OCH_3$ | N | N | $C-CH_3$ | 189 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1059 | 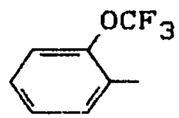 | H | 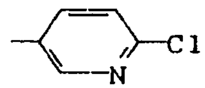 | $OCH_3$ | N | N | $C-CH_3$ | 117-120 |
| 1060 | 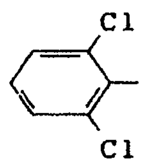 | H | 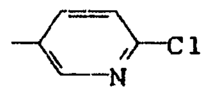 | $OCH_3$ | N | N | $C-CH_3$ | 211-216 |
| 1061 | 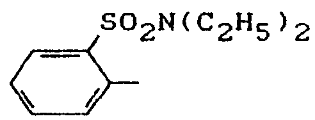 | $CH_3$ | $C_2H_5$ | $OCH_3$ | N | N | $C-OCH_3$ | 156-160 |
| 1062 | 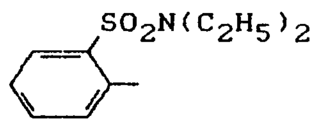 | H | $-C(CH_3)=CHC_2H_5$ | $OCH_3$ | N | N | $C-OCH_3$ | 176 |
| 1063 | 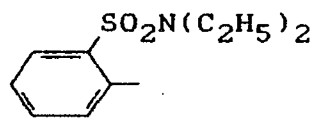 | $CH_3$ | $CH_2OCH_3$ | $OCH_3$ | N | N | $C-OCH_3$ | 210 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1064 | 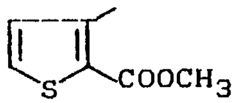 | $CH_3$ | $CH_2CH(CH_3)_2$ | $OCH_3$ | N | N | $C-CH_3$ | 115 |
| 1065 | 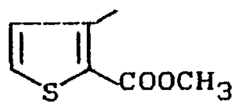 | H | $C(CH_3)_3$ | $OCH_3$ | N | N | $C-CH_3$ | 214 |
| 1066 | 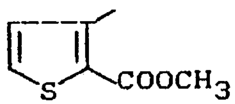 | H | $CH(C_2H_5)_2$ | $OCH_3$ | N | N | $C-CH_3$ | 159 |
| 1067 | 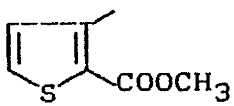 | $CH_3$ | $C_2H_5$ | $OCH_3$ | N | N | $C-CH_3$ | 137 |
| 1068 | 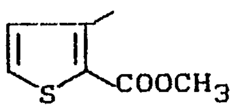 | H | $-C(CH_3)=CHC_2H_5$ | $OCH_3$ | N | N | $C-CH_3$ | 178-185 |
| 1069 | 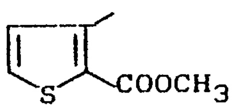 | $CH_3$ | $CH_2OCH_3$ | $OCH_3$ | N | N | $C-CH_3$ | 134-138 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1070 | 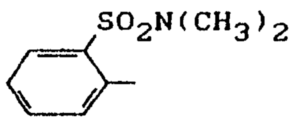 | H | $C_2H_5$ | $OCH_3$ | N | N | $C-CH_3$ | 165-166 |
| 1071 | 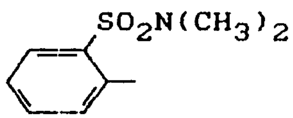 | H | $C_9H_{19}$-n | $OCH_3$ | N | N | $C-CH_3$ | 111-115 |
| 1072 | 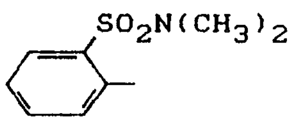 | H | $CH_2-C_6H_5$ | $OCH_3$ | N | N | $C-CH_3$ | 97-104 |
| 1073 | 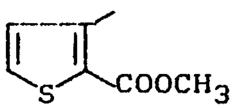 | H | $C_9H_{19}$-n | $OCH_3$ | N | N | $C-CH_3$ | 112-113 |
| 1074 | 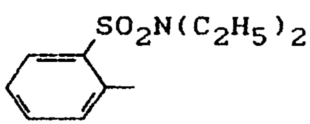 | H | $CH(C_2H_5)_2$ | $OCH_3$ | N | N | $C-OCH_3$ | 120 |
| 1075 | 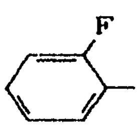 | $CH_3$ | $CH_3$ | $SCH_3$ | N | N | $C-CH_3$ | 166 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1076 | 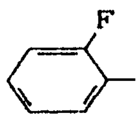 | $CH_3$ | $CH_3$ | $OC_2H_5$ | N | N | $C-CH_3$ | 144 |
| 1077 | 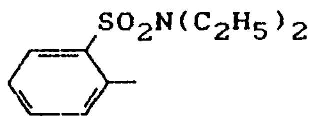 | H | $C_2H_5$ | $OCH_3$ | N | N | $C-OCH_3$ | 125 |
| 1078 | 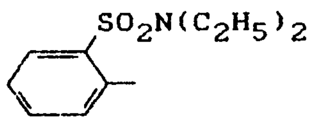 | H | $-(CH_2)_8-CH_3$ | $OCH_3$ | N | N | $C-OCH_3$ | 104 |
| 1079 | 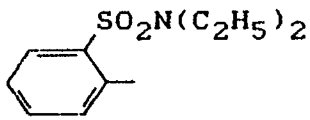 | H | $-CH_2-C_6H_5$ | $OCH_3$ | N | N | $C-OCH_3$ | 158 |
| 1080 | 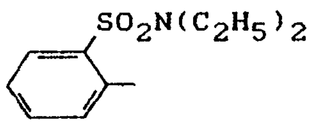 | $CH_3$ | $CH_2CH(CH_3)_2$ | $OCH_3$ | N | N | $C-CH_3$ | 158 |
| 1081 | 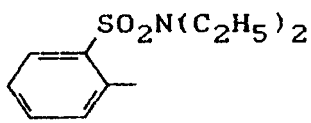 | H | $CH_3$ | $OCH_3$ | N | N | $C-CH_3$ | 158 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1082 | 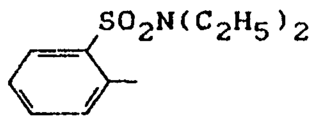 | H | 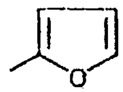 | $OCH_3$ | N | N | $C-CH_3$ | 230 |
| 1083 | 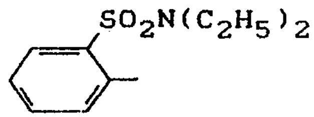 | H | 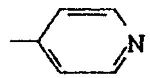 | $OCH_3$ | N | N | $C-CH_3$ | 196 |
| 1084 | 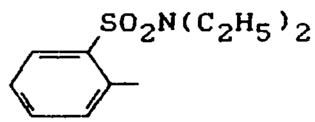 | H | 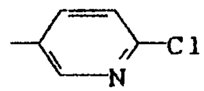 | $OCH_3$ | N | N | $C-CH_3$ | 240 |
| 1085 | 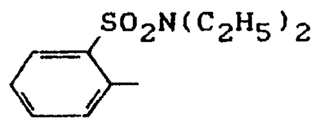 | H | 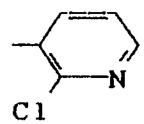 | $OCH_3$ | N | N | $C-CH_3$ | 195 |
| 1086 | 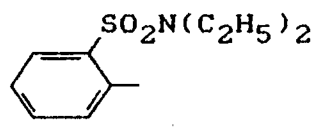 | H | 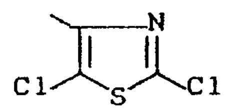 | $OCH_3$ | N | N | $C-CH_3$ | 166 |
| 1087 | 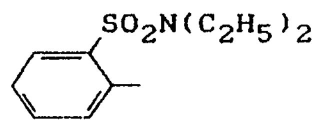 | H | 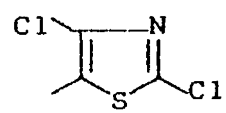 | $OCH_3$ | N | N | $C-CH_3$ | 179 |

Tabelle 3 - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 1088 | 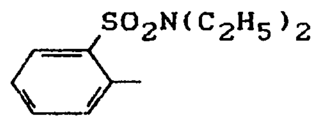 | H | $C_2H_5$ | $OCH_3$ | N | N | $C-CH_3$ | 160 |
| 1089 | 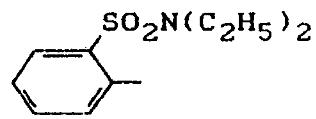 | H | $-(CH_2)_8-CH_3$ | $OCH_3$ | N | N | $C-CH_3$ | 98 |
| 1090 | 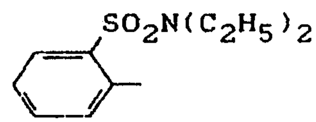 | H | $-CH_2-C_6H_5$ | $OCH_3$ | N | N | $C-CH_3$ | 180 |
| 1091 | 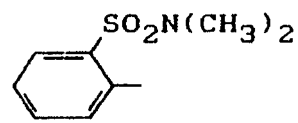 | H | $CH(C_2H_5)_2$ | $OCH_3$ | N | N | $C-CH_3$ | 124 |
| 1092 | 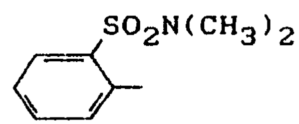 | $CH_3$ | $C_2H_5$ | $OCH_3$ | N | N | $C-CH_3$ | 190 |
| 1093 | 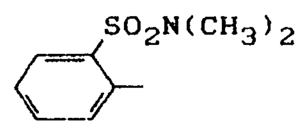 | H | $-\underset{CH_3}{\underset{\vert}{C}}=CHC_2H_5$ | $OCH_3$ | N | N | $C-CH_3$ | 145 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1094 | 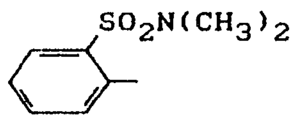 | $CH_3$ | $CH_2OCH_3$ | $OCH_3$ | N | N | $C-CH_3$ | 195 |
| 1095 | 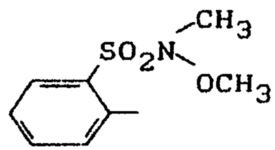 | H | 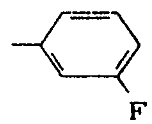 | $OCH_3$ | N | N | $C-OCH_3$ | 164 |
| 1096 | 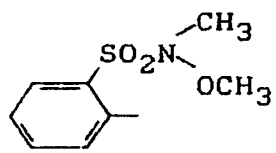 | H | 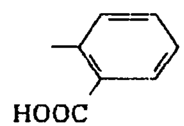 | $OCH_3$ | N | N | $C-OCH_3$ | 198 |
| 1097 | 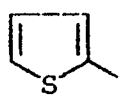 | $CH_3$ | $CH_3$ | $OCH_3$ | N | N | $C-CH_3$ | 200 |
| 1098 | 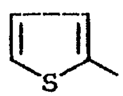 | H | $C_6H_5$ | $OCH_3$ | N | N | $C-CH_3$ | 212 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1099 | 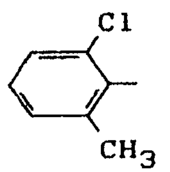 | H | 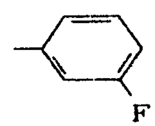 | $OCH_3$ | N | N | $C-CH_3$ | 188 |
| 1100 | 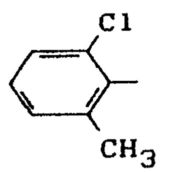 | H | 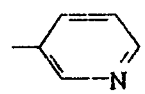 | $OCH_3$ | N | N | $C-CH_3$ | 172 |
| 1101 | 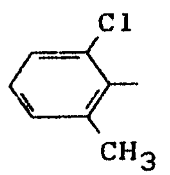 | H | 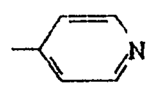 | $OCH_3$ | N | N | $C-CH_3$ | 196 |
| 1102 | 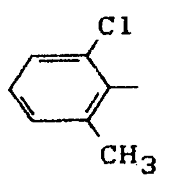 | H | 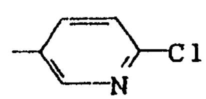 | $OCH_3$ | N | N | $C-CH_3$ | 134 |
| 1103 | 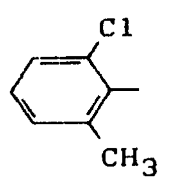 | H | 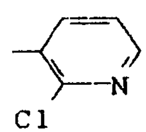 | $OCH_3$ | N | N | $C-CH_3$ | 198 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1104 | 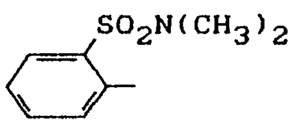 | H | $C(CH_3)_3$ | $OCH_3$ | N | N | $C-CH_3$ | 160 |
| 1105 | 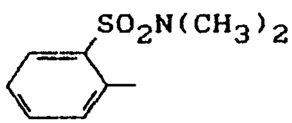 | H | $C_2H_5$ | $OCH_3$ | N | N | $C-OCH_3$ | 150 |
| 1106 | 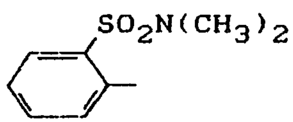 | H | $-(CH_2)_8-CH_3$ | $OCH_3$ | N | N | $C-OCH_3$ | 99 |
| 1107 | 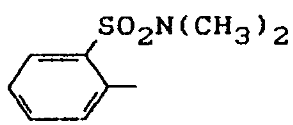 | H | $-CH_2-C_6H_5$ | $OCH_3$ | N | N | $C-OCH_3$ | 124 |
| 1108 | 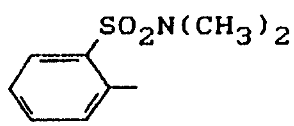 | H | $-\underset{CH_3}{\underset{\vert}{C}}=CHC_2H_5$ | $OCH_3$ | N | N | $C-OCH_3$ | 159 |
| 1109 | 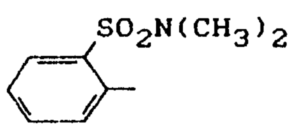 | $CH_3$ | $CH_2OCH_3$ | $OCH_3$ | N | N | $C-OCH_3$ | 166 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1110 | 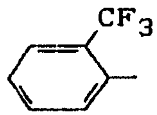 | H | 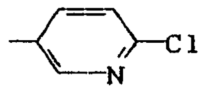 | $OCH_3$ | N | N | $C-CH_3$ | 176 |
| 1111 | 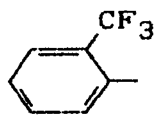 | H | 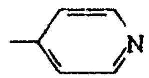 | $OCH_3$ | N | N | $C-OCH_3$ | 143 |
| 1112 | 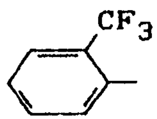 | H | 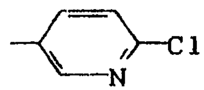 | $OCH_3$ | N | N | $C-OCH_3$ | 163 |
| 1113 | 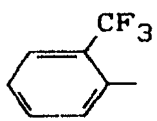 | H | 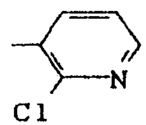 | $OCH_3$ | N | N | $C-OCH_3$ | 149 |
| 1114 | 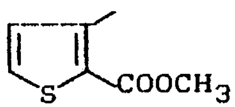 | H | $-CH_2-C_6H_5$ | $OCH_3$ | N | N | $C-CH_3$ | 99 |
| 1115 | 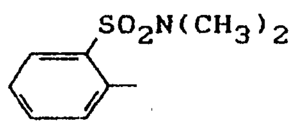 | H | $-(CH_2)_3-CH_3$ | $OCH_3$ | N | N | $C-OCH_3$ | 164 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1116 | 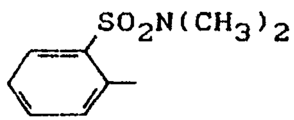 | H | $-(CH_2)_4-CH_3$ | $OCH_3$ | N | N | $C-OCH_3$ | 133 |
| 1117 | 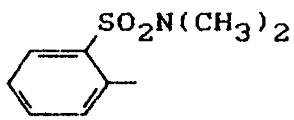 | H | $-(CH_2)_6-CH_3$ | $OCH_3$ | N | N | $C-OCH_3$ | 135 |
| 1118 | 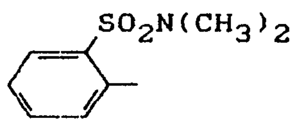 | H | $-(CH_2)_7-CH_3$ | $OCH_3$ | N | N | $C-OCH_3$ | 110 |
| 1119 | 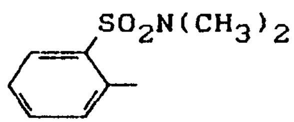 | H | $-(CH_2)_3-CH_3$ | $OCH_3$ | N | N | $C-CH_3$ | 154 |
| 1120 | 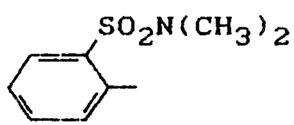 | H | $-(CH_2)_4-CH_3$ | $OCH_3$ | N | N | $C-CH_3$ | 148 |
| 1121 | 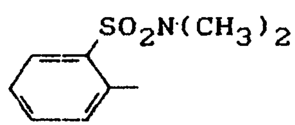 | H | $-(CH_2)_6-CH_3$ | $OCH_3$ | N | N | $C-CH_3$ | 136 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1122 | 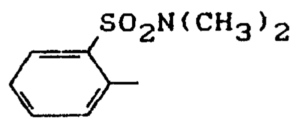 | H | $-(CH_2)_7-CH_3$ | $OCH_3$ | N | N | $C-CH_3$ | 135 |
| 1123 | 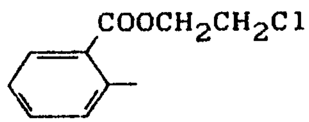 | H | 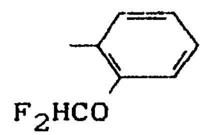 | $OCH_3$ | N | N | $C-CH_3$ | 160 |
| 1124 | 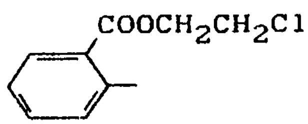 | H | 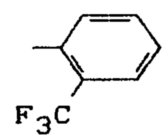 | $OCH_3$ | N | N | $C-CH_3$ | 154 |
| 1125 | 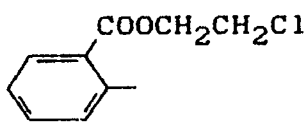 | H | 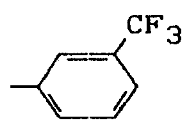 | $OCH_3$ | N | N | $C-CH_3$ | 181 |
| 1126 | 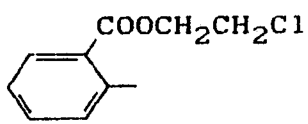 | H | 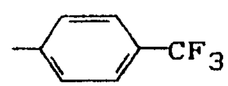 | $OCH_3$ | N | N | $C-CH_3$ | 129 |

Tabelle 3 - Fortsetzung

| Bsp.-Nr. | $R^1$ | $R^2$ | $R^3$ | $R^4$ | X | Y | Z | Schmelzpunkt ($^0C$) |
|---|---|---|---|---|---|---|---|---|
| 1127 | 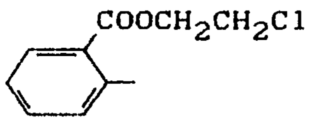 | H | 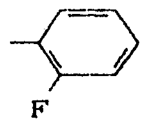 | $OCH_3$ | N | N | $C-CH_3$ | 165 |
| 1128 | 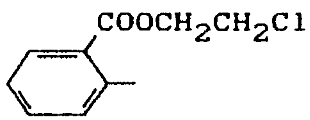 | H | 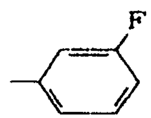 | $OCH_3$ | N | N | $C-CH_3$ | 164 |
| 1129 | 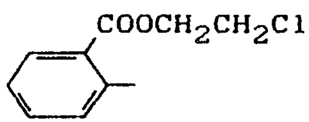 | H | 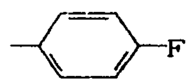 | $OCH_3$ | N | N | $C-CH_3$ | 176 |
| 1130 | 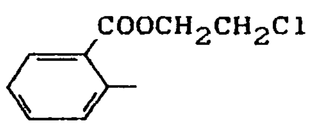 | H | 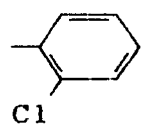 | $OCH_3$ | N | N | $C-CH_3$ | 120 |
| 1131 | 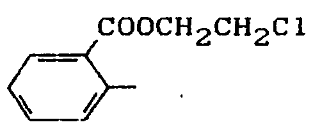 | H | 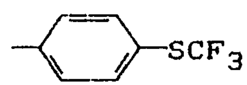 | $OCH_3$ | N | N | $C-CH_3$ | 145 |

Tabelle 3 - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 1132 | 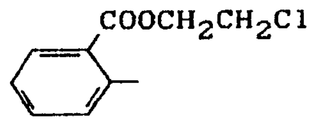 | H | 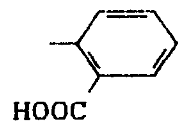 | $OCH_3$ | N | N | $C-CH_3$ | 208 |
| 1133 | 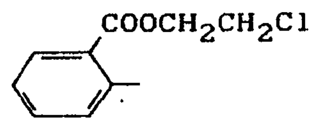 | H | 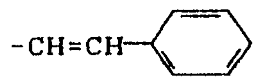 | $OCH_3$ | N | N | $C-CH_3$ | 194 |
| 1134 | 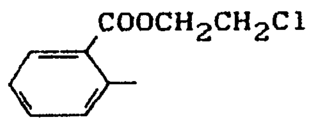 | $-(CH_2)_5-$ | | $OCH_3$ | N | N | $C-CH_3$ | 154 |
| 1135 | 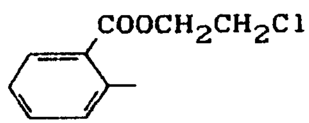 | H | $CH(CH_3)_2$ | $OCH_3$ | N | N | $C-CH_3$ | 141 |
| 1136 | 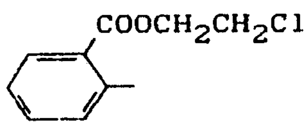 | H | $C(CH_3)_3$ | $OCH_3$ | N | N | $C-CH_3$ | 126 |
| 1137 | 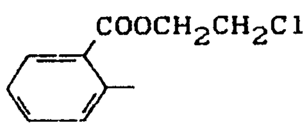 | $CH_3$ | $CH_2CH(CH_3)_2$ | $OCH_3$ | N | N | $C-CH_3$ | 96 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1138 | 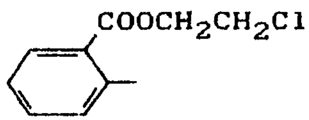 | H | $CH_3$ | $OCH_3$ | N | N | $C-CH_3$ | 134 |
| 1139 | 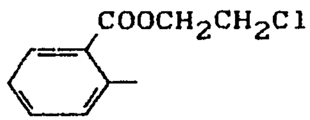 | H | 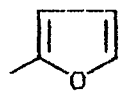 | $OCH_3$ | N | N | $C-CH_3$ | 145 |
| 1140 | 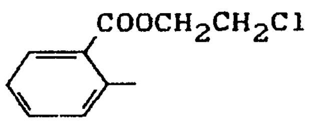 | H | 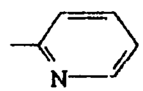 | $OCH_3$ | N | N | $C-CH_3$ | 169 |
| 1141 | 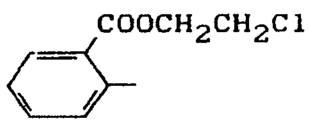 | H | 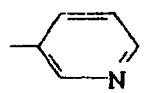 | $OCH_3$ | N | N | $C-CH_3$ | 148 |
| 1142 | 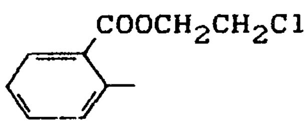 | H | 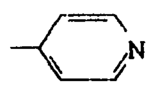 | $OCH_3$ | N | N | $C-CH_3$ | 150 |
| 1143 | 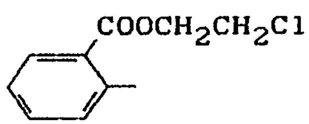 | H | 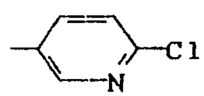 | $OCH_3$ | N | N | $C-CH_3$ | 188 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1144 | 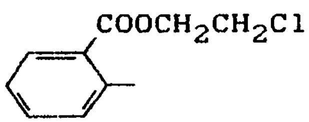 | H | 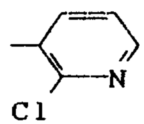 | $OCH_3$ | N | N | $C-CH_3$ | 137 |
| 1145 | 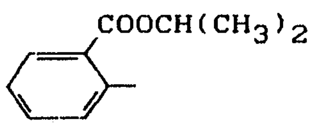 | H | 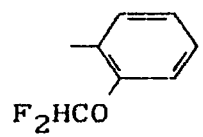 | $OCH_3$ | N | N | $C-CH_3$ | 184 |
| 1146 | 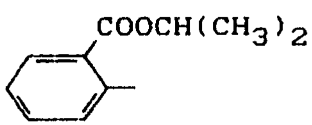 | H | 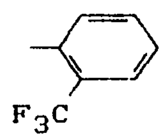 | $OCH_3$ | N | N | $C-CH_3$ | 139 |
| 1147 | 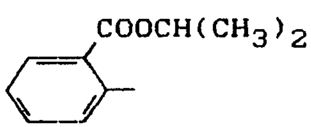 | H | 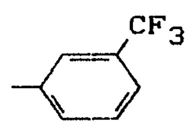 | $OCH_3$ | N | N | $C-CH_3$ | 170 |
| 1148 | 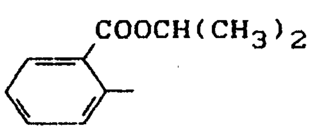 | H | 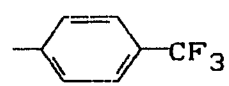 | $OCH_3$ | N | N | $C-CH_3$ | 215 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1149 | 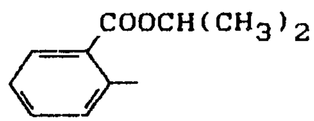 | H | 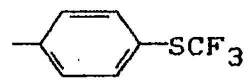 | $OCH_3$ | N | N | $C-CH_3$ | 174 |
| 1150 | 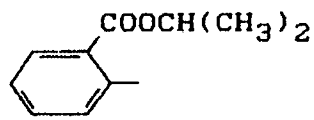 | H | 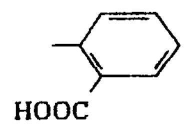 | $OCH_3$ | N | N | $C-CH_3$ | 209 |
| 1151 | 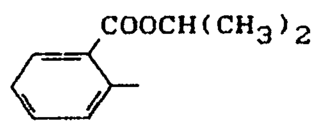 | $-(CH_2)_5-$ | | $OCH_3$ | N | N | $C-CH_3$ | 186 |
| 1152 | 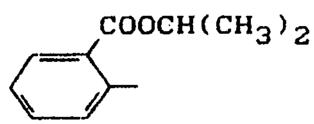 | H | 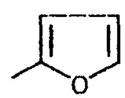 | $OCH_3$ | N | N | $C-CH_3$ | 144 |
| 1153 | 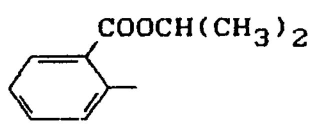 | H | 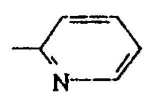 | $OCH_3$ | N | N | $C-CH_3$ | 185 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1154 | 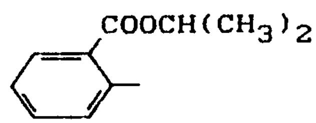 | H | 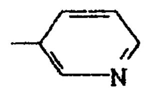 | $OCH_3$ | N | N | $C-CH_3$ | 181 |
| 1155 | 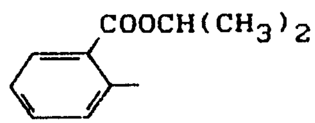 | H | 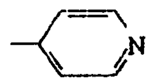 | $OCH_3$ | N | N | $C-CH_3$ | 183 |
| 1156 | 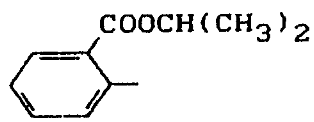 | H | 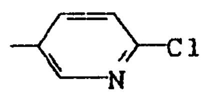 | $OCH_3$ | N | N | $C-CH_3$ | 182 |
| 1157 | 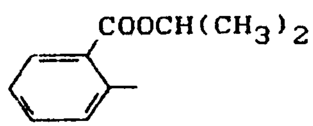 | H | 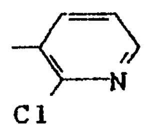 | $OCH_3$ | N | N | $C-CH_3$ | 157 |
| 1158 | 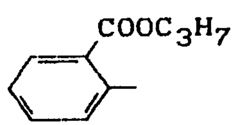 | H | 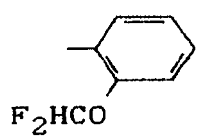 | $OCH_3$ | N | N | $C-CH_3$ | 143 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1159 | 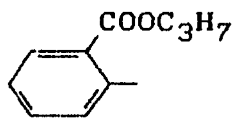 | H | 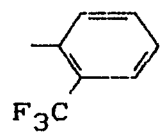 | $OCH_3$ | N | N | $C-CH_3$ | 150 |
| 1160 | 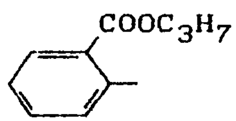 | H | 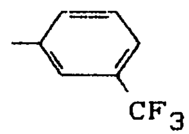 | $OCH_3$ | N | N | $C-CH_3$ | 158 |
| 1161 | 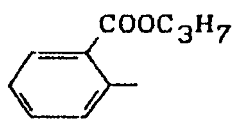 | H | 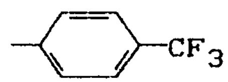 | $OCH_3$ | N | N | $C-CH_3$ | 169 |
| 1162 | 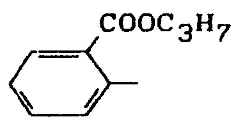 | H | 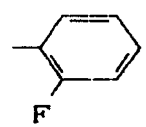 | $OCH_3$ | N | N | $C-CH_3$ | 144 |
| 1163 | 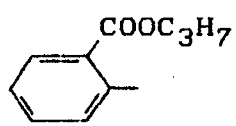 | H | 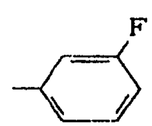 | $OCH_3$ | N | N | $C-CH_3$ | 156 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1164 | 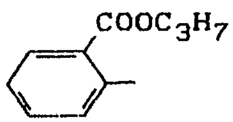 | H | 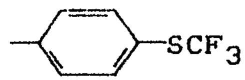 | $OCH_3$ | N | N | $C-CH_3$ | 143 |
| 1165 | 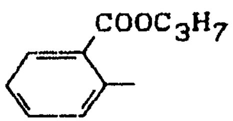 | $-(CH_2)_5-$ | | $OCH_3$ | N | N | $C-CH_3$ | 134 |
| 1166 | 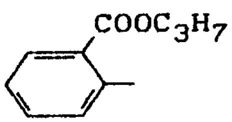 | H | 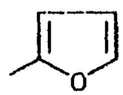 | $OCH_3$ | N | N | $C-CH_3$ | 141 |
| 1167 | 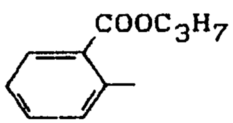 | H | 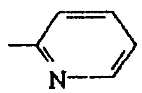 | $OCH_3$ | N | N | $C-CH_3$ | 129 |
| 1168 | 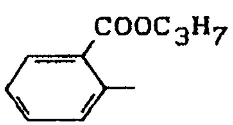 | H | 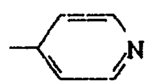 | $OCH_3$ | N | N | $C-CH_3$ | 142 |
| 1169 | 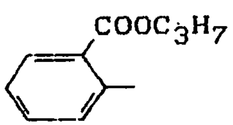 | H | 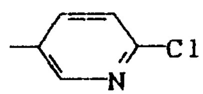 | $OCH_3$ | N | N | $C-CH_3$ | 164 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1170 | 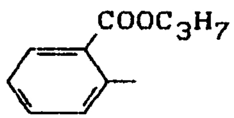 | H | 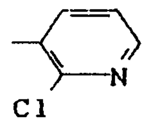 | $OCH_3$ | N | N | $C-CH_3$ | 139 |
| 1171 | 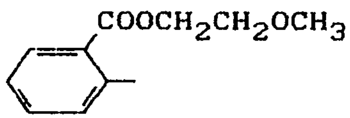 | H | 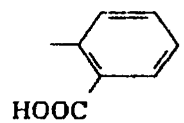 | $OCH_3$ | N | N | $C-CH_3$ | 189 |
| 1172 | 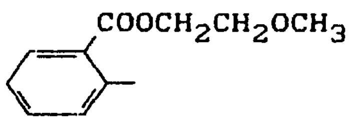 | H | 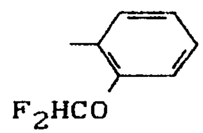 | $OCH_3$ | N | N | $C-CH_3$ | 154 |
| 1173 | 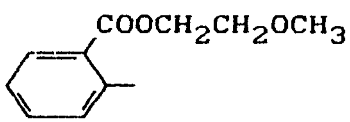 | H | 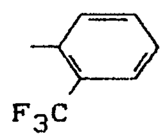 | $OCH_3$ | N | N | $C-CH_3$ | 167 |
| 1174 | 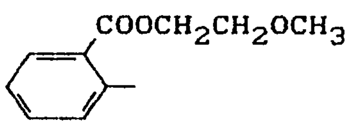 | H | 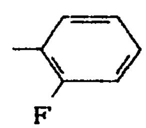 | $OCH_3$ | N | N | $C-CH_3$ | 144 |

**Tabelle 3** - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1175 | 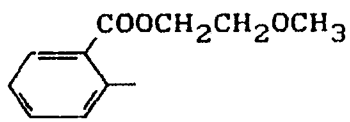 | H | 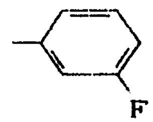 | $OCH_3$ | N | N | $C-CH_3$ | 154 |
| 1176 | 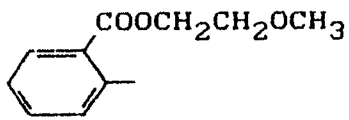 | H | 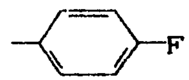 | $OCH_3$ | N | N | $C-CH_3$ | 190 |
| 1177 | 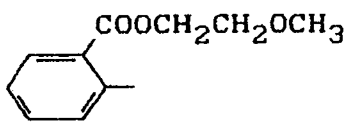 | H | 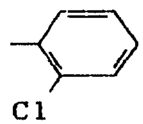 | $OCH_3$ | N | N | $C-CH_3$ | 141 |
| 1178 | 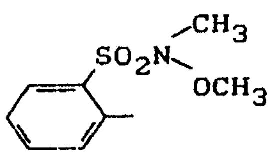 | H | 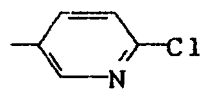 | $OCH_3$ | N | N | $C-OCH_3$ | 211 |
| 1179 | 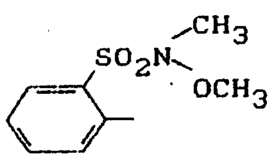 | H | 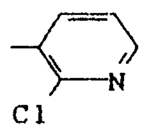 | $OCH_3$ | N | N | $C-OCH_3$ | 199 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1180 | 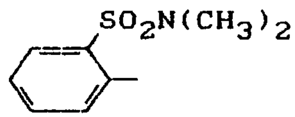 | H | $CH_3$ | $OCH_3$ | N | N | $C-OCH_3$ | 167 |
| 1181 | 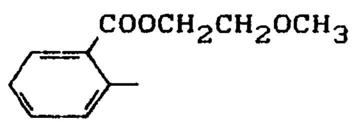 | H | 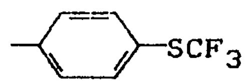 | $OCH_3$ | N | N | $C-CH_3$ | 161 |
| 1182 | 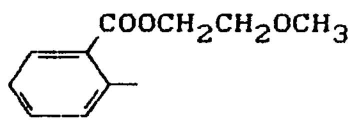 | H | 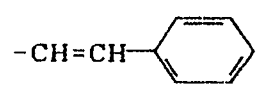 | $OCH_3$ | N | N | $C-CH_3$ | 189 |
| 1183 | 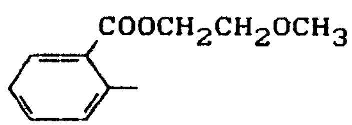 | $-(CH_2)_5-$ | | $OCH_3$ | N | N | $C-CH_3$ | 138 |
| 1184 | 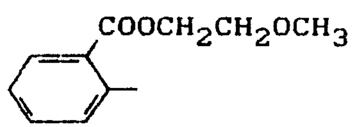 | H | $CH(CH_3)_2$ | $OCH_3$ | N | N | $C-CH_3$ | 127 |
| 1185 | 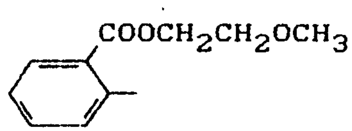 | H | $C(CH_3)_3$ | $OCH_3$ | N | N | $C-CH_3$ | 129 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1186 | 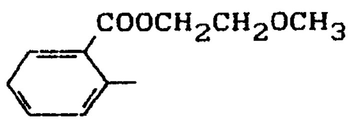 | $CH_3$ | $-CH_2CH(CH_3)_2$ | $OCH_3$ | N | N | $C-CH_3$ | 128 |
| 1187 | 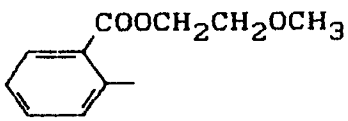 | H | $CH_3$ | $OCH_3$ | N | N | $C-CH_3$ | 151 |
| 1188 | 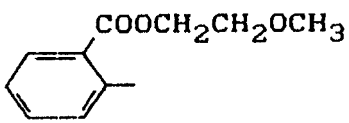 | H | 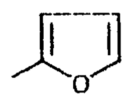 | $OCH_3$ | N | N | $C-CH_3$ | 140 |
| 1189 | 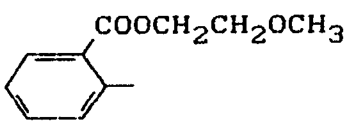 | H | 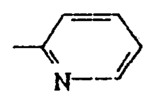 | $OCH_3$ | N | N | $C-CH_3$ | 146 |
| 1190 | 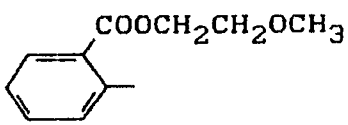 | H | 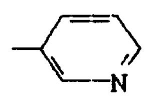 | $OCH_3$ | N | N | $C-CH_3$ | 119 |
| 1191 | 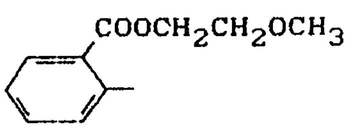 | H | 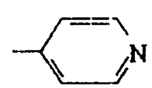 | $OCH_3$ | N | N | $C-CH_3$ | 138 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1192 | 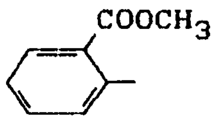 | H | $CH_3$ | $OCH_3$ | N | N | $C-CH_3$ | 153 |
| 1193 | 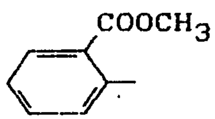 | H | 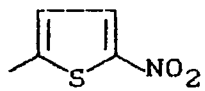 | $OCH_3$ | N | N | $C-CH_3$ | 208 |
| 1194 | 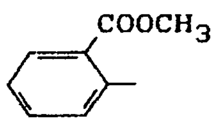 | $CH_3$ | 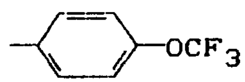 | $OCH_3$ | N | N | $C-CH_3$ | 222 |
| 1195 | 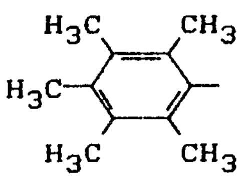 | $CH_3$ | $CH_3$ | $OCH_3$ | N | N | $C-CH_3$ | 224 |
| 1196 | 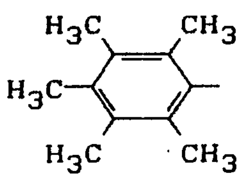 | H | $C_6H_5$ | $OCH_3$ | N | N | $C-CH_3$ | 205 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1197 | 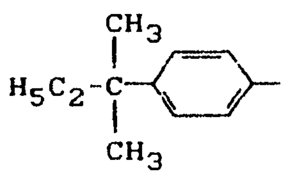 | $CH_3$ | $CH_3$ | $OCH_3$ | N | N | $C-CH_3$ | 136 |
| 1198 | 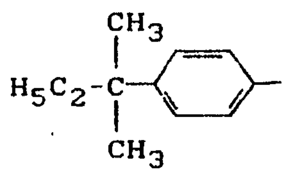 | H | $C_6H_5$ | $OCH_3$ | N | N | $C-CH_3$ | 231 |
| 1199 | 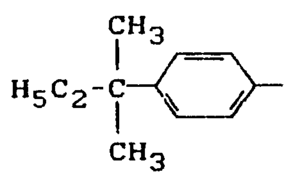 | $CH_3$ | $CH_3$ | $OCH_3$ | N | N | $C-OCH_3$ | 136 |
| 1200 | 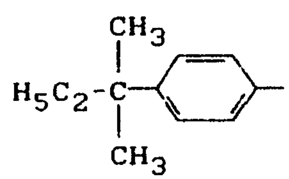 | H | $C_6H_5$ | $OCH_3$ | N | N | $C-OCH_3$ | 220 |
| 1201 | 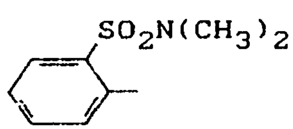 | $CH_3$ | $CH_3$ | $CH_3$ | N | CH | CH | 204 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1202 | 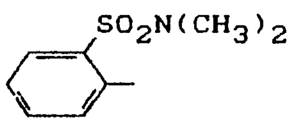 | H | 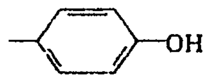 | $CH_3$ | N | CH | CH | 143 |
| 1203 | 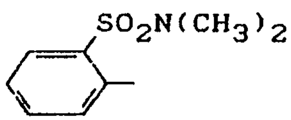 | H | 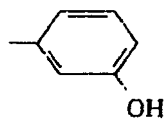 | $CH_3$ | N | CH | CH | 158 |
| 1204 | 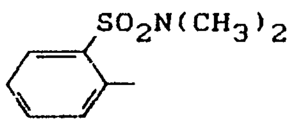 | H | 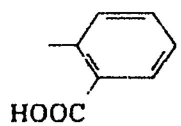 | $CH_3$ | N | CH | CH | 220 |
| 1205 | 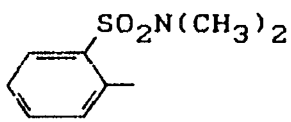 | H | 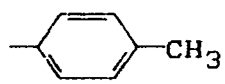 | $CH_3$ | N | CH | CH | 234 |
| 1206 | 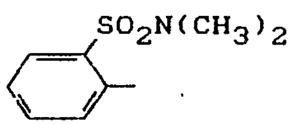 | H | 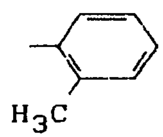 | $CH_3$ | N | CH | CH | 216 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1207 | 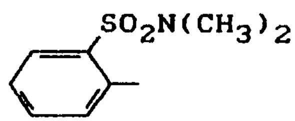 | H | 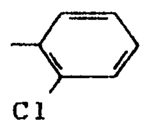 | $CH_3$ | N | CH | CH | 171 |
| 1208 | 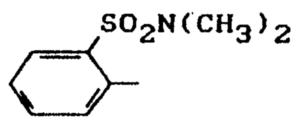 | H | 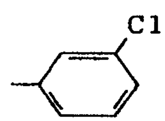 | $CH_3$ | N | CH | CH | 218 |
| 1209 | 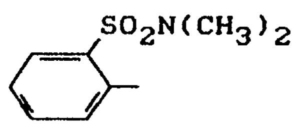 | H | 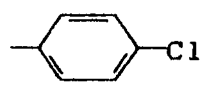 | $CH_3$ | N | CH | CH | 234 |
| 1210 | 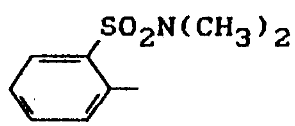 | H | 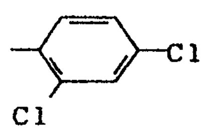 | $CH_3$ | N | CH | CH | 221 |
| 1211 | 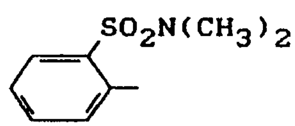 | H | 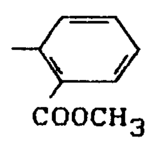 | $CH_3$ | N | CH | CH | 238 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1212 | 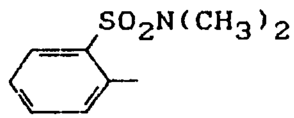 | $C_6H_5$ | $C_6H_5$ | $CH_3$ | N | CH | CH | 228 |
| 1213 | 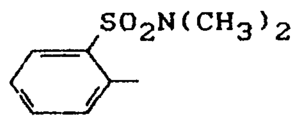 | $CH_3$ | $C_6H_5$ | $CH_3$ | N | CH | CH | 243 |
| 1214 | 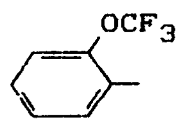 | H | $C_6H_5$ | $OCH_3$ | N | CH | $C-CH_3$ | 213 |
| 1215 | 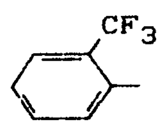 | H | $C_6H_5$ | $OCH_3$ | N | CH | $C-CH_3$ | 214 |
| 1216 | 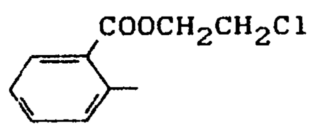 | H | $C_6H_5$ | $-CH_2OCH_3$ | N | CH | $C-OCH_3$ | 192 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1217 | 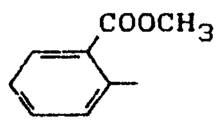 | $-(CH_2)_5-$ | | $OCH_3$ | N | CH | $-COCH_3$ | 196 |
| 1218 | 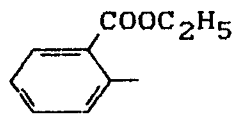 | H | $C_6H_5$ | $-CH_2OCH_3$ | N | CH | $C-OCH_3$ | 177 |
| 1219 | 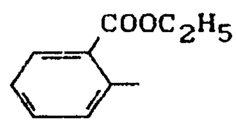 | $-(CH_2)_5-$ | | $-CH_2OCH_3$ | N | CH | $C-OCH_3$ | 75 |
| 1220 | 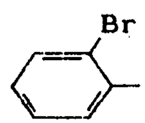 | H | $C_6H_5$ | $-CH_2OCH_3$ | N | CH | $C-OCH_3$ | 215 |
| 1221 | 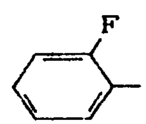 | H | $C_6H_5$ | $-CH_2OCH_3$ | N | CH | $C-OCH_3$ | 228 |
| 1222 | 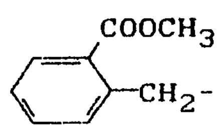 | H | $C_6H_5$ | $OCH_3$ | N | CH | $C-OCH_3$ | 188 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1223 | 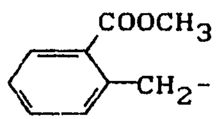 | H | 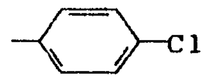 | $OCH_3$ | N | CH | $C-OCH_3$ | 212 |
| 1224 | 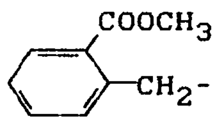 | H | 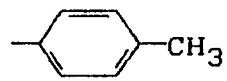 | $OCH_3$ | N | CH | $C-OCH_3$ | 194 |
| 1225 | 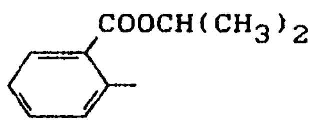 | $CH_3$ | $CH_3$ | $OCH_3$ | N | CH | $C-OCH_3$ | 192 |
| 1226 | 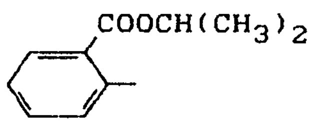 | $CH_3$ | $CH_3$ | $OCH_3$ | N | N | $C-OCH_3$ | 187 |
| 1227 | 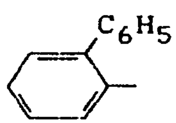 | $CH_3$ | $CH_3$ | $OCH_3$ | N | CH | $C-CH_3$ | 193 |
| 1228 | 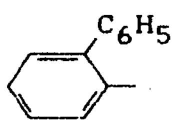 | H | $C_6H_5$ | $OCH_3$ | N | CH | $C-CH_3$ | 92 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1229 | 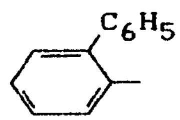 | H | $N(CH_3)_2$ | $OCH_3$ | N | CH | $C-CH_3$ | 134 |
| 1230 | 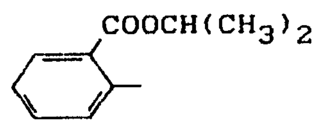 | H | $C_6H_5$ | $OCH_3$ | N | CH | $C-OCH_3$ | 197 |
| 1231 | 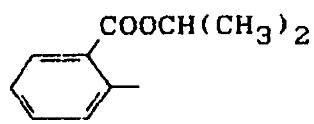 | $CH_3$ | $CH_3$ | $CH_3$ | N | CH | $C-CH_3$ | 171 |
| 1232 | 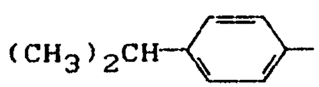 | H | $C_6H_5$ | $CH_3$ | N | CH | $C-CH_3$ | 201 |
| 1233 | 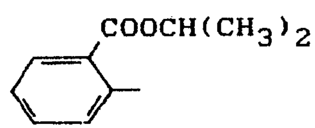 | H | $C_6H_5$ | $CH_3$ | N | CH | $C-CH_3$ | 208 |
| 1234 | 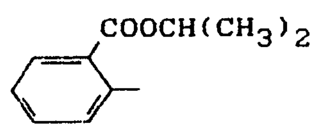 | H | $C_6H_5$ | $OCH_3$ | N | N | $C-OCH_3$ | 182 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1235 | 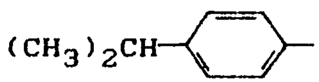 | H | $C_6H_5$ | $OCH_3$ | N | CH | $C-OCH_3$ | 182 |
| 1236 | 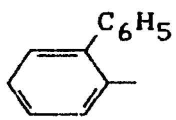 | H | 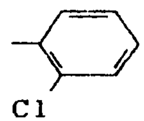 | $OCH_3$ | N | CH | $C-OCH_3$ | 205 |
| 1237 | 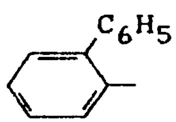 | H | 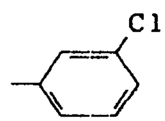 | $OCH_3$ | N | CH | $C-OCH_3$ | 178 |
| 1238 | 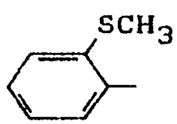 | $CH_3$ | $CH_3$ | $OCH_3$ | N | N | $C-CH_3$ | 144 |
| 1239 | 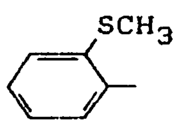 | H | $C_6H_5$ | $OCH_3$ | N | N | $C-CH_3$ | 228 |
| 1240 | 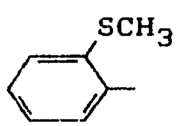 | H | $C(CH_3)_3$ | $OCH_3$ | N | N | $C-OCH_3$ | 168 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1241 | 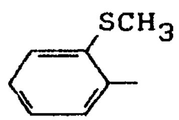 | H | 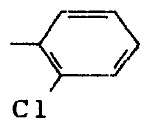 | $OCH_3$ | N | N | $C-OCH_3$ | 214 |
| 1242 | 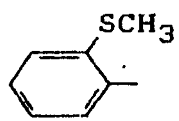 | H | 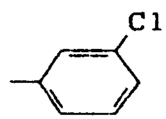 | $OCH_3$ | N | N | $C-OCH_3$ | 178 |
| 1243 | 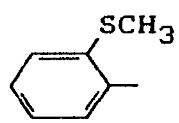 | H | 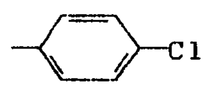 | $OCH_3$ | N | N | $C-OCH_3$ | 207 |
| 1244 | 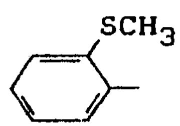 | H | 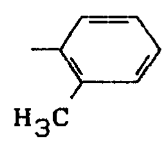 | $OCH_3$ | N | N | $C-OCH_3$ | 192 |
| 1245 | 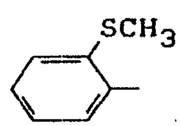 | H | 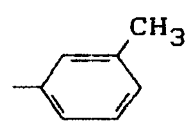 | $OCH_3$ | N | N | $C-OCH_3$ | 193 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1246 | 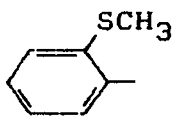 | H | 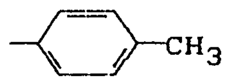 | $OCH_3$ | N | N | C-$OCH_3$ | 216 |
| 1247 | 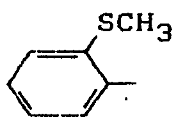 | H | 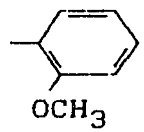 | $OCH_3$ | N | N | C-$OCH_3$ | 221 |
| 1248 | 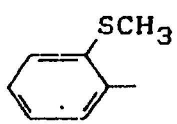 | H | 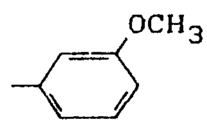 | $OCH_3$ | N | N | C-$OCH_3$ | 213 |
| 1249 | 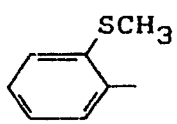 | H | 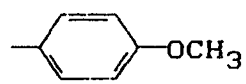 | $OCH_3$ | N | N | C-$OCH_3$ | 208 |
| 1250 | 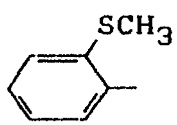 | H | 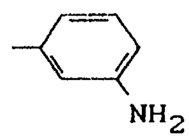 | $OCH_3$ | N | N | C-$OCH_3$ | (amorph) |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1251 | 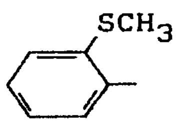 | H | 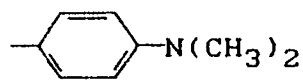 | $OCH_3$ | N | N | $C-OCH_3$ | 198 |
| 1252 | 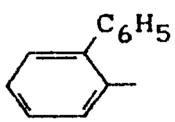 | H | 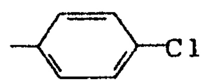 | $OCH_3$ | N | CH | $C-OCH_3$ | 228 |
| 1253 | 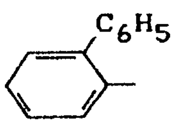 | H | 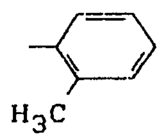 | $OCH_3$ | N | CH | $C-OCH_3$ | 192 |
| 1254 | 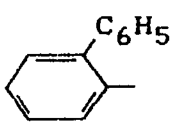 | H | 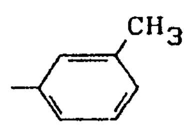 | $OCH_3$ | N | CH | $C-OCH_3$ | 189 |
| 1255 | 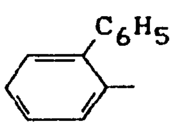 | H | 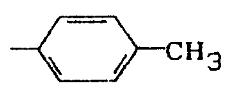 | $OCH_3$ | N | CH | $C-OCH_3$ | 208 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1256 | 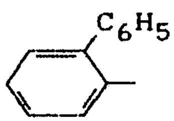 | H | 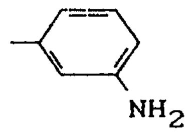 | $OCH_3$ | N | CH | C-$OCH_3$ | 202 |
| 1257 | 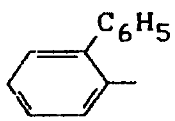 | H | 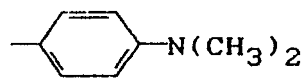 | $OCH_3$ | N | CH | C-$OCH_3$ | 232 |
| 1258 | 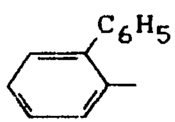 | H | 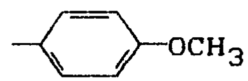 | $OCH_3$ | N | CH | C-$OCH_3$ | 224 |
| 1259 | 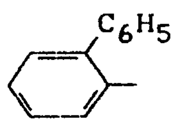 | H | 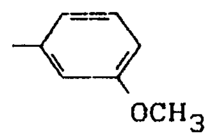 | $OCH_3$ | N | CH | C-$OCH_3$ | 201 |
| 1260 | 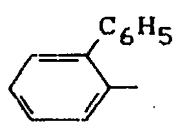 | H | 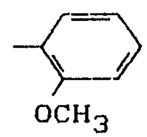 | $OCH_3$ | N | CH | C-$OCH_3$ | 198 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1261 | 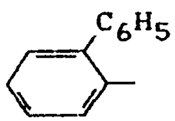 | H | 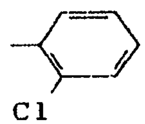 | $OCH_3$ | N | N | $C-OCH_3$ | 206 |
| 1262 | 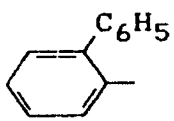 | H | 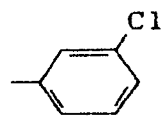 | $OCH_3$ | N | N | $C-OCH_3$ | 112 |
| 1263 | 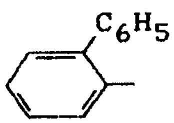 | H | 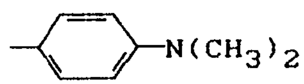 | $OCH_3$ | N | N | $C-OCH_3$ | 211 |
| 1264 | 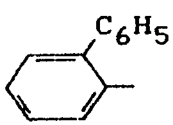 | H | 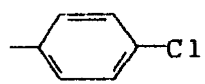 | $OCH_3$ | N | N | $C-OCH_3$ | 204 |
| 1265 | 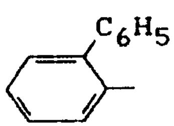 | H | 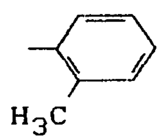 | $OCH_3$ | N | N | $C-OCH_3$ | 201 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1266 | 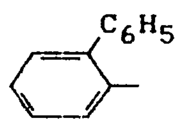 | H | 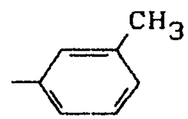 | $OCH_3$ | N | N | $C-OCH_3$ | 176 |
| 1267 | 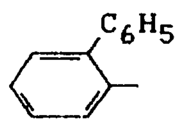 | H | 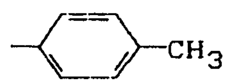 | $OCH_3$ | N | N | $C-OCH_3$ | 187 |
| 1268 | 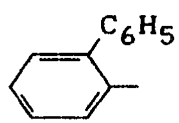 | H | 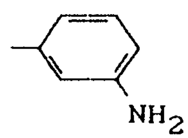 | $OCH_3$ | N | N | $C-OCH_3$ | 169 |
| 1269 | 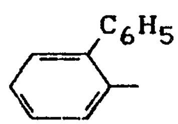 | H | 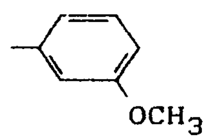 | $OCH_3$ | N | N | $C-OCH_3$ | 176 |
| 1270 | 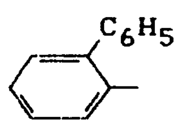 | H | 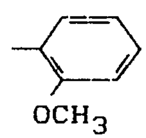 | $OCH_3$ | N | N | $C-OCH_3$ | 214 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1271 | 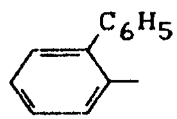 | H | 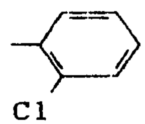 | $OCH_3$ | N | N | $C-CH_3$ | 199 |
| 1272 | 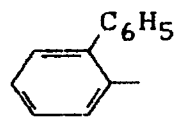 | H | 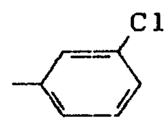 | $OCH_3$ | N | N | $C-CH_3$ | 124 |
| 1273 | 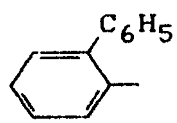 | H | 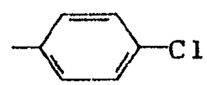 | $OCH_3$ | N | N | $C-CH_3$ | 198 |
| 1274 | 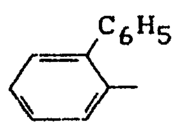 | H | 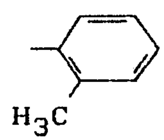 | $OCH_3$ | N | N | $C-CH_3$ | 204 |
| 1275 | 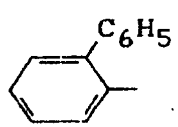 | H | 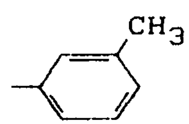 | $OCH_3$ | N | N | $C-CH_3$ | 198 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1276 | 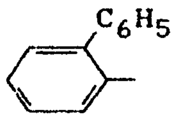 | H | 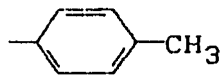 | $OCH_3$ | N | N | $C-CH_3$ | 203 |
| 1277 | 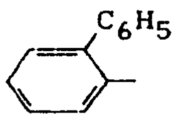 | H | 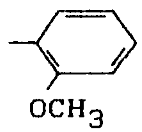 | $OCH_3$ | N | N | $C-CH_3$ | 216 |
| 1278 | 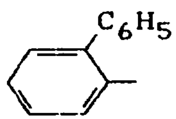 | H | 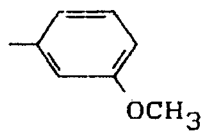 | $OCH_3$ | N | N | $C-CH_3$ | 193 |
| 1279 | 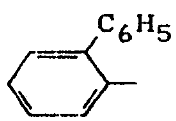 | H | 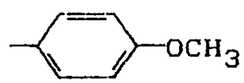 | $OCH_3$ | N | N | $C-CH_3$ | 207 |
| 1280 | 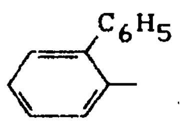 | H | 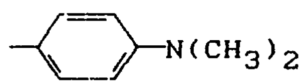 | $OCH_3$ | N | N | $C-CH_3$ | 219 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1281 | 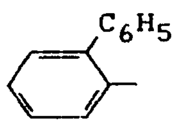 | H | $C_6H_5$ | $CH_3$ | N | N | $C-CH_3$ | 208 |
| 1282 | 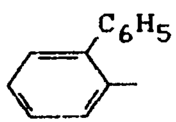 | H | 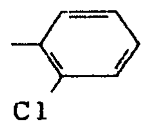 | $CH_3$ | N | N | $C-CH_3$ | 204 |
| 1283 | 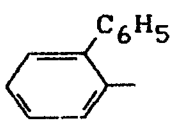 | H | 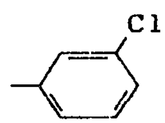 | $CH_3$ | N | N | $C-CH_3$ | 189 |
| 1284 | 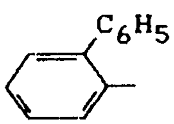 | H | 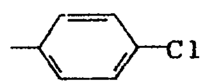 | $CH_3$ | N | N | $C-CH_3$ | 232 |
| 1285 | 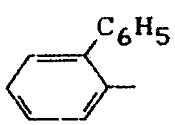 | H | 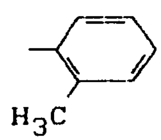 | $CH_3$ | N | N | $C-CH_3$ | 209 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1286 | 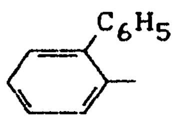 | H | 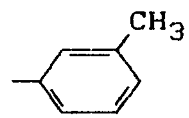 | $CH_3$ | N | N | $C-CH_3$ | 212 |
| 1287 | 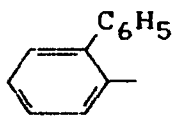 | H | 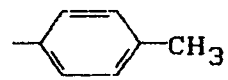 | $CH_3$ | N | N | $C-CH_3$ | 217 |
| 1288 | 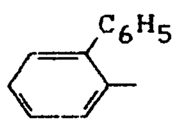 | H | 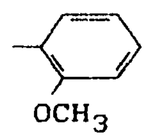 | $CH_3$ | N | N | $C-CH_3$ | 205 |
| 1289 | 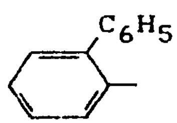 | $CH_3$ | $CH_3$ | $CH_3$ | N | N | $C-CH_3$ | 206 |
| 1290 | 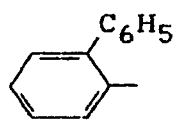 | H | 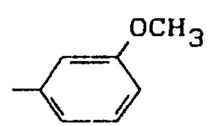 | $CH_3$ | N | N | $C-CH_3$ | 204 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1291 | 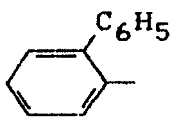 | H | 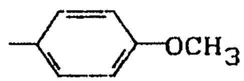 | $CH_3$ | N | N | $C-CH_3$ | 208 |
| 1292 | 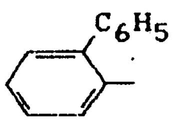 | H | 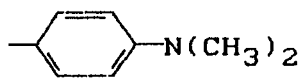 | $CH_3$ | N | N | $C-CH_3$ | 206 |
| 1293 | 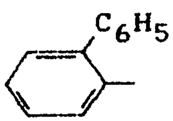 | $CH_3$ | $CH_3$ | $OC_2H_5$ | N | N | $C-CH_3$ | 185 |
| 1294 | 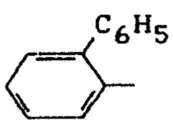 | H | $C_6H_5$ | $OC_2H_5$ | N | N | $C-CH_3$ | 158 |
| 1295 | 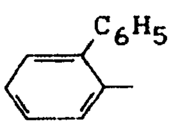 | H | 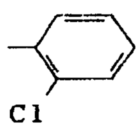 | $OC_2H_5$ | N | N | $C-CH_3$ | 182 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1296 | 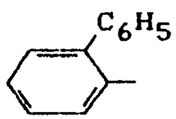 | H | 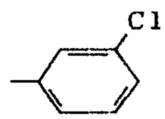 | $OC_2H_5$ | N | N | $C-CH_3$ | 108 |
| 1297 | 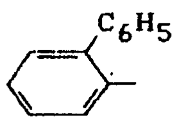 | H | 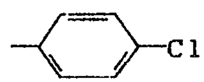 | $OC_2H_5$ | N | N | $C-CH_3$ | 165 |
| 1298 | 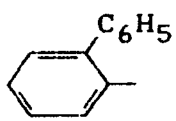 | H | 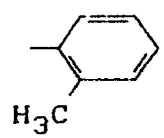 | $OC_2H_5$ | N | N | $C-CH_3$ | 182 |
| 1299 | 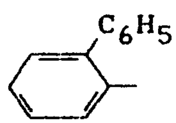 | H | 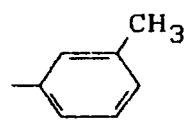 | $OC_2H_5$ | N | N | $C-CH_3$ | 164 |
| 1300 | 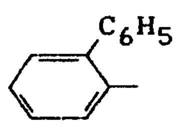 | H | 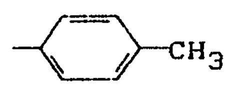 | $OC_2H_5$ | N | N | $C-CH_3$ | 174 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1301 | 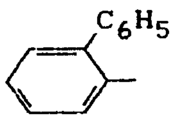 | H | 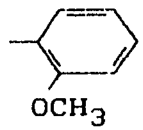 | $OC_2H_5$ | N | N | $C-CH_3$ | 211 |
| 1302 | 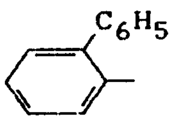 | H | 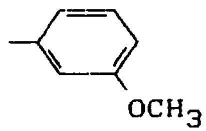 | $OC_2H_5$ | N | N | $C-CH_3$ | 162 |
| 1303 | 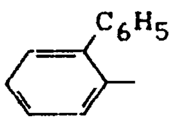 | H | 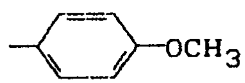 | $OC_2H_5$ | N | N | $C-CH_3$ | 176 |
| 1304 | 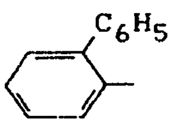 | H | 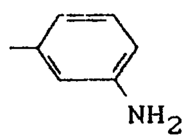 | $CH_3$ | N | N | $C-CH_3$ | 131 |
| 1305 | 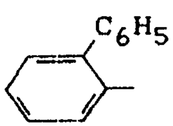 | $CH_3$ | $CH_3$ | $OC_2H_5$ | N | CH | $C-OC_2H_5$ | 194 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1306 | 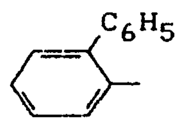 | H | $C_6H_5$ | $OC_2H_5$ | N | CH | $C-OC_2H_5$ | 201 |
| 1307 | 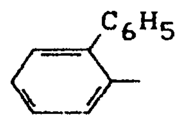 | H | 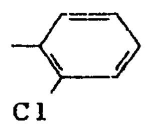 | $OC_2H_5$ | N | CH | $C-OC_2H_5$ | 204 |
| 1308 | 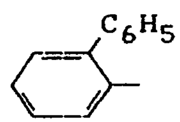 | H | 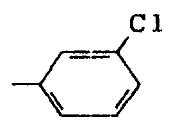 | $OC_2H_5$ | N | CH | $C-OC_2H_5$ | 182 |
| 1309 | 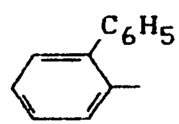 | H | 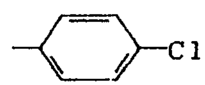 | $OC_2H_5$ | N | CH | $C-OC_2H_5$ | 224 |
| 1310 | 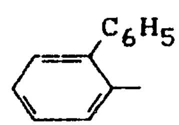 | H | 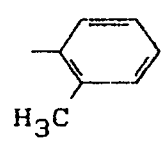 | $OC_2H_5$ | N | CH | $C-OC_2H_5$ | 197 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1311 | 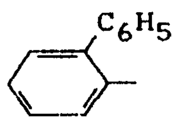 | H | 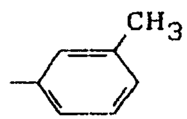 | $OC_2H_5$ | N | CH | $C-OC_2H_5$ | 177 |
| 1312 | 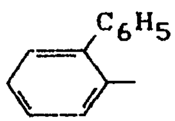 | H | 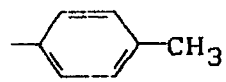 | $OC_2H_5$ | N | CH | $C-OC_2H_5$ | 218 |
| 1313 | 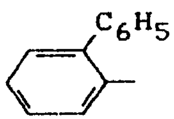 | H | 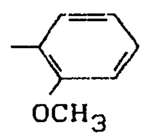 | $OC_2H_5$ | N | CH | $C-OC_2H_5$ | 202 |
| 1314 | 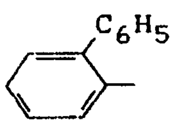 | H | 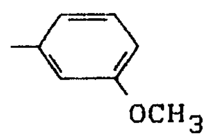 | $OC_2H_5$ | N | CH | $C-OC_2H_5$ | 175 |
| 1315 | 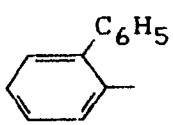 | H | 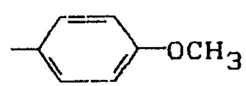 | $OC_2H_5$ | N | CH | $C-OC_2H_5$ | 165 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1316 | 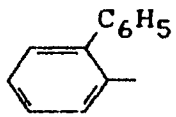 | H | 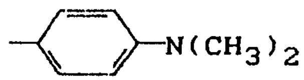 | $OC_2H_5$ | N | CH | $C-OC_2H_5$ | 232 |
| 1317 | 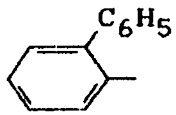 | H | 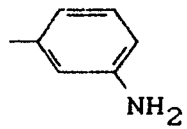 | $OC_2H_5$ | N | CH | $C-OC_2H_5$ | 175 |
| 1318 | 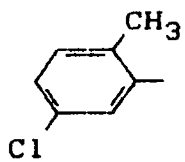 | $CH_3$ | $CH_3$ | $OCH_3$ | N | CH | $C-OCH_3$ | 182 |
| 1319 | 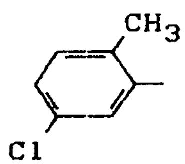 | H | $C_6H_5$ | $OCH_3$ | N | CH | $C-OCH_3$ | 192 |
| 1320 | 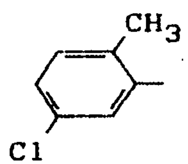 | H | 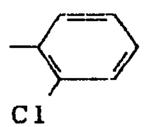 | $OCH_3$ | N | CH | $C-OCH_3$ | 208 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1321 | 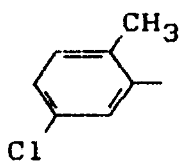 | H | 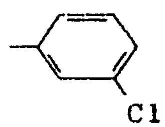 | $OCH_3$ | N | CH | $C-OCH_3$ | 215 |
| 1322 | 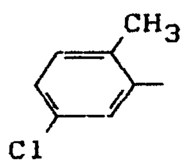 | H | 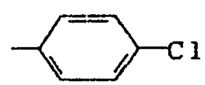 | $OCH_3$ | N | CH | $C-OCH_3$ | 207 |
| 1323 | 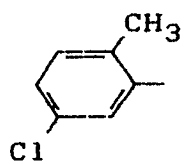 | H | 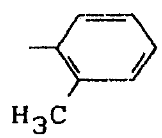 | $OCH_3$ | N | CH | $C-OCH_3$ | 206 |
| 1324 | 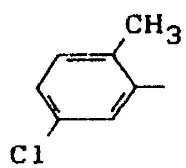 | H | 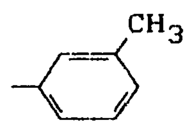 | $OCH_3$ | N | CH | $C-OCH_3$ | 217 |
| 1325 | 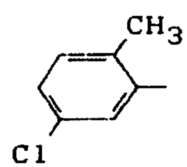 | H | 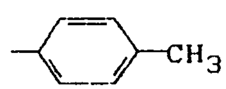 | $OCH_3$ | N | CH | $C-OCH_3$ | 219 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1326 | 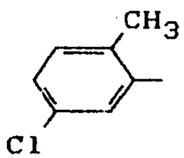 | H | 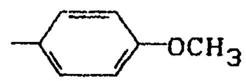 | $OCH_3$ | N | CH | $C-OCH_3$ | 238 |
| 1327 | 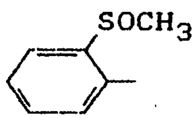 | H | 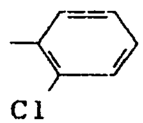 | $OCH_3$ | N | CH | $C-OCH_3$ | 215 |
| 1328 | 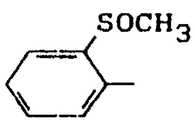 | H | 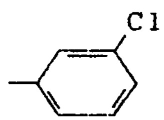 | $OCH_3$ | N | CH | $C-OCH_3$ | 222 |
| 1329 | 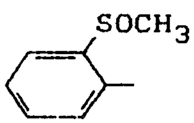 | H | 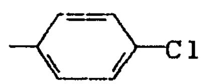 | $OCH_3$ | N | CH | $C-OCH_3$ | 233 |
| 1330 | 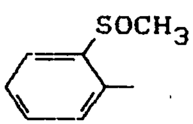 | H | 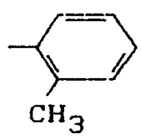 | $OCH_3$ | N | CH | $C-OCH_3$ | 224 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1331 | 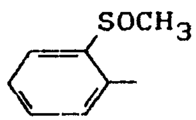 | H | 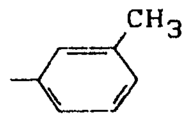 | $OCH_3$ | N | CH | $C-OCH_3$ | 202 |
| 1332 | 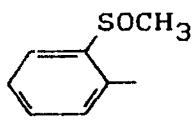 | H | 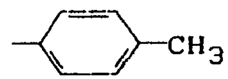 | $OCH_3$ | N | CH | $C-OCH_3$ | 230 |
| 1333 | 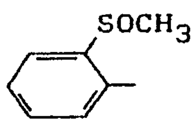 | H | 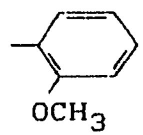 | $OCH_3$ | N | CH | $C-OCH_3$ | 210 |
| 1334 | 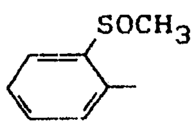 | H | 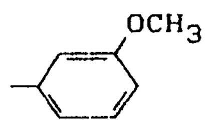 | $OCH_3$ | N | CH | $C-OCH_3$ | 213 |
| 1335 | 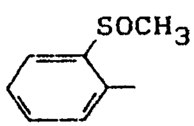 | H | 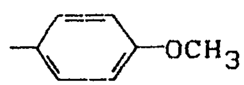 | $OCH_3$ | N | CH | $C-OCH_3$ | 246 |
| 1336 | 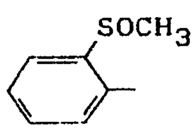 | H | 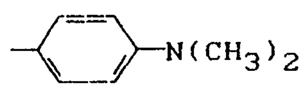 | $OCH_3$ | N | CH | $C-OCH_3$ | 226 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1337 | 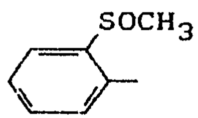 | H | 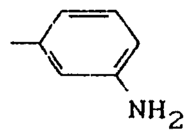 | $OCH_3$ | N | CH | $C-OCH_3$ | 205 |
| 1338 | 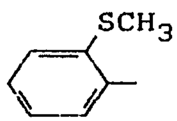 | $CH_3$ | $CH_3$ | $OC_2H_5$ | N | CH | $C-OC_2H_5$ | 175 |
| 1339 | 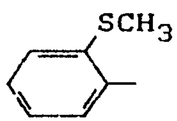 | H | $C_6H_5$ | $OC_2H_5$ | N | CH | $C-OC_2H_5$ | 183 |
| 1340 | 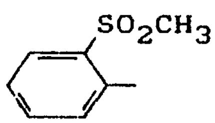 | $CH_3$ | $CH_3$ | $OCH_3$ | N | CH | $C-OCH_3$ | 222 |
| 1341 | 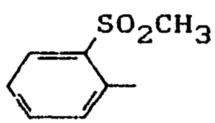 | H | $C_6H_5$ | $OCH_3$ | N | CH | $C-OCH_3$ | 211 |
| 1342 | 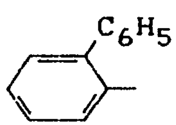 | $CH_3$ | $CH_3$ | $OC_2H_5$ | N | N | $C-OCH_3$ | 160 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1343 | 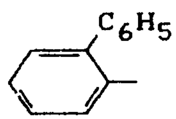 | H | $C_6H_5$ | $OC_2H_5$ | N | N | $C-OCH_3$ | 112 |
| 1344 | 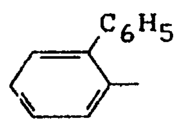 | H | 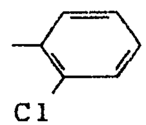 | $OC_2H_5$ | N | N | $C-OCH_3$ | 181 |
| 1345 | 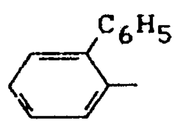 | H | 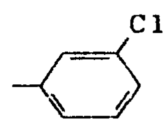 | $OC_2H_5$ | N | N | $C-OCH_3$ | 151 |
| 1346 | 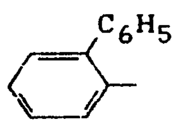 | H | 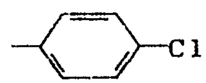 | $OC_2H_5$ | N | N | $C-OCH_3$ | 177 |
| 1347 | 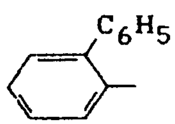 | H | 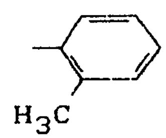 | $OC_2H_5$ | N | N | $C-OCH_3$ | 177 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1348 | 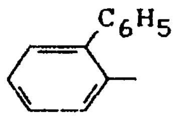 | H | 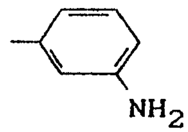 | $OC_2H_5$ | N | N | $C-CH_3$ | 149 |
| 1349 | 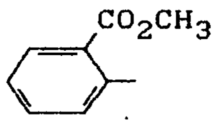 | $CH_3$ | $CH_3$ | $CH_3$ | N | N | $C-CH_3$ | |
| 1350 | 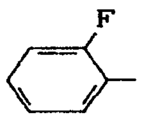 | $CH_3$ | $CH_3$ | $CH_3$ | N | N | $C-CH_3$ | |
| 1351 | 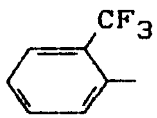 | $CH_3$ | $CH_3$ | $CH_3$ | N | N | $C-CH_3$ | |
| 1352 | 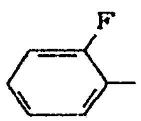 | H | $C_6H_5$ | $OCH_3$ | N | N | $C-OCH_3$ | 208 |
| 1353 | 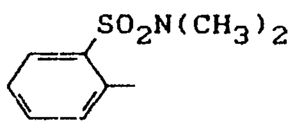 | H | $C_6H_5$ | $CH_3$ | N | N | $C-CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1354 | 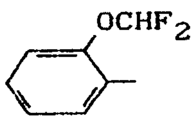 | H | $C_6H_5$ | $OCH_3$ | N | N | $C-C_2H_5$ | |
| 1355 | 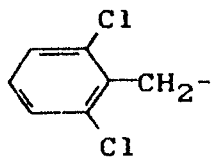 | $CH_3$ | $CH_3$ | $CH_3$ | N | N | $C-CH_3$ | |
| 1356 | 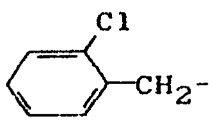 | H | $C_6H_5$ | $OCH_3$ | N | N | $C-CH_3$ | |
| 1357 | 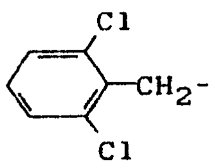 | H | $C_6H_5$ | $CH_3$ | N | N | $C-CH_3$ | |
| 1358 | 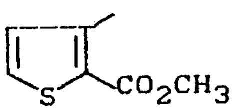 | $CH_3$ | $CH_3$ | $CH_3$ | N | N | $C-CH_3$ | |
| 1359 | 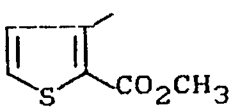 | H | $C_6H_5$ | $CH_3$ | N | N | $C-CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1360 | 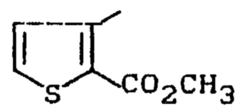 | $CH_3$ | $CH_3$ | $OCH_3$ | N | N | $C-C_2H_5$ | |
| 1361 | 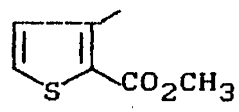 | H | $C_6H_5$ | $OCH_3$ | N | N | $C-C_2H_5$ | |
| 1362 | 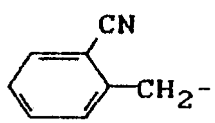 | $CH_3$ | $CH_3$ | $OCH_3$ | N | N | $C-CH_3$ | |
| 1363 | 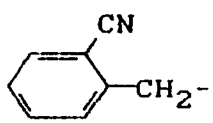 | H | $C_6H_5$ | $OCH_3$ | N | N | $C-CH_3$ | |
| 1364 | 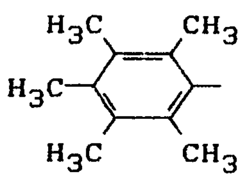 | $CH_3$ | $CH_3$ | $OCH_3$ | N | N | $C-OCH_3$ | 211 |
| 1365 | 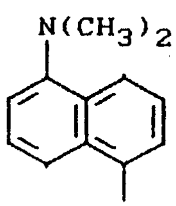 | $CH_3$ | $CH_3$ | $OCH_3$ | N | N | $C-OCH_3$ | 154 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1366 | 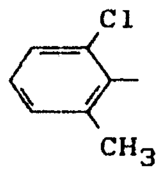 | $CH_3$ | $CH_3$ | $OCH_3$ | N | N | $C-OCH_3$ | 200 |
| 1367 | 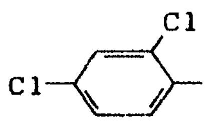 | $CH_3$ | $CH_3$ | $OCH_3$ | N | N | $C-CH_3$ | 100 |
| 1368 | 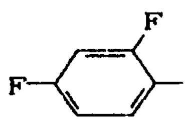 | $CH_3$ | $CH_3$ | $OCH_3$ | N | N | $C-CH_3$ | 165 |
| 1369 | 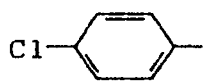 | $CH_3$ | $CH_3$ | $OCH_3$ | N | N | $C-CH_3$ | 152 |
| 1370 | 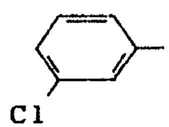 | $CH_3$ | $CH_3$ | $OCH_3$ | N | N | $C-CH_3$ | 142 |
| 1371 | 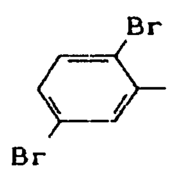 | $CH_3$ | $CH_3$ | $OCH_3$ | N | N | $C-CH_3$ | 194 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1372 | 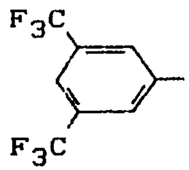 | $CH_3$ | $CH_3$ | $OCH_3$ | N | N | $C-OCH_3$ | |
| 1373 | 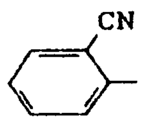 | $CH_3$ | $CH_3$ | $OCH_3$ | N | N | $C-OCH_3$ | |
| 1374 | 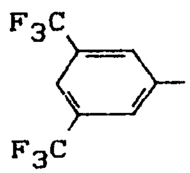 | $CH_3$ | $CH_3$ | $OCH_3$ | N | N | $C-CH_3$ | 172 |
| 1375 | 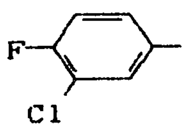 | $CH_3$ | $CH_3$ | $OCH_3$ | N | N | $C-CH_3$ | |
| 1376 | 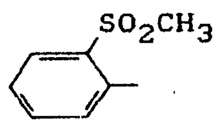 | $CH_3$ | $CH_3$ | $OCH_3$ | N | N | $C-CH_3$ | 224 |
| 1377 | 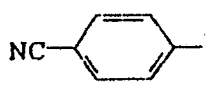 | $CH_3$ | $CH_3$ | $OCH_3$ | N | N | $C-CH_3$ | 165 |

<u>Tabelle 3</u> - Fortsetzung

| Bsp.-Nr. | $R^1$ | $R^2$ | $R^3$ | $R^4$ | X | Y | Z | Schmelz-punkt (°C) |
|---|---|---|---|---|---|---|---|---|
| 1378 | 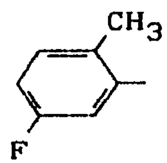 | $CH_3$ | $CH_3$ | $OCH_3$ | N | N | $C-OCH_3$ | |
| 1379 | 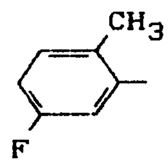 | $CH_3$ | $CH_3$ | $OCH_3$ | N | N | $C-CH_3$ | |
| 1380 | 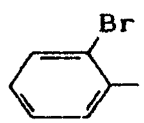 | $CH_3$ | $CH_3$ | $OCH_3$ | N | N | $C-CH_3$ | |
| 1381 | 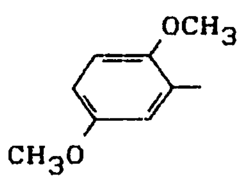 | $CH_3$ | $CH_3$ | $OCH_3$ | N | N | $C-OCH_3$ | 184 |
| 1382 | 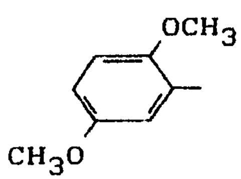 | $CH_3$ | $CH_3$ | $OCH_3$ | N | N | $C-CH_3$ | 164 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1383 | 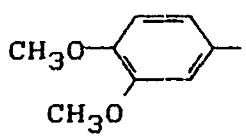 | $CH_3$ | $CH_3$ | $OCH_3$ | N | N | C-$OCH_3$ | 116 |
| 1384 | 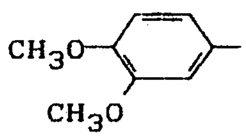 | $CH_3$ | $CH_3$ | $OCH_3$ | N | N | C-$CH_3$ | 216 |
| 1385 | 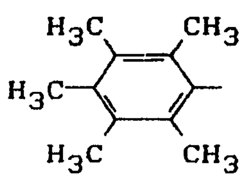 | H | $C_6H_5$ | $OCH_3$ | N | N | C-$OCH_3$ | 208 |
| 1386 | 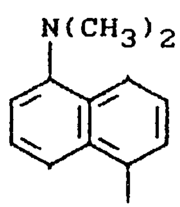 | H | $C_6H_5$ | $OCH_3$ | N | N | C-$OCH_3$ | 197 |
| 1387 | 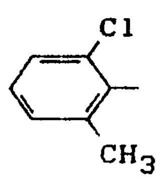 | H | $C_6H_5$ | $OCH_3$ | N | N | C-$OCH_3$ | 198 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1388 | 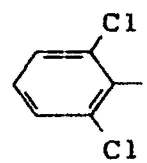 | H | $C_6H_5$ | $OCH_3$ | N | N | $C-OCH_3$ | 206 |
| 1389 | 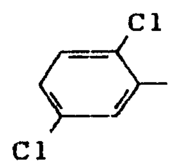 | H | $C_6H_5$ | $OCH_3$ | N | N | $C-OCH_3$ | 131 |
| 1390 | 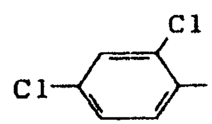 | H | $C_6H_5$ | $OCH_3$ | N | N | $C-OCH_3$ | 157 |
| 1391 | 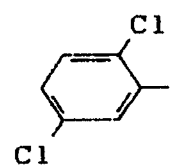 | H | $C_6H_5$ | $OCH_3$ | N | N | $C-CH_3$ | |
| 1392 | 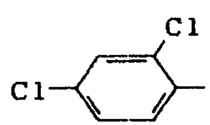 | H | $C_6H_5$ | $OCH_3$ | N | N | $C-CH_3$ | 163 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1393 | 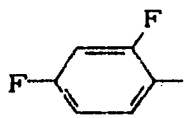 | H | $C_6H_5$ | $OCH_3$ | N | N | $C-OCH_3$ | |
| 1394 | 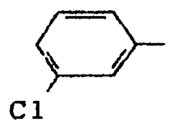 | H | $C_6H_5$ | $OCH_3$ | N | N | $C-OCH_3$ | 142 |
| 1395 | 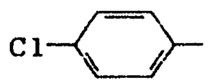 | H | $C_6H_5$ | $OCH_3$ | N | N | $C-OCH_3$ | 163 |
| 1396 | 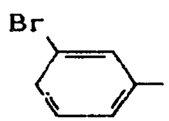 | H | $C_6H_5$ | $OCH_3$ | N | N | $C-OCH_3$ | |
| 1397 | 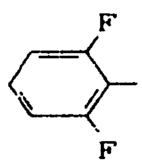 | H | $C_6H_5$ | $OCH_3$ | N | N | $C-OCH_3$ | 196 |
| 1398 | 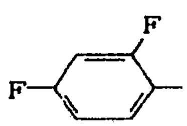 | H | $C_6H_5$ | $OCH_3$ | N | N | $C-CH_3$ | 216 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1399 | 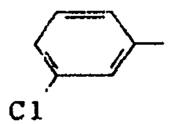 | H | $C_6H_5$ | $OCH_3$ | N | N | $C-CH_3$ | 162 |
| 1400 | 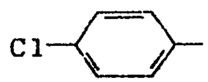 | H | $C_6H_5$ | $OCH_3$ | N | N | $C-CH_3$ | 204 |
| 1401 | 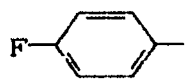 | H | $C_6H_5$ | $OCH_3$ | N | N | $C-CH_3$ | 200 |
| 1402 | 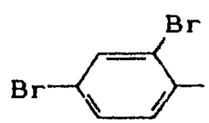 | H | $C_6H_5$ | $OCH_3$ | N | N | $C-CH_3$ | 182 |
| 1403 | 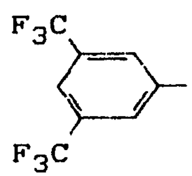 | H | $C_6H_5$ | $OCH_3$ | N | N | $C-OCH_3$ | |
| 1404 | 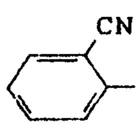 | H | $C_6H_5$ | $OCH_3$ | N | N | $C-OCH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1405 | 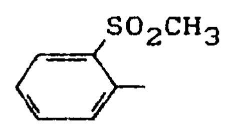 | H | $C_6H_5$ | $OCH_3$ | N | N | C-$OCH_3$ | |
| 1406 | 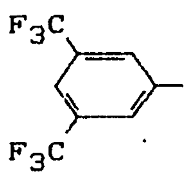 | H | $C_6H_5$ | $OCH_3$ | N | N | C-$CH_3$ | 187 |
| 1407 | 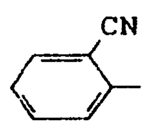 | H | $C_6H_5$ | $OCH_3$ | N | N | C-$CH_3$ | |
| 1408 | 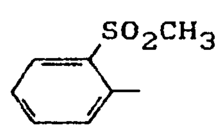 | H | $C_6H_5$ | $OCH_3$ | N | N | C-$CH_3$ | 205 |
| 1409 | 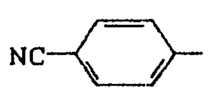 | H | $C_6H_5$ | $OCH_3$ | N | N | C-$CH_3$ | 243 |
| 1410 | 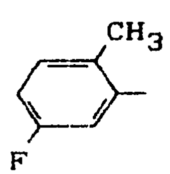 | H | $C_6H_5$ | $OCH_3$ | N | N | C-$OCH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1411 | 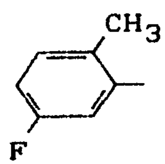 | H | $C_6H_5$ | $OCH_3$ | N | N | $C-CH_3$ | |
| 1412 | 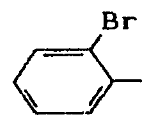 | H | $C_6H_5$ | $OCH_3$ | N | N | $C-OCH_3$ | 192 |
| 1413 | 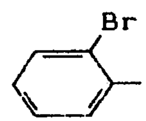 | H | $C_6H_5$ | $OCH_3$ | N | N | $C-CH_3$ | |
| 1414 | 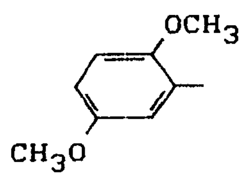 | H | $C_6H_5$ | $OCH_3$ | N | N | $C-OCH_3$ | 215 |
| 1415 | 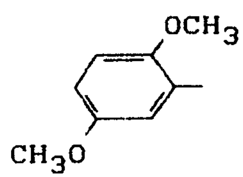 | H | $C_6H_5$ | $OCH_3$ | N | N | $C-CH_3$ | 193 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1416 | 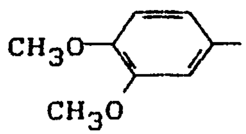 | H | $C_6H_5$ | $OCH_3$ | N | N | $C-OCH_3$ | 159 |
| 1417 | 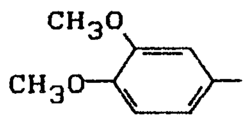 | H | $C_6H_5$ | $OCH_3$ | N | N | $C-CH_3$ | 174 |
| 1418 | 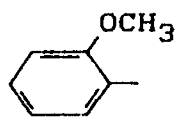 | H | $C_6H_5$ | $OCH_3$ | N | N | $C-OCH_3$ | |
| 1419 | 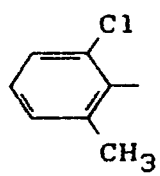 | $CH_3$ | $CH_2CH(CH_3)_2$ | $OCH_3$ | N | N | $C-OCH_3$ | |
| 1420 | 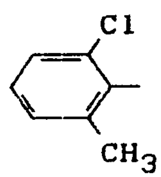 | H | $CH_3$ | $OCH_3$ | N | N | $C-OCH_3$ | |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1421 | 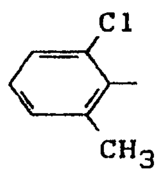 | H | 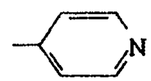 | $OCH_3$ | N | N | $C-OCH_3$ | 185 |
| 1422 | 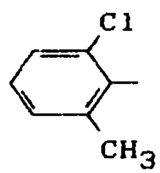 | H | 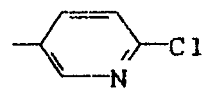 | $OCH_3$ | N | N | $C-OCH_3$ | 191 |
| 1423 | 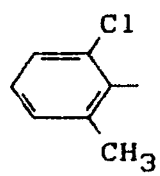 | H | 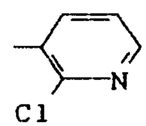 | $OCH_3$ | N | N | $C-OCH_3$ | 189 |
| 1424 | 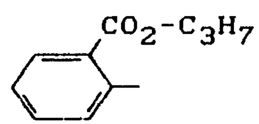 | H | 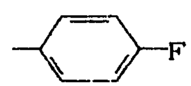 | $OCH_3$ | N | N | $C-CH_3$ | 167 |
| 1425 | 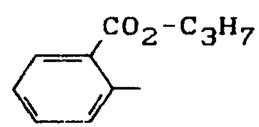 | H | 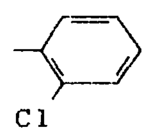 | $OCH_3$ | N | N | $C-CH_3$ | 143 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1426 | 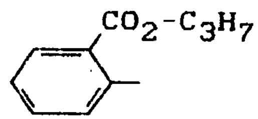 | H | 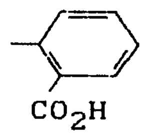 | $OCH_3$ | N | N | $C-CH_3$ | |
| 1427 | 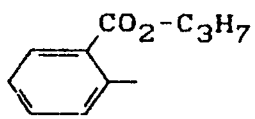 | H | 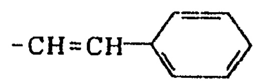 | $OCH_3$ | N | N | $C-CH_3$ | 182 |
| 1428 | 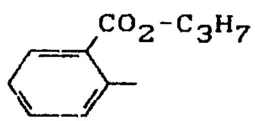 | H | $CH(CH_3)_2$ | $OCH_3$ | N | N | $C-CH_3$ | 145 |
| 1429 | 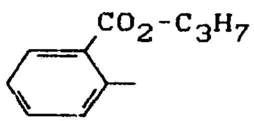 | H | $-C(CH_3)_3$ | $OCH_3$ | N | N | $C-CH_3$ | 149 |
| 1430 | 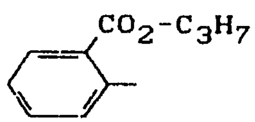 | $CH_3$ | $CH_2CH(CH_3)_2$ | $OCH_3$ | N | N | $C-CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1431 | 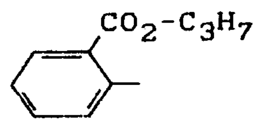 | H | $CH_3$ | $OCH_3$ | N | N | $C-CH_3$ | 134 |
| 1432 | 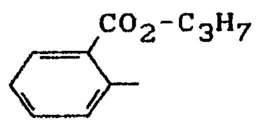 | H | 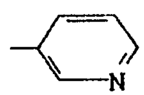 | $OCH_3$ | N | N | $C-CH_3$ | 150 |
| 1433 | 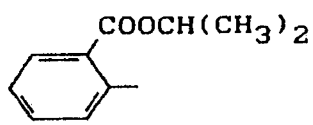 | H | 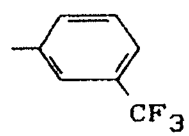 | $OCH_3$ | N | N | $C-CH_3$ | |
| 1434 | 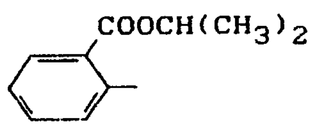 | H | 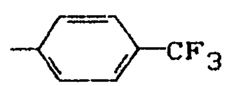 | $OCH_3$ | N | N | $C-CH_3$ | 168 |
| 1435 | 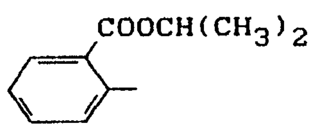 | H | 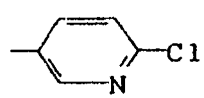 | $OCH_3$ | N | N | $C-CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1436 | 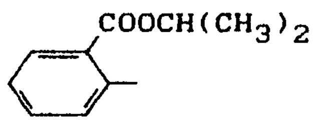 | H | 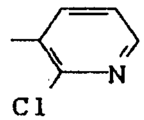 | $OCH_3$ | N | N | $C-CH_3$ | |
| 1437 | 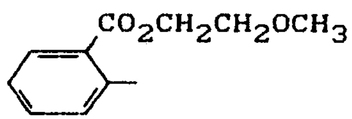 | H | 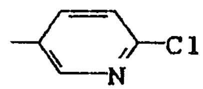 | $OCH_3$ | N | N | $C-CH_3$ | 134 |
| 1438 | 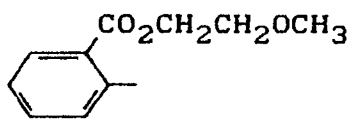 | H | 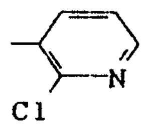 | $OCH_3$ | N | N | $C-CH_3$ | 131 |
| 1439 | 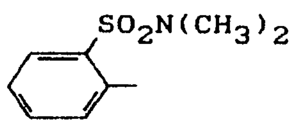 | H | $CH_3CH_2CH_2$ | $OCH_3$ | N | N | $C-OCH_3$ | |
| 1440 | 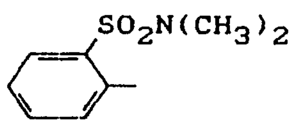 | H | $CH_3SCH_2CH_2$ | $OCH_3$ | N | N | $C-OCH_3$ | 172 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1441 | 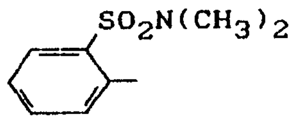 | H | $CH_3CH_2CH_2$ | $OCH_3$ | N | N | $C-CH_3$ | 167 |
| 1442 | 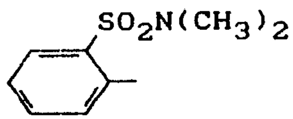 | H | $CH_3SCH_2CH_2$ | $OCH_3$ | N | N | $C-CH_3$ | 161 |
| 1443 | 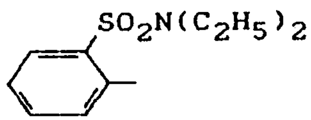 | H | $CH_3$ | $OCH_3$ | N | N | $C-OCH_3$ | |
| 1444 | 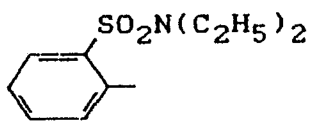 | $CH_3$ | $CH_2CH(CH_3)_2$ | $OCH_3$ | N | N | $C-OCH_3$ | |
| 1445 | 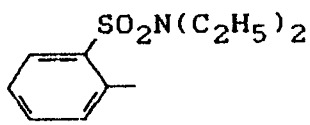 | H | $-C(CH_3)_3$ | $OCH_3$ | N | N | $C-OCH_3$ | |
| 1446 | 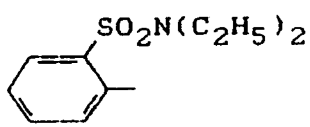 | $-(CH_2)_5-$ | | $OCH_3$ | N | N | $C-OCH_3$ | |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1447 | 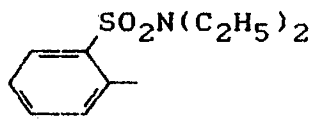 | H | $-CH(CH_3)_2$ | $OCH_3$ | N | N | $C-OCH_3$ | |
| 1448 | 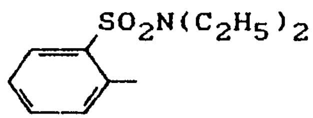 | H | $CH_3CH_2CH_2-$ | $OCH_3$ | N | N | $C-OCH_3$ | 154 |
| 1449 | 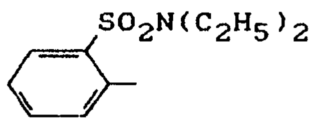 | H | $CH_3SCH_2CH_2-$ | $OCH_3$ | N | N | $C-OCH_3$ | 141 |
| 1450 | 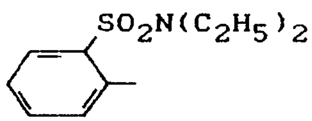 | $CH_3$ | $-CH_2CH(CH_3)_2$ | $OCH_3$ | N | N | $C-CH_3$ | |
| 1451 | 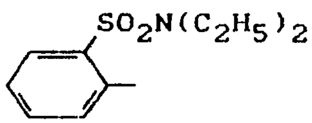 | H | $C(CH_3)_3$ | $OCH_3$ | N | N | $C-CH_3$ | |
| 1452 | 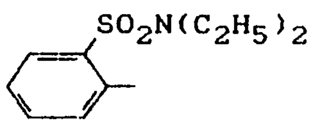 | $-(CH_2)_5-$ | | $OCH_3$ | N | N | $C-CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1453 | 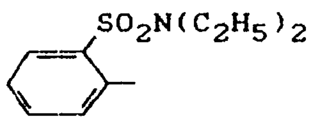 | H | $-CH(CH_3)_2$ | $OCH_3$ | N | N | $C-CH_3$ | |
| 1454 | 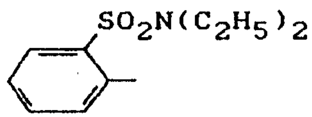 | H | $CH_3CH_2CH_2-$ | $OCH_3$ | N | N | $C-CH_3$ | 163 |
| 1455 | 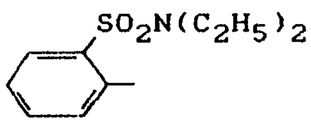 | H | $CH_3SCH_2CH_2-$ | $OCH_3$ | N | N | $C-CH_3$ | 153 |
| 1456 | 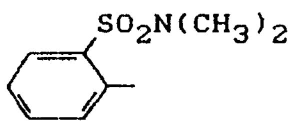 | H | $CH_3(CH_2)_5-$ | $OCH_3$ | N | N | $C-OCH_3$ | 102 |
| 1457 | 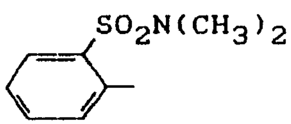 | H | $CH_3(CH_2)_9-$ | $OCH_3$ | N | N | $C-OCH_3$ | 108 |
| 1458 | 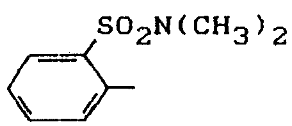 | H | $CH_3(CH_2)_{10}-$ | $OCH_3$ | N | N | $C-OCH_3$ | 112 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1459 | 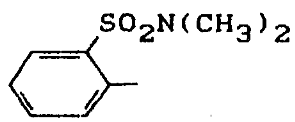 | H | $CH_3(CH_2)_{11}$- | $OCH_3$ | N | N | C-$OCH_3$ | 105 |
| 1460 | 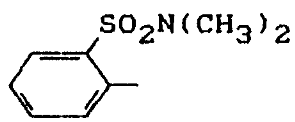 | H | $CH_3(CH_2)_{12}$- | $OCH_3$ | N | N | C-$OCH_3$ | |
| 1461 | 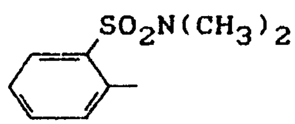 | H | $CH_3(CH_2)_5$- | $OCH_3$ | N | N | C-$CH_3$ | 108 |
| 1462 | 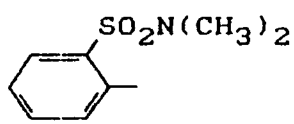 | H | $CH_3(CH_2)_9$- | $OCH_3$ | N | N | C-$CH_3$ | 105 |
| 1463 | 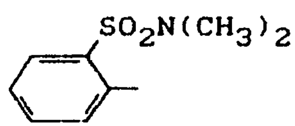 | H | $CH_3(CH_2)_{10}$- | $OCH_3$ | N | N | C-$CH_3$ | 113 |
| 1464 | 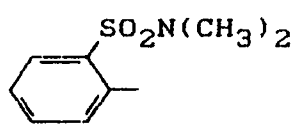 | H | $CH_3(CH_2)_{11}$- | $OCH_3$ | N | N | C-$CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1465 | 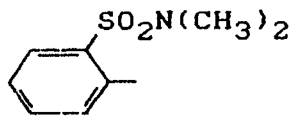 | H | $CH_3(CH_2)_{12}$- | $OCH_3$ | N | N | C-$CH_3$ | 105 |
| 1466 | 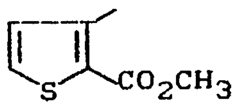 | H | $CH_3CH_2$- | $OCH_3$ | N | N | C-$CH_3$ | |
| 1467 | 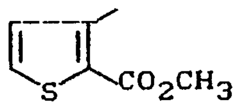 | H | $CH_3CH_2CH_2$- | $OCH_3$ | N | N | C-$CH_3$ | 149 |
| 1468 | 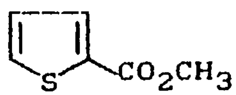 | H | $CH_3SCH_2CH_2$- | $OCH_3$ | N | N | C-$CH_3$ | 150 |
| 1469 | 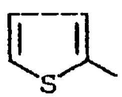 | $CH_3$ | $CH_3$ | $OCH_3$ | N | N | C-$OCH_3$ | |
| 1470 | 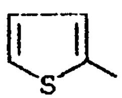 | H | $C_6H_5$ | $OCH_3$ | N | N | C-$OCH_3$ | |
| 1471 | 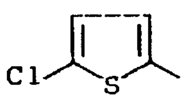 | $CH_3$ | $CH_3$ | $OCH_3$ | N | N | C-$OCH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1472 | 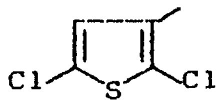 | H | $C_6H_5$ | $OCH_3$ | N | N | $C-CH_3$ | |
| 1473 | 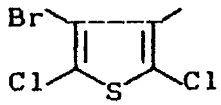 | $CH_3$ | $CH_3$ | $OCH_3$ | N | N | $C-OCH_3$ | |
| 1474 | 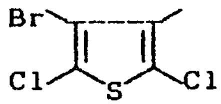 | $CH_3$ | $CH_3$ | $OCH_3$ | N | N | $C-CH_3$ | |
| 1475 | 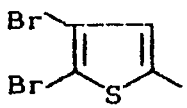 | $CH_3$ | $CH_3$ | $OCH_3$ | N | N | $C-OCH_3$ | |
| 1476 | 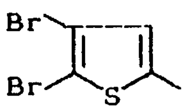 | $CH_3$ | $CH_3$ | $OCH_3$ | N | N | $C-CH_3$ | |
| 1477 | 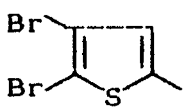 | H | $C_6H_5$ | $OCH_3$ | N | N | $C-OCH_3$ | |
| 1478 | 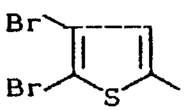 | H | $C_6H_5$ | $OCH_3$ | N | N | $C-CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1479 | 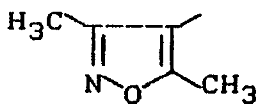 | $CH_3$ | $CH_3$ | $OCH_3$ | N | N | $C-OCH_3$ | |
| 1480 | 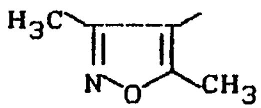 | $CH_3$ | $CH_3$ | $OCH_3$ | N | N | $C-CH_3$ | |
| 1481 | 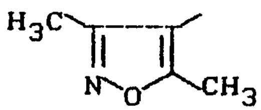 | H | $C_6H_5$ | $OCH_3$ | N | N | $C-OCH_3$ | |
| 1482 | 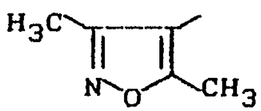 | H | $C_6H_5$ | $OCH_3$ | N | N | $C-CH_3$ | |
| 1483 | 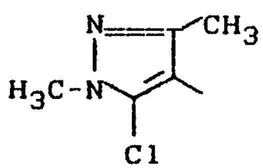 | $CH_3$ | $CH_3$ | $OCH_3$ | N | N | $C-OCH_3$ | |
| 1484 | 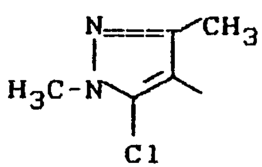 | $CH_3$ | $CH_3$ | $OCH_3$ | N | N | $C-CH_3$ | |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1485 | 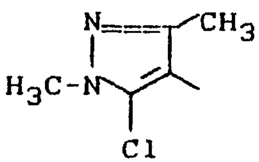 | H | $C_6H_5$ | $OCH_3$ | N | N | $C-OCH_3$ | |
| 1486 | 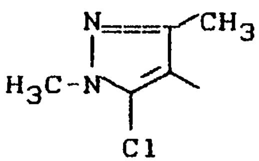 | H | $C_6H_5$ | $OCH_3$ | N | N | $C-CH_3$ | |
| 1487 | 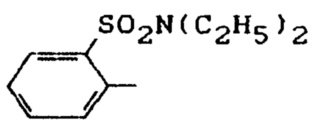 | H | 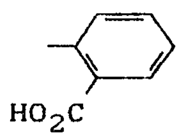 | $OCH_3$ | N | N | $C-CH_3$ | 212 |
| 1488 | 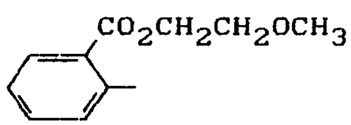 | H | 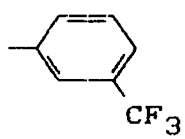 | $OCH_3$ | N | N | $C-CH_3$ | 144 |
| 1489 | 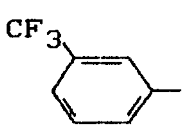 | $CH_3$ | $CH_3$ | $OCH_3$ | N | N | $C-CH_3$ | 109 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1490 | 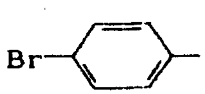 | $CH_3$ | $CH_3$ | $OCH_3$ | N | N | $C-CH_3$ | 134 |
| 1491 | 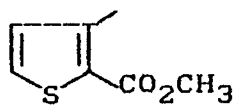 | H | $CH_3(CH_3)_3-$ | $OCH_3$ | N | N | $C-CH_3$ | 134 |
| 1492 | 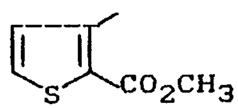 | H | $CH_3(CH_2)_4-$ | $OCH_3$ | N | N | $C-CH_3$ | 120 |
| 1493 | 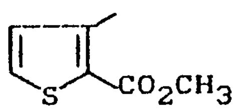 | H | $CH_3(CH_2)_{12}-$ | $OCH_3$ | N | N | $C-CH_3$ | 189 |
| 1494 | 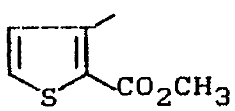 | H | $CH_3(CH_2)_{10}-$ | $OCH_3$ | N | N | $C-CH_3$ | 110 |
| 1495 | 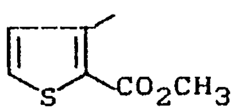 | H | $CH_3(CH_2)_{11}-$ | $OCH_3$ | N | N | $C-CH_3$ | 103 |
| 1496 | 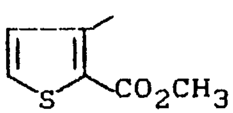 | H | $CH_3(CH_2)_9-$ | $OCH_3$ | N | N | $C-CH_3$ | 120 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1497 | 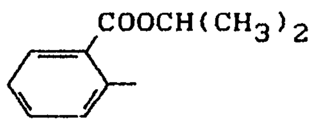 | H | 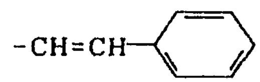 | $OCH_3$ | N | N | $C-CH_3$ | 173 |
| 1498 | 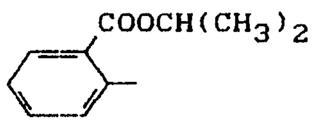 | H | $-CH(CH_3)_2$ | $OCH_3$ | N | N | $C-CH_3$ | 178 |
| 1499 | 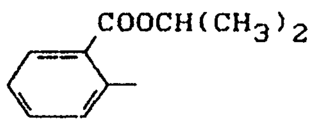 | H | $-C(CH_3)_3$ | $OCH_3$ | N | N | $C-CH_3$ | 166 |
| 1500 | 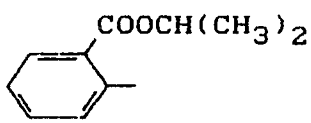 | $CH_3$ | $-CH_2CH(CH_3)_2$ | $OCH_3$ | N | N | $C-CH_3$ | 154 |
| 1501 | 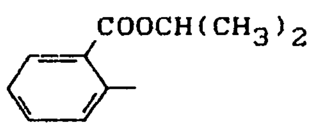 | H | 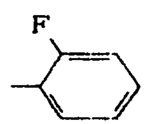 | $OCH_3$ | N | N | $C-CH_3$ | 188 |
| 1502 | 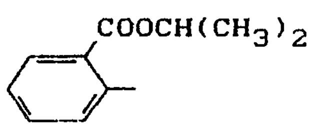 | H | 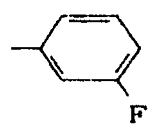 | $OCH_3$ | N | N | $C-CH_3$ | 184 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1503 | 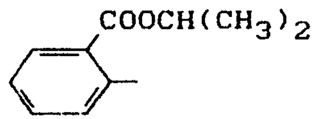 | H | 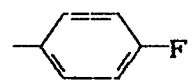 | $OCH_3$ | N | N | $C-CH_3$ | 206 |
| 1504 | 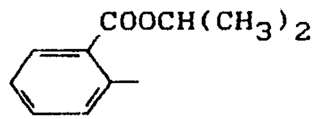 | H | 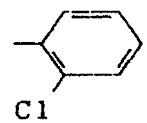 | $OCH_3$ | N | N | $C-CH_3$ | 168 |
| 1505 | 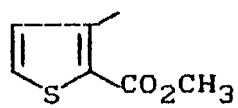 | H | $-(CH_2)_5CH_3$ | $OCH_3$ | N | N | $C-CH_3$ | 117 |
| 1506 | 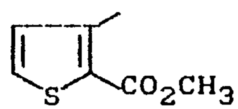 | H | $-(CH_2)_7CH_3$ | $OCH_3$ | N | N | $C-CH_3$ | 111 |
| 1507 | 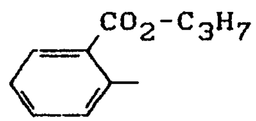 | H | 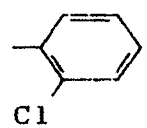 | $OCH_3$ | N | N | $C-CH_3$ | 143 |
| 1508 | 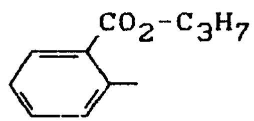 | H | 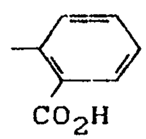 | $OCH_3$ | N | N | $C-CH_3$ | |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1509 | 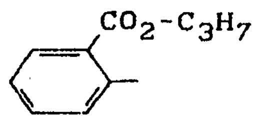 | H | $-CH=CH-$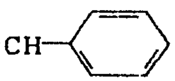 | $OCH_3$ | N | N | $C-CH_3$ | 182 |
| 1510 | 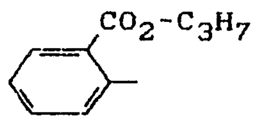 | H | $CH(CH_3)_2$ | $OCH_3$ | N | N | $C-CH_3$ | 145 |
| 1511 | 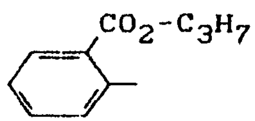 | H | $-C(CH_3)_3$ | $OCH_3$ | N | N | $C-CH_3$ | 149 |
| 1512 | 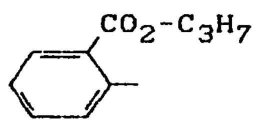 | $CH_3$ | $CH_2CH(CH_3)_2$ | $OCH_3$ | N | N | $C-CH_3$ | 136 |
| 1513 | 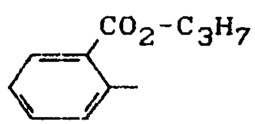 | H | $CH_3$ | $OCH_3$ | N | N | $C-CH_3$ | 134 |
| 1514 | 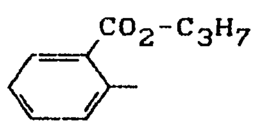 | H | 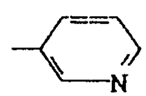 | $OCH_3$ | N | N | $C-CH_3$ | 150 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1515 | 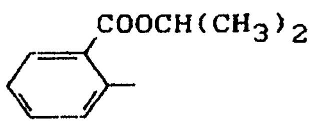 | H | 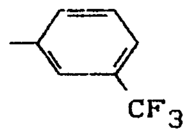 | $OCH_3$ | N | N | $C-CH_3$ | 170 |
| 1516 | 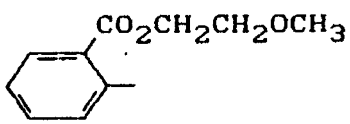 | H | 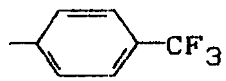 | $OCH_3$ | N | N | $C-CH_3$ | 168 |
| 1517 | 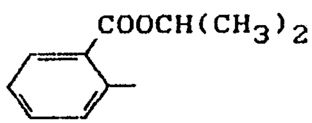 | H | 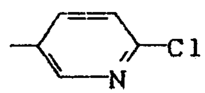 | $OCH_3$ | N | N | $C-CH_3$ | 182 |
| 1518 | 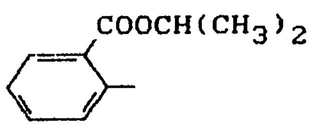 | H | 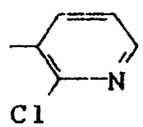 | $OCH_3$ | N | N | $C-CH_3$ | 157 |
| 1519 | 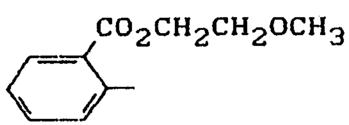 | H | 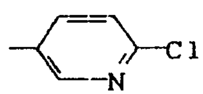 | $OCH_3$ | N | N | $C-CH_3$ | 134 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1520 | 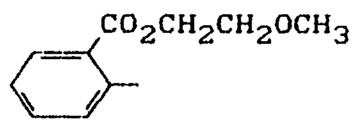 | H | 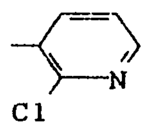 | $OCH_3$ | N | N | $C-CH_3$ | 131 |
| 1521 | 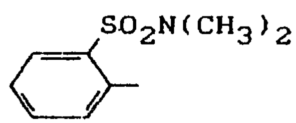 | H | $CH_3CH_2CH_2$ | $OCH_3$ | N | N | $C-OCH_3$ | |
| 1522 | 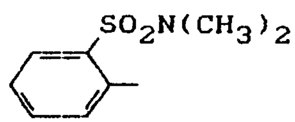 | H | $CH_3SCH_2CH_2$ | $OCH_3$ | N | N | $C-OCH_3$ | 172 |
| 1523 | 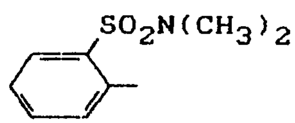 | H | $CH_3CH_2CH_2$ | $OCH_3$ | N | N | $C-CH_3$ | 167 |
| 1524 | 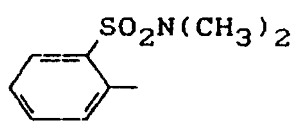 | H | $CH_3SCH_2CH_2$ | $OCH_3$ | N | N | $C-CH_3$ | 161 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1525 | 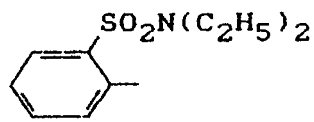 | H | $CH_3$ | $OCH_3$ | N | N | $C-OCH_3$ | |
| 1526 | 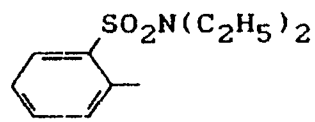 | $CH_3$ | $CH_2CH(CH_3)_2$ | $OCH_3$ | N | N | $C-OCH_3$ | |
| 1527 | 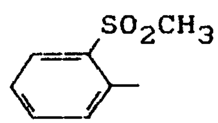 | $CH_3$ | $CH_3$ | $CH_3$ | N | CH | $C-CH_3$ | 287 |
| 1528 | 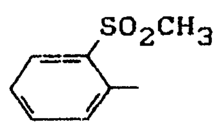 | H | $C_6H_5$ | $CH_3$ | N | CH | $C-CH_3$ | 273 |
| 1529 | 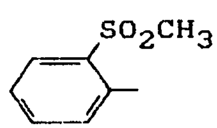 | H | 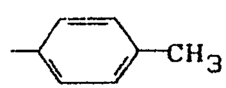 | $CH_3$ | N | CH | $C-CH_3$ | 248 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1530 | 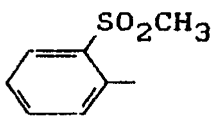 | H | 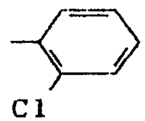 | $CH_3$ | N | CH | $C-CH_3$ | 248 |
| 1531 | 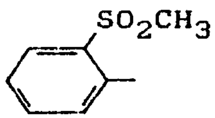 | H | 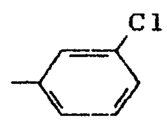 | $CH_3$ | N | CH | $C-CH_3$ | 265 |
| 1532 | 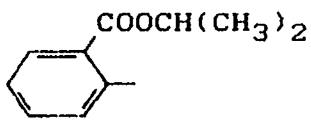 | H | $CH_3$ | $OCH_3$ | N | N | $C-CH_3$ | |
| 1533 | 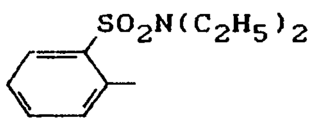 | H | 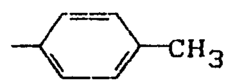 | $OCH_3$ | N | N | $C-CH_3$ | |
| 1534 | 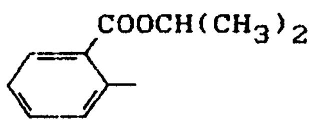 | H | 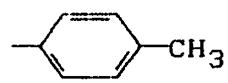 | $OCH_3$ | N | N | $C-CH_3$ | |

<u>Tabelle 3</u> - Fortsetzung

| Bsp.-Nr. | $R^1$ | $R^2$ | $R^3$ | $R^4$ | X | Y | Z | Schmelzpunkt (°C) |
| --- | --- | --- | --- | --- | --- | --- | --- | --- |
| 1535 | 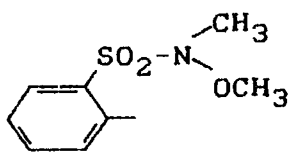 | H | 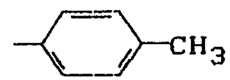 | $OCH_3$ | N | N | $C-CH_3$ | |
| 1536 | 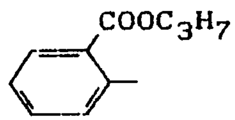 | H | 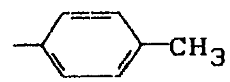 | $OCH_3$ | N | N | $C-CH_3$ | |
| 1537 | 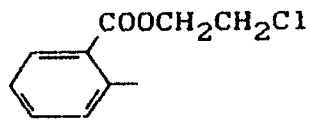 | H | 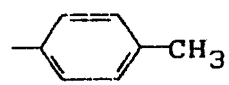 | $OCH_3$ | N | N | $C-CH_3$ | |
| 1538 | 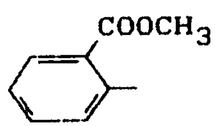 | H | 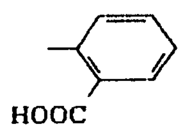 | $OCH_3$ | N | N | $C-OCH_3$ | |
| 1539 | 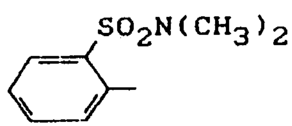 | $C_2H_5$ | $C_2H_5$ | $CH_3$ | N | CH | CH | 167 |

<u>Tabelle 3</u> - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1540 | 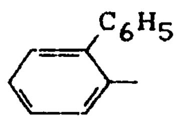 | H | 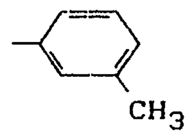 | $OC_2H_5$ | N | N | $C-OCH_3$ | 150 |
| 1541 | 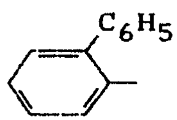 | H | 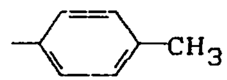 | $OC_2H_5$ | N | N | $C-OCH_3$ | 151 |
| 1542 | 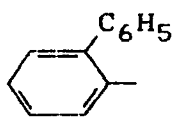 | H | 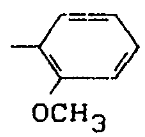 | $OC_2H_5$ | N | N | $C-OCH_3$ | 189 |
| 1543 | 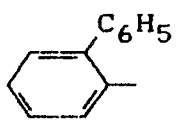 | H | 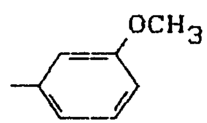 | $OC_2H_5$ | N | N | $C-OCH_3$ | 170 |
| 1544 | 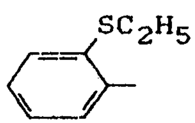 | H | $C_6H_5$ | $OCH_3$ | N | CH | $C-OCH_3$ | 231 |

Tabelle 3 - Fortsetzung

| Bsp.-Nr. | $R^1$ | $R^2$ | $R^3$ | $R^4$ | X | Y | Z | Schmelzpunkt (°C) |
|---|---|---|---|---|---|---|---|---|
| 1545 | 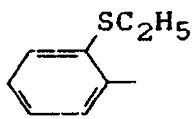 | H | 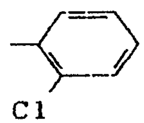 | $OCH_3$ | N | CH | $C-OCH_3$ | 200 |
| 1546 | 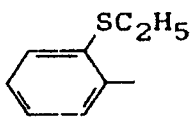 | H | 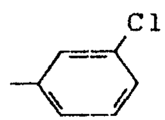 | $OCH_3$ | N | CH | $C-OCH_3$ | 210 |
| 1547 | 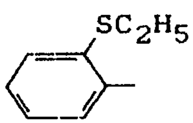 | H | 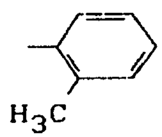 | $OCH_3$ | N | CH | $C-OCH_3$ | 210 |
| 1548 | 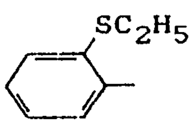 | H | 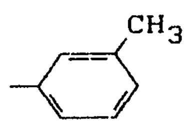 | $OCH_3$ | N | CH | $C-OCH_3$ | 212 |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Ausgangsstoffe der Formel (II):

Beispiel (II-1)

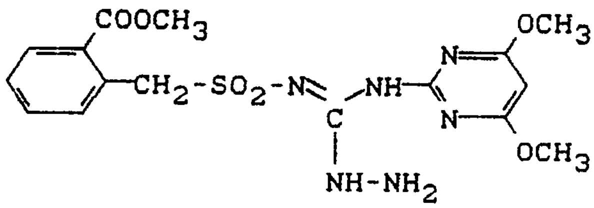

(II-1)

1,3 g (0,025 Mol) Hydrazinhydrat werden bei anfangs 20 °C zu einer Suspension von 15,9 g (0,025 Mol) N′-(4,6-Dimethoxy-pyrimidin-2-yl)-N″-methoxy-N″-(2-methoxycarbonyl-phenylsulfonyl)-N‴-(2-methoxycarbonyl-benzylsulfonyl) guanidin in 100 ml Methanol unter Rühren gegeben, wobei sich die Reaktionsmischung auf 30 °C erwärmt und eine klare Lösung entsteht. Das nach vierstündigem Rühren bei 20 °C bis 30 °C kristallin abgeschiedene Produkt wird durch Absaugen isoliert.

Man erhält 9,5 g (89 % der Theorie) N′-(4,6-Dimethoxy-pyrimidin-2-yl)-N″-amino-N‴-(2-methoxycarbonyl-benzylsulfonyl)-guanidin vom Schmelzpunkt 166 °C.

Beispiel (II-2)

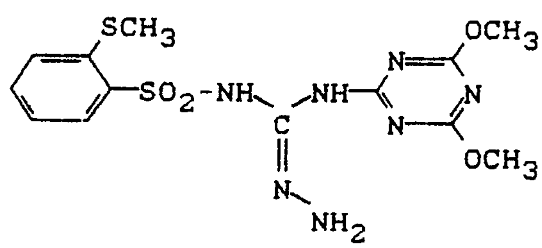

1,0 g (0,02 Mol) Hydrazinhydrat werden unter Rühren bei 20 °C zu einer Mischung aus 8,3 g (0,02 Mol) N′-(4,6-Dimethoxy-s-triazin-2-yl)-N″-(2-methylthio-phenylsulfonyl)-S-methyl-isothioharnstoff und 80 ml Methylenchlorid gegeben und diese Mischung wird 30 minuten bei 20 °C gerührt. Dann wird eingeengt, der Rückstand mit Ethanol verrieben und das kristallin angefallene Produkt durch Absaugen isoliert.

Man erhält 5,5 g (69% der Theorie) N′-(4,6-Dimethoxy-s-triazin-2-yl)-N″-amino-N‴-(2-methylthio-phenylsulfonyl)-guanidin vom Schmelzpunkt 147 °C.

Analog zu den Beispielen (II-1) und (II-2) können beispielsweise auch die in der nachstehenden Tabelle 4 aufgeführten Verbindungen der Formel (II) hergestellt werden, ("Zers." Bedeutet "Zersetzung").

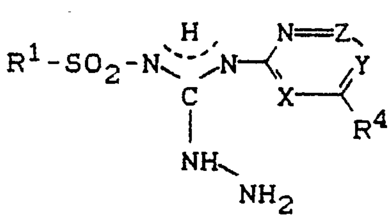

(II)

Die obigen Verbindungen (II-1) und (II-2) sowie die einzelnen in Tabelle 4 genannten Verbindungen (II-3) bis (II-94) sind als solche neu und besitzen auch selbst herbizide Eigenschaften; diese Verbindungen sind ebenfalls Gegenstand der Erfindung.

Tabelle 4: Beispiele für die Ausgangsstoffe der Formel (II)

| Bsp. Nr. | $R^1$ | $R^4$ | X | Y | Z | Schmelzpunkt (°C) |
|---|---|---|---|---|---|---|
| II-3 | 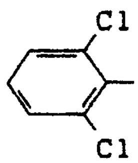 | $OCH_3$ | N | N | $C-OCH_3$ | 132 (Zers.) |
| II-4 | 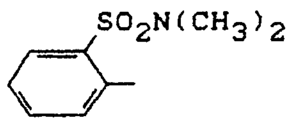 | $OCH_3$ | N | N | $C-OCH_3$ | 143 (Zers.) |
| II-5 | 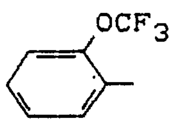 | $CH_3$ | N | N | $C-OC_2H_5$ | 224 |
| II-6 | 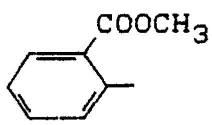 | $CH_3$ | N | N | $C-OC_2H_5$ | 121 (Zers.) |
| II-7 | 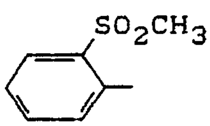 | $OCH_3$ | N | N | $C-OCH_3$ | 153 (Zers.) |
| II-8 | 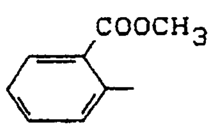 | $OCH_3$ | N | N | $C-OCH_3$ | 170 (Zers.) |
| II-9 | 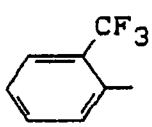 | $OCH_3$ | N | N | $C-OCH_3$ | 155 (Zers.) |
| II-10 | 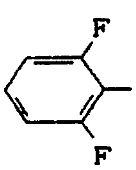 | $OCH_3$ | N | N | $C-OCH_3$ | 124 (Zers.) |

<u>Tabelle 4</u> - Fortsetzung

| Bsp.-Nr. | $R^1$ | $R^4$ | X | Y | Z | Schmelzpunkt (°C) |
|---|---|---|---|---|---|---|
| II-11 | 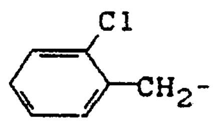 | $OCH_3$ | N | N | $C-OCH_3$ | 170 (Zers.) |
| II-12 | 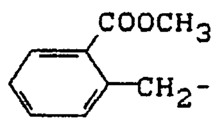 | $CH_3$ | N | N | $C-OCH_3$ | 150 (Zers.) |
| II-13 | 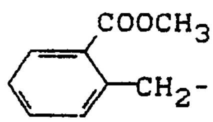 | $OCH_3$ | N | N | $C-OCH_3$ | 178 (Zers.) |
| II-14 | 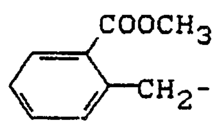 | $CH_3$ | N | N | $C-CH_3$ | 138 (Zers.) |
| II-15 | 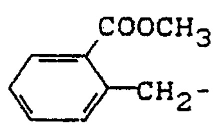 | $C_2H_5$ | N | N | $C-OCH_3$ | 150 (Zers.) |
| II-16 | 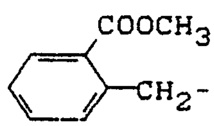 | $CH_3$ | N | N | $C-OC_2H_5$ | 141 (Zers.) |
| II-17 | 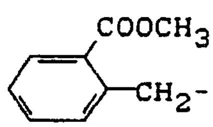 | $CH_3$ | N | N | $C-N(CH_3)_2$ | 193 (Zers.) |
| II-18 | 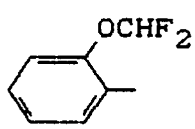 | $C_2H_5$ | N | N | $C-OCH_3$ | 212 (Zers.) |

Tabelle 4 - Fortsetzung

| Bsp. Nr. | $R^1$ | $R^4$ | X | Y | Z | Schmelzpunkt (°C) |
|---|---|---|---|---|---|---|
| II-19 | 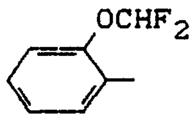 | $CH_3$ | N | N | $C-OCH_3$ | 225 |
| II-20 | 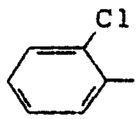 | $C_2H_5$ | N | N | $C-OCH_3$ | 133 (Zers.) |
| II-21 | 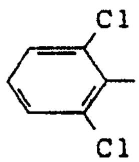 | $C_2H_5$ | N | N | $C-OCH_3$ | 105 (Zers.) |
| II-22 | 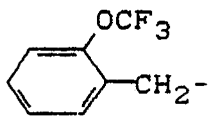 | $CH_3$ | N | N | $C-SCH_3$ | 217 |
| II-23 | 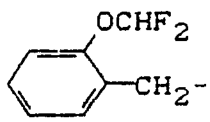 | $C_2H_5$ | N | N | $C-OCH_3$ | 190 |
| II-24 | 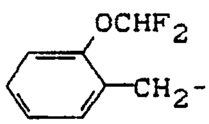 | $CH_3$ | N | N | $C-OC_2H_5$ | 188 |
| II-25 | 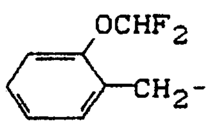 | $CH_3$ | N | N | $C-SCH_3$ | 148 (Zers.) |
| II-26 | 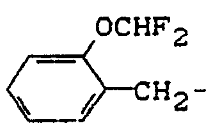 | $CH_3$ | N | N | $C-CH_3$ | 133 (Zers.) |

Tabelle 4 - Fortsetzung

| Bsp.-Nr. | $R^1$ | $R^4$ | X | Y | Z | Schmelzpunkt (°C) |
|---|---|---|---|---|---|---|
| II-27 | 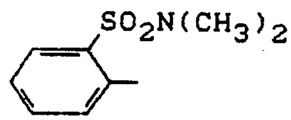 | $CH_3$ | N | N | $C-CH_3$ | 145 (Zers.) |
| II-28 | 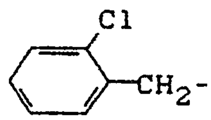 | $CH_3$ | N | N | $C-OC_2H_5$ | 136 (Zers.) |
| II-29 | 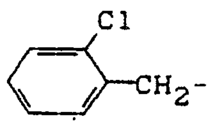 | $CH_3$ | N | N | $C-SCH_3$ | 169 (Zers.) |
| II-30 | 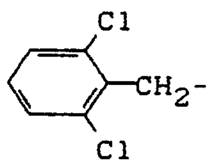 | $CH_3$ | N | N | $C-OC_2H_5$ | 177 |
| II-31 | 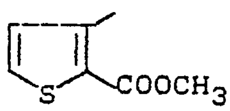 | $CH_3$ | N | N | $C-SCH_3$ | 137 |
| II-32 | 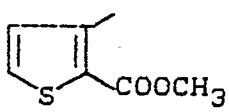 | $CH_3$ | N | N | $C-OC_2H_5$ | 65 |
| II-33 | 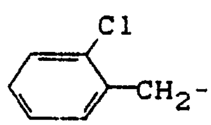 | $CH_3$ | N | N | $C-OCH_3$ | 163 (Zers.) |
| II-34 | 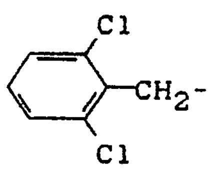 | $CH_3$ | N | N | $C-OCH_3$ | 166 (Zers.) |

Tabelle 4 - Fortsetzung

| Bsp.-Nr. | $R^1$ | $R^4$ | X | Y | Z | Schmelzpunkt (°C) |
|---|---|---|---|---|---|---|
| II-35 | 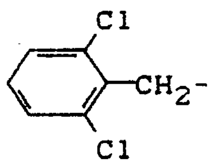 | $OCH_3$ | N | N | $C-OCH_3$ | 164 (Zers.) |
| II-36 | 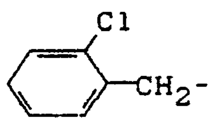 | $CH_3$ | N | N | $C-CH_3$ | 167 (Zers.) |
| II-37 | 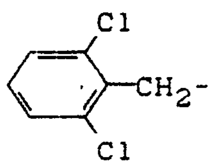 | $CH_3$ | N | N | $C-SCH_3$ | 238 (Zers.) |
| II-38 | 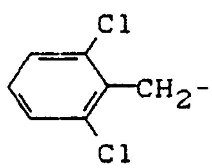 | $C_2H_5$ | N | N | $C-OCH_3$ | 218 |
| II-39 | 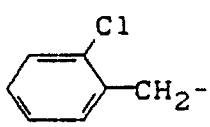 | $C_2H_5$ | N | N | $C-OCH_3$ | 203 |
| II-40 | 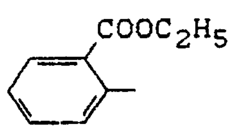 | $CH_3$ | N | N | $C-SCH_3$ | 125 (Zers.) |
| II-41 | 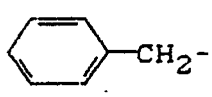 | $CH_3$ | N | N | $C-OCH_3$ | 217 (Zers.) |
| II-42 | 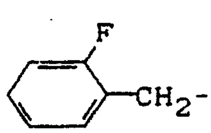 | $CH_3$ | N | N | $C-OCH_3$ | 204 |

Tabelle 4 - Fortsetzung

| Bsp.-Nr. | $R^1$ | $R^4$ | X | Y | Z | Schmelzpunkt (°C) |
|---|---|---|---|---|---|---|
| II-43 | 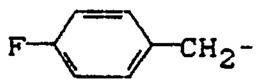 | $CH_3$ | N | N | $C-OCH_3$ | 218 (Zers.) |
| II-44 | 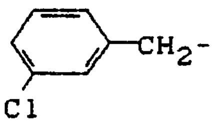 | $CH_3$ | N | N | $C-OCH_3$ | 195 (Zers.) |
| II-45 | 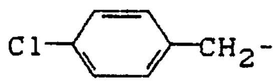 | $CH_3$ | N | N | $C-OCH_3$ | 232 (Zers.) |
| II-46 | 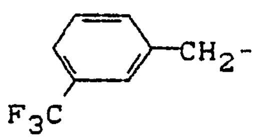 | $CH_3$ | N | N | $C-OCH_3$ | 211 (Zers.) |
| II-47 | 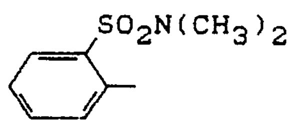 | $CH_3$ | N | N | $C-OCH_3$ | 136 (Zers.) |
| II-48 | 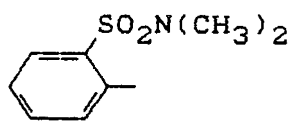 | $OCH_3$ | N | CH | $C-OCH_3$ | 142 |
| II-49 | 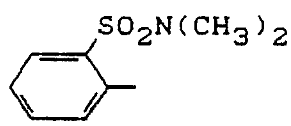 | $CH_3$ | N | CH | CH | 172 |
| II-50 | 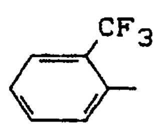 | $CH_3$ | N | CH | CH | 148 |

<u>Tabelle 4</u> - Fortsetzung

| Bsp.-Nr. | $R^1$ | $R^4$ | X | Y | Z | Schmelzpunkt (°C) |
|---|---|---|---|---|---|---|
| II-51 | 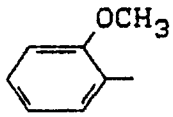 | $CH_3$ | N | CH | $C-CH_3$ | 141 |
| II-52 | 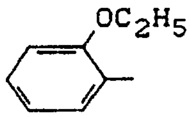 | $CH_3$ | N | CH | $C-CH_3$ | 171 |
| II-53 | 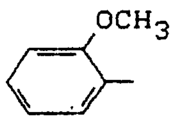 | $OCH_3$ | N | CH | $C-OCH_3$ | 194 |
| II-54 | 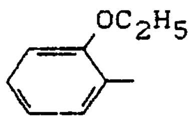 | $OCH_3$ | N | CH | $C-OCH_3$ | 186 |
| II-55 | 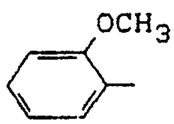 | $CH_3$ | N | CH | CH | 124 |
| II-56 | 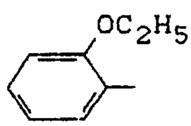 | $CH_3$ | N | CH | CH | 178 |
| II-57 | 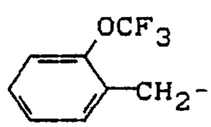 | $CH_3$ | N | CH | $C-CH_3$ | 151 |
| II-58 | 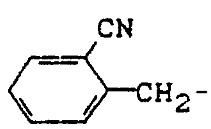 | $CH_3$ | N | CH | $C-CH_3$ | 162 |

Tabelle 4 - Fortsetzung

| Bsp.-Nr. | $R^1$ | $R^4$ | X | Y | Z | Schmelzpunkt (°C) |
|---|---|---|---|---|---|---|
| II-59 | 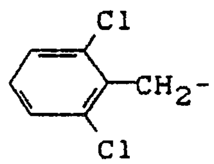 | $CH_3$ | N | CH | $C-CH_3$ | 205 |
| II-60 | 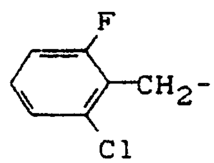 | $CH_3$ | N | CH | $C-CH_3$ | 197 |
| II-61 | 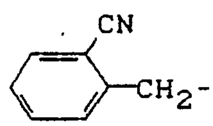 | $CH_3$ | N | CH | $C-OCH_3$ | 192 |
| II-62 | 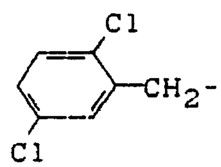 | $CH_3$ | N | CH | $C-OCH_3$ | 204 |
| II-63 | 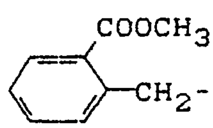 | $CH_3$ | N | CH | $C-OCH_3$ | 166 |
| II-64 | 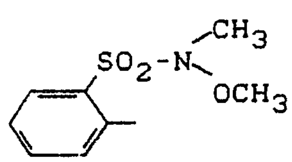 | $CH_3$ | N | CH | $C-OCH_3$ | 176 |
| II-65 | 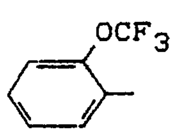 | $OCH_3$ | N | CH | $C-CH_2OCH_3$ | 225 |

Tabelle 4 - Fortsetzung

| Bsp.-Nr. | $R^1$ | $R^4$ | X | Y | Z | Schmelzpunkt (°C) |
|---|---|---|---|---|---|---|
| II-66 | 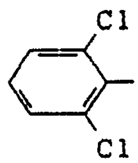 | $CH_3$ | N | CH | CH | 162 |
| II-67 | 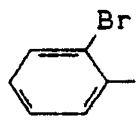 | $CH_3$ | N | CH | CH | 131 |
| II-68 | 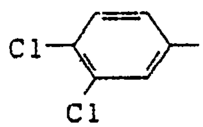 | $CH_3$ | N | CH | CH | 132 |
| II-69 | 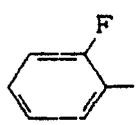 | $CH_3$ | N | CH | CH | 108 |
| II-70 | 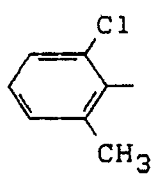 | $CH_3$ | N | CH | CH | 102 |
| II-71 | 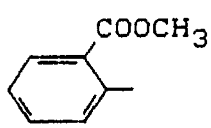 | $OCH_3$ | N | $C-CH_3$ | CH | 153 |
| II-72 | 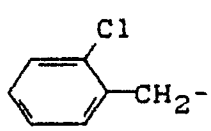 | $CH_3$ | N | CH | CH | 154 |
| II-73 | 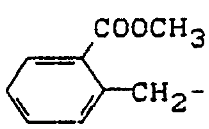 | $OCH_3$ | N | CH | C-Cl | 145 |

Tabelle 4 - Fortsetzung

| Bsp.-Nr. | $R^1$ | $R^4$ | X | Y | Z | Schmelzpunkt (°C) |
|---|---|---|---|---|---|---|
| II-74 | 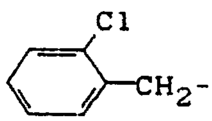 | $OCH_3$ | N | CH | $C-OCH_3$ | 150 |
| II-75 | 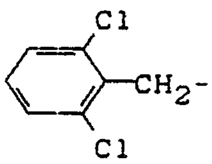 | $OCH_3$ | N | CH | $C-OCH_3$ | 188 |
| II-76 | 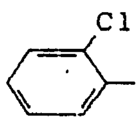 | $OCH_3$ | N | CH | $C-OCH_3$ | 153 |
| II-77 | 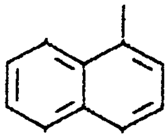 | $OCH_3$ | N | N | $C-OCH_3$ | 89 |
| II-78 | 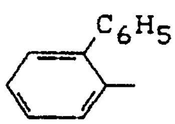 | $CH_3$ | N | CH | $C-CH_3$ | 164 |
| II-79 | 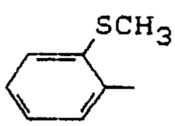 | $OCH_3$ | N | CH | $C-OCH_3$ | 198 |
| II-80 | 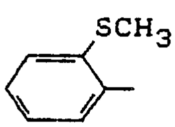 | $CH_3$ | N | CH | $C-CH_3$ | 146 |
| II-81 | 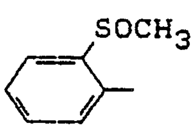 | $CH_3$ | N | CH | $C-CH_3$ | 192 |

Tabelle 4 - Fortsetzung

| Bsp.-Nr. | $R^1$ | $R^4$ | X | Y | Z | Schmelzpunkt (°C) |
|---|---|---|---|---|---|---|
| II-82 | 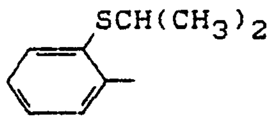 | $OCH_3$ | N | CH | $C-OCH_3$ | 194 |
| II-83 | 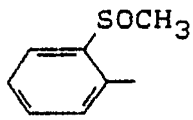 | $OCH_3$ | N | CH | $C-OCH_3$ | 149 |
| II-84 | 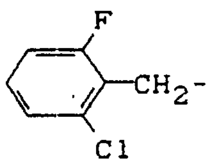 | $CH_3$ | N | N | $C-OCH_3$ | 231 (Zers.) |
| II-85 | 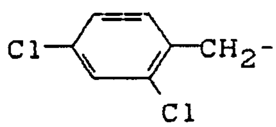 | $CH_3$ | N | N | $C-OCH_3$ | 233 (Zers.) |
| II-86 | 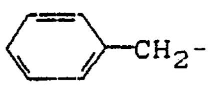 | $OCH_3$ | N | N | $C-OCH_3$ | 156 (Zers.) |
| II-87 | 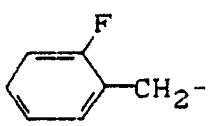 | $OCH_3$ | N | N | $C-OCH_3$ | 218 |
| II-88 | 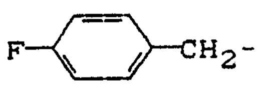 | $OCH_3$ | N | N | $C-OCH_3$ | 225 |
| II-89 | 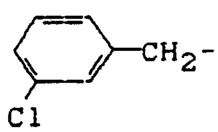 | $OCH_3$ | N | N | $C-OCH_3$ | 220 |

Tabelle 4 - Fortsetzung

| Bsp.-Nr. | $R^1$ | $R^4$ | X | Y | Z | Schmelzpunkt (°C) |
|---|---|---|---|---|---|---|
| II-90 | 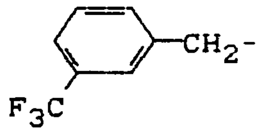 | $OCH_3$ | N | N | $C-OCH_3$ | 189 |
| II-91 | 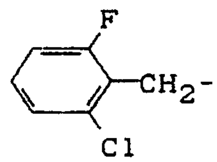 | $OCH_3$ | N | N | $C-OCH_3$ | 214 |
| II-92 | 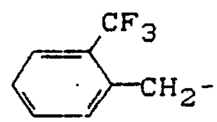 | $CH_3$ | N | CH | $C-OCH_3$ | 187 |
| II-93 | 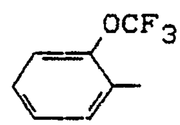 | $CH_3$ | N | CH | $C-OCH_3$ | 135 |
| II-94 | 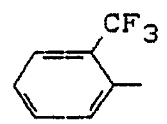 | $CH_3$ | N | CH | $C-OCH_3$ | 140 |

Anwendungsbeispiele

In den folgenden Anwendungsbeispielen wird die nachstehend aufgeführte Verbindung als Vergleichssubstanz herangezogen:

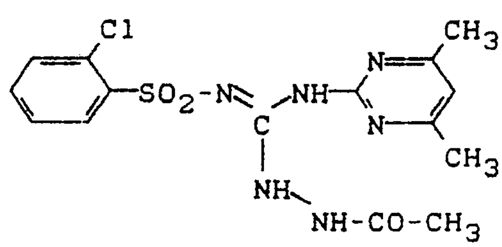

(A)

N'-(4,4-Dimethyl-pyrimidin-2-yl)-N''-acetylamino-N'''-(2-chlor-phenylsulfonyl)-guanidin (bekannt aus EP-A 121 082).

Die Formeln der für die Anwendungsbeispiele herangezogenen erfindungsgemäßen Verbindungen sind - mit der Numerierung der Herstellungsbeispiele ("Bsp.-Nr") -nachstehend einzeln aufgeführt.

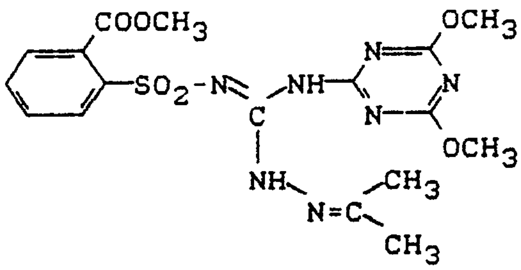

(3)

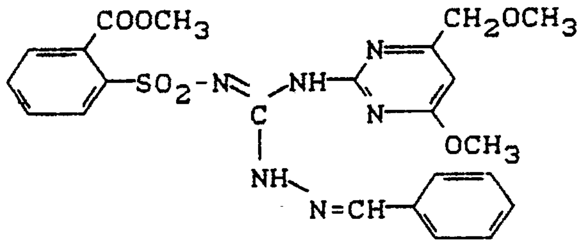

(5)

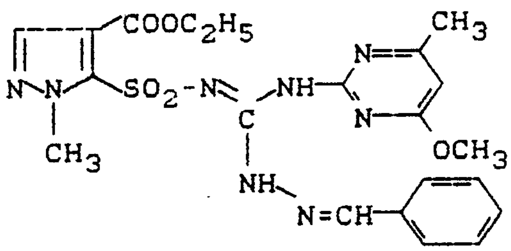

(9)

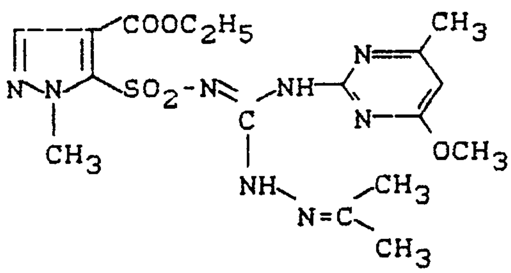

(10)

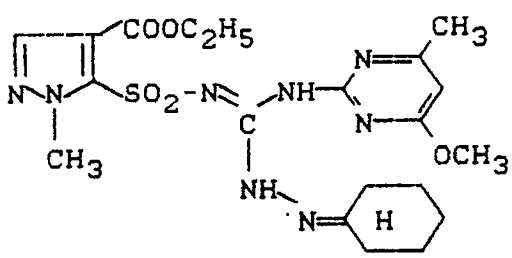

(11)

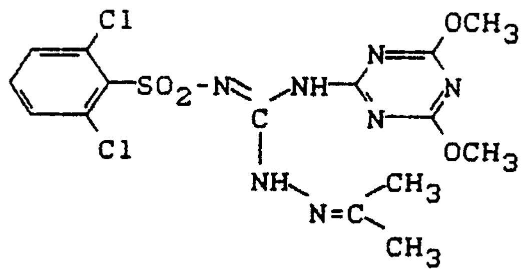

(67)

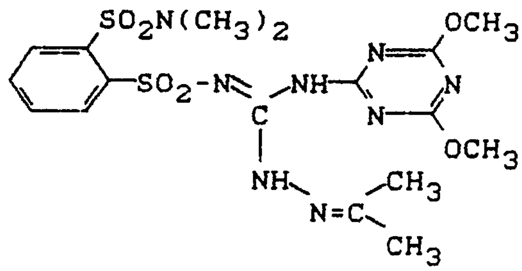

(68)

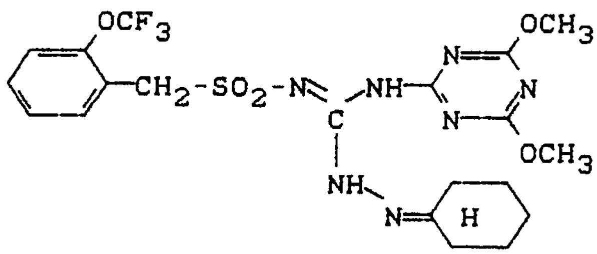

(72)

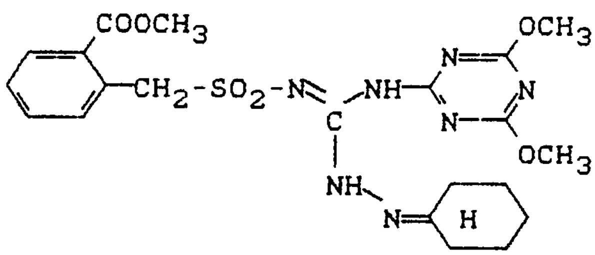

(71)

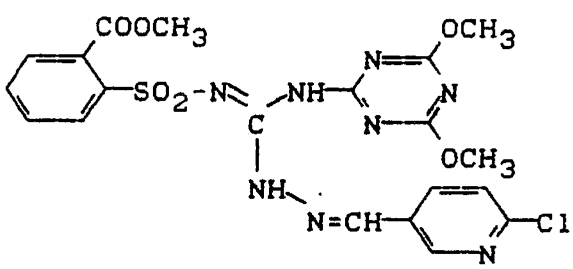

(73)

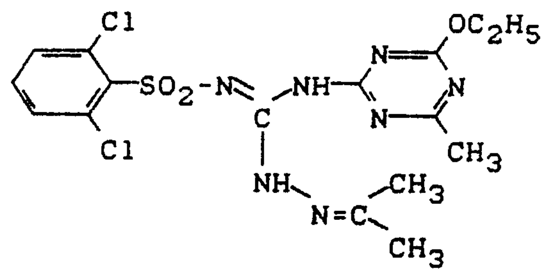

(80)

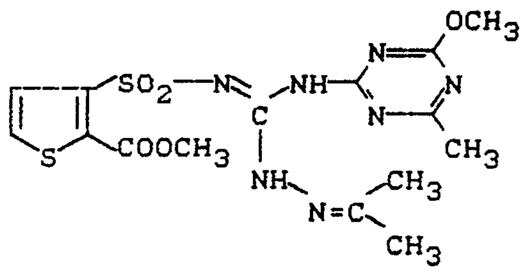

(83)

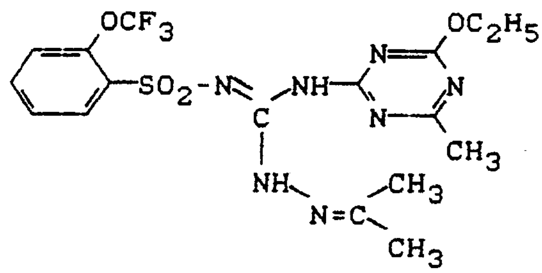

(85)

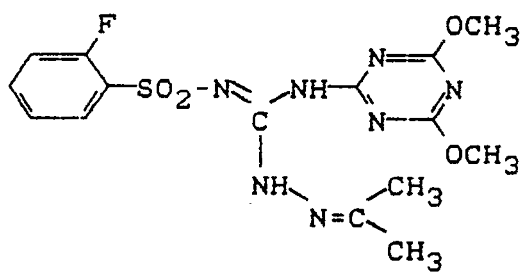

(86)

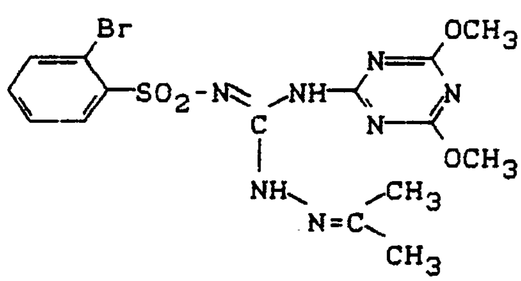

(87)

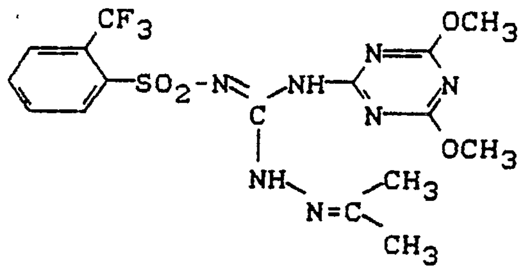

(88)

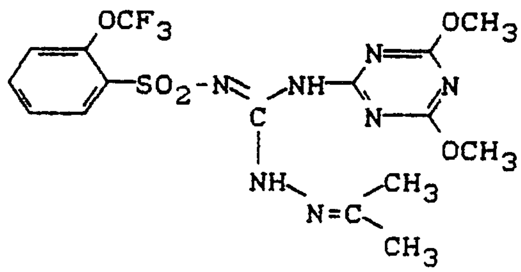

(92)

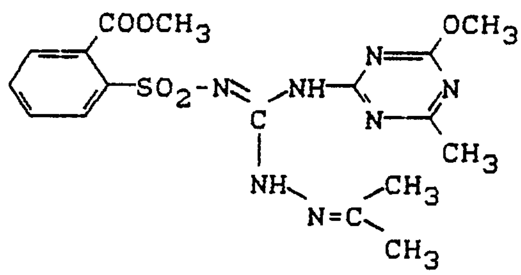

(93)

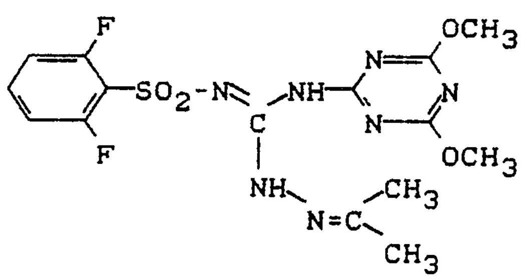

(95)

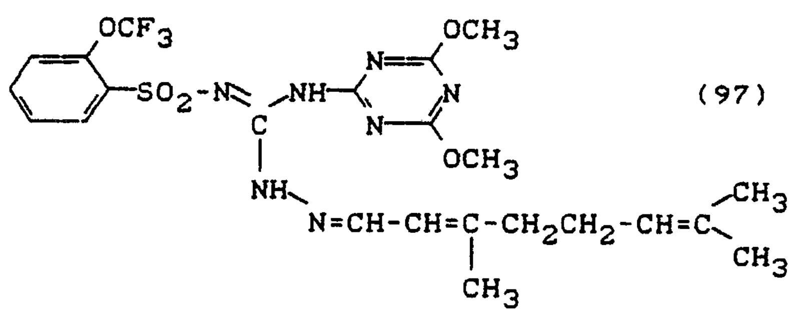

(97)

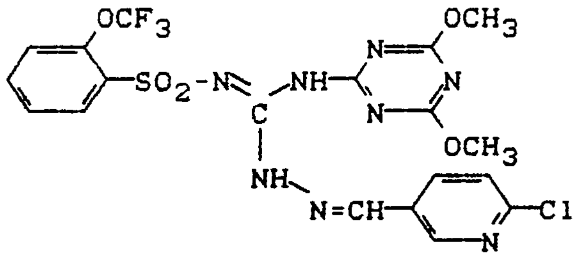

(98)

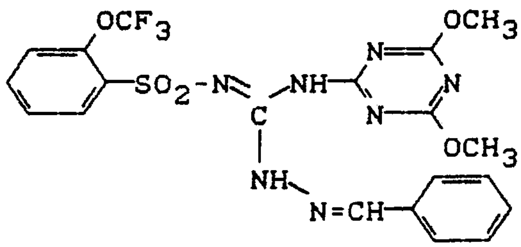

(99)

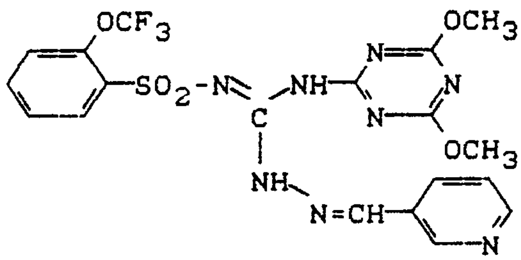

(101)

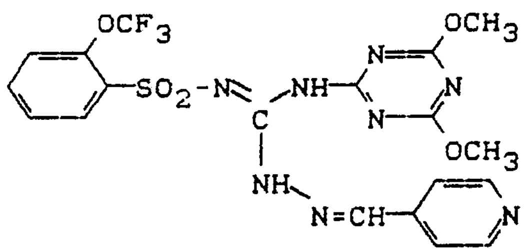

(100)

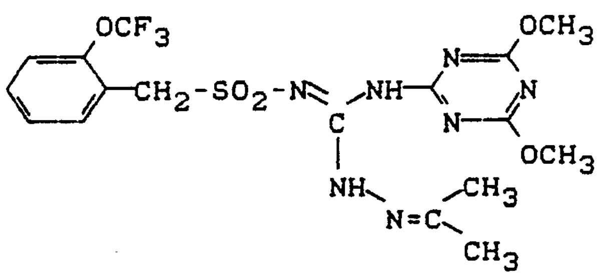

(102)

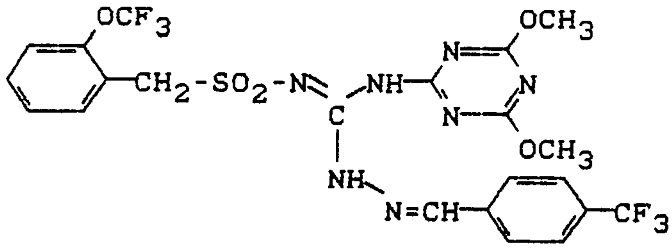

(106)

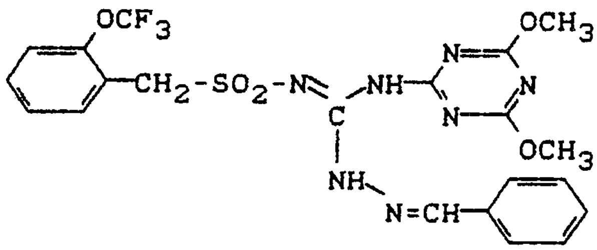

(107)

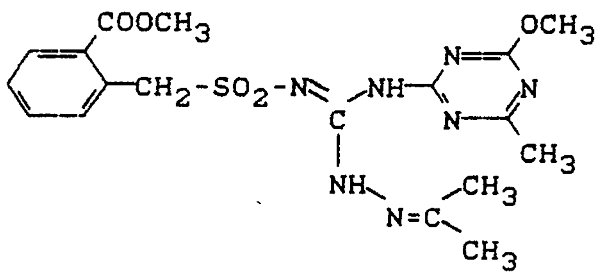

(108)

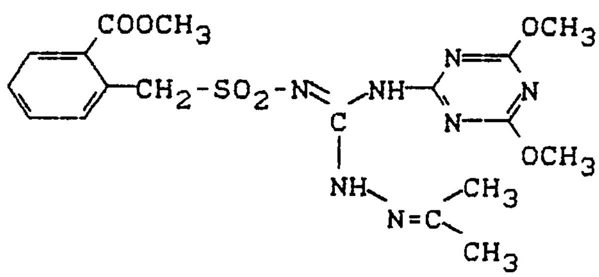

(109)

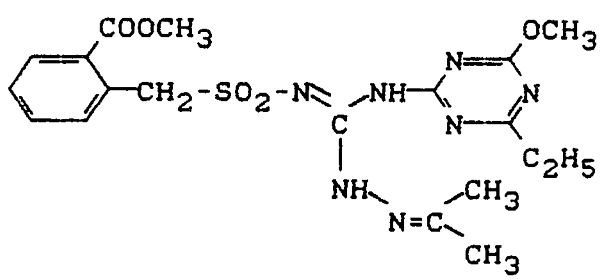

(111)

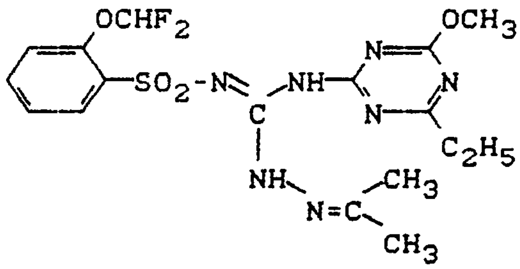

(115)

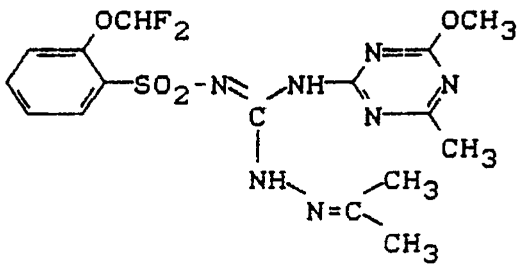

(116)

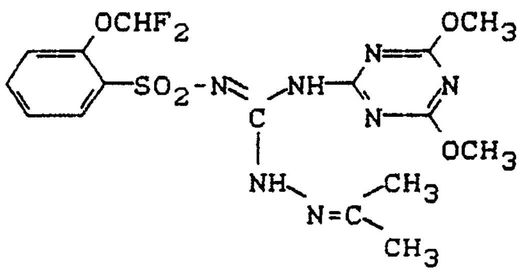

(117)

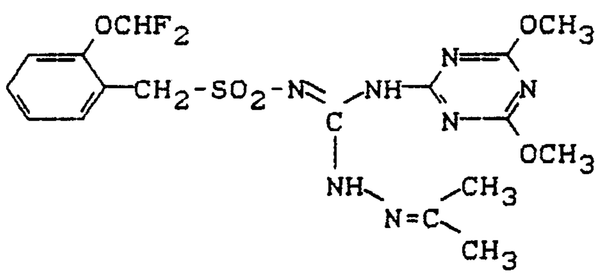

(123)

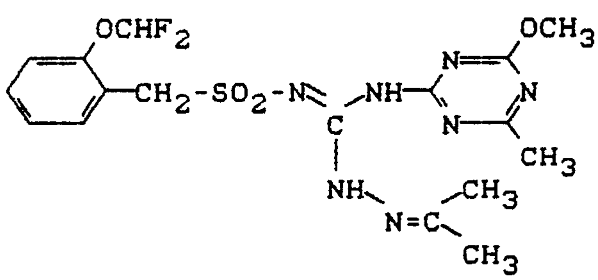

(125)

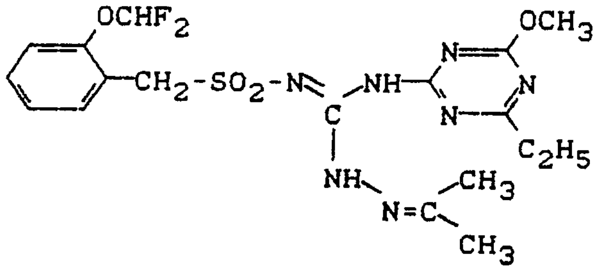

(126)

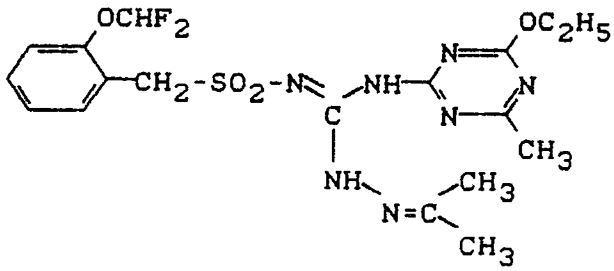

(127)

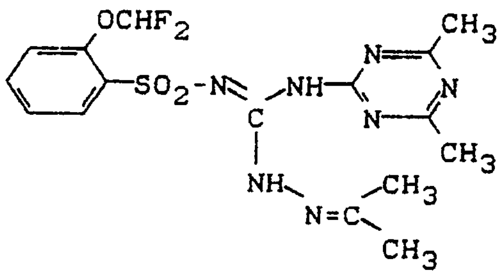

(128)

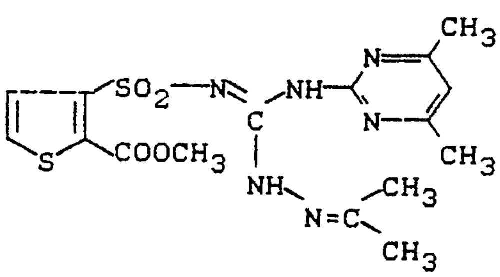

(257)

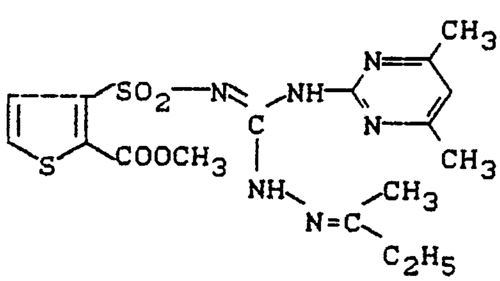

(258)

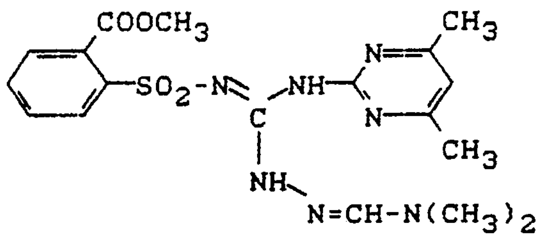

(269)

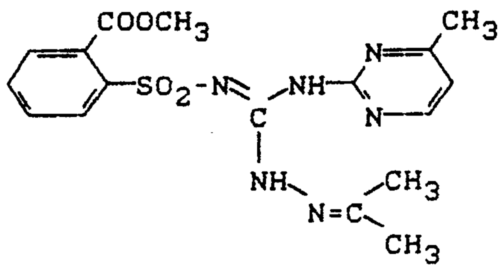

(270)

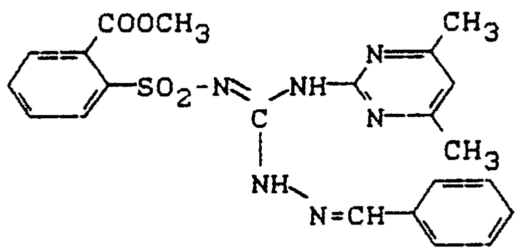

(271)

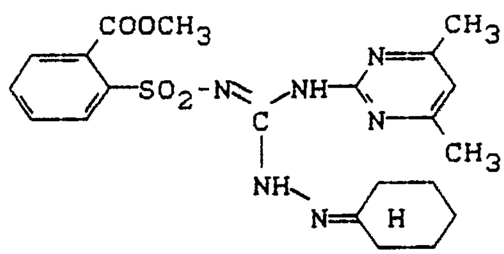

(272)

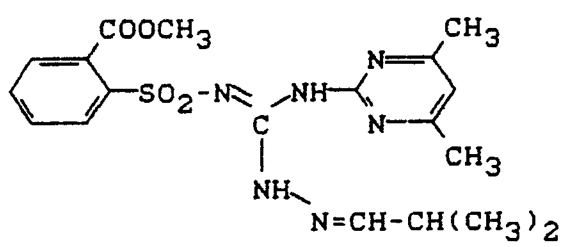

(273)

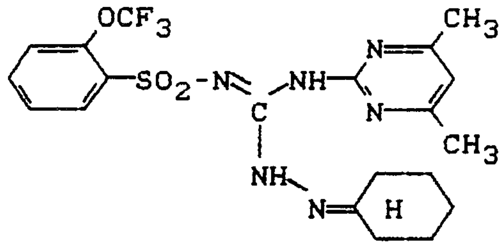

(275)

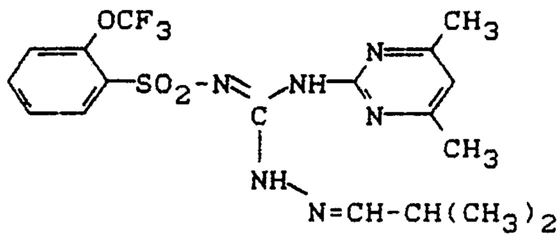

(276)

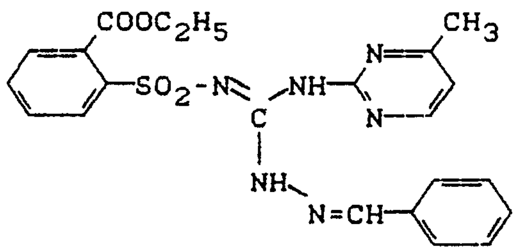

(278)

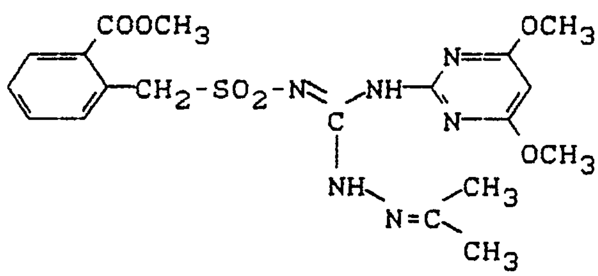

(283)

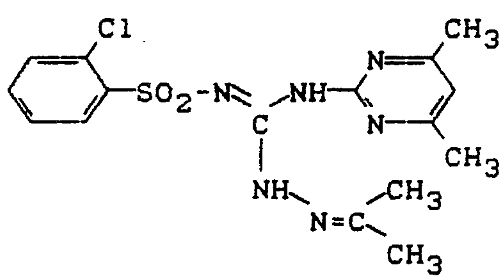

(284)

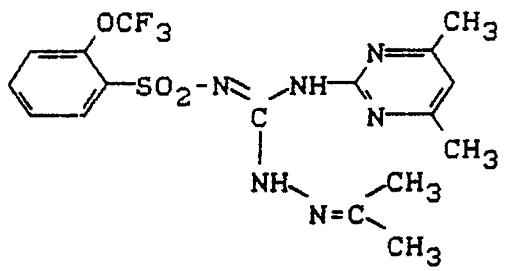

(285)

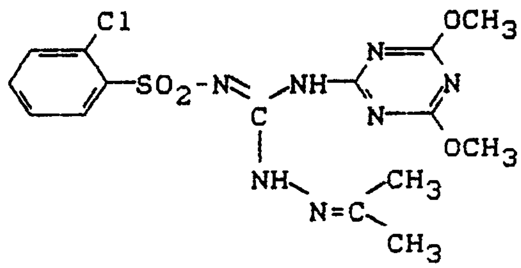

(286)

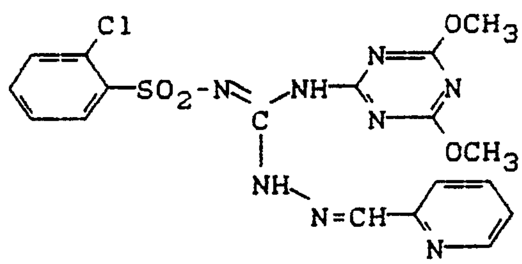

(287)

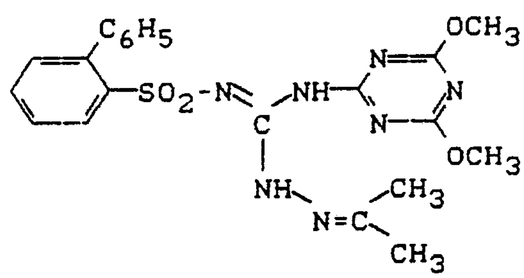

(291)

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit gegenüber dem Stand der Technik zeigen in diesem Test z.B. die Verbindungen gemäß den Herstellungsbeispielen: 3, 67, 68, 80, 83, 85, 87, 88, 92, 93, 95, 99,

101, 108, 116, 117, 258, 269, 270, 271, 272, 273, 275, 276, 278, 284, 285 und 286.

Beispiel B
Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, das in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
O % = keine Wirkung (wie unbehandelte Kontrolle)
100 % = totale Vernichtung

Eine deutliche Überlegenheit in der Wirksamkeit gegenüber dem Stand der Technik zeigen in diesem Test z.B. die Verbindungen gemäß den Herstellungsbeispielen: 5, 9, 10, 11, 67, 68, 71, 72, 73, 80, 83, 86, 87, 88, 93, 95, 97, 98, 99, 100, 101, 102, 106, 107, 108, 109, 111, 115, 116, 117, 123, 125, 126, 127, 128, 257, 269, 270, 276, 278, 283, 286, 287 und 291.

## Ansprüche

1. Substituierte Sulfonylamidinohydrazone der allgemeinen Formel (I),

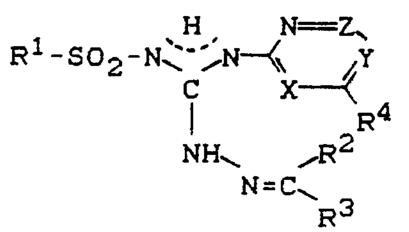

(I)

in welcher
$R^1$ für jeweils gegebenenfalls substituiertes Aryl, Aralkyl oder Heteroaryl steht,
$R^2$ für Wasserstoff oder für jeweils gegebenenfalls substituiertes Alkyl, Aryl oder Aralkyl steht,
$R^3$ für Wasserstoff oder für jeweils gegebenenfalls substituiertes Alkyl, Alkenyl, Alkadienyl, Alkinyl, (Hetero)-Aryl, Aralkyl, Aralkenyl, Alkoxy, Alkoxycarbonyl oder Dialkylamino steht, oder zusammen mit $R^2$ für gegebenenfalls substituiertes Alkandiyl steht,
$R^4$ für Wasserstoff, Halogen, Hydroxy, Alkyl, Halogenalkyl, Alkoxyalkyl, Alkoxy, Halogenalkoxy, Alkylthio, Halogenalkylthio, Amino, Alkylamino oder Dialkylamino steht,
X für Stickstoff oder eine -CH-Gruppierung steht,
Y für Stickstoff oder eine $-CR^5$-Gruppierung steht,
worin
$R^5$ für Wasserstoff, Halogen, Cyano, Alkyl, Formyl, Alkyl-carbonyl oder Alkoxycarbonyl steht, und
Z für Stickstoff oder eine $-CR^6$-Gruppierung steht,
worin
$R^6$ für Wasserstoff, Halogen, Hydroxy, Alkyl, Halogenalkyl, Alkoxy, Halogenalkoxy, Alkylthio, Alkylamino oder Dialkylamino steht.

2. Verfahren zur Herstellung von substituierten Sulfonylamidinohydrazone der allgemeinen Formel (I) gemäß Anspruch 1, dadurch gekennzeichnet, das man
(a) Aminoguanidine der allgemeinen Formel (II)

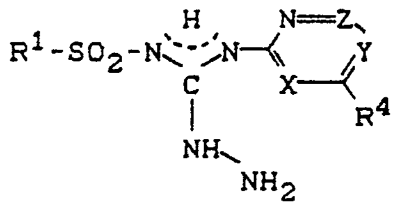 (II)

in welcher
$R^1$, $R^4$, X, Y und Z die in Anspruch 1 angegebenen Bedeutungen haben,
mit Carbonylverbindungen der allgemeinen Formel (III)

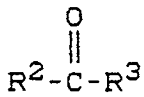 (III)

in welcher
$R^2$ und $R^3$ die in Anspruch 1 angegebenen Bedeutungen haben,
oder mit N,N-Dialkyl-carbonsäureamid-acetalen bzw. -ketalen
gegebenenfalls in Gegenwart eines Kondensationshilfsmittels und gegebenenfalls in in Gegenwart eines Verdünnungsmittels umsetzt, oder das man
(b) Sulfonylverbindungen der allgemeinen Formel (IV)

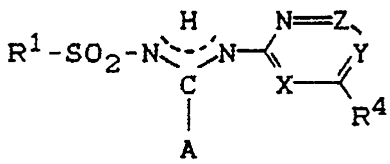 (IV)

in welcher
$R^1$, $R^4$, X, Y und Z die in Anspruch 1 angegebenen Bedeutungen haben und
A für Halogen oder eine der nachstehend angegebenen Abgangsgruppen

$$R^1-SO_2-\underset{|}{N}-O-R^7 \text{ oder } -Q-R^8 \text{ steht,}$$

worin
$R^1$ die oben angegebene Bedeutung hat,
$R^7$ für Alkyl, Alkenyl oder Aralkyl steht,
$R^8$ für jeweils gegebenenfalls substituiertes Alkyl, Aralkyl oder Aryl steht und
Q für Sauerstoff oder Schwefel steht,
mit Hydrazonen der allgemeinen Formel (V),

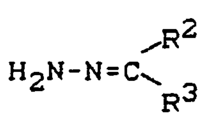 (V)

in welcher
$R^2$ und $R^3$ die in Anspruch 1 angegebenen Bedeutungen haben,
gegebenenfalls in Gegenwart eines Verdünnungsmittels umsetzt.

3. Herbizide Mittel, gekennzeichnet durch einen Gehalt an mindestens einem substituiertem Sulfonylamidinohydrazon der Formel (I) gemäß Anspruch 1.

4. Verfahren zur Bekämpfung von Unkräutern, dadurch gekenzeichnet, daß man substituierte Sulfonylamidinohydrazone der Formel (I) gemäß Anspruch 1 auf die Unkräuter und/oder ihren Lebensraum einwirken läßt.
5. Verwendung von substituierten Sulfonylamidinohydrazonen der Formel (I) gemäß Anspruch 1 zur Bekämpfung von Unkräutern.
6. Verfahren zur Herstellung von herbiziden Mitteln, dadurch gekennzeichnet, das man substituierte Sulfonylamidinohydrazone der Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

**EP 90 11 8519**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | EP-A-0 121 082 (BAYER AG)<br>* Anspruch 1; Seite 131, Zeilen 2-9 *<br>- - - - - | 1 | C 07 D 403/12<br>C 07 D 251/46<br>A 01 N 47/44<br>C 07 D 409/12<br>C 07 D 239/47<br>C 07 D 239/52<br>C 07 D 251/16<br>C 07 D 239/42<br>C 07 D 405/12<br>C 07 D 401/12 |

| RECHERCHIERTE SACHGEBIETE (Int. Cl.5) |
|---|
| C 07 D 403/00<br>C 07 C 251/00<br>C 07 D 409/00<br>C 07 D 239/00<br>C 07 D 405/00<br>C 07 D 401/00 |

Der vorliegende Recherchenbericht wurde für alle Patentansprüche erstellt

| Recherchenort | Abschlußdatum der Recherche | Prüfer |
|---|---|---|
| Den Haag | 08 Januar 91 | DE JONG B.S. |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument